# EUROPEAN PATENT APPLICATION

(11) **EP 1 813 603 A1**
(43) Date of publication of application: **01.08.2007**
(21) Application number: 05806912.1
(22) Date of filing: 15.11.2005
(51) Int. Cl.: C07D 243/14, C07D 401/06, C07D 413/06, C07D 403/06, C07D 413/14, C07D 407/06, C07D 409/06, A61K 31/5513, A61P 13/02, A61P 43/00

(54) **AROMATIC AMIDE DERIVATIVES, MEDICINAL COMPOSITIONS CONTAINING THE SAME, MEDICAL USES OF BOTH**

(30) Priority: 17.11.2004 JP 2004333802
(71) Applicant: Kissei Pharmaceutical Co., Ltd., Matsumoto-shi Nagano 399-8710 (JP)
(72) Inventor: YOKOYAMA, K., Kissei Pharmaceutical Co. Ltd, Azumino-shi, Nagano 3998304 (JP); SUZUKI, R., Kissei Pharmaceutical Co. Ltd, Azumino-shi, Nagano 3998304 (JP); KONDO, T., Kissei Pharmaceutical Co. Ltd, Azumino-shi, Nagano 3998304 (JP); KONDO, A., Kissei Pharmaceutical Co. Ltd, Azumino-shi, Nagano 3998304 (JP); KOBAYASHI, H., Kissei Pharmaceutical Co. Ltd, Azumino-shi, Nagano 3998304 (JP)
(74) Representative: Hayes, Adrian Chetwynd
(86) International application number: PCT/JP2005/020970
(87) International publication number: WO 2006/054560

(57) **Abstract**

A present invention provides aromatic amide derivatives which have an agonism of V2 receptor, are useful as agents for the treatment or prevention of diabetes insipidus, nocturia, nocturnal enuresis, overactive bladder or the like, and are represented by the general formula (I): 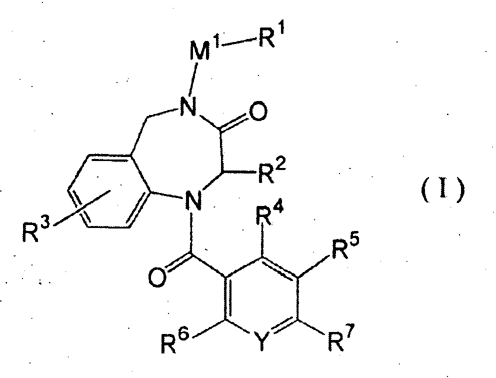 wherein R¹ represents a hydrogen atom or a C₁₋₆ alkyl group which may have a substituent, R² is a hydrogen atom or a C₁₋₆ alkyl group, R³ is a hydrogen atom, a C₁₋₆ alkyl group or the like, R⁴, R⁵ and R⁶ are independently a hydrogen atom, a halogen atom or the like, R⁷ is a hydrogen atom, a heteroaryl group which may have a substituent, a C₃₋₈ cycloalkyl group', an amino group which may have a substituent or a C₁₋₆ alkoxy group which may have a substituted group;
M¹ is a single bond, a C₁₋₄ alkylene group or the like
Y is N or CR^{F} (in the formula, R^{F} represents a hydrogen atom, a C₁₋₆ alkyl group or the like
or a pharmaceutically acceptable salt thereof, or a prodrug thereof, or pharmaceutical compositions comprising the same and pharmaceutical uses thereof.

## Description

### Technical Field

The present invention relates to aromatic amide derivatives or pharmaceutically acceptable salts thereof, or prodrugs thereof which are useful as medicaments, or pharmaceutical compositions comprising the same and pharmaceutical uses thereof.

More particularly, the present invention relates to novel aromatic amide derivatives having an agonism of a type 2 arginine vasopressin receptor (hereinafter called V2 receptor), or pharmaceutically acceptable salts thereof, or prodrugs thereof, or pharmaceutical compositions comprising the same and pharmaceutical uses thereof.

### Background Art

Arginine vasopressin is one of neurohormones, which is biosynthesizedinthe hypothalamus and is released from the posterior pituitary gland. Arginine vasopressin receptors were classified to V1a, V1b and V2 subtypes. An arginine vasopressin is called an antidiuretic hormone because an arginine vasopressin decreases urine volume due to enhancing water reabsorption at collecting ducts, in which V2 receptor exists and arginine vasopressin shows agonism of V2 receptor via binding this receptor (see Non-patent Reference 1). Therefore, the patients suffer from polyuria because of a deficiency of arginine vasopressin, as a concrete example, central diabetes insipidus, nocturnal enuresis in children, nocturia with aging and the like can be illustrated. (see Non-patent References 2 and 3).

Heretofore, peptide-type compounds(see Non-patent Reference 3; desmopressin (1-desamino, D-Arg8) vasopressin, DDAVP) have been used for the treatment of central diabetes insipidus or nocturnal enuresis as a V2 agonist. However, concerning an absorption rate in gastrointestinal tract, it is known that peptide-type compounds have wide individual variability in absorption and the wide variability in plasma concentration of the compounds has been reported (see Non-patent Reference 4). Therefore, it is feared the adverse effects due to these variability will occur and clinical use of the compounds was not necessarily satisfied with safety. It is most preferable to use clinically non-peptide drug, that is, a low molecular V2 agonist for the patients with disorders as mentioned above.

Recently, any drugs were administered simultaneously or at different time to a patient due to a diversity of medication or an aging society. The drug-drug interactions due to multidrug therapy that is aspect of medical care for the elderly person in the present field are unavoidable problems. Generally, the drug interaction is classified to a pharmacodynamic and a pharmacokinetic interaction. Many of the pharmacokinetic drug interactions occur via a metabolic process of drugs clinically. The drug interactions related to drug metabolism occur via inhibition of cytochrome P-450s (CYP) mainly existing in liver and so on, and are also most concerned clinically. The disturbance of the pharmacokinetics induced by the inhibition of CYP not only cause a loss of ability to medicate adequately but also is fearful of the occurrence of sever side effects consequently. Therefore, the development of the oral agents that have weak inhibitory effect on CYP that metabolizes many drugs is hoped in the development of the drugs, which are safe and are easy to control in the present field.

As some non-peptide compounds that have an agonism of vasopressin receptors, the compounds represented by the following general formulas (II-1) and (II-2) have been reported in Patent References 1 and 2.

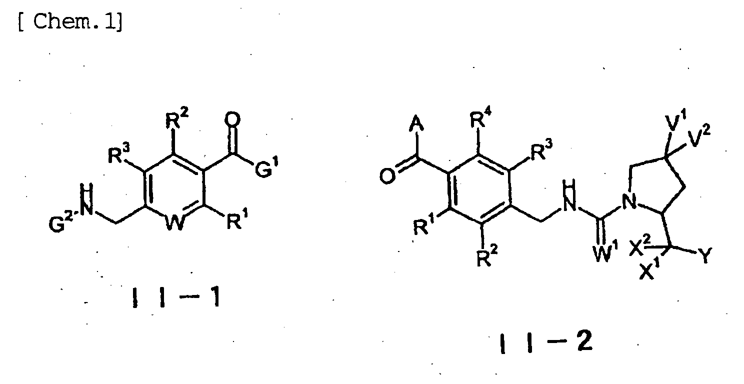

(in the formula, the sign refers to the Patent Reference.)

In addition, as the compounds which have an agonism of vasopressin receptors and an antagonism of vasopressin/oxytocin, the compounds represented by the following general formula (III-1) have been reported in Patent References 3.

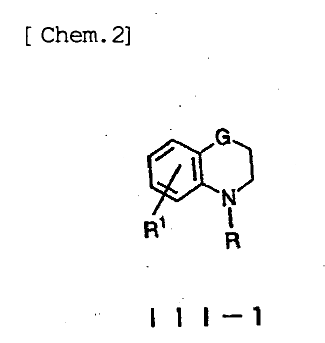

(in the formula, the sign refers to the Patent-Reference.)

Also, as some compounds that have antagonism of vasopressin, the compounds represented by the following general formula (IV-1), (IV-2), (IV-3) and (IV-4) have been reported in Patent References 4 to 7.

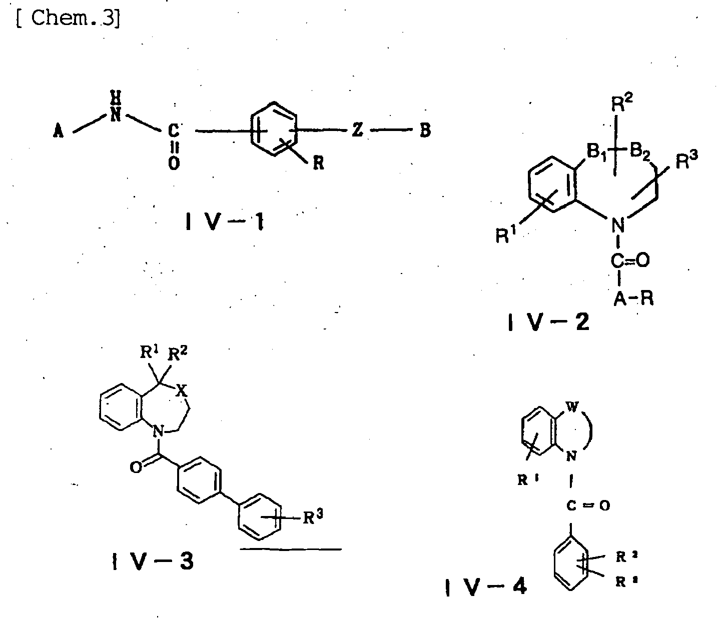

(in the formula, the sign refers to the Patent Reference.)

However, there is no disclosure in any Patent References that mentioned benzodiazepinon derivatives, which have an amide bond in the ring. It has not been reported that the aromatic amide derivatives as described by the following disclosure of the invention are real new benzodiazepine derivatives, which have amide bond in the aromatic ring and have an agonism of V2 receptor and useful for the treatment or prevention for diabetes insipidus, nocturnal enuresis in children and nocturia with aging.

Patent Reference 1: International Publication WO2002/000626 pamphlet;
Patent Reference 2: International Publication WO2001/049682 pamphlet;
Patent Reference 3: International Publication WO95/34540 pamphlet;
Patent Reference 4: International Publication WO99/37637 pamphlet;
Patent Reference 5: International Publication WO94/04525 pamphlet;
Patent Reference 6: Japanese Patent Publication H06-016643;
Patent Reference 7: Japanese Patent Publication H04-321669;
Non-patent Reference 1: Goodman&Gilman's, The Pharmacological Basis of Therpeutics(Tenth Edition), published by McGraw-Hill Co., Ltd. ;
Non-patent Reference 2: Tsutomu Akikawa and 2 persons, Scand. J. Urol. Nephrol. Suppl, 1999, Vol.202, pp.47-49;
Non-patent Reference 3: Jeffrey P. Weiss and 1 person, J. Urol., Vol.163, 2000, pp.5-12;
Non-patent Reference 4: Mogens Hammer and 1 person, J. Pharmacol. Exp. Ther., Vol.234, 1985, pp.754-760.

### Disclosure of the Invention

### Problem to be solved by the Invention

The object of the present invention is to provide a novel compound having an agoism of V2 receptor.

### Means of solving the Problems

As a result that the present inventors have studied earnestly on compounds having an agonism of V2 receptor, they acquired the surprising knowledge that a certain aromatic amide derivatives represented by a general formula (I) described below have an agonism of V2 receptor and are superior medicines having a decreasing activity of urine volume, thereby forming the bases of the present invention.

That is, the present invention relates to:
An aromatic amide derivative represented by the general formula:

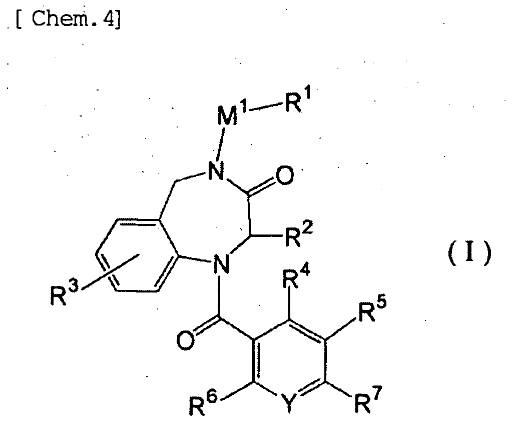

wherein R¹ represents a hydrogen atom or a C₁₋₆ alkyl group which may have a substituent selected from the following (Substituent group A);
(Substituent group A)
a hydroxy group, a halogen atom, a thiol group, a cyano group, a C₃₋₁₀ cycloalkyl group, a C₁₋₆ alkoxy group, a halo (C₁₋₆ alkyl) group, a C₆₋₁₀ aryl group, a C₆₋₁₀ aryloxy group, -COOR^{A1} (in the formula, R^{A1} is a hydrogen atom, a C₃₋₁₀ cycloalkyl group, a C₁₋₆ alkyl group or a C₆₋₁₀ aryl(C₁₋₆ alkyl) group), -CONHNR^{A2}R^{A3} (in the formula, R^{A2} and R^{A3} are independently a hydrogen atom, a formyl group, a C₂₋₇ acyl group, a C₁₋₆ alkoxy(C₂₋₇ acyl) group, a heteroarylcarbonyl group, an alicyclic amino (C₂₋₇ acyl) group, a C₁₋₆ alkoxycarbonyl (C₂₋₇ acyl) group or a C₆₋₁₀ arylcarbonyl group), -CONR^{A4}R^{A5} (in the formula, R^{A4} and R^{A5} are independently a hydrogen atom, a C₁₋₆ alkyl group, a C₁₋₆ alkoxy(C₁₋₆ alkyl) group, a hydroxy(C₁₋₆ alkyl) group, a C₃₋₁₀ cycloalkyl group, a hydroxy(C₃₋₁₀ cycloalkyl) group, a C₆₋₁₀ aryl(C₁₋₆ alkyl) group, a carboxy (C₁₋₆ alkyl) group, a C₁₋₆ alkoxycarbonyl (C₁₋₆ alkyl) group, a heteroaryl group or a C₆₋₁₀ aryl group, or -NR^{A4}R^{A5} forms an alicyclic amino group), -NR^{A6}R^{A7} (in the formula, R^{A6} and R^{A7} are independently a hydrogen atom, a C₁₋₆ alkyl group, a C₁₋₆ alkoxy(C₁₋₆ alkyl) group, a C₁₋₆ alkoxy(C₂₋₇ acyl) group, a C₂₋₇ acyl group, a C₆₋₁₀ aryl group, a C₆₋₁₀ arylcarbonyl group, a C₁₋₆ alkylsulfonyl group, a C₆₋₁₀ arylsulfonyl group or a heteroarylcarbonyl group, or -NR^{A6}R^{A7} forms an alicyclic amino group), -SO₂NR^{A8}R^{A9} (in the formula, R^{A8} and R^{A9} are independently a hydrogen atom, a C₁₋₆ alkyl group or a C₁₋₆ alkoxy(C₁₋₆ alkyl) group), a heterocycloalkyl group, a group represented by a general formula:

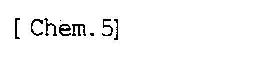

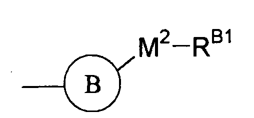

wherein B ring is a C₆₋₁₀ aryl group or a heteroaryl group, R^{B1} is a hydrogen atom, a halogen atom, a cyano group, a C₃₋₁₀ cycloalkyl group, a C₁₋₆ alkoxy group, a halo(C₁₋₆ alkyl) group, a C₁₋₆ alkoxy(C₁₋₆ alkyl) group, a hydroxy(C₁₋₆ alkyl) group, -COOR^{B11} (in the formula, R^{B21} is a hydrogen atom, a C₁₋₆ alkyl group or a C₆₋₁₀ aryl (C₁₋₆ alkyl) group), -CONR^{B12}R^{B13} (in the formula, R^{B12} and R^{B13} are independently a hydrogen atom, a C₁₋₆ alkyl group, a C₁₋₆ alkoxy(C₁₋₆ alkyl) group or a hydroxy(C₁₋₆ alkyl) group, or -NR^{B12}R^{B13} forms an alicyclic amino group), -NR^{B14}R^{B15} (in the formula, R^{B14} and R^{B15} are independently a hydrogen atom, a C₁₋₆ alkyl group, a C₂₋₇ acyl group, a C₆₋₁₀ arylcarbonyl group, a C₆₋₁₀ aryl group, a heteroaryl group, an alicyclic amino-subsutituted (C₁₋₆alkyl) group, a C₁₋₆ alkylsulfonyl group or a C₆₋₁₀ arylsulfonyl group, or -NR^{B14}R^{B15} forms an alicyclic amino group), or -SO₂NR^{B16}R^{B17} (in the formula, R^{B16} and R^{B17} are independently a hydrogen atom, a C₁-₆ alkyl group or a C₁-₆ alkoxy (C₁-₆alkyl) group, or -NR^{B16}R^{B17} forms an alicyclic amino group), NI² is a single bond or a C₁-₄ alkylene group; or a group represented by the general formula:

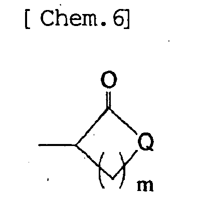

or

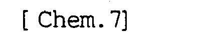

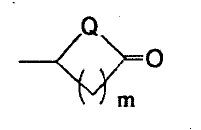

wherein Q is -O- or -NR^{C}- (in the formula, R^{C} is a hydrogen atom or a C₁₋₆ alkyl group), m is an integer from 1 to 4,
R² is a hydrogen atom or C₁₋₆ alkyl group;
R³ is a hydrogen atom, a halogen atom, a hydroxy group, a C₁₋₆ alkyl group or a C₁₋₆ alkoxy group;
R⁴, R⁵ and R⁶ are independently a hydrogen atom, a halogen atom, a C₁₋₆ alkyl group, a C₁₋₆ alkoxy group or a halo(C₁₋₆ alkyl) group;
R⁷ is a hydrogen atom, a halogen atom, a hydroxy group, a C₁₋₆ alkyl group, a halo(C₁₋₆ alkyl) group, a hydroxy(C₁₋₆ alkyl) group, a halo(C₁₋₆ alkoxy)C₁₋₆ alkyl group, a C₆₋₁₀ aryl group, a heteroaryl group, a heterocycloalkyl group, -NR^{D1}R^{D2} (in the formula, R^{D1} and R^{D2} are independently a hydrogen atom, a C₁₋₆alkyl group, a hydroxy(C₁₋₆ alkyl) group, a halo(C₁₋₆ alkyl) group or a C₁₋₆ alkoxy(C₁₋₆ alkyl) group, or -NR^{D1}R^{D2} forms an alicyclic amino group), -O-R^{D3} (in the formula, R^{D3} is a hydrogen atom, a C₁₋₆ alkyl group, a C₂₋₇ acyloxy-substituted(C₁₋₆ alkyl) group, a hydroxy(C₁₋₆ alkyl) group, a C₁₋₆ alkoxy(C₁₋₆ alkyl) group, a halo (C₁₋₆ alkyl) group, a halo(C₁₋₆ alkoxy)C₁₋₆ alkyl group, a C₁₋₆ alkoxycarbonyl(C₁₋₆ alkyl) group, a C₆₋₁₀ aryl group or a heteroaryl group), a C₆₋₁₀ aryl[C₁₋₆ alkoxy(C₁₋₆ alkyl)] group or a C₃₋₈ cycloalkyl group;
M¹ is a single bond, a C₁₋₄ alkylene group, -CO-, -NR^{E}- (in the formula,
R^{E} is a hydrogen atom, a C₁₋₆ alkyl group or a C₂₋₇ acyl group) or -SO₂-;
Y is N or CR^{E} (in the formula, R^{E} is a hydrogen atom, a halogen atom, a C₁₋₆ alkyl group or a halo(C₁₋₆ alkyl) group;
or a pharmaceutically acceptable salt thereof, or a prodrug thereof.

And in another situation, the present invention relates to a pharmaceutical composition comprising as an active ingredient an aromatic amide derivative represented by the above general formula (I) or a pharmaceutically acceptable salt thereof, or a prodrug thereof.

Moreover in another situation, the present invention relates to an agent for the treatment or prevention of a disease associated with an increseing of urine volume or an increasing of number of micturition, comprising as an active ingredient an aromatic amide derivative represented by the above general formula (I) or a pharmaceutically acceptable salt thereof, or a prodrug thereof.

Moreover in another situation, the present invention relates to a pharmaceutical composition comprising as an active ingredient a human V2 agonist an aromatic amide derivative represented by the above general formula (I) which is a human V2 agonist or a pharmaceutically acceptable salt thereof, or a prodrug thereof.

Moreover in another situation, the present invention relates to an agent for the treatment or prevention of a disease associated with diabetes insipidus (more preferably central diabetes insipidus), nocturia, nocturnal enuresis, overactive bladder, hemophilia or von-Wiliebrand's disease, comprising as an active ingredient an aromatic amide derivative represented by the above general formula (I) or a pharmaceutically acceptable salt thereof, or a prodrug thereof.

Moreover in another situation, the present invention relates to a pharmaceutical composition comprising as an active ingredient an aromatic amide derivative represented by the above general formula (I) or a pharmaceutically acceptable salt thereof, or a prodrug thereof, in combination with at least one agent selected from a group consisting of agents for the treatment of diabetes insipidus, nocturia and nocturnal enuresis other than a V2 agonist.

Moreover in another situation, the present invention relates to a pharmaceutical composition comprising as an active ingredient an aromatic amide derivative represented by the above general formula (I) or a pharmaceutically acceptable salt thereof, or a prodrug thereof, wherein the agent selected from a group consisting of agents for the treatment of diabetes insipidus, nocturia, nocturnal enuresis and overactive bladder other than a V2 agonist are an α₁-adrenoceptor blocker, an anticholinergic agent, a cholinergic agent, an antispasmodic agent, an anti-androgen'agent, an antidepressant, a calcium antagonist, a potassium-channel opener, a sensory nerve blocking agent, a β-adrenergic agonist, an acetylcholinesterase inhibitor or anti-inflammatory agent. As the agent selected, an α₁-adrenoceptor blocker, a calcium antagonist, a potassium-channel opener, a β-adrenergic agonist or an acetylcholinesterase inhibitor is preferable.

Moreover in another situation, the present invention relates to a use of an aromatic amide derivative represented by the above general formula (I) or a pharmaceutically acceptable salt thereof, or a prodrug thereof as claimed in any one of claims 1 to 8, for the manufacture of an agent for the treatment or prevention of diabetes insipidus (more preferably central diabetes insipidus), nocturia, nocturnal enuresis, overactive bladder, hemophilia or von-Wiliebrand's disease.

In the compounds represented by the above general formula (I) of the present invention, an agonism of V2 receptor can be confirmed by using a cell expressing human V2 receptor, and in the compounds of the present invention, a strongagonismof V2 receptorwas confirmed. In addition, the compounds represented by the above general formula (I) of the present invention having a strong antidiuretic effect were confirmed by antidiuretic effect-the confirmation study of antidiuretic effect on the diuretic activity induced by loading hypotonic solution in the anesthetized rats infused with hypotonic solution.

In the present invention, the following terms have the following meanings if not otherwise specified especially.

The term "halogen atom" means a fluorine atom, a chlorine atom, a bromine atom or an iodine atom. A fluorine atom, a chlorine atom or a bromine atom is preferable, and a chlorine atom or a fluorine atom is more preferable.

The term "C₁₋₆ alkyl group" or "C₁₋₆ alkyl-" means a straight-chained or branched alkyl group having 1 to 6 carbon atoms such as a methyl group, an ethyl group, a propyl group, an isopropyl group, a n-butyl group, an isobutyl group, a sec-butyl group, a tert-butyl group, a n-pentyl group, an isopentyl group, a neopentyl group, a tert-pentyl group, an 1-methylbutyl group, 2-methylbutyl group, a 1, 2-dimethylpropyl group, a hexyl group, an isohexyl group or the like. The C₁₋₆ alkyl group in R¹, R³, R⁴, R⁵, R⁶ and R⁷ is an alkyl group represented by a "C₁₋₃ alkyl group" having 1 to 3 carbon atoms preferably, and an ethyl group, a propyl group, an isopropyl group or a tert-butyl group is more preferable. The C₁₋₆ alkyl group in R² is an alkyl group represented by a "C₁₋₃ alkyl group" having 1 to 3 carbon atoms preferably, and a methyl group is more preferable.

The term "halo (C₁₋₆ alkyl) group" or "halo (C₁₋₆ alkyl)-" means C₁₋₆ alkyl group substituted by the same or different 1 to 3 halogen atoms as defined above such as a trifluoromethyl group, a 2-chloroethyl group, a 2-fluoroethyl group, a 2,2,2-trifluoroethyl group, a 2,2,2-trichloroethyl group or the like. C₁₋₆ alkyl group substituted by a fluorine atom is preferable, and a trifluoromethyl group, a 2-fluoroethyl group or a 2,2,2-trifluoroethyl group is more preferable.

The term "C₃₋₁₀ cycloalkyl group" or "C₃₋₁₀ cycloalkyl-" means a monocyclic aliphatic alkyl group having 3 to 10 carbon atoms such as a cyclopropyl group, a cyclobutyl group, a cyclopentyl group, a cyclohexyl group, a cycloheptyl group, a cyclooctyl group, a cyclononyl group, a cyclodecyl group or the like, or a cyclopentyl group or a cyclohexyl group fused with a benzene ring.

The term "heterocycloalkyl group" or "heterocycloalkyl-"means a 3 to 7-membered heterocyclic group containing any 1 to 4 hetero atoms selected from a group consisting of an oxygen atom, a sulfur atom and a nitrogen atom in the ring, for example, a tetrahydrofuryl group, a tetrahydropyranyl group, a tetrahydrothienyl group, a dihydrooxazolyl group, a dihydrothiazolylgroup, a dihydroimidazolyl. group, a piperidinyl group, a morpholinyl group, a homopiperazinyl group, a tetrahydropyrimidinyl group or the like can be illustrated.

The term "C₁₋₆ alkoxy group" or "C₁₋₆ alkoxy-" means a straight-chained or branched alkoxy group having 1 to 6 carbon atoms such as a methoxy group, an ethoxy group, a propoxy group, an isopropoxy group, a butoxy group, an isobutoxy group, a sec-butoxy group, a tert-butoxy group, a pentyloxy group, a hexyloxy group or the like. The alkoxy group having 1 to 4 carbon atoms is preferable, and a methoxy group, an ethoxy group or a propoxy group is more preferable.

The term "C₂₋₇ acyl group" means a straight-chained or branched acyl group having 2 to 7 carbon atoms such as an acetyl group, a propionyl group, a butyryl group, an isobutyryl group, a valeryl group, a pivaloyl group, a hexanoyl group or the like.

The term "C₆₋₁₀ aryl group" or "C₆₋₁₀ aryl-" means an aromatic hydrocarbon group having 6 to 10 carbon atoms such as a phenyl group, a naphthyl group or the like, unsubstituted or substituted by 1 to 5 groups independently selected from Group C described blow. A phenyl group substituted independently by 1 to 3 groups selected from a group consisting of Group C described blow or without is preferable.
Group C: a halogen atom, a cyano group, a nitro group, a C₁₋₆ alkyl group, halo(C₁₋₆ alkyl) group, C₁₋₆ alkoxy group, -OW¹, -OCOW², -COOW³, -NW⁴W⁵, -NW⁶COW⁷, -CONW⁸W⁹, -SO₂NW¹⁰W¹¹ or -NW¹²-SO₂W¹³, W¹ to W¹³ independently represent a hydrogen atom, a C₁₋₆ alkyl group, a hydroxy(C₁₋₆ alkyl) group or a C₆₋₁₀ aryl(C₁₋₆ alkyl), or W⁴ and W⁵, W⁶ and W⁷, W⁸ and W⁹, W¹⁰ and W¹¹, and W¹² and W¹³ may form an alicyclic amino group which contains the bound nitrogen atom.

The term "C₆₋₁₀ aryl (C₁₋₆ alkyl) group" means the above C₁₋₆ alkyl group substituted by the above C₆₋₁₀ aryl group. A benzyl group or a phenethyl group, each of which is unsubstituted or substituted by 1 to 3 groups independently selected from the above Group C on it's benzen-ring is preferable.

The term "C₆₋₁₀ aryloxy group" means a group represented by a C₆₋₁₀ aryl-O-, which substituted by the above C₆₋₁₀ aryl group. A phenoxy group, which is unsubstituted or substituted by 1 to 3 groups independently selected from the above Group C on it's benzen ring is preferable.

The term "heteroaryl group" means a 5 to 10-membered aromatic heterocyclic group containing 1 to 4 optional hetero atoms other than the binding position selected from a group consisting of an oxygen atom, a sulfur atom and a nitrogen atom in the ring, which is derived from thiazole, triazole, oxazole, isothiazole, isoxazole, pyridine, pyrimidine, pyrazine, pyridazine, furan, pyrrole, thiophene, imidazole, pyrazole, oxadiazole, thiodiazole, tetrazole, furazan or the like, or an aromatic heterocyclic group consisting of a 6-membered ring fused with a 5 or 6-membered ring containing 1 to 4 optional hetero atoms selected from a group consisting of an oxygen atom, a sulfur atom and a nitrogen atom in the ring other than the binding position, which is derived from indole, isoindole, benzofuran, isobenzofuran, benzothiophen, benzoxazole, benzothiazole, indazole, benzimidazole, quinoline, isoquinoline, phthalazine, quinoxaline, quinazoline, cinnoline, indolizine, naphthyridine, pteridine, phthalimide or the like. These aromatic heterocyclic groups are unsubstituted or substituted by 1 to 4 groups selected from the above Group C or without. In addition, these aromatic heterocyclic groups can be considered all regioisomers (for example, 2-pyridiyl group, 3-pyridiyl group, 4-pyridiyl group and the like). The 5 or 6-membered above aromatic heterocyclic group is preferable. In the compounds represented by the above general formula (I) of the present invention, the heteroaryl group at B ring is preferably the 5-membered above aromatic heterocyclic group.

The term "C₆₋₁₀ arylcarbonyl group" means a carbonyl group substituted by the above C₆₋₁₀ aryl group which is represented by a (C₆₋₁₀ aryl)-CO-, such as a benzoyl group, a 1-naphthlycarbonyl group, a 2-naphthylcarbonyl group and the like. A benzoyl group, which is unsubstituted or substituted by 1 to 3 groups independently selected from the above Group C on it's benzen ring is preferable.

The term "C₁₋₆ alkylsulfonyl group" means a sulfonyl group substituted by the above Cₗ-₆ alkyl group which is represented by a (C₁₋₆alkyl)-SO_{Z}-, suchasamethanesulfonylgroup, anethanesulfonyl group, a propanesulfonyl group, a butanesulfonyl group, a pentanesulfonyl group, hexanesulfonyl group and the like.

The term "C₆₋₁₀ arylsulfonyl group" means a sulfonyl group substituted by the above C₆₋₁₀ aryl group which is represented by a (C₆₋₁₀ aryl)-SO₂-. A phenylsulfonyl group group, which is unsubstituted or substituted by 1 to 3 groups independently selected from the above Group C on it's benzen ring is preferable.

The term "C₁₋₆ alkoxy(C₁₋₆ alkyl) group" means a C₁₋₆ alkyl group substituted by the above C₁₋₆ alkoxy group. A methoxymethyl group, an ethoxymethyl group, a propoxymethyl group, an isopropoxymethyl group, a butoxymethyl group, a tert-butoxymethyl group, a 2-methoxyethyl group, a 2-ethoxyethyl group, a 3-methoxypropoxy group or a 3-ethoxypropoxy group is preferable, a methoxyethyl group, an 2-methoxyethyl group or 2-ethoxyethyl group is more preferable.

The term "C₆₋₁₀ aryloxy(C₁₋₆ alkyl) group" means the above C₁₋₆ alkyl group substituted by the above C₆₋₁₀ aryloxy group. A phenoxymethyl group or 2-phenoxyethyl group, each of which is unsubstituted or substituted by 1 to 3 groups independently selected from the above Group C on it's benzen ring is preferable.

The term "C₆₋₁₀ aryl[C₁₋₆ alkoxy(C₁₋₆ alkyl)] group" means the above (C₁₋₆ alkoxy)C₁₋₆ alkyl group substituted by the above C₆₋₁₀ aryl group.

The term "hydroxy(C₁₋₆ alkyl) group" means the above Cₗ-₆ alkyl group substituted by a hydroxy group such as a hydroxymethyl group, a 2-hydroxyethyl group, a 1-hydroxyethyl group, a 3-hydroxypropyl group, a 2-hydroxypropyl group, a 1-hydroxypropyl group, . 4-hydroxybutyl group or the like. A hydroxymethyl group, a 2-hydroxyethyl group or a 3-hydroxypropyl group is preferable.

The term "halo (C₁₋₆ alkoxy) C₁₋₆ alkyl group" means the above C₁₋₆ alkyl group substituted by the above halo (C₁₋₆ alkoxy) group. The above C₁₋₆ alkyl group substituted by the above halo (C₁₋₆ alkoxy) group substituted by a fluorine atom is more preferable.

The term "alicyclic amino group" or "alicyclic amino-" means a 5 or 6-membered alicyclic amino group which may contain one hetero atom other than the nitrogen atom at the binding position selected from a group consisting of an oxygen atom, a sulfur atom and nitrogen atom in the ring, such as a morpholino group, a thiomorpholino group, a 1-aziridinyl group, a 1-azetidinyl group, a 1-pyrrolidinyl group, a 3-hydroxy-1-pyrrolidinyl group, a 3-methyl-1-pyrrolidinyl group, a piperidino group, 1-imidazolidinyl group, a 1-piperazinyl group, a pyrazolidinyl group or the like, which may substituted independently by 1 to 3 groups selected from a group consisting of the above Group C.

The term "C₁₋₄ alkylene group" means a straight-chained or branched alkylene group having 1 to 4 carbon atoms such as -CH₂-, -CH₂CH₂-, -CH(CH₃)-, -CH₂CH₂CH₂-, -CH(CH₃)CH₂-, -CH₂CH(CH₃)-, -C(CH₃)₂-, -CH(CH₂CH₃)-, -CH₂CH₂CH₂CH₂- or the like. A preferable group is -CH₂-, -CH₂CH₂- or -CH₂CH₂CH₂-.

The term "alicyclic amino (C₂₋₇ acyl) group" means the above C₂₋₇ acyl group substituted by the above alicyclic amino group.

The term "hydroxy (C₃₋₁₀ cycloalkyl) group" means the above C₃₋₁₀ cycloalkyl group substituted by a hydroxy group such as a 2-hydroxycyclopropyl group, a 2-hydroxycyclobutyl group, a 3-hydroxycyclobutyl group, a 2-hydroxycyclopentyl group, a 3-hydroxycyclopentyl group, a 2-hydroxycyclohexyl group, a 3-hydroxycyclohexyl group, a 4-hydroxycyclohexyl group or the like. A 2-hydroxycyclopentyl group is preferable.

The term "C₁₋₆ alkoxycarbonyl (C₁₋₆ alkyl) group" means a C₁₋₆ alkyl group substituted by a C₁₋₆ alkoxycarbonyl group.

The term "carboxy (C₁₋₆ alkyl) group" means a C₁₋₆ alkyl group substituted by a carboxyl group.

The term "C₁₋₆ alkoxy (C₂₋₇ acyl) group" means a C₂₋₇ acyl group substituted by the above C₁₋₆ alkoxy group.

The term "heteroarylcarbonyl group" means a arbonyl group substituted by the above heteroaryl group.

As the compounds represented by the above general formula (I) of the present invention have one and more asymmetric carbon atom, R-isomer or S-isomer at each asymmetric carbon atom, or an optional mixture of both isomers can be also employed in the present invention. In addition, each of a racemic compound, a racemic mixture, a single enantiomer and a diastereomeric compound can be employed in the present invention. As the compounds represented by the above general formula (I) of the present invention have one and more geometrical isomer, each of cis-isomer, trans-isomer and an optional mixture of both isomers can be also employed in the present invention. Moreover, the compounds represented by the above general formula (I) of the present invention include a hydrate and a solvate with a pharmaceutically acceptable solvent such as ethanol or the like.

In the present invention, the term "prodrug" means a compound obtained by modifying a parent compound with a pharmaceutically acceptable group generally used in a prodrug, and such compound can be expected, for example, to have additional characteristics such as improved stability, long action or the like and exert an efficacy after being converted into the parent compound in the body. The prodrugs of the compound represented by the above general formula (I) of the present invention can be prepared by suitably introducing a group forming a prodrug into one or more group optionally selected from a group consisting of a hydroxy group, a carboxy group, an amino group, another group acceptable to form a prodrug of a compound represented by the above general formula (I) using an agent to form a prodrug such as the corresponding halide compound or the like in the usual way and then optionally isolating and purifying in the usual way as an occasion demand (see "Gekkan-yekuji The clinical pharmacokinetics for proper uses of pharmaceutical drugs", Extra edition, March 2000, Vol.42, No.4, pp. 669-707 and "New drug delivery system", issued by CMC Co. Ltd., January 31, 2000, pp.67-173).

For example, in case that the compound represented by the above general formula (I) of the present invention have a carboxy group, as the prodrug, an ester which can be formed by replacing a hydrogen atom of the carboxy group by the following group: a C₁₋₆ alkyl group (for example, a methyl group, an ethyl group, a propyl group,an isopropyl group, a butyl group, a tert-butyl group and the like); a C₁₋₆ acyloxymethyl group (for example, a pivaloyloxymethyl group and the like); a 1-(C₂₋₇ acyloxy)ethyl group (for example', a 1-(pivaloyloxy)ethyl group and the like); a C₃₋₈ cycloalkoxycarbonyloxy(C₁₋₆ alkyl) group (for example, a 1-cyclohexyloxycarbonylethyl group and the like); a C₁₋₆ alkoxycarbonyloxymethyl group (for example, a tert-butoxycarbonyloxymethyl group) ; and a 1-(C₁₋₆ alkoxycarbonyloxy) ethyl group (for example, a 1-(tert-butoxycarbonyloxy)ethyl group); or a 3-phthalidyl group can be illustrated.

In addition, in case that the compound represented by the above general formula (I) of the present invention has a hydroxy group, as the prodrug, a compound which can be formed by replacing a hydrogen atom of the hydroxy group by the following group: a C₂₋₇ acyl group (for example, an acetyl group, a propionyl group, a butyryl group, an isobutyryl group, a pivaloyl group and the like); a C₁₋₆ alkoxycarbonyl group (for example, a methoxycarbonyl group, an ethoxycarbonyl group,a propoxycarbonyl group,an isopropoxycarbonyl group, a tert-butoxycarbonyl group and the like) ; a succinoyl group; a C₂₋₇ acyloxymethyl group (for example, a pivaloyloxymethyl group and the like); a 1-(C₂₋₇ acyloxy) ethyl group (for example, a 1-(pivaloyloxy)ethyl group and the like); or a C₁₋₆ alkoxycarbonyloxymethyl group (for example, tert-butoxycarbonyloxymethyl group); a C₃₋₈ cycloalkoxycarbonyl group (for example, a cyclohexyloxycarbonyl group and the like) can be illustrated.

In addition, in case that the compound represented by the above general formula (I) of the present invention have an amino group, as the prodrug, a compound which can be formed by replacing a hydrogen atom of the amino group by the following group: a C₂₋₇ acyl group (for example, an acetyl group, a propionyl group, a butyryl group, an isobutyryl group, a pivaloyl group and the like); a C₁₋₆ alkoxycarbonyl group (for example, a methoxycarbonyl group, an ethoxycarbonyl group, a propoxycarbonyl group, an isopropoxycarbonyl group, a tert-butoxycarbonyl group and the like); a C₃₋₈ cycloalkoxycarbonyl group (for example, a cyclohexyloxycarbonyl group and the like) can be illustrated.

Other preferable examples of the present invention are an aromatic amide derivative represented by a general formula (Ia):

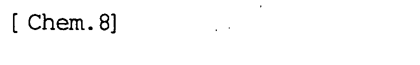

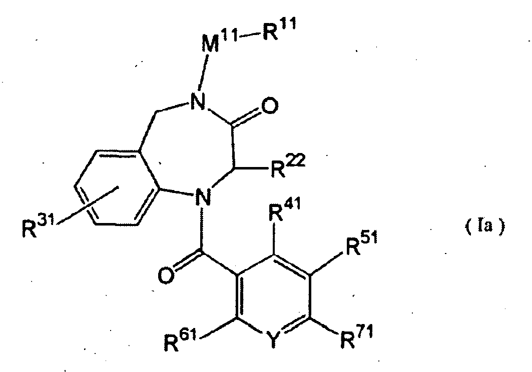

wherein R¹¹ is a hydrogen atom or a C₁₋₆ alkyl group which may have a substituent selected from the following (Substituent group A1);
(Substituent group A1) a hydroxy group, a halogen atom, a thiol group, a cyano group, a C₃₋₁₀ cycloalkyl group, a C₁₋₆ alkoxy group, a halo (C₁₋₆ alkyl) group, a C₆₋₁₀ aryl group, a C₆₋₁₀ aryloxy group, -COOR^{A1} (in the formula, R^{A1} is a hydrogen atom, a C₃₋₁₀ cycloalkyl group, a C₁₋₆ alkyl group or a C₆₋₁₀ aryl (C₁₋₆ alkyl) group), -CONHNR^{A2}R^{A3} (in the formula, R^{A2} and R^{A3} are independently a hydrogen atom, a C₂₋₇ acyl group, a C₁₋₆ alkoxy(C₂₋₇ acyl) group, a C₁₋₆ alkoxycarbonyl-subsutituted (C₂₋₇ acyl) group or C₆₋₁₀ arylcarbonyl group), -CONR^{A41}R^{A51} (in the formula, R^{A41} is a hydrogen atom, and R^{A51} is a C₁₋₆ alkyl group, a C₁₋₆alkoxy(C₁₋₆ alkyl) group, a hydroxy(C₁₋₆ alkyl) group, a heteroaryl group or a C₆₋₁₀ aryl group), -NR^{A6}R^{A7} (in the formula, in case that R^{A6} is a hydrogen atom, R^{A7} is a C₂₋₇ acyl group, a C₆₋₁₀ arylcarbonyl group or a heteroarylcarbonyl group, or in case that R^{A6} is a C₁₋₆ alkyl group, a C₆₋₁₀ aryl group or a heteroarylcarbonyl group, R^{A7} is a C₁₋₆ alkylsulfonyl group or a C₆₋₁₀ arylsulfonyl group), -SO₂NR^{A8}R^{A9} (in the formula, R^{A8} is a hydrogen atom, R^{A9} is a C₁₋₆ alkyl group or a C₁₋₆ alkoxy(C₁₋₆ alkyl) group), a heterocycloalkyl group, a group represented by the general formula:

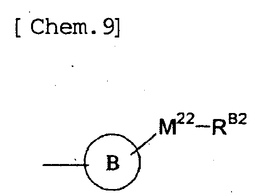

wherein B ring is a C₆₋₁₀ aryl group or a heteroaryl group, R^{B2} is a hydrogen atom, a halogen atom, a cyano group, a C₃₋₁₀ cycloalkyl group, a C₁₋₆ alkoxy group, a halo (C₁₋₆ alkyl) group, a C₁₋₆ alkoxy(C₁₋₆ alkyl) group, a hydroxy(C₁₋₆ alkyl) group, -COOR^{B21} (in the formula, R^{B21} is a hydrogen atom, a C₁₋₆ alkyl group or a C₆₋₁₀ aryl (C₁₋₆ alkyl) group), -CONR^{B22}R^{B23} (in the formula, R^{B22} and R^{B23} are independently a hydrogen atom, a C₁₋₆ alkyl group, a C₁₋₆ alkoxy(C₁₋₆ alkyl) group or a hydroxy (C₁₋₆ alkyl) group, or -NR^{B22}R^{B23} forms an alicyclic amino group), -NR^{B24}R^{B25} (in the formula, R^{B24} and R^{B25} are independently a hydrogen atom, a C₁₋₆ alkyl group, a C₂₋₇ acyl group, a C₆₋₁₀ arylcarbonyl group, C₁₋₆ alkylsulfonyl group or a C₆₋₁₀ arylsulfonyl group, or -NR^{B24}R^{B25} forms an alicyclic amino group), -SO₂NR^{B26}R^{B27} (in the formula, R^{B26} and R^{B27} are independently a hydrogen atom, a C₁₋₆ alkyl group or a C₁₋₆ alkoxy(C₁₋₆ alkyl) group, or -NR^{B26}R^{B27} forms an alicyclic amino group), M²² is a single bond or a C₁₋₄ alkylene group, or a group represented by the general formula:

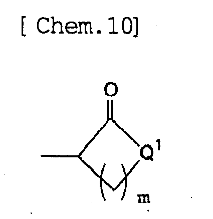

or 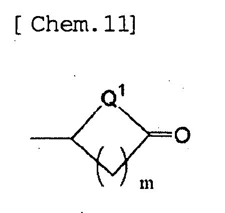 wherein Q¹ is -NR^{C}- (in the formula, R^{C} is a hydrogen atom or a C₁₋₆ alkyl group), and m is an integer from 1 to 4,
R²² is a hydrogen atom or a methyl group;
R31 is a hydrogen atom, a halogen atom, a hydroxy group or a C₁₋₆ alkyl group;
R⁴¹, R⁵¹ and R⁶¹ are independently a hydrogen atom, a halogen atom, a C₁₋₃ alkyl group, a C₁₋₆ alkoxy group or a halo (C₁₋₃ alkyl) group; R⁷¹ is a hydrogen atom, a halogen atom, a hydroxy group, a C₁₋₆ alkyl group, a halo (C₁₋₆ alkyl) group, a hydroxy (Cₗ-₆ alkyl) group, a halo(C₁₋₆ alkoxyl)C₁₋₆ alkyl group, a C₆₋₁₀ aryl group, a heteroaryl group, a C₃₋₈ cycloalkyl group, a heterocycloalkyl group, -NR^{D11}R^{D22} (in the formula, R^{D11} and R^{D22} are independently a hydrogen atom, a C₁₋₆ alkyl group, a hydroxy(C₁₋₆ alkyl) group, a halo (C₁₋₆ alkyl) group or a C₁₋₆ alkoxy(C₁₋₆ alkyl) group, or-NR^{D11}R^{D22} forms an alicyclic amino group) , -O-R^{D33} [ in the formula, R^{D33} is a hydrogen atom, a C₁₋₆ alkyl group, a C₂₋₇ acyloxy-substituted (C₁₋₆ alkyl) group, a hydroxy(C₁₋₆ alkyl) group, a C₁₋₆ alkoxy (C₁₋₆ alkyl) group, a halo (C₁₋₆ alkyl) group or a halo(C₁₋₆ alkoxy)C₁₋₆ alkyl group)], or a C₆₋₁₀ aryl[C₁₋₆ alkoxy(C₁₋₆ alkyl)] group;
M¹¹ is a single bond or a C₁₋₄ alkylene group;
Y is N or CR^{F} (in the formula, R^{F} is a hydrogen atom, a halogen atom, a C₁₋₆ alkyl group or a halo(C₁₋₆ alkyl) group.

As the compound represented by the above general formula (Ia), R¹¹ is preferably a hydrogen atom or a C₁₋₆ alkyl group which may have a substituent selected from a group consisting of the following (Substituent group A2),
(Substituent group A2) a hydroxy group, a C₃₋₁₀ cycloalkyl group, a C₁₋₆ alkoxy group, a halo(C₁₋₆ alkyl) group, -COOR^{A1} (in the formula, R^{A1} is a hydrogen atom, a C₃₋₁₀ cycloalkyl group, a C₁₋₆ alkyl group or a C₆₋₁₀ aryl (C₁₋₆ alkyl) group), -CONR^{A41}R^{A51} (in the formula, R^{A41} is a hydrogen atom, and R^{A51} is a C₁₋₆ alkyl group, a C₁₋₆ alkoxy(C₁₋₆ alkyl) group, a hydroxyl(C₁₋₆ alkyl) group, a heteroaryl group or a C₆₋₁₀ aryl group), or a group represented by the general formula:

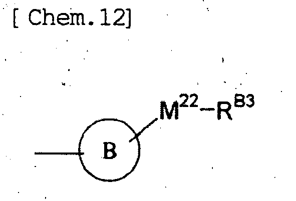

wherein B ring is a C₆₋₁₀ aryl group or a heteroaryl group, R^{B3} is a hydrogen atom, a C₃₋₁₀ cycloalkyl group, a C₁₋₆ alkoxy group, a halo(C₁₋₆ alkyl) group, -COOR^{B21} (in the formula, R^{B21} is a hydrogen atom, a C₁₋₆ alkyl group or a C₆₋₁₀ aryl(C₁₋₆ alkyl) group), -CONR^{B22}R^{B23} (in the formula, R^{B22} and R^{B23} are independently a hydrogen atom, a C₁₋₆ alkyl group, a C₁₋₆ alkoxy(C₁₋₆ alkyl) group or a hydroxyl(C₁₋₆ alkyl) group, or -NR^{B22}R^{B23} forms an alicyclic amino group), a C₁₋₆ alkoxy(C₁₋₆ alkyl) group or a hydroxy(C₁₋₆ alkyl) group, and M²² is a single bond or a C₁₋₄ alkylene group,
R²² is preferably a hydrogen atom,
R³¹ is preferably a hydrogen atom,
R⁴¹, R⁵¹ and R⁶¹ are preferably independently a hydrogen atom or a halogen atom,
R⁷¹ is preferably a hydroxy(C₁₋₆ alkyl) group, a halo (C₁₋₆ alkoxyl) C₁₋₆ alkyl group, a C₆₋₁₀ aryl group, a heteroaryl group, a heterocycloalkyl group, -NR^{D11}R^{D22} (in the formula, R^{D11} and R^{D22} are independently a hydrogen atom, a C₁₋₆ alkyl group, a hydroxy(C₁₋₆ alkyl) group, a halo(C₁₋₆ alkyl) group or a C₁₋₆ alkoxy(C₁₋₆ alkyl) group, or -NR^{D11}R^{D22} forms an alicyclic amino group), -O-R^{D33} [in the formula, R^{D33} is a hydrogen atom, a C₁₋₆ alkyl group, a C₂₋₇ acyloxy-substituted (C₁₋₆ alkyl) group, a hydroxy(C₁₋₆ alkyl) group, a C₁₋₆ alkoxy(C₁₋₆ alkyl) group, a halo(C₁₋₆ alkyl) group or a halo(C₁₋₆ alkoxy)C₁₋₆ alkyl group], or a C₆₋₁₀ aryl[C₁₋₆ alkoxy(C₁₋₆ alkyl)] group, a more preferable group is a C₆₋₁₀ aryl group, a heteroaryl group, a heterocycloalkyl group, -NR^{D11}R^{D22} (in the formula, R^{D11} and R^{D22} are independently a hydrogen atom, a C₁₋₆ alkyl group, a hydroxy(C₁₋₆ alkyl) group, a halo(C₁₋₆ alkyl) group or a C₁₋₆ alkoxy(C₁₋₆ alkyl) group, or -NR^{D11}R^{D22} forms an alicyclic amino group), -O-R^{D33} [in the formula, R^{D33} is a hydrogen atom, a C₁₋₆ alkyl group, a C₂₋₇ acyloxy-substituted (C₁₋₆ alkyl) group, a hydroxy(C₁₋₆ alkyl) group, a C₁₋₆ alkoxy(C₁₋₆ alkyl) group, a halo (C₁₋₆ alkyl) group or a halo(C₁₋₆ alkoxy)C₁₋₆ alkyl group],
M¹¹ is preferably a C₁₋₄ alkylene group;
Y is preferably CR^{F} (in the formula, R^{F} is a hydrogen atom, a halogen atom, a C₁₋₆ alkyl group or a halo(C₁₋₆ alkyl) group, R^{F} is more preferably a hydrogen atom).

Among the substituent of (Substituent group A2) described the above R¹¹, the substituent of (Substituent group A3) described below is more preferable.
(Substituent group A3)
a hydroxy group, a C₃₋₁₀ cycloalkyl group, a C₁₋₆ alkoxy group, a halo(C₁₋₆ alkyl) group, -COOR^{A1} [in the formula, R^{A1} is a hydrogen atom, a C₃₋₁₀ cycloalkyl group, a C₁₋₆ alkyl group or a C₆₋₁₀ aryl (C₁₋₆ alkyl) group], -CONR^{A41}R^{A51} [in the formula, R^{A41} is a hydrogen atom, and R^{A51} is a C₁₋₆ alkyl group, a C₁₋₆ alkoxy(C₁₋₆ alkyl) group, a hydroxy(C₁₋₆ alkyl) group, a heteroaryl group or a C₆₋₁₀ aryl group], or a group represented by the general formula:

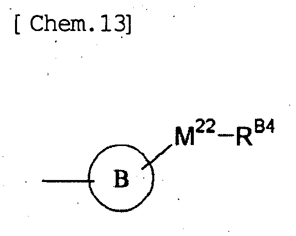

wherein a ring B is a C₆₋₁₀ aryl group or a heteroaryl group, R^{B4} is a hydrogen atom, a C₃₋₁₀ cycloalkyl group, a C₁₋₆ alkoxy group or a halo (C₁₋₆ alkyl) group, and M²² is a single bond or a C₁₋₄ alkylene group.

As a concrete preferable example of the present invention, compounds described in Examples or the like can be illustrated. Among them, compounds selected from the following group, or pharmaceutically acceptable salts thereof are preferable.

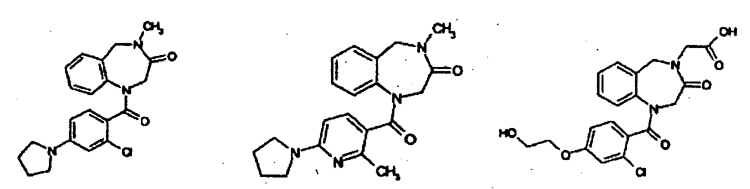

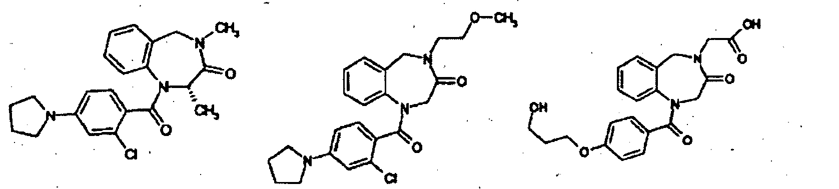

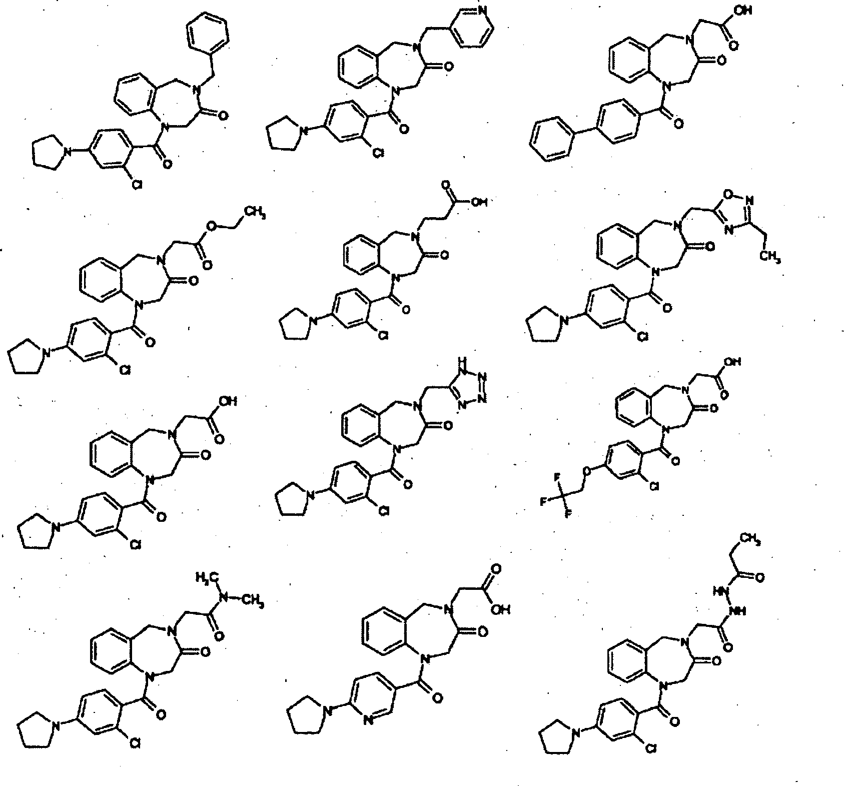

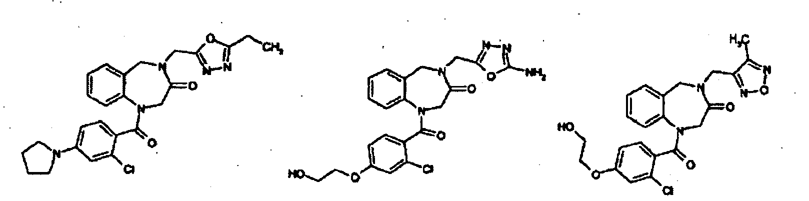

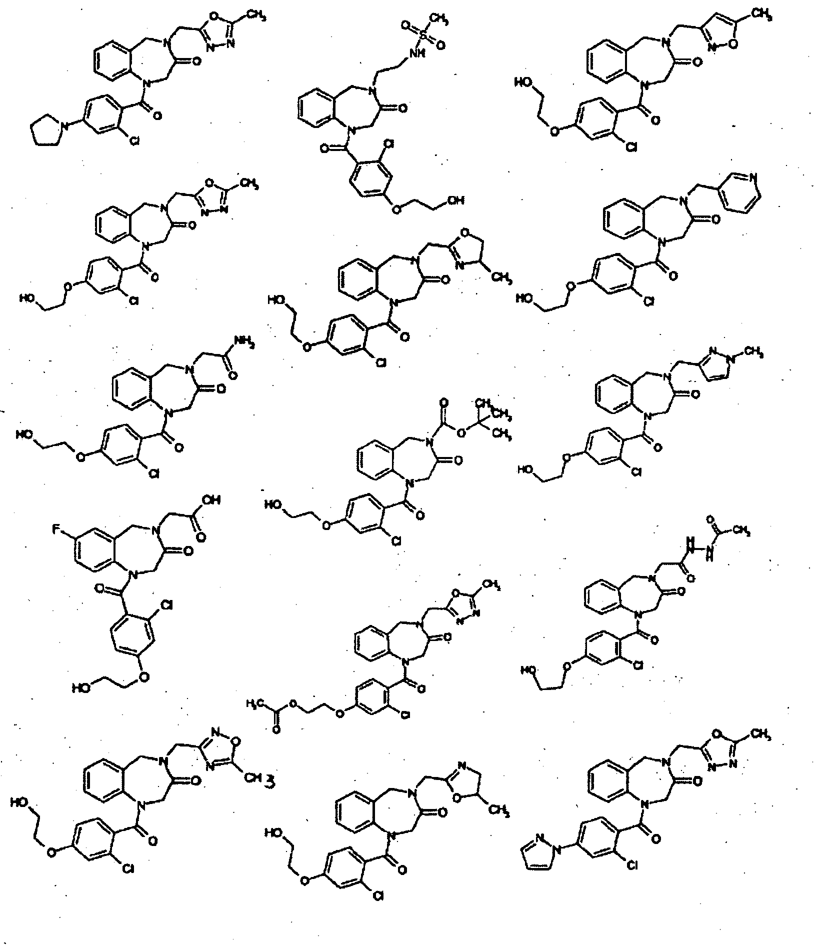

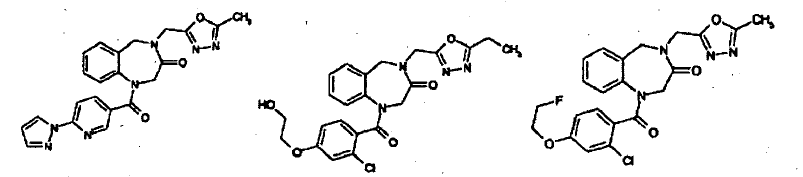

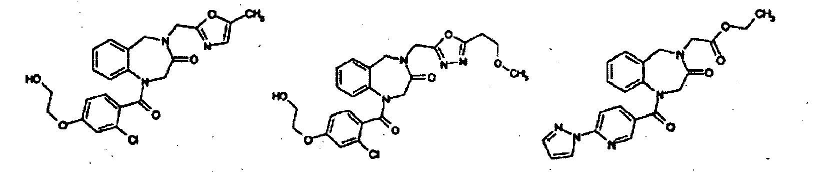

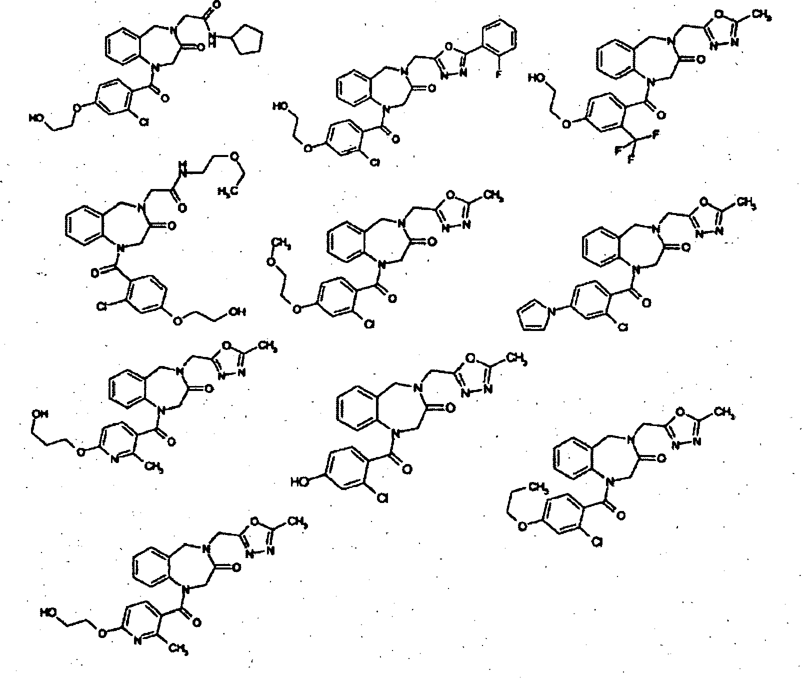

The compounds represented by the above general formula (I) of the present invention can be prepared, for example, in methods described below in schemes 1 to 6 or similar methods, or methods described in literatures or similar methods or the like. In addition, in case that a protective group is necessary in accordance with a kind of a functional group, introduction and removal procedures can be optionally combined in the usual way. About a kind of a protective group, introduction and removal procedures, for example, the methods described in "Protective Groups inOrganic Synthesis (third edition) " written and edited by Green & Wuts can be illustrated.

The typical methods of manufacture are shown below. There is a case that each process of each scheme described below is executed in combination with multistep reaction, and may be combined with any process selected by those in the art.

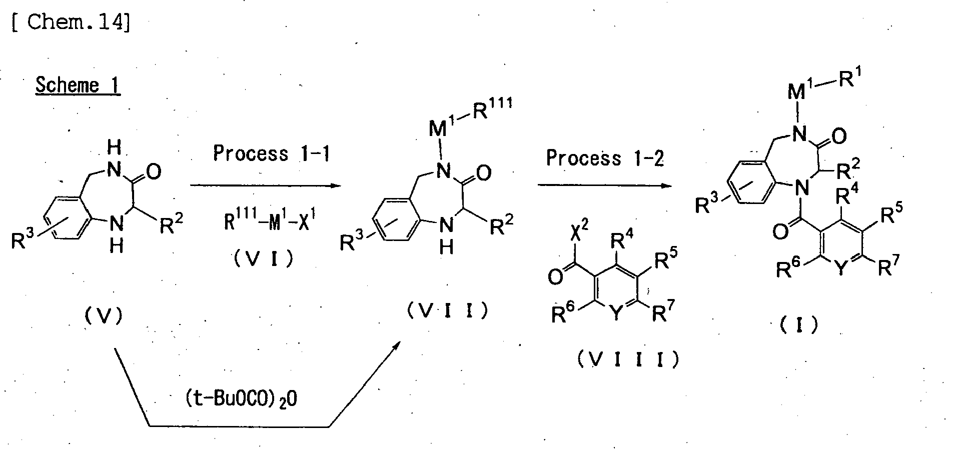

In the formula, X¹ is a halogen atom, a trifluoromethanesulfonyloxy group, a methanesulfonyloxy group or p-toluenesulfonyloxy group; X² is a halogen atom; and R¹¹¹ is a C₁₋₆ alkyl group which may have a substituent selected from the following (Substituent group A) allowed to have a protective group.
(Substituent group A)
a hydroxy group, a halogen atom, a thiol group, a cyano group, a C₃₋₁₀ cycloalkyl group, a C₁₋₆ alkoxy group, a halo(C₁₋₆ alkyl) group, a C₆₋₁₀ aryl group, a C₆₋₁₀ aryloxy group, -COOR^{A1} (in the formula, R^{A1} is a hydrogen atom, a C₃₋₁₀ cycloalkyl group, a C₁₋₆ alkyl group or a C₆₋₁₀ aryl(C₁₋₆ alkyl) group), -CONHNR^{A2}R^{A3} (in the formula, R^{A2} and R^{A3} are independently a hydrogen atom, a C₂₋₇ acyl group, a C₁₋₆ alkoxy(C₂₋₇ acyl) group, an alicyclic amino (C₂₋₇ acyl) group, a C₁₋₆ alkoxycarbonyl(C₂₋₇ acyl) group or a C₆₋₁₀ arylcarbonyl group), -CONR^{A4}R^{A5} (in the formula, R^{A4} and R^{A5} are independently a hydrogen atom, a C₁₋₆ alkyl group, a C₁₋₆ alkoxy(C₁₋₆ alkyl) group, a hydroxy(C₁₋₆ alkyl) group, a heteroaryl group or a C₆₋₁₀ aryl group, or -NR^{A4}R^{A5} forms an alicyclic amino group), -NR^{A6}R^{A7} (in the formula, R^{A6} and R^{A7} are independently a hydrogen atom, a C₁₋₆ alkyl group, a C₁₋₆ alkoxy(C₁₋₆ alkyl) group, a C₁₋₆ alkoxy(C₂₋₇ acyl) group, a C₂₋₇ acyl group, a C₆₋₁₀ aryl group, a C₆₋₁₀ arylcarbonyl group, a C₁₋₆ alkylsulfonyl group, a C₆₋₁₀ arylsulfonyl group or a heteroarylcarbonyl group, or -NR^{A6}R^{A7} forms an alicyclic amino group), -SO₂NR^{A8}R^{A9} (in the formula, R^{A8} and R^{A9} are independently a hydrogen atom, a C₁₋₆ alkyl group, a C₁₋₆ alkoxy(C₁₋₆ alkyl) group or a C₃₋₁₀ cycloalkyl group), a heterocycloalkyl group, a group represented by a general formula: 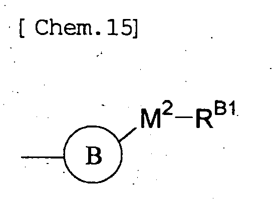 wherein B ring is a C₆₋₁₀ aryl group or a heteroaryl group, R^{B1} is a hydrogen atom, a halogen atom, a cyano group, a C₃₋₁₀ cycloalkyl group, a C₁₋₆ alkoxy group, a halo(C₁₋₆ alkyl) group, a C₁₋₆ alkoxy(C₁₋₆ alkyl) group, a hydroxy(C₁₋₆ alkyl) group, -COOR^{B11} (in the formula, R ^{B11} is a hydrogen atom, a C₁₋₆ alkyl group or a C₆₋₁₀ aryl (C₁₋₆ alkyl) group), -CONR^{B12}R^{B13} (in the formula, R^{B12} and R^{B13} are independently a hydrogen atom, a C₁₋₆ alkyl group, a C₁₋₆ alkoxy(C₁₋₆ alkyl) group or a hydroxy(C₁₋₆ alkyl) group, or -NR^{B12}R^{B13} forms an alicyclic amino group), -NR^{B14}R^{B15} (in the formula, R^{B14} and R^{B15} are independently a hydrogen atom, a C₁₋₆ alkyl group, a C₂₋₇ acyl group, a C₆₋₁₀ arylcarbonyl group, a C₆₋₁₀ aryl group, a C₁₋₆ alkylsulfonyl group or a C₆₋₁₀ arylsulfonyl group, or -NR^{B14}R^{B15} forms an alicyclic amino group), or -SO₂NR^{B16}R^{B17} (in the formula, R^{B16} and R^{B17} are independently a hydrogen atom, a C₁₋₆ alkyl group or a C₁₋₆ alkoxy(C₁₋₆ alkyl) group, or -NR^{B16}R^{B17} forms an alicyclic amino group) M² is a single bond or a C₁₋₄ alkylene group; or a group represented by the general formula: 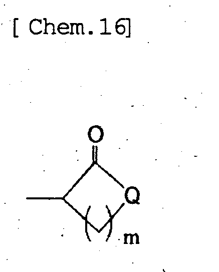 or 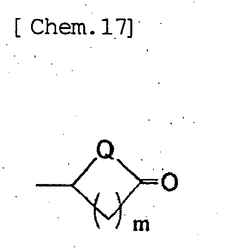 wherein Q is -O- or -NR^{C}- (in the formula, R^{C} is a hydrogen atom or a C₁₋₆ alkyl group), m is an integer from 1 to 4, and R², R³, R⁴, R⁵, R⁶, R⁷, M¹ and Y have the same meanings as defined above.

### Process 1-1

A compound (VII) can be obtained prepared by subjecting a compound represented by the general formula (V) to condensation of a compound represented by the above general formula R¹¹¹-M¹-X¹ (VI) or di-tert-butyl dicarboxylate in the presence of a base in a solvent. As the used solvent, *N,N*-dimethylformamide, tetrahydrofuran, a mixed solvent thereof or the like can be illustrated, as a base, sodium hydride, potassium hydroxide, potassium tert-butoxide, 4-dimethylaminopyridine, lithium bis(trimethylsilyl)amide, benzyltrimethylammonium hydroxide or the like can be illustrated. It is preferable to use 1 to 5 amounts of the base for the compound (V). The reaction temperature is usually from-20°C to solvent reflux temperature, and the reaction time is usually from 30 minutes to 3 days, varying based on a used starting material, solvent and reaction temperature.

Process 1-1 can be conducted by the desired method selected those in the art usually, for example, by the followingprocess selected optionally or by exchange an order of process.

Reaction (a): a reduction reaction
Reaction (b): a hydrolysis reaction
Reaction (c) : a reaction deriving -COOH in R¹¹¹ to -CONHNR^{A2}R^{A3} (in the formula, R^{A2} and R^{A3} have the same meanings as defined above) or -CONR^{A4}R^{A5} (in the formula, R^{A4} and R^{A5} have the same meanings as defined above)
Reaction (d) : a reaction converting a cyano group in R¹¹¹ into a tetrazolyl group
Reaction (e) : an esterification reaction of converting -COOH in R¹¹¹
Reaction (f): a reaction converting an ester group or a carbamoyl group in R¹¹¹ into a 1,2,4-oxadiazole group
Reaction (g) : a reaction converting -CONHNR^{A2}R^{A3} in _{R}¹¹¹ (in the formula, R^{A2} and R^{A3} have the same meanings as defined above) into a 1, 3, 4-oxadiazole group
Reaction (h) : a reaction converting a carbamoyl group in R¹¹¹ into a cyano group
Reaction (i): an alkylation reaction of a hydroxy group
Reaction (j): a reaction converting a hydroxy group into an amino group
Reaction (k): an amidation reaction of an amino group
Reaction (1): an oxidation reaction
Reaction (m): a hydrogenolysis reaction
Reaction (n) : in case that R¹¹¹ is -CONHR^{A10} (in the formula, R^{A10} is a hydroxy (C₁₋₆ alkyl) group and however a position of hydroxy group is β) and M¹ is a C₁₋₄ alkylene group, a reaction converting -CONHR^{A10} into an oxazoline group
Reaction (o) : a reaction converting a cyano group in R¹¹¹ into a 1,2,4-oxadiazole group
Reaction (p) : a reaction converting -COOH into a urea group
Reaction (q): a reaction converting an ester group into a hydrazide group

The reduction reaction of reaction (a) can be used by the reduction method which those in the art can be usually selected. For example, as the reduction used for a metal, a metal salt, a hydride compound, an enzyme, a microorganism or the like can be illustrated, these can be conducted by the method described in JIKKEN KAGAKU KOUZA (Experimental Chemistry, the forth edition) edited by The Chemical Society of Japan, Vol.26, 1990, MARUZEN CO., LTD publication or the like.

The reaction hydrolyzing carboxylic acid derivatives(an ester, a lactone, an acid halide, an acid anhydrate, an acid amide, an acid hydrazide, a nitrile or the like) of reaction (b) can be used the hydrolysis method which those in the art can be selected usually. For example, an acid or alkali hydrolysis, a hydrolysis at neutral condition using a halogen ion, a thiolate or the like, or a catalysed hydrolysis can be illustrated, for example, these can be conducted by the method described in JIKKEN KAGAKU KOUZA (Experimental Chemistry, the forth edition) Ed. by The Chemical Society of Japan, Vol.26, 1990, MARUZEN CO., LTDpublication, JIKKENKAGAKUKOUZA (Experimental Chemistry, the forth edition) Ed. by The Chemical Society of Japan, Vo1.26, 1990, MARUZEN CO., LTD publication or the like.

The converting a carboxylic acid or the reactive functional derivatives (an acid halide, an acid anhydrate, an active ester or the like) into an amide of reaction (c) can be used the amidation method which those in the art can be usually selected, for example, by the amidation a carboxy group or the reactive functional derivatives, with hydrazine derivatives (for example, NH₂NR^{A2}R^{A3}, in the formula, R^{A2} and R^{A3} have the same meanings as defined above) or amine derivatives (for example, NHR^{A4}R^{A5}, in in the formula, R^{A4} and R^{A5} have the same meanings as defined above), -CONHNR^{A2}R^{A3} (in the formula, R^{A2} and R^{A3} have the same meanings as defined above) or -CONR^{A4}R^{A5} (in the formula, R^{A4} and R^{A5} have the same meanings as defined above) can be derived. for example, these can be conducted by the method described in JIKKEN KAGAKU KOUZA (Experimental Chemistry, the 4th edition) edited by The Chemical Society of Japan, Vol.22, 1990, MARUZEN CO., LTD publication or the like.

The reaction converting a cyano group (a nitrile group, a halogeno cyan, a alkoxy cyan or the like) or a carboxylic amidrazone into a tetrazolyl group of reaction (d) can be used the ring closure reaction which those in the art can be selected usually, for example, these can be conducted by the method described in SHINHEN HETEROKANKAGOUBUTSU (New editon of heterocyclic compound), applied chapter, edited by Hiroshi Yamanaka 2004, MARUZEN CO., LTD publication or the like.

The esterification reaction of carboxylic acid of reaction (e) can be used the esterification reaction which those in the art can be selected usually. Forexample, the esterification of carboxylic acids with alcohols by acid-catalyst, the esterification of reactive functional derivatives (acid halides, acid anhydrates, active esteres or the like) and alcohols, esterification used alkylating agents in the presence or absence of bases or the like can be illustrated. These can be conducted by the method described in JIKKEN KAGAKU KOUZA (Experimental Chemistry, the 4th edition) edited by The Chemical Society of Japan, Vo1.22, 1990, MARUZEN CO., LTD publication or the like.

Of reaction (f), the reaction converting an ester group or a carbamoyl group into a 1,2, 4-oxadiazole group can be used the ring closure reaction which those in the art can be usually selected. For example, the reaction can be conducted by allowing the ester derivatives to react with amidoxime derivatives in the presence of a base. As the solvent, water, toluene, ethanol, tetrahydrofuran or the like can be illustrated. As the base, triethylamine, pyridine, N-methylmorphorine, sodium hydride, sodium hydroxide or the like can be illustrated. The reaction temperature is usually from room temperature to solvent reflux temperature, and the reaction time is usually from 10 minutes to 12 hours.

Of reaction (g), the reaction converting an acy hydrazide or -CONHNR^{A2}R^{A3} (in the formula, R^{A2} and R^{A3} have the same meanings as defined above) into 1,3,4-oxadiazole group can be used Paal-Knorr type cyclization reaction which those in the art canbe usually selected. For example, these can be conducted by the method described in SHINHEN HETEROKANKAGOUBUSTU (New editon of heterocyclic compound), applied chapter, edited by Hiroshi Yamanaka 2004, KOUDANSHA Ltd publication or the like.

Of reaction (h), the method converting a carbamoyl group into a cyano group can be used the dehydration reaction which those in the art can be usually selected. For example, the group can be dehydrated in combination of an organic solvent, an acylating agent or sulfonylating agent, a base and a dehydrating agent optionally. As the used organic solvent, dichloromethane, toluene, diethylether, tetrahydrofuran, dioxane or the like can be illustrated. As an acylating agent or a sulfonylating agent, acetic anhydride, trifluoroacetic anhydride, methanesulfonic anhydride, p-toluenesulfonyl chloride or the like can be illustrated. As a base, triethylamine, pyridine, N-methylmorphorine or the like can be illustrated. In addition, as a dehydrating agent, phosphorus pentoxide, phosphoryl chloride or the like can be illustrated. The reaction temperature is usually from room temperature to solvent reflux temperature, and the reaction time is usually from 10 minutes to 12 hours. These can be conducted, for example, by the method described in SHIN JIKKEN KAGAKU KOUZA (New Experimental Chemistry Lecture) edited by The Chemical Society of Japan, Vol.14 III, 1978, published by MARUZEN CO., LTD. or the like.

Of reaction (i), the reaction converting a hydroxy group into an ether group can be used the reaction which those in the art can be selected usually, the reaction can be conducted, for example, by the method described in JIKKEN KAGAKU KOUZA (Experimental Chemistry Lecture, the 4th Edition) edited by The Chemical Society of Japan, Vo1.20, 1990, published by MARUZEN CO., LTD. or the like.

Of reaction (j), the reaction converting a hydroxy group into an amino group can be used the reaction which those in the art can be selected usually. The converting reaction from a hydroxy group into an amino group directly can be conducted by using in the presence of a metal catalyst such as copper(I) oxide, dichlorotris(triphenylphosphine)ruthenium(II) or the like. On the other, after converting a hydroxy group into a good leaving group, and then the group can be also converted into an amino group. As a good leaving group, a halogen atom such as iodide, bromide, chloride or the like, methanesulfonyl group, p-toluenesulfonyl group can be illustrated, the reaction can be conducted, for example, by the method described in JIKKEN KAGAKU KOUZA (Experimental Chemistry Lecture, the 4th Edition) edited by The Chemical Society of Japan, Vol.20, 1990, published by MARUZEN CO., LTD. or the like.

Of reaction (k), the reaction converting an amino group into an amide group can be used the reaction which those in the art can be selected usually, the reaction can be conducted, for example, by the method described in JIKKEN KAGAKU KOUZA (Experimental Chemistry Lecture, the 4th Edition) edited by The Chemical Society of Japan, Vol.20, 1990, published by MARUZEN CO., LTD. or the like.

Of reaction (1), the oxidation reaction can be used the oxidation reaction which those in the art can be selected usually. For example, the reaction can be conducted by using a metal oxidating agent, peracid or peroxide, a metal catalyst using catalytic dehydrogenation,in stoichiometric or catalytic amounts,for example, the reaction can be conducted by the method described in JIKKEN KAGAKU KOUZA (Experimental Chemistry Lecture, the 4th Edition) edited by The Chemical Society of Japan, Vol.23, 1990, published by MARUZEN CO., LTD. or the like.

Of reaction (m), the hydrogenolysis reaction can be used the reduction reaction which those in the art can be usually selected. For example, the reaction can be conducted in an organic solvent, in the presence of metal catalyst, under a hydrogen atmosphere. As a metal catalyst, palladium on carbon, Raney nickel, platinum or the like can be illustrated. As an organic solvent, tetrahydrofuran, dichloromethane, ethyl acetate, acetic acid, ethanol or the like can be illustarted. The reaction temperature is usually from room temperature to solvent reflux temperature. These, for example, can be conducted by the method described in JIKKEN KAGAKU KOUZA (Experimental Chemistry Lecture, the 4th Edition) edited by The Chemical Society of Japan, Vol.26, 1990, published by MARUZEN CO., LTD. or the like.

Of reaction (n), the reaction converting a β-hydroxyaminoacyl group into an oxazoline can be used the ring closure reaction which those in the art can be selected usually. For example, the reaction can be conducted in an organic solvent under a dehydrating condition. As a dehydrating condition, a single application of an oxalyl chloride, thionyl chloride, Burgess reagent or the like, or a combination of an methanesulfonyl chloride, trifluoroacetic anhydrate or the like and a base such as N,N-diisopropylethylamine, pyridine or the like, can be used. The reaction temperature is usually from ice cooling temperature to solvent reflux temperature, and the reaction time is usually from 10 minutes to 12 hours.

The reaction (o) : the reaction converting a cyano group into a 1,2,4-oxadiazole group can be used the ring closure reaction which those in the art can be selected usually. The reaction can be conducted by the method described in SHINHEN HETEROKANKAGOUBUSTU (New edition of heterocyclic compound), applied chapter, edited by Hiroshi Yamanaka 2004, published by KOUDANSHA Ltd or the like, for example, after converting a cyano group into an imido group, the group can converted into a 1,2,4-oxadiazole group under acidic condition, basic condition, heat condition or the like on ring closure reaction.

The reaction (p) : the reaction converting a carboxylic acid into an urea group can be used the reaction which those in the art can be usually selected. For example, an acylazide derivative obtained as a result of a carboxy group or the reactive functional derivative reacts with diphenylphosphoryl azide or sodium azide can be conducted to react with the amine derivative.

The reaction (q) : the reaction converting an ester group into a hydrazide group can be used the reaction which those in the art can be selected usually. For example, the reaction can be conducted by allowing an ester derivative to react with a hydrazine derivative in an organic solvent. As the used solvent, an alcohol solvent such as methanol, ethanol or the like can be illustrated.

### Process 1-2

A compound represented by the above general formula (I) can be prepared by subjecting a reactive functional derivative represented by the general formula (VII) to condensation with a compound represented by the above general formula (VIII) in the presence of a base such as N, N-diisopropylethylamine or triethylamine or the like in the solvent, optionally by removing a protective group. As a solvent used in the reaction, dichloromethane, tetrahydrofuran, a mixed solvent thereof or the like can be illustrated. The reaction temperature is usually from 0°C to solvent reflux temperature, and the reaction time is usually from 30 minutes to 3 days, varying based on a used starting material, solvent and reaction temperature. It is preferable to use 0.5 to 1.5 amounts of a reactive functional derivative for the compound (VII), and it is preferable to use 1 to 5 amounts of a base for the compound (VII).

Process 1-2 can be usually conducted by the desired method selected those in the art, for example, by the above-mentioned process (a) to (q) selected optionally or by exchanging an order of process (a) to (q).

The compounds represented by the above general formula (I) of the present invention can be prepared in the following methods described in schemes 2.

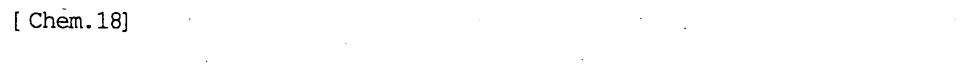

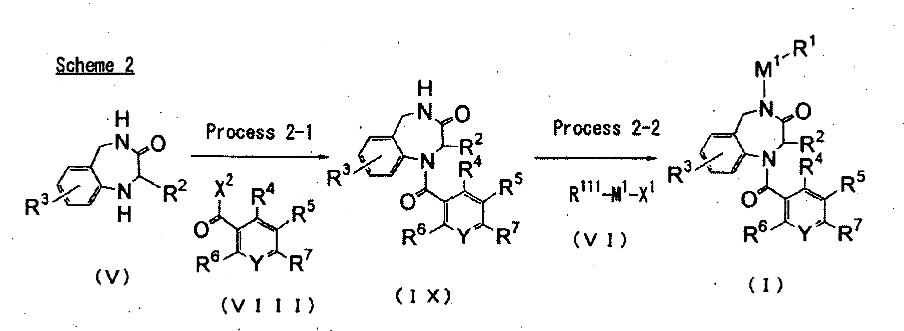

In the formula, R¹, R², R³, R⁴, R⁵, R⁶, R⁷, R¹¹¹, M¹, Y, X¹ and X² have the same meanings as defined above.

### Process 2-1

A compound represented by the above general formula (IX) can be prepared by subjecting a reactive functional derivative represented by the general formula (VIII) to condensation with a compound represented by the above general formula (V) in the presence of a base such as N,N-diisopropylethylamine or triethylamine or the like in the solvent. As a solvent used in the reaction, dichloromethane, tetrahydrofuran, a mixed solvent thereof or the like can be illustrated. The reaction temperature is usually from. 0°C to solvent reflux temperature, and the reaction time is usually from 30 minutes to 3 days, varying based on a used starting material, solvent and reaction temperature. It is preferable to use 0.5 to 1.5 amounts of a reactive functional derivative for the compound (V), and it is preferable to use 1 to 5 amounts of a base for the compound (V).

### Process 2-2

A compound represented by the above general formula (I) can be prepared by subjecting a compound represented by the above general formula (IX) to condensation with a reactive functional derivative (VI) represented by the general formula R¹¹¹-M¹-X¹ in the presence of a base in the solvent. As a solvent used in the reaction, N,N-dimethylformamide, tetrahydrofuran, a mixed solvent thereof or the like can be illustrated, as a base, sodium hydride, potassium hydroxide, potassium tert-butoxide, lithium bis (trimethylsilyl) amide, benzyltrimethylammonium hydroxide or the like can be illustrated. It is preferable to use 1 to 5 amounts of a base for the compound (IX) . The reaction temperature is usually from -20°C to solvent reflux temperature, and the reaction time is usually from 30 minutes to 3 days, varying based on a used starting material, solvent and reaction temperature.

Process 2-2 can be usually conducted by the desired method selected those in the art, for example, by the above-mentioned process (a) to (q) selected optionally or by exchanging an order of process (a) to (q).

The material compounds represented by the above general formula (V) used in the above scheme can be prepared in accordance with a knownmethod described in literatures or a similar method. For example, they can be prepared in the following methods described in schemes 3.

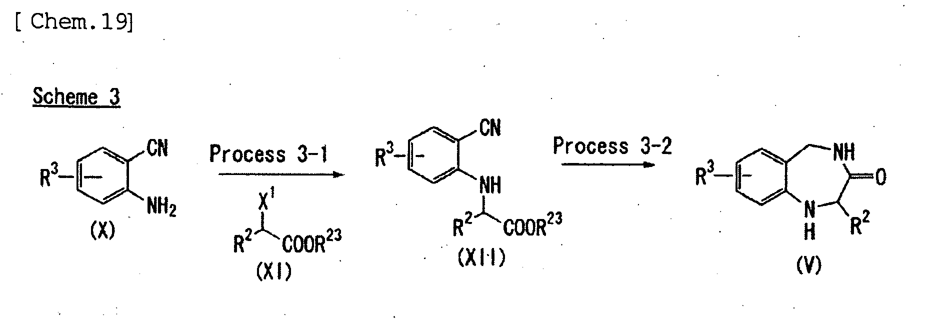

In the formula, R²³ is a C₁₋₆ alkyl group; R³, R³, and X¹ have same meanings as defined above.

### Process 3-1

A compound represented by the above general formula (XII) can be prepared by subjecting a compound represented by the above general formula (X) to alkylation with a reactive functional derivative represented by the general formula (XI) in the presence of a base such as N,N-diisopropylethylamine or potassium carbonate or the like in the solvent, and optionally by removing a protective group. As a solvent used in the reaction, N,N-dimethylformamide, dimethylsulfoxide, a mixed solvent thereof or the like can be illustrated. It is preferable to use 1 to 5 amounts of both a reactive functional derivative represented by the general formula (XI) and a base for the compound (X). The reaction temperature is usually from 0°C to solvent reflux temperature, and the reaction time is usually from 30 minutes to 3 days, varying based on a used starting material, solvent and reaction temperature.

### Process 3-2

Compound (V) can be prepared by subjecting a compound represented by the above general formula (XII) to reduction in an organic solvent, in the presence of a metal catalyst, under a hydrogen atmosphere, and optionally by deprotecting. As a solvent used in the reaction, methanol, ethanol, tetrahydrofuran, a mixed solvent thereof or the like canbe illustrated. As a metal catalyst, palladium on carbon, Raney nickel or the like can be illustrated. It is preferable to use a catalystic amount to 1 amount of a metal catalyst for the compound (XII). The reaction temperature is usually from -20°C to solvent reflux temperature, and the reaction time is usually from 30 minutes to 3 days, varying based on a used starting material, solvent and reaction temperature.

In addition, the material compound represented by the above general formula (VII) can be also prepared in the following methods described in schemes 4.

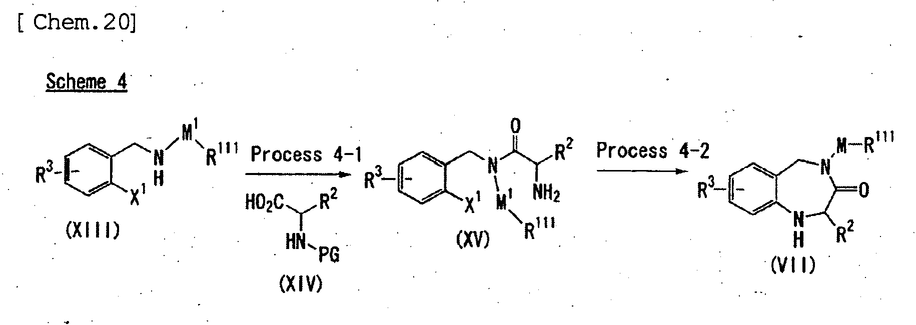

In the formula, PG is a protective group for amino group, such as a benzyl group, a tert-butoxycarbonyl group, a benzyloxycarbonyl group, an acetyl group or the like; R¹¹¹, R², R³, M¹ and X¹ have same meanings as defined above.

### Process 4-1

A compound represented by the above general formula (XV) can be prepared by subjecting a compound represented by the above general formula (XIII) to condensation with a compound represented by the above general formula (XIV) in the presence of a condensation agent such as 1-ethyl-3-(N,N-dimethylaminopropyl)carbodiimide hydrochloride or the like in the solvent, optionally to adding a base such as 4-dimethylaminopyridine, triethylamine or the like, optionally by removing a protective group. As a solvent used in the reaction, N,N-dimethylformamide, dichloromethane, a mixed solvent thereof or the like can be illustrated. It is preferable to use 1 to 5 amounts of a compound represented by the general formula (XIV), a condensation agent and a base for the compound (XIII). The reaction temperature is usually from 0°C to solvent reflux temperature, and the reaction time is usually from 30 minutes to 3 days, varying based on a used starting material, solvent and reaction temperature.

### Process 4-2

A compound represented by the above general formula (VII) can be prepared by subjecting a compound represented by the above general formula (XV) to cyclization in the presence of a base such as cesium carbonate, sodium tert-butoxide or the like, and a ligand such as tri-tert-butylphosphine, 2,2'-bis(diphenylphosphino)-1,1'-binaphthyl or the like, by using a catalyst such as palladium(II) acetate, bis(benzylideneacetone) palladium(0) or the like, optionally by removing a protective group. As a solvent used in the reaction, toluene, xylene, a mixed solvent thereof or the like can be illustrated. It is preferable to use 1 to 5 amounts of a base for the compound (XV) . It is preferable to use a catalystic amount to 1 amount of a catalyst and a ligand for the compound (XV). The reaction temperature is usually from 0°C to solvent reflux temperature, and the reaction time is usually from 30 minutes hour to 3 days, varying based on a used starting material, solvent and reaction temperature.

In addition, the material compound represented by the above general formula (V) can be also prepared in the following methods described in schemes 5.

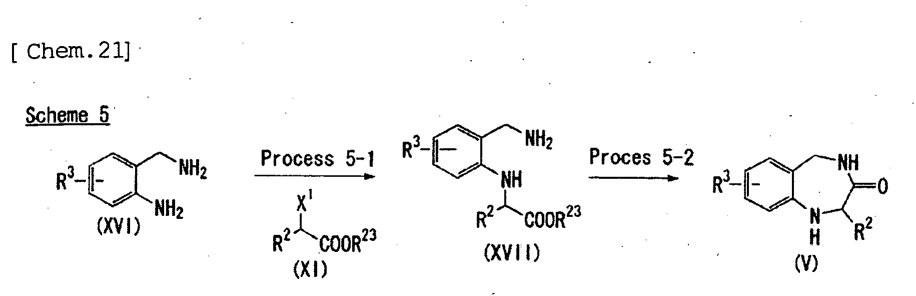

In the formula, PG, R², R³, R²³, and X¹ have same meanings as defined above.

### Process 5-1

A compound represented by the above general formula (XVII) can be prepared by subjecting a compound represented by the above general formula (XVI) to alkylation with a reactive functional derivative represented by the general formula (XI) in the presence of a base such as N,N-diisopropylethylamine or potassium carbonate or the like in a solvent, and optionally by removing a protective group. As a solvent used in the reaction, N,N-dimethylformamide, dimethylsulfoxide, a mixed solvent thereof or the like can be illustrated. It is preferable to use 1 to 5' amounts of both a reactive functional derivative represented by the general formula (XI) and a base for the compound (XVI). The reaction temperature is usually from 0°C to solvent reflux temperature, and the reaction time is usually from 30 minutes to 3 days, varying based on a used starting material, solvent and reaction temperature.

### Process 5-2

A compound represented by the above general formula (V) can be prepared by subjecting a compound represented by the above general formula (XVII) to cyclization optionally in the presence of a base such as sodium hydroxide, potassium hydroxide or the like, or an acid such as concentrated hydrochloric acid, concentrated sulfuric acid or the like in a solvent, optionally by removing a protective group. As a solvent used in the reaction, water, methanol, ethanol, a mixed solvent thereof or the like can be illustrated. It is preferable to use 1 to 5 amounts of a base for the compound (XVII). The reaction temperature is usually from 0°C to solvent reflux temperature, and the reaction time is usually from 30 minutes hour to 3 days, varying based on a used starting material, solvent and reaction temperature.

The reactive functional derivative represented by the above general formula (VIII) used in the above scheme can be prepared in accordance with a known method described in literatures or a similar method. For example, they can be prepared in the following methods described in scheme 6.

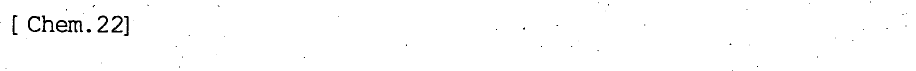

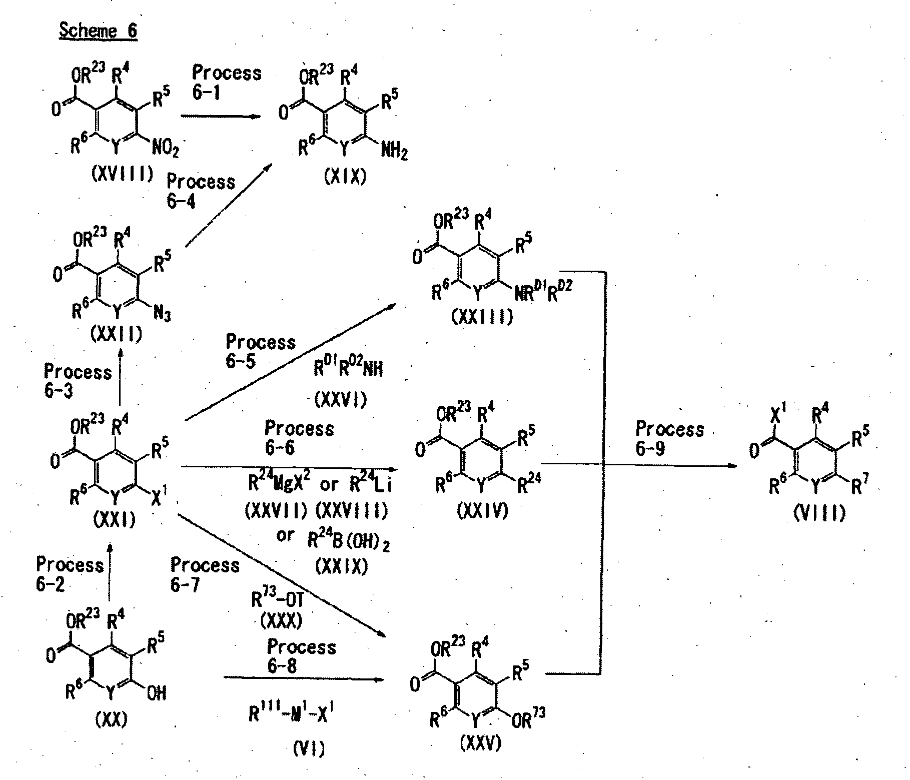

In the formula, R²³ have same meanings as defined above; R⁷³ is a 2,2,2-trifluoroethyl group, ω-benzyloxyalkyl group, ω-acyloxyalkyl group or the like; T is a sodium atom, a potassium atom, a lithium atom; R²⁴ is a C₆₋₁₀ aryl group; R¹¹¹, R⁴, R⁵, R⁶, R⁷, R^{D1}, R^{D2}, M¹, X¹ and X² have same meanings as defined above.

### Process 6-1

The present process can be conducted by method (a) or (b) described below.

(a) Compound (XIX) can be prepared by subjecting a compound represented by the above general formula (XVIII) to reduction with a metal catalyst in an organic solvent, under hydrogen atmosphere, in the presence or absence of an acid, and optionally by deprotecting. As a solvent usedin the reaction, methanol, ethanol, tetrahydrofuran, a mixed solvent thereof or the like can be illustrated. As an acid, hydrochloric acid, acetic acid or the like can be illustrated. As a metal catalyst, palladium on carbon, Raney nickel or the like can be illustrated. It is preferable to use a catalystic amount to 1 amount of a metal catalyst for the compound (XVIII). The reaction temperature is usually-from -20°C to solvent reflux temperature, and the reaction time is usually from 30 minutes hour to 3 days, varying based on a used starting material, solvent and reaction temperature.

(b) Compound (XIX) can be prepared by subjecting a compound represented by the above general formula (XVIII) to reduction with a metal catalyst in an organic solvent, under the acidic condition as using a hydrochloric acid, a sulfuric acid; ammonium chloride or the like, by using a metal such as an iron, a zinc or the like, or by using a metal salt such as tin(II) chloride or the like, and optionally by deprotecting. As a solvent used in the reaction, methanol, ethanol, acetic acid, a mixed solvent thereof or the like can be illustrated. It is preferable to use 1 to 5 amounts of a metal or metal salt for the compound (XVIII). The reaction temperature is usually from -20°C to solvent reflux temperature, and the reaction time is usually from 30 minutes to 3 days, varying based on a used starting material, solvent and reaction temperature.

### Process 6-2

Compound (XXI) can be prepared by subjecting a compound represented by the above general formula (XX) to halogenation using a halogenated phosphorus compound such as phosphoryl chloride, phosphoryl bromide or the like, without or in an organic solvent, and optionally by deprotecting. It is preferable to use 1 to 5 amounts of a halogenated phosphorus compound for the compound (XX). The reaction temperature is usually from -20°C to solvent reflux temperature, and the reaction time is usually from 30 minutes to 3 days, varying based on a used starting material, solvent and reaction temperature.

### Process 6-3

A compound represented by the above general formula (XXII) can be prepared by subjecting a compound represented by the above general formula (XXI) to azidation with an azidating reagent such as sodium azide, lithium azide or the like in an organic solvent, and optionally by removing a protective group. As a solvent used in the reaction, N,N-dimethylformamide, dimethylsulfoxide, a mixed solvent thereof or the like can be illustrated. It is preferable to use 1 to 5 amounts of an azidating reagent for the compound (XXI) . The reaction temperature is usually from 0°C to solvent reflux temperature, and the reaction time is usually from 30 minutes hour to 3 days, varying based on a used starting material, solvent and reaction temperature.

### Process 6-4

Compound (XIX) can be prepared by subjecting a compound represented by the above general formula (XXII) to reduction in the presence of a metal catalyst in a solvent, under a hydrogen atmosphere, and optionally by deprotecting. As a solvent used in the reaction, methanol, ethanol, tetrahydrofuran, a mixed solvent thereof or the like can be illustrated. As a metal catalyst, palladium on carbon, platinum oxide or the like can be illustrated. It is preferable to use a metal catalyst of a catalystic amount to 1 amount for the compound (XXII). The reaction temperature is usually from -20°C to solvent reflux temperature, and the reaction time is usually from 30 minutes hour to 3 days, varying based on a used starting material, solvent and reaction temperature.

### Process 6-5

The present process can be conducted by method (a) or (b) described below.

(a) A compound represented by the above general formula (XXIII) can be prepared by allowing a compound represented by the above general formula (XXI) to react with a compound represented by the above general formula (XXVI H-NR^{D1}R^{D2}) in a solvent, in the presence of a base such as cesium carbonate, sodium tert-butoxide or the like, and a ligand such as tri-tert-butylphosphine, 2,2'-bis(diphenylphosphino)-1,1'-binaphthyl or the like, by using a catalyst such as palladium(II) acetate, bis(benzylideneacetone) palladium(0) or the like, optionally by removing a protective group. As a solvent used in the reaction, toluene, xylene, a mixed solvent thereof or the like can be illustrated. It is preferable to use 1 to 5 amounts of both a base and a compound represented by the above general formula (XXVI) for the compound (XXI). It is preferable to use a catalystic to 1 amount of a catalyst and a ligand for the compound (XXI). The reaction temperature is usually from 0°C to solvent reflux temperature, and the reaction time is usually from 30 minutes to 3 days, varying based on a used starting material, solvent and reaction temperature.

(b) A compound represented by the above general formula (XXIII) can be prepared by subjecting a compound represented by the above general formula (XXI) to aminaton using a compound represented by the above general formula (XXVI H-NR^{D1}R^{D2}) in a solvent, optionally in the presence of a base such as triethylamine, potassium carbonate or the like, optionally by removing a protective group. As a solvent used in the reaction, N,N-dimethylformamide, N-methylpyrrolidone, tetrahydrofuran, a mixed solvent thereof or the like canbe illustrated. It is preferable to use 1 to 5 amounts of both a compound represented by the above general formula (XXVI) and a base for the compound (XXI). The reaction temperature is usually from 0°C to solvent reflux temperature, and the reaction time is usually from 30 minutes hour to 3 days, varying based on a used starting material, solvent and reaction temperature. In addition, the present reaction can be accelerated by using a catalyst such as copper, copper salt or the like.

### Process 6-6

The present process can be conducted by any method of (a), (b) or (c) described below.

(a) A compound represented by the above general formula (XXIV) can be prepared by allowing a compound represented by the above general formula (XXI) to react with a Grignard reagent (R²⁴MgX²) represented by the above general formula (XXVII) in a solvent, and optionally by removing a protective group. As a solvent used in the reaction, tetrahydrofuran, diethylether, a mixed solvent thereof or the like can be illustrated. It is preferable to use 1 to 5 amounts of a Grignard reagent represented by the above general formula (XXVII) for the compound (XXI). The reaction temperature is usually from -78 °C to solvent reflux temperature, and the reaction time is usually from 30 minutes to 3 days, varying based on a used starting material, solvent and reaction temperature.

(b) A compound represented by the above general formula (XXIV) can be prepared by allowing a compound represented by the above general formula (XXI) to react with an organic lithium reagent (R²⁴Li) represented by the above general formula (XXVIII) in a solvent, and optionally by removing a protective group. As a solvent used in the reaction, tetrahydrofuran, diethylether, a mixed solvent thereof or the like can be illustrated. It is preferable to use 1 to 5 amounts of an alkyllithium reagent represented by the above general .formula (XXVIII)for the compound (XXI). The reaction temperature is usually from -78 °C to solvent reflux temperature, and the reaction time is usually from 30 minutes to 3 days, varying based on a used starting material, solvent and reaction temperature.

(c) A compound represented by the above general formula (XXIV) can be prepared by allowing a compound represented by the above general formula (XXI) to react with a boronic acid derivative represented by the above general formula (XXIX) in a solvent, in the presence of a base such as sodium carbonate, cesium fluoride, sodium tert-butoxide or the like, and a palladium catalyst such as tetrakis(triphenylphosphine)palladium(0), dichlorobis(triphenylphosphine)palladium(II) or the like, and optionally by removing a protective group. As a solvent used in the reaction, water, toluene, 1,4-dioxane, N,N-dimethylformamide, a mixed solvent thereof or the like can be illustrated. It is preferable to use 1 to 5 amounts of a base for the compound (XXI). In addition, it is preferable to use a catalystic amount to 1 amount of a palladium catalyst for the compound (XXI). The reaction temperature is usually from 0°C to solvent reflux temperature, and the reaction time is usually from 30 minutes to 3 days, varying based on a used starting material, solvent and reaction temperature.

### Process 6-7

A compound represented by the above general formula (XXV) can be prepared by allowing a compound represented by the above general formula (XXI) to react with a metal alkoxide or a metal aryloxide represented by the above general formula (XXX R⁷³-O-T) in a solvent, and optionally by removing a protective group. As a solvent used in the reaction, tetrahydrofuran, methanol, ethanol, N,N-dimethylformamide, a mixed solvent thereof or the like can be illustrated. It is preferable to use 1 to 5 amounts of a metal alkoxide or a metal aryloxide represented by the above general formula (XXX R⁷³-O-T) for the compound (XXI). The reaction temperature is usually from -78 °C to solvent reflux temperature, and the reaction time is usually from 30 minutes to 3 days, varying based on a used starting material, solvent and reaction temperature.

### Process 6-8

A compound represented by the above general formula (XXV) can be prepared by allowing a compound represented by the above general formula (XX) to react with reactive functional derivative (VI) represented by the above general formula (R¹¹¹-M¹-X¹) in a solvent, in the presence of abase such as cesium carbonate, potassium carbonate, optionally by removing a protective group. As a solvent used in the reaction, N,N-dimethylformamide, acetone, a mixed solvent thereof or the like can be illustrated. It is preferable to use 1 to 5 amounts of a reactive functional derivative represented by the above general formula (VI) and a base for the compound (XX): The reaction temperature is usually from 0°C to solvent reflux temperature, and the reaction time is usually from 30 minutes to 3 days, varying based on a used starting material, solvent and reaction temperature. In addition, the present reaction can be accelerated by adding a halogenated alkali metal such as sodium iodide, potassium iodide or the like.

### Process 6-9

After hydrolyzing or hydrogenolyzing a compound represented by the above general formula (XXIII), (XXIV) or (XXV), a reactive functional derivative represented by the above general formula (VIII) can be prepared by subjecting them to halogenation using a halogenating reagent such as thionyl chloride, oxalyl chloride or the like in an organic solvent, optionally by removing a protective group. As a solvent used in the reaction, chloroform, dichloromethane, a mixed solvent thereof or the like can be illustrated. It is preferable to use 1 to 20 amounts of a halogenating reagent for the compound (XXIII), (XXIV) or (XXV). The reaction temperature is usually from 0°C to solvent reflux temperature, and the reaction time is usually from 30 minutes to 3 days, varying based on a used starting material, solvent and reaction temperature. In addition, the present reaction can be accelerated by adding N-methylpyrrolidone, N,N-dimethylformamide or the like.

In addition, the compound represented by the above general formula (I) of the present invention can be prepared in the following methods described in schemes 7.

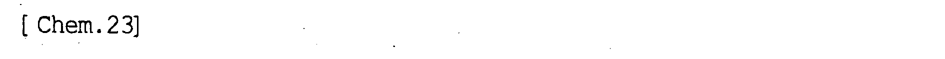

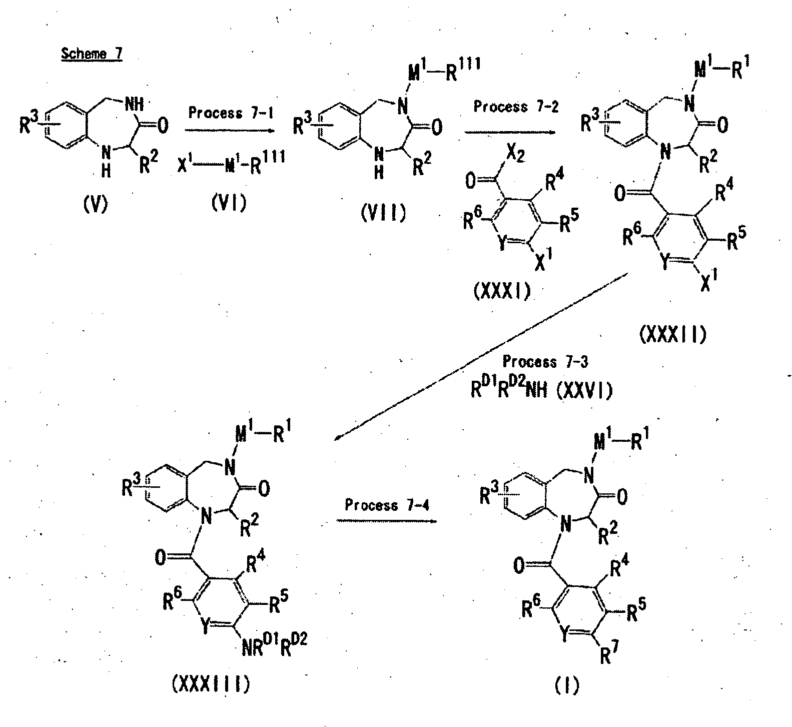

In the formula, R¹, R², R³, R⁴, R⁵, R⁶, R⁷, R¹¹¹, R^{D1}, R^{D2}, X¹, X², M¹ and Y have same meanings as defined above.

### Process 7-1

A compound represented by the above general formula (VII) can be prepared by using the similar method of process 1-1.

### Process 7-2

A compound represented by the above general formula (XXXII) can be prepared by allowing a compound represented by the above general formula (VII) to react with a compound represented by the above general formula (XXXI), by using the similar method of process 1-2.

### Process 7-3

A compound represented by the above general formula (XXXIII) can be prepared by allowing a compound represented by the above general formula (XXXII)- to react with a compound represented by the above general formula (XXVI) which R^{D1} and R^{D2} are appropriately protected, by using the same similar method of process 6-5.

### Process 7-4

A compound represented by the above general formula (I) can be prepared by subjecting a compound represented by the above general formula (XXXIII) to optionally removing a protective group.

A reactive functional derivative (VI) represented by the above general formula (R¹¹¹-M¹-X¹) which uses in the above schemes can be prepared in known or similar methods described in literatures or the like, and may be commercially available.

The compound represented by the above general formula (I) of the present invention which can be prepared in the above schemes can be optionally converted into their pharmaceutically acceptable salts in the usual way. Examples of such salts include acid addition salts with mineral acids such as hydrochloric acid, hydrobromic acid, hydroiodic acid, nitric acid, sulfuric acid, phosphoric acid and the like, acid addition salts with organic acids such as formic acid, acetic acid, adipic acid, citric acid, fumaric acid, maleic acid, oleicacid, lactic acid, stearic acid, succininc acid, tartaric acid, propionic acid, butyric acid, oxalic acid, malonic acid, malic acid, carbonic acid, glutamic acid, aspartic acid, methanesulfonic acid, benzenesulfonic acid, p-toluenesulfonic acid and the like, salts with organic bases such as 2-aminoethanol, piperidine, morpholine, pyrrolidine, *N*-methyl-D-glucamin, *N,N*'-dibenzylethylenediamine, tris(hydroxymethyl)aminomethane, arginine, lysine and the like, salts with inorganic bases such as sodium salt, potassium salt, calcium salts, magnesium salts and the like can be illustrated.

The scheme shown in the above is any illustration of the method for manufacturing the compound of the present invention or the intermediates thereof in production. They can be variously modified to the scheme easily understood to the those in the art.

The compound represented by the above general formula (I) of the present invention and intermediates thereof for use in manufacturing the compound can be isolated and purified, optionally by using a operation of a solvent extraction, a recrystallization, chromatography and a preparative high performance liquid chromatography known in the those in the art in the field as the method of isolation and purification.

The pharmaceutical compositions comprising as an active ingredient a compound represented by the above general formula (I) of the present invention, or pharmaceutically acceptable salts thereof are used various dosage forms, according to the usage. As the dosage forms, for example, powders, fine granules, granules, dry syrups, tablets, capsules, injections, solutions, ointments, suppositories, poultices, sublingual formulation or the like can be illustrated, these are administered in oral or parenteral.

These pharmaceutical compositions can be prepared by suitably admixing or by diluting and dissolving with appropriate pharmaceutical additives such as excipients, disintegrators, binders, lubricants, diluents, buffers, isotonicities, antiseptics, moistening agents, emulsifiers, dispersing agents, stabilizing agents, dissolving aids and the like by method well-known in the galenical pharmacy depending on the formulation.

The V2 receptor agonist of the present invention means an agent having an agonism of V2 receptor and acting as agonist or partial agonist of V2 receptor. The compound represented by the above general formula (I) of the present invention can act as agonist or- partial agonist of V2 receptor.

The compound represented by the above general formula (I) of the present invention, for example, by a binding experiment for human V2 receptor or a study to confirm the agonism of human V2 receptor, is shown a strong agonism of human V2 receptor. Thence the compound represented by the above general formula (I) of the present invention has an antidiuretic activity wherein urine volume decreases significantly. Therefore the compound represented by the above general formula (I) of the present invention can be applied as an agent for the treatment or prevention of a disease associated with an unusual increseing of urine volume or increseing of number of micturition. The disease associated with an increseing of urine volume or increasing of number of micturition means, for example, a various disease pointing to dysuria and a large volume of urine, the compound represented by the above general formula (I) is preferably an agent for the treatment or prevention of a disease associated with micturition, urinary incontinence, enuresis, diabetes insipidus (for example, central diabetes insipidus, nephrogenic diabetes insipidus or the like can be illustrated, central diabetes insipidus is more preferably), nocturia, overactive bladder (for example, neurogenic overactive bladder, detrusor overactivity, sudden bladder overactivity or the like), nocturnal enuresis (for example, nocturnal enuresis in children or the like) or the like. In addition, the compound represented by the above general formula (I) of the present invention has a release activity of coagulation factor VIII and von-Wiliebrand factor, can be used in the treatment or prevention of a bleeding disease. For example, the treatment or prevention of hemophilia, von-Wiliebrand disease, congenital/acquired dysfunction of blood platelets, spontaneous bleeding or the like is-preferably. The compound of the present invention has a very weak inhibition activity against cytochrome P-450 (CYP) enzyme, and can be used without anxiety, in case of using for the elderly person or combination with other agents.

The compound represented by the above general formula (I) of the present invention or the pharmaceutically acceptable salt thereof can be used in combination with at least one agent selected from a group consisting of agents for the treatment of diabetes insipidus, nocturia, nocturnal enuresis, overactive bladder, hemophilia, other than an agent for V2 agonist. As these agents for the treatment of diabetes insipidus, nocturia, nocturnal enuresis, overactive bladder, hemophilia, other than a human V2 agonist, an α₁-adrenoceptor blocker, a cholinergic blocking agent, a cholinergic agent,an antispasmodic agent,an anti-androgen agent, an antidepressant, a calcium antagonist, a potassium-channel opener, a sensory nerve blocking agent, a β-adrenergic agonist, an acetylcholinesterase inhibitor, anti-inflammatory agent and the like can be illustrated.

In case of uses of the compound represented by the above general formula (I) of the present invention in combination with the above one or more other drugs, either dosage form of simultaneous administration as a single preparation or separated preparations in way of the same or different administration route, and administration at different dosage intervals as separated preparations in way of the same or different administration route can be adopted, a pharmaceutical composition comprising in combination with the compound of the present invention and the above agent can adopt dosage form of a single preparation or combination with separated preparations as follows.

The compounds of the present invention can obtain more advantageous effects than additive effects in the prevention or treatment of the above diseases using suitably in combination with the above one or more drugs. Also, the administration dose can be decreased in comparison with administration of either drug alone, or adverse effects of coadministrated drugs can be avoided or declined.

The concrete compounds as the drugs used for combination and preferable diseases to be treated are exemplified as follows. However, the present invention is not limited thereto, and the concrete compounds include their free compounds, and their or other pharmaceutically acceptable salts.

As an α₁-adrenoceptor blocker, for example, terazosin, bunazosin, urapidil, tamsulosin, bunitrolol, doxazosin, prazosin, carvedilol, bevantolol, WY-21901, naftopidil, alfuzosin, levobunolol, silodosin, IDR-16804, fiduxosin, SPM-969, (S)-doxazosin, KRG-3332 or the like can be illustrated.

As an anticholinergic agent, for example, propiverine, oxybutynin, tolterodine, solifenacin or the like can be illustrated.

As a cholinergic drug, for example, besacolin or the like can be illustrated.

As an antispasmodic agent, for example, flavoxate or the like can be illustrated.

As an anti-androgen drug, for example, chlormadinone acetate, allylestrenol or the like can be illustrated.

As an antidepressant, for example, imipramine or the like can be illustrated.

As a calcium antagonist, for example, fasudil, nifedipine, nimodipine, nilvadipine, bepridil, manidipine, barnidipine, nitrendipine,benidipine;isradipine,nicardipine,lercanidipine, amlodipine, nisoldipine, efonidipine, gallopamil, diltiazem, cilnidipine, azelnidipine, felodipine, lacidipine, aranidipine, pranidipine, ranolazine, IQB-875D, iganidipine or the can be illustrated.

As a potassium-channel opner, for example, NS-8, nicorandil, tilisolol, pinacidil, levcromakalim, GKE-841, PNU-83757, NN-414, KCO-912, AZD-0947 ABT-598 or the like can be illustrated.

As a sensory nerve blocking agent, for example, KW-7158 or the like can be illustrated.

As a (β-adrenergic agonist, for example, a selective β2-adrenergic agonist, a non-selective β2-adrenergic agonist, a selective β3-adrenergic agonist, a non-selective β3-adrenergic agonist, a (β2+β3)-adrenergic agonist or the like can be illustrated. Among them, a selective β3-adrenergic agonist is preferable. As a (β2+β3)-adrenergic agonist means a β-adrenergic agonist having β2-adrenergic effect and β3-adrenergic effect. As a concrete β-adrenergic agonist, mabuterol, ritodrine, fenoterol, denopamine, docarpamine, clenbuterol, formoterol, procaterol, pirbuterol, KWD-2183, xamoterol, terbutaline, tulobuterol, salmeterol, dopexamine, levalbuterol, ephedrine, meluadrine, SR-58611, arformoterol, CHF-4226, KUR-1246, KUC-7483, YM-178, QAB-149, TD-3327, LY-362884, GW-427353, N-5984, KUL-7211 or the like canbe illustrated.

As an acetylcholinesterase inhibitor, for example, donepezil, itopride, rivastigmine, metrifonate, galantamine, phenoserine, KA-672, CHF-2819, T-82, EN-101, ZT-1, TAK-802, ladostigil or the like can be illustrated.

As an anti-inflammatory agent, suplatast tosilate or the like can be illustrated.

The dosage of a compound represented by the above general formula (I) or a pharmaceutically acceptable salt thereof is appropriately decided depending on the age, sex, body weight and degree of symptoms and treatment of each patient, which is approximately within the range of 0.01 to 1,000 mg per day per adult human in the case of oral administration and approximately within the range of from 0.01 to 1,000 mg per day per adult human in the case of parenteral administration, and the daily dose can be divided into one to several doses per day and administered suitably.

As a drug which the compound represented by the above general formula (I) or a pharmaceutically acceptable salt thereof in combination with at least one agent selected from a group consisting of a therapeutic agent for diabetes insipidus, nocturia and nocturnal enuresis other than an agent for human V2 agonist, the dosage of an agent can be appropriately selected depending on the age, sex, body weight of each patient, the symptom, a dosing period, a dosage form, an administration method, a combination of agents.

### Effect of the Invention

The compound represented by the above general formula (I) of the present invention, for example, by a binding experiment for human V2 receptor or a study to confirm the agonism of human V2 receptor, showed a strong stimulatory activity against human V2 receptor. Thence the compound represented by the above general formula (I) of the present invention can decrease urine volume significantly. Therefore the compound represented by the above general formula (I) of the present invention has an antidiuretic activity on the profile based on the present activity and a release activity of coagulation factor VIII and von-Wiliebrand factor, is useful for various dysuria, a large volume of urine or bleeding tendency, is preferably as an agent for the treatment or prevention of a disease associated with micturition, urinary incontinence, enuresis, central diabetes insipidus, nephrogenic diabetes insipidus, nocturia, nocturnal enuresis, overactive bladder, hemophilia, von-Wiliebrand disease, congenital/acquired dysfunction of blood platelets, spontaneous bleeding or the like. In addition, the compound of the present invention has a very weak inhibition activity against cytochrome P-450 (CYP) enzyme, and can be used without anxiety, in case of using for the elderly person or combination with other agents.

### Best Mode to practice the Invention

The present invention is further illustrated in more detail by way of the following Test Examples. However, the present invention is not limited thereto. In addition, among signs using in Tables, "1H-NMR" represents ¹H-NMR, "Solvent" represents a measuring solvent of ¹H-NMR, "CDCl3" means CDCl₃, "DMSO-d6" means DMSO-d₆, and "CD3OD" means CD₃OD. In addition, "MS" means the mass spectrometry.

### Examples

### Reference example 1

### Ethyl (3-oxo-1,2,3,5-tetrahydrobenzo[e]-1,4-diazepin-4-yl)acetate

A solution of 1, 2, 4, 5-tetrahydrobenzo[e] -1,4-diazepin-3-one (0.570 g) in N,N-dimethylformamide (7.0 mL) was added dropwise to a stirred suspension of sodium hydride (ca 60% : 0.169 g) in N,N-dimethylformamide (5.0 mL) under ice-cooling. After the mixture was allowed to stirr at roomtemperature for an hour, ethyl bromoacetate (0.429 mL) was added to the stirred mixture under ice-cooling. The mixture was stirred at room temperature overnight. The reaction mixture was poured into water and extracted with ethyl acetate. The organic layer was washed with water. The organic layer was dried over anhydrous magnesium sulfate, after filtration, the filtrate was concentrated under reduced pressure. The obtained crude product was purified by column chromatography on silica gel (eluent:ethyl acetate-hexane) to give ethyl (3-oxo-1,2,3,5-tetrahydrobenzo[e]-1,4-diazepin-4-yl)acetate (0.655 g).

¹_{H}-NMR (CDCl₃) δ ppm:
1.23 (3H, t, J=7.5Hz), 4.00-4.30 (7H, m), 4.61 (2H; s), 6.50-6.60 (1H, m), 6.60-6.75 (1H, m), 6.85-6.95 (1H, m), 7.05-7.15 (1H, m)

### Reference examples 2-1 to 2-26

The following compounds of Reference examples 2-1 to 2-26 were prepared with the use of 1,2,4,5-tetrahydrobenzo[e]-1,4-diazepin-3-one and the corresponding alkylating reagents instead of ethyl bromoacetate in a similar manner to that described in Reference example 1. The structure formla and physical data of these compounds were shown in Table 1 to 7.

**[Table 1]**

| Reference example | Structure formula | Compound name | 1H-NMR (solvent) or MS(m/z) |
|---|---|---|---|
| 2-1 | 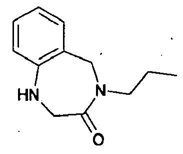 | 4-propyl-1,2,4,5-tetrahydrobenzo[e]-1,4-diazepin-3-one | 1H-NMR (CDCl3) δ ppm: 0.85 (3H, t, J=7.3Hz), 1.50-1.60 (2H, m), 3.40-3.50 (2H, m), 4.00-4.10 (1H, m), 4.14 (2H, d, J=5.7Hz), 4.53 (2H, s), 6.50-6.55 (1H, m), 6.60-6.70 (1 H, m), 6.85-6.95 (1H, m), 7.00-7.10 (1H, m) |
| 2-2 | 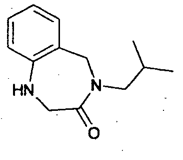 | 4-isobutyl-1,2,4,5-tetrahydrobenzo[e]-1,4-diazepin-3-one | 1H-NMR (CDCl3) δ ppm: 0.85 (6H, d, J=6.7Hz), 1.85-2.00 (1H, m), 3.31 (2H, d, J=7.5Hz), 4.10-4.20 (3H, m), 4.53 (2H, s), 6.50-6.55 (1H, m), 6.60-6.65 (1H, m), 6.85-6.95 (1H, m), 7.00-7.10 (1H, m) |
| 2-3 | 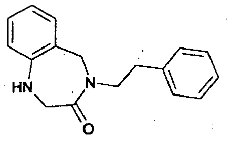 | 4-phenethyl-1,2,4,5-tetrahydrobenzo[e]-1,4-diazepin-3-one | 1H-NMR (CDCl3) δ ppm: 2.84 (2H, t, J=7.3Hz), 3.70-3.75 (2H, m), 4.00-4.15 (3H, m), 4.42 (2H, s), 6.45-6.55 (1H, m), 6.55-6.65 (1H, m), 6.75-6.85 (1H, m), 7.00-7.10 (1H, m), 7.10-7.35 (5H, m) |
| 2-4 | 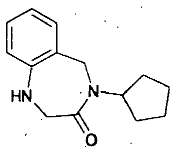 | 4-cyclopentyl-1,2,4,5-tetrahydrobenzo[e]-1,4-diazepin-3-one | 1H-NMR (CDCl3) δ ppm: 1.35-1.95 (8H, m), 4.00-4.20 (3H, m), 4.48 (2H, s), 4.98 (1 H, quint, J=8.4Hz), 6.45-6.55 (1H, m), 6.60-6.70 (1H, m), 6.85-6.95 (1H, m), 7.00-7.10 (1H, m) |

**[Table 2]**

| Reference example | Structure formula | Compound name | 1H-NMR(solvent) or MS(m/z) |
|---|---|---|---|
| 2-5 | 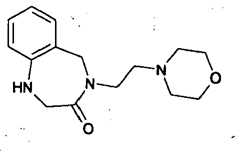 | 4-(2-morpholin-4-yfethyl)-1,2,4, 5-tetrahydrobenzo[e]-1,4-diazepin-3-one | 1H-NMR (CDCI3) δ ppm: 2.30-2.45 (4H, m), 2.47 (2H, t, J=6.6Hz), 3.55-3.65 (6H, m), 4.10-4.30 (3H, m), 4.58 (2H, s), 6.50-6.55 (1H, m), 6.55-6.65 (1H, m), 6.85-6.95 (1H, m), 7.00-7.10 (1H, m) |
| 2-6 | 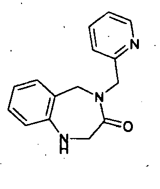 | 4-(pyridin-2-ylmethyl)-1,2,4, 5-tetrahydrobenzo[e]-1,4-diazepin-3-one | 1H-NMR (CDCI3) δ ppm: 4.05-4.15 (1H, m), 4.22 (2H, d, J=5.9Hz), 4.62 (2H, s), 4.81 (2H, s), 6.50-6.60 (2H, m), 6.70-6.80 (1H, m), 7.00-7.10 (1H, m), 7.10-7.20 (1H, m), 7.20-7.30 (1H, m), 7.50-7.60 (1H, m), 8.50-8.60 (1H, m) |
| 2-7 | 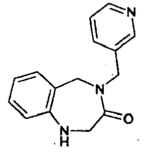 | 4-(pyridin-3-ylmethyl)-1,2,4, 5-tetrahydrobenzo[e]-1,4-diazepin-3-one | 1 H-NMR (CDC13) δ ppm: 4.05-4.15 (1H, m), 4.22 (2H, d, J=5.6Hz), 4.48 (2H, s), 4.70 (2H, s), 6.50-6.75 (3H, m), 7.00-7.10 (1H, m), 7.15-7.25 (1H, m), 7.50-7.60 (1H, m), 8.45-8.55 (2H, m) |
| 2-8 | 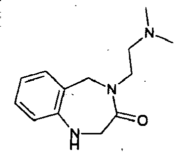 | 4-(2-N,N-dimethylaminoethyl)-1,2,4,5-tetrahydrobenzo[e]-1,4-diazepin-3-one | 1H-NMR (CDCl3) δ ppm: 2.23 (6H, s), 2.43 (2H, t, J=7.0Hz). 3.59 (2H, t, J=7.0Hz), 4.10-4.20 (3H, m), 4.57 (2H, s), 6.50-6.55 (1H, m), 6.60-6.70 (1 H, m), 6.85-6.95 (1H, m), 7.00-7.10 (1H, m) |

**[Table 3]**

| Reference example | Structure formula | Compound name | 1 H-NMR(solvent) or MS(m/z) |
|---|---|---|---|
| 2-9 | 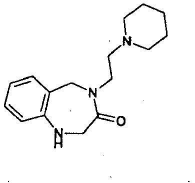 | 4-(2-piperidin-1-ylethyl)-1,2,4, 5-tetrahydrobenzo[e]-1,4-diazepin-3-one | 1H-NMR (CDCl3) δ ppm: 1.35-1.45 (2H, m), 1.50-1.60 (4H, m), 2.25-2.50 (6H, m), 3.61 (2H, t, J=7.0Hz), 4.00-4.10 (1H, m), 4.13 (2H, d, J=6.0Hz), 4.59 (2H, s), 6.50-6.55 (1H, m), 6.60-6.70 (1H, m), 6.85-6.95 (1H, m), 7.00-7.10 (1H, m) |
| 2-10 | 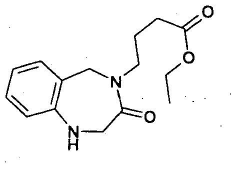 | ethyl 4-(3-oxo-1,2,3,5-tetrahydrobenzo[e]-1,4-diazepin-4-yl)butylate | 1H-NMR (CDCl3) δ ppm: 1.24 (3H, t, J=7.3Hz), 1.80-1.90 (2H, m), 2.26 (2H, t, J=7.3Hz), 3.53 (2H, t, J=7.3Hz), 4.05-4.20 (5H, m), 4.54 (2H, s), 6.50-6.55 (1H, m), 6.60-6.70 (1H, m), 6.85-6.95 (1H, m), 7.00-7.10 (1H, m) |
| 2-11 1 | 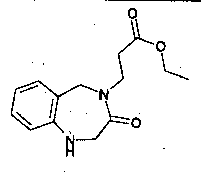 | ethyl 3-(3-oxo-1,2,3,5-tetrahydrobenzo[e]-1,4-diazepin-4-yl)propionate | 1H-NMR (CDCI3) δ ppm: 1.21 (3H, t, J=7.3Hz), 2.58 (2H, t, J=6.7Hz), 3.77 (2H, t, J=6.7Hz), 4.00-4.15 (5H, m), 4.61 (2H, s), 6.50-6.55 (1H, m), 6.60-6.70 (1H, m), 6.85-6.95 (1H, m), 7.00-7.10 (1H, m) |
| 2-12 | 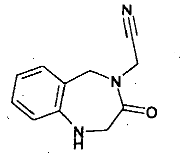 | (3-oxo-1,2,3,5-tetrahydrobenzo[e]-1,4-diazepin-4-yl)acetonitrile | 1H-NMR (CDCl3) δ ppm: 4.00-4.15 (1H, m), 4.15-4.25 (2H, m), 4.45 (2H, s), 4.67 (2H, s), 6.55-6.60 (1H, m), 6.65-6.75 (1H, m), 6.95-7.05 (1H, m), 7.10-7.15 (1H, m) MS(ESI, m/z): 202(M+H)+ |

**[Table 4]**

| Reference example | Structure formula | Compound name | 1 H-NMR(solvent) or MS(m/z) |
|---|---|---|---|
| 2-13 | 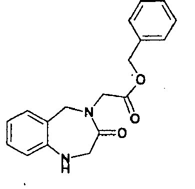 | benzyl (3-oxo-1,2,3,5-tetrahydrobenzo[e]-1,4-diazepin-4-yl)acetate | 1H-NMR (CDCl3) δ ppm: 4.00-4.10 (1H, m), 4.19 (2H, d, J=5.4Hz), 4.31 (2H, s), 4.61 (2H, s), 5.15 (2H, s), 6.50-6.60 (1H, m), 6.60-6.70 (1H, m), 6.85-6.90 (1H, m), 7.05-7.15 (1H, m), 7.25-7.45 (5H, m) |
| 2-14 | 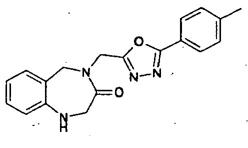 | 4-[5-(4-methylphenyl)-1,3,4-oxadiazol-2-ylmethyl]-1,2,4,5-tetrahydrobenzo[e]-1,4-diazepin-3-one | 1 H-NMR (CDCl3) δ ppm: 2.41, (3H, s), 4.17 (1H, t, J=5.5 Hz), 4.24 (2H, d, J=5.5 Hz), 4.67 (2H, s), 4.99 (2H, s), 6.50-6.60 (2H, m), 6.75-6.85 (1H, m), 6.95-7.05 (1H, m), 7.25 (2H, d, J=8:2 Hz), 7.77 (2H, d, J=8.2 Hz) MS(ESI, m/z): 335(M+H)+ |
| 2-15 | 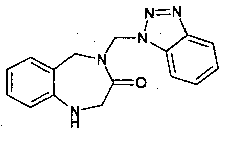 | 4-benzotriazol-1-ylmethyl-1,2,4,5-tetrahydrobenzo[e]-1,4-diazepin-3-one | 1 H-NMR (CDCI3) δ ppm: 4.00-4.10 (1H, m), 4.17 (2H, d, J=5.7Hz), 4.69 (2H, s), 6.29 (2H, s), 6.40-6.50 (1H, m), 6.55-6.60 (1H, m), 6.80-6.90 (1H, m), 6.95-7.05 (1H, m), 7.30-7.50 (2H, m), 7.80-7.90 (1H, m), 7.95-8.05 (1 H, m) |

**[Table 5]**

| Reference example | Structure formula | Compound name | 1H-NMR(solvent) or MS(m/z) |
|---|---|---|---|
| 2-16 | 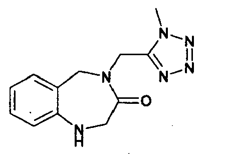 | 4-(1-methyl-1H-tetrazol-5-ylmethyl)-1,2,4,5-tetrahydrobenzo[e]-1,4-diazepin-3-one | 1H-NMR (CDCl3) δ ppm: 3.88 (3H, s), 4.05-4.10 (1H, m), 4.20 (2H, d, J=5.7Hz), 4.64 (2H, s), 5.01 (2H, s), 6.50-6.70 (3H, m), 7.00-7.10 (1H, m) |
| 2-17 | 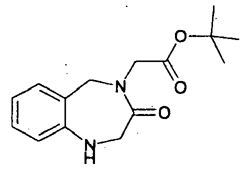 | tert-butyl (3-oxo-1,2,3,5-tetrahydrobenzo[e]-1,4-diazepin-4-yl)acetate | 1H-NMR (CDCl3) δ ppm: 1.42 (9H, s), 4.00-4.10 (1H, m), 4.15 (2H, s), 4.19 (2H, d, J=5.7Hz), 4.60 (2H, s), 6.50-6.70 (2H, m), 6.85-6.95 (1H, m), 7.00-7.15 (1H, m) |
| 2-18 | 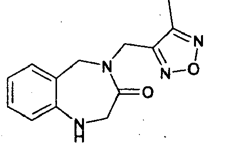 | 4-(4-methylfurazan-3-ylmethyl)-1,2,4,5-tetrahydrobenzo[e]-1,4-diazepin-3-one | 1H-NMR (CD3OD) 6 ppm: 2.18 (3H, s), 4.05-4.15 (1H, m), 4.21 (2H, d, J=5.6Hz), 4.55 (2H, s), 4.87 (2H, s), 6.45-6.70 (3H, m), 7.00-7.10 (1H, m) MS(ESI, m/z) : 259(M+H)+ |
| 2-19 | 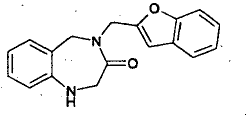 | 4-benzofuran-2-ylmethyl-1,2,4,5-tetrahydrobenzo[e]-1,4-diazepin-3-one | 1 H-NMR (CDCI3) δ ppm: , 4.10-4.15 (1H, m), 4.20 (2H, d, . J=5.4Hz), 4.63 (2H, s), 4.81 (2H, s), 6.50-6.65 (3H, m), 6.80-6.90 (1H, m), 7.00-7.10 (1H, m), 7.15-7.30 (2H, m), 7.35-7.55 (2H, m) MS(ESI, m/z): 293(M+H)+ |

**[Table 6]**

| Reference example | Structure formula | Compound name | 1H-NMR (solvent) or MS(m/z) |
|---|---|---|---|
| 2-20 | 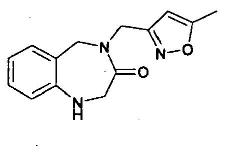 | 4-(5-methyl-isoxazol-3-ylmethyl)-1,2,4,5-tetrahydrobenzo[e]-1,4-diazepine-3-one | 1H-NMR (CD3OD) δ ppm: 2.30-2.35 (3H, m), 4.05-4.15 (1H, m), 4.18 (2H, d, J=5.5Hz), 4.54 (2H, s), 4.66 (2H, m), 5.80-5.90 (1H, m), 6.50-6.55 (1H, m), 6.55-6.65 (1H, m), 6.80-6.85 (1H, m), 7.00-7.10 (1H, m) MS(ESI, m/z): 258(M+H)+ |
| 2-21 | 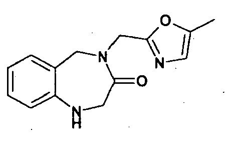 | 4-(5-methyl-oxazol-2-ylmethyl)-1,2,4,5-tetrahydrobenzo[e]-1,4-diazepine-3-one | 1H-NMR (CD3OD) 6 ppm: 2.17 (3H, s), 4.05-4.15 (1 H, m), 4.21 (2H, d, J=5.6Hz), 4.60 (2H, s), 4.75 (2H, s), 6.50-6.70 (3H, m), 6.75-6.85 (1H, m), 7.00-7.15 (1H, m) |
| 2-22 | 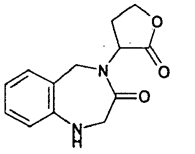 | 4-(2-oxo-tetrahydro-furan-3-yl)-1,2,4,5-tetrahydrobenzo[e]-1,4-diazepin-3-one | MS(ESI, m/z) : 247(M+H)+ |
| 2-23 | 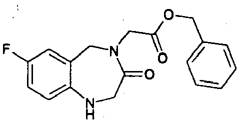 | benzyl (7-tluoro-3-oxo-1,2,3,5-tetrahydrobenzo[e]-1,4-diazepin-4-yl)acetate | 1H-NMR (CDCl3) δ ppm : 4.05-4.15 (3H, m), 4.30 (2H, s), 4.55 (2H, s), 5.14 (2H, s), 6.45-6.55 (1H, m), 6.60-6.65 (1H, m), 6.75-6.85 (1H, m), 7.20-7.45 (5H, m) |

**[Table 7]**

| Reference example | Structure formula | Compound name | 1 H-NMR(solvent) or MS(m/z) |
|---|---|---|---|
| 2-24 | 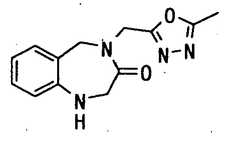 | 4-(5-methyl-1,3,4-oxadiazol-2-ylmethyl)-1,2,4,5-tetrahydrobenzo[e]-1,4-diazepin-3-one | 1H-NMR (CDCl3) δ ppm : 2.35 (3H, s), 4.10-4.20 (1H, br), 4.21 (2H, d, J=5.6Hz), 4.61 (2H, s), 4.88 (2H, s), 6.50-6.65 (2H, m), 6.70-6.80 (1H, m), 7.00-7.10 (1H, m) MS(ESI, m/z): 259(M+H)+ |
| 2-25 ' | 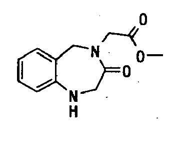 | methyl (3-oxo-1,2,3,5-tetrahydrobenzo[e]-1,4-diazepin-4-yl)acetate | 1H-NMR (CDCl3) δ ppm : 3.71 (3H, s), 4.10-4.25 (2H, m), 4.26 (2H, s), 4.60 (2H, s), 6.56 (1H, d, J=8.0Hz), 6.60-6.70 (1H, m), 6.89 (1H, d, J=7.5Hz), 7.05-7.15 (1H, m) MS(ESI, m/z) : 235(M+H)+ |
| 2-26 | 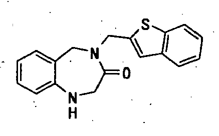 | 4-benzo[b]thiophen-2-ylmethyl-1,2,4,5-tetrahydrotienzo[e]-1,4-diazepin-3-one | 1H-NMR (CDCl3) δ ppm : 4.21 (2H, s), 4.55 (2H, s), 4.91 (2H, d, J=0.8Hz), 6.50-7.95 (9H, m) |

### Reference example 3

### tert-Butyl {[benzyl(2-bromobenzyl)carbamoyl]methyl}carbamate

To a stirred solution of benzyl (2-bromobenzyl) amine (347 mg), tert-butoxycarbonylaminoacetic acid (242 mg) and 4-dimethylaminopyridine (169 mg) in N,N-dimethylformamide (3.9 mL) was added 1-ethyl-3-(N,N-dimethylaminopropyl)carbodiimide hydrochloride (289 mg) and allowed to stirr at room temperature for 4 days. To the reaction mixture were added water and ethyl acetate, and the organic layer was separated. The organic layer was washed with water, 1 mol/L hydrochloric acid, water, a saturated solution of sodium hydrogen carbonate, water and brine, and dried over anhydrous magnesium sulfate, and filtered. The filtrate was concentrated under reduced pressure to give tert-butyl {[benzyl(2-bromobenzyl)carbamoyl]methyl}carbamate (525 mg).

¹H-NMR (CDCl₃) δ ppm:
1.40-1.50 (9H, m), 3.95-4.15 (2H, m), 4.40-4.80 (4H, m), 5.50-5.60 (1H, m), 7.00-7.65 (9H, m)

### Refernce example 4

### 2-Amino-N-benzyl-N-(2-bromobenzyl)acetamide

To tert-butyl {[benzyl(2-bromobenzyl)carbamoyl]-methyl} carbamate (521 mg) was added 20 wt% hydrochloric acid-ethanol (3.6 mL) under ice-cooling, and stirred at room temperature for 13 hours. To the stirred solution was added conc-hydrochloric acid (1.0 mL) under ice-cooling, and stirred at room temperature for an hour. The reaction mixture was concentrated under reduced pressure. To the obtained residue were added ethyl acetate and a saturated aqueous solution of sodium hydrogen carbonate. The organic layer was separated and the aqueous layer was extracted with ethyl acetate. The organic layer was combined, and washed with brine, and dried over anhydrous magnesium sulfate, and then filtered. The filtrate was concentrated under reduced pressure to give 2-amino-N-benzyl-N- (2-bromobenzyl)-acetamide (386 mg).

¹H-NMR (CDC1₃) δ ppm:
3.40-3.65 (2H, m), 4.40-4.80 (4H, m), 7.05-7.65 (9H, m)

### Reference example 5

### 2-Amino-N-(2-bromobenzyl)-N-methylacetamide hydrochloride

2-Amino-N-(2-bromobenzyl)-N-methylacetamide hydrochloride was prepared with the use of tert-butyl {[(2-bromobenzyl)-methylcarbamoyl]methyl}carbamate instead of tert-butyl {[benzyl(2-bromobenzyl)carbamoyl]methyl}carbamate in a similar manner to that described in Reference example 4.

¹H-NMR (DMSO-d6) δ ppm:
2.85-3.05 (3H, m), 3.80-4.05 (2H, m), 4.59 (2H, s), 7.15-7.50 (3H, m), 7.60-7.75 (1H, m), 8.27 (3H, s)

### Reference example 6

### 4-Methyl-1,2,4,5-tetrahydrobenzo[e]-1,4-diazepin-3-one

A mixture of 2-amino-N-(2-bromobenzyl)-N-methylacetamide hydrochloride (1.00 g), (S)-2,2'-bis(diphenylphosphino)-1,1'-binaphtyl (159 mg), palladium acetate (II) (38.2 mg), sodium tert-butoxide (687mg) and toluene (15mL) was stirred at 85 °C overnight under an argon atmosphere. To the reaction mixture was added water, and extracted with dichloromethane. The organic layer was concentrated under reduced pressure, the obtained residue was purified by column chromatography on aminopropylsilylated silica gel (eluent:ethyl acetate-hexane) to give 4-methyl-1,2,4,5-tetrahydrobenzo[e]-1,4-diazepin-3-one (303 mg).

¹H-NMR (CDCl₃) δ ppm:
3.07 (3H, s), 4.00-4.10 (1H, m), 4.14 (2H, d, J=5.7Hz), 4.52 (2H, s), 6.50-6.60 (1H, m), 6.60-6.70 (1H, m), 6.85-6.95 (1H, m), 7.05-7.15 (1H, m)

### Reference examples 7-1 to 7-3

The following compounds of Reference examples 7-1 to 7-3 were prepared with the use of the corresponding materials in a similar manner to that described in Reference example 6. The structure formula and physical data of these compounds were shown in Table 8.

**[Table 8]**

| Reference example | Structure formula | Compound name | 1H-NMR (solvent) |
|---|---|---|---|
| 7-1 | 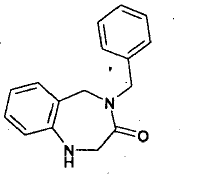 | 4-benzyl-1, 2,4, 5-tetrahydrobenzo[e]-1,4-diazepin-3-one | 1 H-NMR (CDCI3) δ ppm : 4.05-4.15 (1 H, m), 4.20-4.25 (2H, m), 4.44 (1 H, s), 4.69 (1 H, s), 6.50-6.65 (2H, m), 6.70-6.75 (1H, m), 7.00-7.10 (1 H, m), 7.20-7.35 (4H, m) |
| 7-2 | 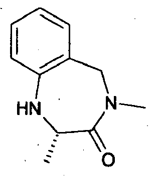 | (2S)-methyl-4-methyl-1,2,4,5-tetrahydrobenzo[e]-1,4-diazepin-3-one | 1 H-NMR (CDCI3) δ ppm: 1.40 (3H, d, J=6.2Hz), 3.07 (3H, s), 3.50-3.60 (1 H, m), 3.69 (1 H, d, J=16.5Hz), 4.70-4.80 (1H, m), 5.40 (1 H, d, J=16.5Hz), 6.45-6.55 (1H, m), 6.60-6.65 (1H, m), 6.85-6.95 (1H, m), 7.00-7.10 (1H, m) |
| 7-3 | 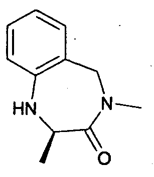 | (2R)-methyl-4-methyl-1,2,4,5-tetrahydrobenzo[e]-1,4-diazepin-3-one | 1H-NMR (CDCl3) δ ppm: 1.40 (3H, d, J=6.3Hz); 3.07 (3H, s), 3.50-3.60 (1 H, m), 3.69 (1H, d, J=16.5Hz), 4.70-4.80 (1H, m), 5.40 (1H, d, J=16.5Hz), 6.45-6.55 (1H, m), 6.60-6.65 (1 H, m), 6.85-6.95 (1 H, m), 7.00-7:10 (1H, m) |

### Reference example 8

### (2-Hydroxypropylcarbamoyl)methyl acetate

To a stirred solution of acetoxyacetic acid (1.00 g), 1-aminopropan-2-ol (0.655 g), and hydroxybenzotriazole monohydrate (1.36 g) in N,N-dimethylformamide (15 mL) was added 1-ethyl-3-(dimethylaminopropyl)carbodiimide hydrochloride (1.70 g) at room temperture, and the mixture was allowed to stirr at room temperature for 12 hours. The solvent was removed under reduced pressure. The obtained crude product was purified by column chromatography on aminopropylsilylated silica gel (eluent:ethyl acetate-methanol) to give (2-hydroxypropylcarbamoyl) methyl acetate (1.56 g).

¹H-NMR (CD₃OD) δ ppm:
1.22 (3H, d, J=6.3Hz), 2.18 (3H, s), 3.10-3.30 (2H, m), 3.45-3.60 (1H, m), 3.90-4.05 (1H, br), 4.59 (2H, s), 6.50-6.70 (1H, br)

### Reference example 9

### (2-Oxopropylcarbamoyl)methyl acetate

To a stirred solution of (2-hydroxypropylcarbamoyl)methyl acetate (1.37 g) in dichloromethane (30 mL) was added Dess-Martin reagent (3.98 g) at room temperature, and the mixture was stirred at room temperature for an hour. Dichloromethane was added to the suspension, and filtered through Celite. The filtrate was concentrated under reduced pressure. The obtained crude product was purified by column chromatography on silica gel (eluent:ethyl acetate-methanol) . To the obtained product was added diethyl ether, and then filtered off. The filtrate was concentrated under reduced pressure to give (2-oxopropylcarbamyl)methyl acetate (0.517 g).

¹H-NMR (CD₃OD) δ ppm:
2.20 (3H, s), 2.24 (3H, s), 4.21 (2H, d, J=4.6Hz), 4.60 (2H, s), 6.80-6.95 (1H, br)

### Reference example 10

### 5-Methyloxazol-2-ylmethyl acetate

A solution of (2-oxopropylcarbamoyl)methyl acetate (0.517 g) in phosphorus oxychloride (4.58 g) was heated to reflux for 0.5 hour. After left to cool to room temperature, the solution was concentrated under reduced pressure. Ethyl acetate was added to the residue, and the mixture was poured onto ice-water. The pH value was adjusted to pH 9 by adding potassium carbonate to the mixture under ice-cooling, and the mixture was stirred at the same condition for 0.5 hour. To the mixture was added ethylacetate, and the organic layer was separated. The aqueous layer was extracted with ethyl acetate, and the collected organic layer was dried over anhydrous magnesium sulfate. The solvent was removed under reduced pressure to give 5-methyloxazol-2-ylmethyl acetate (326 mg).

¹H-NMR (CDCl₃) δ ppm:
2.14 (3H, s), 2.33-(3H, d, J=1.1Hz), 5.11 (2H, s), 6.70-6.75 (1H, m)

### Reference example 11

### (5-Methyloxazol-2-yl)methanol

To a stirred solution of 5-methyloxazol-2-ylmethyl acetate (326 mg) in methanol was added 5 mol/L aqueous solution of sodium hydroxide (0.84 mL) at room temperature, the solution was stirred at room temperature for 0.5 hour. The reaction was quenched by addition of 2 mol/L hydrochloric acid (2.10 mL). To the solution were added ethyl acetate and water, and the organic layer was separated. The aqueous layer was extracted with ethyl acetate, the collected organic layer was dried over brine. The solvent was removed under reduced pressure to give (5-methyloxazol-2-yl)methanol (125 mg).

¹H-NMR (CD₃OD) δ ppm:
2.35-2.40 (3H, m), 4.76 (2H, s), 6.80-6.90 (1H, m)

### Reference example 12 ,

### tert-Butyl 3-oxo-1,2,3,5-tetrahydrobenzo[e]-1,4-diazepine-4-carboxylic acid

A solution of di-tert-butyl dicarboxylate (387 mg) in dichloromethane (3 mL) was added to a stirred solution of 1,2,4,5-tetrahydrobenzo[e]-1,4-diazepin-3-one (250 mg), diisopropylethylamine (195 mg) and N,N-dimethyl-4-aminopyridine (18.7 mg) in dichloromethane (2 mL) at room temperature. The reaction mixture was allowed to stirr for 2 days, and concentrated under reduced pressure. The residue was purified by column chromatography on silica gel (eluent:dichloromethane-methanol) to give tert-butyl 3-oxo-1,2,3,5-tetrahydrobenzo[e]-1,4-diazepine-4-carboxylate (327 mg).

¹H-NMR (CDCl₃) δ ppm:
1.51 (9H, s), 4.05-4.15 (1H, m), 4.24 (2H, d, J=5.8Hz), 5.00 (2H, s), 6.50-6.60 (1H, m), 6.65-6.75 (1H, m), 6.95-7.05 (1H, m), 7.05-7.15 (1H, m)

### Reference example 13

### 2-(2-Chloromethyl)-5-methyloxazole

Thionyl chloride (0.615 g) was added to a solution of 2-(2-hydroxymethyl)-5-methyloxazole (0.117 g) in dichloromethane (2 mL) under ice-cooling, and then the solution was stirred at room temperature for 1.5 hours. The solvent was removed under reduced pressure to give 2-(2-chloromethyl)-5-methyloxazole (0.116 g).

¹H-NMR (CDCl₃) δ ppm:
2.38 (3H, d, J=1.1Hz), 4.67 (2H, s), 6.85 (1H, d, J=1.1Hz)

### Reference example 14-1

### Methyl 4-(2-benzyloxyethoxy)-2-chlorobenzoate

To a stirred mixture of methyl 2-chloro-4-hydroxybenzoate (0.959 g), cesium carbonate (2.51 g), sodium iodide (0.847 g) and N,N-dimethylformamide (15 mL) was added (2-bromoethoxymethyl)-benzene (0.975 mL) at room temperature, and the reaction mixture was stirred at 80°C overnight. To the reaction mixture were added water and ethyl acetate, and the organic layer was separated. The organic layer was washed with water and brine. The organic layer was dried over anhydrous magnesium sulfate, and after filtration, the filtrate was concentrated under reduced pressure. The obtained crude product was purified by column chromatography on aminopropylsilylated silica gel (eluent:ethyl acetate-hexane) to give methyl 4-(2-benzyloxyethoxy)-2-chlorobenzoate (1.66 g).

¹H-NMR(CDCl₃) δ ppm:
3.80-3.85 (2H, m), 3.89 (3H, s), 4.15-4.25 (2H, m), 4.62 (2H, s), 6.84 (1H, dd, J=8.8, 2.5Hz), 7.00 (1H, d, J=2.5Hz), 7.25-7.40 (5H, m), 7.87 (1H, d, J=8.8Hz)

### Reference examples 14-2 to 14-8

The following compounds of Reference example 14-2 to 14-8 were prepared with the use of the corresponding materials in a similar manner to that described in Reference example 14-1. The structure formula and physical data of these compounds were shown in Table 9 and 10.

**[Table 9]**

| Reference example | Structure formula | Compound name | 1H-NMR (solvent) |
|---|---|---|---|
| 14-2 | 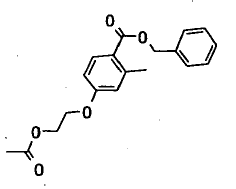 | benzyl 4-(2-acetoxyethoxy)-2-methylbenzoate | 1H-NMR (CDCl3) δ ppm: 2.05-2.15 (3H, m), 2.61 (3H, s), 4.20 (2H, t, J=4.7Hz), 4.43 (2H, t, J=4.7Hz), 5.31 (2H, s), 6.70-6.80 (2H, m), 7.30-7.50 (5H, m), 7.98 (1 H, d, J=8.5Hz) |
| 14-3 | 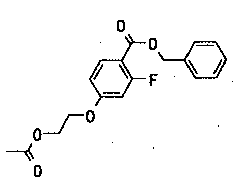 | benzyl 4-(2-acetoxyethoxy)-2-fluorobenzoate | 1H-NMR (CDCl3) δ ppm: 2.11 (3H, s), 4.20 (2H, t, J=4.8Hz), 4.43 (2H, t, J=4.8Hz), 5.36 (2H, s), 6.60-6.80 (2H, m), 7.30-7.50 (5H, m), 7.90-8.00 (1 H, m) |
| 14-4 | 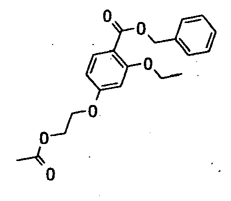 | benzyl 4-(2-acetoxyethoxy)-2-ethoxybenzoate | 1H-NMR (CDCl3) δ ppm: 1.44 (3H, t, J=7.0Hz), 2.11 (3H, s), 4.08 (2H, q, J=7.0Hz), 4.20 (2H, t, J=4.7Hz), 4.43 (2H, t, J=4.7Hz), 5.32 (2H, s), 6.40-6.55 (2H, m), 7.25-7.50 (5H, m), 7.89 (1 H, d, J=8.7Hz) |
| 14-5 | 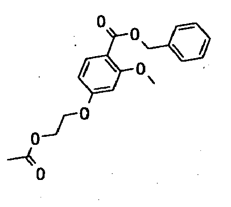 | benzyl4-(2-acetoxyethoxy)-2-methoxybenzoate | 1H-NMR (CDCl3) δ ppm: 2.10 (3H, s), 3.90 (3H, s), 4.21 (2H, t, J=4.8Hz), 4.44 (2H, t, J=4.8Hz), 5.32 (2H, s), 6.47 (1 H, dd, J=8.7, 2.3Hz), 6.52 (1 H, d, J=2.3Hz), 7.25-7.50 (5H, m), 7.89 (1 H, d, J=8.7Hz) |
| 14-6 | 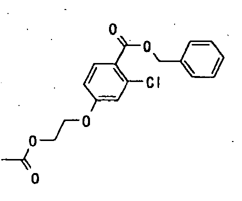 | benzyl 4-(2-acetoxyethoxy)-2-chlorobenzoate | 1H-NMR (CDCl3) δ ppm: 2.05-2.15 (3H, m), 4.15-4.25 (2H, m), 4.40-4.45 (2H, m), 5.35 (2H, s), 6.82 (1H, dd, J=8.8, 2.5Hz), 6.99 (1H, d, J=2.5Hz), 7.30-7.50 (5H, m), 7.91 (1 H, d, J=8.8Hz) |

**[Table 10]**

| Reference example | Structure formula | Compound name | 1H-NMR (solvent) |
|---|---|---|---|
| 14-7 | 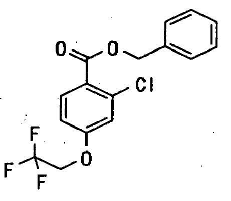 | benzyl 2-chloro-4-(2,2,2-trifluoro-ethoxy)benzoate | 1H-NMR (CDCl3) δ ppm: 4.39 (2H, q, J=7.9Hz), 5.36 (2H, s), 6.87 (1 H, dd, J=8.8, 2.6Hz), 7.03 (1 H, d, J=2.6Hz), 7.30-7.50 (5H, m), 7.94 (1H, d, J=8.8Hz) |
| 14-8 | 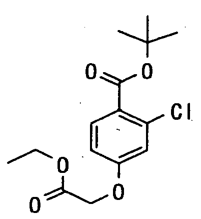 | tert-butyl 2-chloro-4-ethoxycarbonyl-methoxybenzoate | 1H-NMR (CDCl3) δ ppm: 1.30 (3H, t, J=7.1 Hz), 1.59 (9H, s), 4.28 (2H, q, J=7.1Hz), 4.64 (2H, s), 6.81 (1 H, dd, J=8.7, 2.6Hz), 6.95 (1 H, d, J=2.6Hz), 7.79 (1 H, d, J=8.8Hz) |

### Reference example 15-1

### 4-(2-Benzyloxyethoxy)-2-chlorobenzoic acid

A solution of methyl 4-(2-benzyloxyethoxy)-2-chlorobenzoate (1.66 g) and 5 mol/L aqueous solution of sodium hydroxide (5.18 mL) in methanol (15 mL) was heated at reflux for 4.5 hours. After standing to cool, to the reaction mixture was added 2 mol/L hydrochloric acid (12.9 mL), and concentrated under reduced pressure. To the residue were added water and ethyl acetate, and the organic layer was separated. The organic layer was washed with water. The organic layer was dried over anhydrous magnesium sulfate, after filtration, the filtrate was concentrated under reduced pressure to give 4-(2-benzyloxyethoxy)-2-chlorobenzoic acid (1.61 g).

¹H-NMR(CDCl₃) δ ppm:
3.80-3.95 (2H, m), 4.15-4.30 (2H, m), 4.63 (2H, s), 6.88 (1H, dd, J=8.9, 2.5Hz), 7.03 (1H, d, J=2.5Hz), 7.25-7.50 (5H, m), 8.03 (1H, d, J=8.9Hz)

### Reference examples 15-2 to 15-4

The following compounds of Reference examples 15-2 to 15-4 were prepared with the use of the corresponding ester derivatives in a similar manner of hydrolysis to that described in Reference example 15-1. The structure formula and physical data of these compounds were shown in Table 11.

**[Table 11]**

| Referenc example | Structure formula | Compound name | 1 H-NMR (solvent) or MS(m/z) |
|---|---|---|---|
| 15-2 | 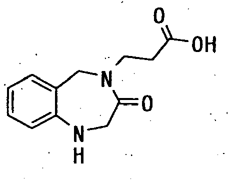 | 3-(3-oxo-1,2,3,5-tetrahydrobenzo[e]-1,4-diazepin-4-yl)propionic acid | 1H-NMR (CD30D) δ ppm: 2.53 (2H, t, J=7.2Hz), 3.73 (2H, t, J=7.2Hz), 4.08 (2H, s), 4.68 (2H, s), 6.50-6.65 (2H, m), 6.90-7.05 (2H, m) |
| 15-3 | 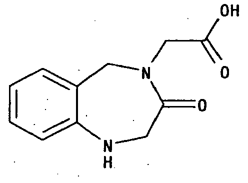 | (3-oxo-1,2,3,5-tetrahydrobenzo[e]-1,4-diazepin-4-yl)acetic acid | MS(ESI, m/z) : 221 (M+H)+ |
| 15-4 | 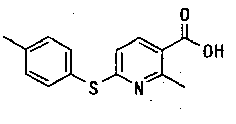 | 2-methyl-6-(4-methylphenyl)sulfanylni cotinic acid | 1H-NMR (CDCl3) δ ppm: 2.43 (3H, s), 2.84 (3H, s), 6.00-6.65 (1H, m), 7.25-7.35 (2H, m), 7.45-7.55 (2H, m), 8.03 (1H, d, J=8.5Hz) |

### Reference example 16-1

### 4-(2-Acetoxyethoxy)-2-chlorobenzoic acid

A suspension of benzyl 4- (2-acetoxyethoxy) -2- chlorobenzoate (1.73 g) and palladium-carbon (10%, 0.35 g) in tetrahydrofuran (20 mL) was stirred at room temperature for 2 hours under a hydrogen atmosphere. The mixture was filtered through Celite, and the filtrate was concentrated under reduced pressure to give 4-(2-acetoxyethoxy)-2-chlorobenzoic acid (1.14 g).

¹H-NMR (CDCl₃) δ ppm:
2.11 (3H, s), 4.23 (2H, t, J=4.7Hz), 4.44 (2H, t, J=4.7Hz), 6.87 (1H, dd, J=8.8, 2.5Hz), 7.03 (1H, d, J=2.5Hz), 8.05 (1H, d, J=8.8Hz)

### Reference examples 16-2 to 16-8

The following compounds of Reference examples 16-2 to 16-8 were prepared with the use of the corresponding materials in a similar manner to that described in Reference example 16-1. The structure formula and physical data of these compounds were shown in Tables 12 and 13.

**[Table 12]**

| Reference example | Structure formula | Compound name | 1 H-NMR (solvent) |
|---|---|---|---|
| 16-2 | 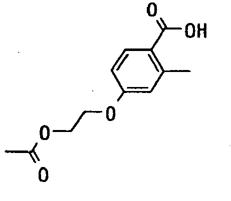 | 4-(2-acetoxyethoxy)-2-methylbenzoic acid | 1H-NMR (CDCl3) δ ppm: 2. 11 (3H, m), 2.64 (3H, s), 4.23 (2H, t, J=4.7Hz), 4.44 (2H, t, J=4.7Hz), 6.70-6.85 (2H, m), 8.00-8.10 (1H, m) |
| 16-3 | 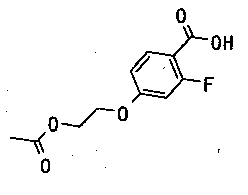 | 4-(2-acetoxyethoxy)-2-fluorobenzoic acid | 1H-NMR (CD3OD) δ ppm: 2.06 (3H, s), 4.20-4.30 (2H, m), 4.35-4.45 (2H, m), 6.75-6.90 (2H, m), 1.85-7.95 (1 H, m) |
| 16-4 | 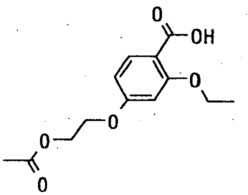 | 4-(2-acetoxyethoxy)-2-ethoxybenzoic acid | 1H-NMR (CDCl3) δ ppm: 1.50-1.60 (3H, m), 2.11 (3H, s), 4.20-4.35 (4H, m), 4.45 (2H, t, J=4.7Hz), 6.57 (1H, d, J=2.2Hz), 6.64 (1 H, dd, J=8.9, 2.2Hz), 8.15 (1H, d, J=8.9Hz), 10.60-10.80 (1H, br) |
| 16-5 | 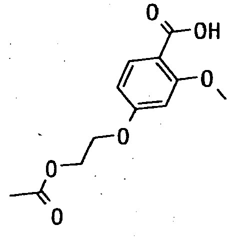 | 4-(2-acetoxyethoxy)-2-methoxybenzoic acid | 1H-NMR (CDCl3) δ ppm: 2.11 (3H, s), 4.05 (3H, s), 4.25 (2H, t, J=4.8Hz), 4.45 (2H; t, J=4.8Hz), 6.59 (1H, d, J=2.2Hz), 6.64 (1H, dd, J=8.8, 2.2Hz), 8.14 (1 H, d, J=8.8Hz), 10.00-11.50 (1H, br) |

**[Table 13]**

| Reference example | Structure formula | Compound name | 1 H-NMR (solvent) |
|---|---|---|---|
| 16-6 | 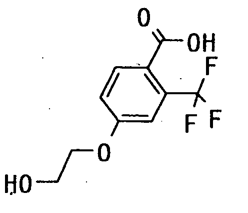 | 4-(2-hydroxyethoxy)-2-trifluoromethyl-benzoic acid | 1H-NMR (CDCl3) δ ppm: 4.00-4.10 (2H, m), 4.15-4.25 (2H, m), 7.11 (1H, dd, J=8.8, 2.5Hz), 7.35 (1H, d, J=2.5Hz), 8.00-8.10 (1H, m) |
| 16-7 | 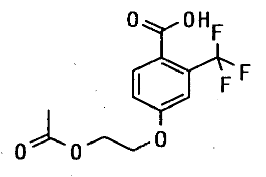 | 4-(2-acetoxyethoxy)-2-trifluoromethyl-benzoic acid | 1H-NMR (CDCl3) δ ppm: 2.12 (3H, s), 4.27 (2H, t, J=4.6Hz), 4.47 (2H, t, J=4.6Hz), 7.09 (1H, dd, J=8.7, 2.4Hz), 7.34 (1H, d, J=2.4Hz), 8.05 (1H, d, J=8.7Hz) |
| 16-8 | 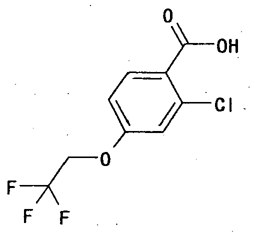 | 2-chloro-4-(2,2,2-trifluoroethoxy)-benzoic acid | 1H-NMR (CDCl3) δ ppm: 4.42 (2H, q, J=7.8Hz), 6.92 (1H, dd, J=8.9, 2.6Hz), 7.07 (1H, d, J=2.6Hz), 8.07 (1H, d, J=8.9Hz) |

### Reference example 17-1

### 6-(2-Benzyloxyethoxy)-2-methylnicotinic acid

To a stirred solution of tert-butyl 6- (2-benzyloxyethoxy) -2-methylnicotinate (0.0646 g) in dichloromethane (1.0 mL) was added trifluoroacetic acid (0.429 g) under ice-cooling, and the reaction mixture was allowed to stirr overnight. To the mixture was added toluene, after trifluoroacetic acid was removed by azeotropy, the residue was concentrated under reduced pressure to give 6-(2-benzyloxyethoxy)-2-methylnicotinic acid (0.0526 g).

¹H-NMR (DMSO-d₆) δ ppm:
2.65 (3H, s), 3.70-3.80 (2H, m), 4.45-4.50 (2H, m), 4.54(2H, s), 6.73. (1H, d, J=8.6Hz), 7.25-7.40 (5H, m), 8.10.(1H, d, J=8.6Hz), 12.50-13.50 (1H, br)

### Reference examples 17-2 to 17-4

The following compounds of Reference example 17-2 to 17-4 was prepared with the use of the corresponding materials in a similar manner to that described in Refernce example 17-1. The structure formula and physical data of these compounds were shown in Table 14.

**[Table 14]**

| Reference example | Structure formula | Compound name | 1 H-NMR (solvent) |
|---|---|---|---|
| 17-2 | 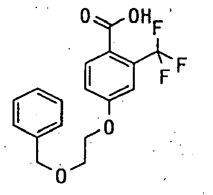 | 4-(2-benzyloxy-ethoxy)-2-trifluoro-methylbenzoic acid | 1 H-NMR (CDCI3) δ ppm: 3.80-3.90 (2H, m), 4.20-4.30 (2H, m), 4.64 (2H, s), 7.10 (1 H, dd, J=8.8, 2.5Hz), 7.25-7.45 (6H, m), 8.03 (1H, d, J=8.8Hz) |
| 17-3 | 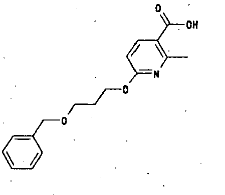 | 6-(3-benzyloxy-propoxy)-2-methylnicotinic acid | 1H-NMR (DMSO-d6) δ ppm: 1.95-2.05 (2H, m), 2.66 (3H, s), 3.57 (2H, t, J=6.4Hz), 4.39 (2H, t, J=6.4Hz), 4.48 (2H, s), 6.68 (1H, d, J=8.6Hz), 7.20-7.40 (5H, m), 8.09 (1 H, d, J=8.6Hz), 12.50-13.00 (1H, br) |
| 17-4 | 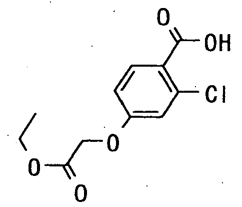 | 2-chloro-4-ethoxy-carbonylmethoxy-benzoic acid | 1 H-NMR (CDCI3) δ ppm: 1.31 (3H, t, J=7.2Hz), 4.30 (2H, q, J=7.2Hz), 4.68 (2H, s), 6:86 (1H, dd, J=8.9, 2.6Hz), 7.02 (1 H, d, J=2.6Hz), 8.06 (1 H, d, J=8.9Hz) |

### Reference example 18-1

### Benzyl 3-(3-oxo-1,2,3,5-tetrahydrobenzo[e]-1,4-diazepin-4-yl)-propionate

A stirred suspension of 3-(3-oxo-1,2,3,5-tetrahydrobenzo[e]-1,4-azepin-4-yl)propionic acid (0.294 g) and cesium carbonate (0.612 g) in N,N-dimethylformamide (8.0 mL) was added benzylbromide (0.257 g) at room temperature, the mixture was stirred at room temperature for an hour. To the mixture were added water and ethyl acetate, then the organic layer was separated. The organic layer was successively washed with water and brine, and dried over anhydrous magnesium sulfate. The solvent was removed under reduced pressure. The obtained crude product was purified by column chromatography on silica gel (eluent: ethyl acetate-hexane) to give benzyl 3-(3-oxo-1,2,3,5-tetrahydrobenzo[e]-1,4-diazepin-4-yl)propionate (0.262 g).

¹H-NMR (CDCl₃) δ ppm:
2.64 (2H, t, J=6.7Hz), 3.78 (2H, t, J=6.7Hz), 4.00-4.15 (3H, m), 4.45-4..60 (2H, m), 5.00-5.10 (2H, m), 6.50 (1H, dd, J=8.1, 0.9Hz), 6.55-6.70 (1H, m), 6.88 (1H, dd, J-7.4, 1.2Hz), 7.00-7.10 (1H, m), 7.25-7.30 (5H, m)
MS (ESI, m/z): MS (ESI, m/z): 325 (M+H)⁺

### Reference examples 18-2 to 18-4

The following compounds of Reference examples 18-2 to 18-4 were prepared with the use of the corresponding materials in a similar manner to that described in Reference example 18-1. The structure formula and physical data of these compounds were shown in Table 15.

**[Table 15]**

| Reference example | Structure formula | Compound name | 1 H-NMR (solvent) or MS(m/z) |
|---|---|---|---|
| 18-2 | 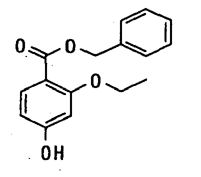 | benzyl 2-ethoxy-4-hydroxybenzoate | 1H-NMR (CDCI3) δ ppm: 1.38 (3H, t, J=7.0Hz), 3.98 (2H, q, J=7.0Hz), 5.32 (2H, s), 6.25-6.45 (3H, m), 7.25-7.50 (5H, m), 7.75-7.85 (1H, m) |
| 18-3 | 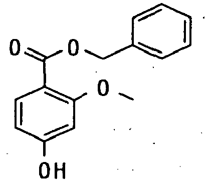 | benzyl 4-hydroxy-2-methoxybenzoate | 1 H-NMR (CDCl3) δ ppm: 3.80 (3H, s), 5.31 (2H, s), 6.25- . 6.35 (1H, brs), 6.35-6.50 (2H, m), 7.25-7.50 (5H, m), 7.83 (1H, d, J=8.6Hz) |
| 18-4 | 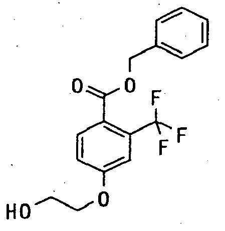 | benzyl 4-(2-hydroxy-ethoxy)-2-trifluoromethyl-benzoate | 1H-NMR (CDCl3) δ ppm: 2.00-2.15 (1H, m), 3.95-4.05 (2H, m), 4.10-4.20 (2H, m), 5.34 (2H, s), 7.07 (1H, dd, J=8.7, 2.5Hz), 7.29 (1H, d, J=2.5Hz), 7.30-7.50 (5H, m), 7.88 (1H, d, J=8.7Hz) |

### Reference example 19

### Ethyl 2-methyl-6-(4-methylphenyl)sulfanylnicotinate

To a stirred solution of 4-methylbenzenethiol (0.0650 g) in N,N-dimethylformamide (0.30 mL) was added potassium tert-butoxide (0.0610 g) at room temperature, and stirred under the same condition for 15 minutes (as a solution A). To a solution of ethyl 6-chloro-2-methylnicotinate in N,N-dimethylformamide (0.50 mL) was gradually added the solution A at an external temperature of -20°C, and stirred under the same condition for an hour. The reaction mixture was poured onto ice-water, and extracted with ethyl acetate. The separated organic layer was washed with water and brine, and dried over anhydrous magnesium sulfate, and filtered. The filtrate was concentrated under reduced pressure, the residue was purified by column chromatography on silica gel (eluent:hexane-ethyl acetate) to give ethyl 2-methyl-6-(4-methylphenyl)sulfanylnicotinate (0.134 g).

¹H-NMR (CDC1₃) δ ppm:
1.36 (3H, t, J=7.2Hz), 2.42 (3H, s), 2.79 (3H, .Ss), 4.33 (2H, q, J=7.2Hz), 6.60 (1H, d, J=8.5Hz), 7.20-7.30 (2H, m), 7.45-7.55 (2H, m), 7.93 (1H, d, J=8.5Hz)

### Reference example 20

### tert-Butyl 2-methyl-6-(4-methylphenyl)sulfanylnicotinate

To a mixed solution of 2-methyl-6-(4-methylphenyl)-sulfanylnicotinic acid (0.200 g) and tert-butyl 2,2,2-trichloroacetoimidate (0.320 g) in tetrahydrofuran (2.8 mL)-dichlorometane (2.8 mL) was added trifluoroborane-diethyl ether complex (10 uL) with stirring under ice-cooling, and stirred under the same condition for 4 hours. To the stirred solution was added trifluoroborane-diethyl ether complex (100 uL) under ice-cooling, and stirred under the same condition for 19 hours. To the stirred reaction mixture was added tert-butyl 2,2,2-trichloroacetimidate (0.0843 g) under ice-cooling, and stirred under the same condition for 2 hours. To the stirred reaction mixture were added water and ethyl acetate under ice-cooling. The separated organic layer was washed with water and brine, and dried over anhydrous magnesium sulfate, and filtered. The filtrate was concentrated under reduced pressure, the residue was purified by column chromatography on silica gel (eluent:hexane-ethyl acetate) to give tert-butyl 2-methyl-6-(4-methylphenyl)sulfanylnicotinate (0.136 g).

¹H-NMR (CDCl₃) δ ppm:
1.56 (9H, s), 2.41 (3H, s), 2.76 (3H, s), 6.58 (1H, d, J=8.4Hz), 7.20-7.30 (2H, m), 7.45-7.55 (2H, m), 7.85 (1H, d, J=8.4Hz)

### Reference example 21

### tert-Butyl 2-methyl-6-[(4-methylphenyl)-4-sulfonyl]nicotinate

To a stirred solution of tert-butyl 2-methyl-6-(4-methylphenyl)sulfanylnicotinate (0.135 g) in dichloromethane (2.2 mL) was added 3-chloroperoxybenzoic acid (0.170 g) under ice-cooling, and stirred at room temperature for 2 hours. To the reaction mixture was added 3-chloroperoxybenzoic acid (0.026 g) with stirring under ice-cooling, and stirred at room temperature for an hour. To the reaction mixture was added aqueous solution of sodium thiosulfate, and extracted with ethyl acetate. The separated organic layer was washed with water, a saturated aqueous solution of sodium hydrogen carbonate and brine successively, and dried over anhydrous magnesium sulfate, and filtered. The filtrate was concentrated under reduced pressure, the residue was purified by column chromatography on silica gel (eluent:hexane-ethyl acetate) to give tert-butyl 2-methyl- 6-[(4-methylphenyl)-4-sulfonyl]nicotinate (0.118 g).

¹H-NMR (CDCl₃) δ ppm:
1.58 (9H, s), 2.42 (3H, s), 2.76 (3H, s), 7.25-7.40 (2H, m), 7.90-8.00 (2H, m), 8.00-8.05 (1H, m), 8.24 (1H, d, J=8.1Hz)

### Reference example 22

### tert-Butyl 6-(2-benzyloxyethoxy)-2-methylnicotinate

To a stirred suspension of sodium hydride (10 mg, purity 50-72%) in tetrahydrofuran (0.70 mL) was added 2-benzyloxyethanol (0.0381 g) at room temperature, and stirred at roomtemperature for 30 minutes. To the stirred reaction mixture was added tert-butyl 2-methyl-[6-(4-methylpheriyl)-4-sulfonyl]nicotinate (0.0580 g) under ice-cooling, and stirred at room temperature for 2 hours. To the stirred reaction mixture were added a suspension of sodium hydride (10 mg, 50-72% purity) and 2-benzyloxyethanol (0.013 g) in tetrahydrofuran, and stirred at room temperature for 1.5 hours. To the reaction mixture was added aqueous solution of citric acid (140 mg) (2 mL), and stirred for a few minutes. To the solution of mixture was added water, and extracted with ethyl acetate. After being washed with water, the organic layer was dried over anhydrous magnesium sulfate, and filtered. The filtrate was concentrated under reduced pressure, the residue was purified by column chromatography on silica gel (eluent:hexane-ethyl acetate) to give tert-butyl 6-(2-benzyloxyethoxy)-2-methylnicotinate (0.0646 g).

¹H-NMR (CDCl₃) δ ppm:
1.58 (9H, s), 2.70 (3H, s), 3.80-3.85 (2H, m), 4.50-4.60 (2H, m), 4,62 (2H, s), 6.62 (1H, d, J=8.6Hz), 7.25-7.40 (5H, m), 8.04 (1H, d, J=8.6Hz)

### Reference example 23

### tert-Butyl 6-(3-benzyloxypropoxy)-2-methylnicotinate

tert-Butyl 6-(3-benzyloxypropoxy)-2-methylnicotinate was prepared with the use of 2-benzyloxypropanol in a similar manner to that described in Reference example 22.

¹H-NMR (CDCl₃) δ ppm:
1.58 (9H, s), 2.00-2. 15 (2H, m), 2.71 (3H, s), 3.65 (2H, t, J=6.3Hz), 4.45 (2H, t, J=6.3Hz), 4. 53 (2H, s), 6. 53 (1H, d, J=8.5Hz), 7.25-7.40 (5H, m), 8.04 (1H, d, J=8.5Hz)

### Reference example 24

### Di-tert-butyl N-(5-fluoro-2-nitrobenzyl)imidodicarbonate

A mixture of 2-bromoethyl-4-fluoro-1-nitrobenzene (0.427 g), sodium hydride (0.117 g, purity 60%) and di-tert-butyl iminodicarboxylate (0.635 g) in N,N-dimethylformamide was stirred at an external temperature of 50 °C for 15 hours. After standing to cool, to the mixture were added water and dichloromethane. The organic layer was separated and concentrated under reduced pressure. The obtained crude product was purified by column chromatography on silica gel (eluent:hexane-ethyl acetate) to give di-tert-butyl (5-fluoro-2-nitrobenzyl)carbamate (0.538 g).

¹H-NMR (CDCl₃) δ ppm:
1.46 (18H, s), 5.18 (2H, s), 7'.00-7.15 (2H, m), 8.15 (1H, m)

### Reference example 25

### Di-tert-butyl N-(2-amino-5-fluorobenzyl)imidodicarbonate

The mixture of di-tert-butyl (5-fluoro-2-nitrobenzyl)-carbamate (0.538 g) and 10% palladium-carbon (80.0 mg) in tetrahydrofuran (1.0 mL)-ethanol (2.0 mL) was stirred at room temperature for 6 hours under a hydrogen atmosphere. The reaction mixture was filtered, and the filtrate was concentrated under reduced pressure to give di-tert-butyl (5-fluoro-2- aminobenzyl)carbamate (0.499 g).

¹H-NMR (CDC1₃) δ ppm:
1.48 (18H, s), 4.10-4.25 (2H, br), 4.66 (2H, s), 6.50-6.60 (1H, m), 6.75-6.85 (1H, m), 6.90-7.00 (1H, m)

### Reference example 26

### Ethyl {2-[bis(tert-butoxycarbonyl)aminomethyl]-5-fluorophenylamino}acetate

To a stirred solution of di-tert-butyl (5-fluoro-2-aminobenzyl)carbamate (0.499 g) and diisopropylethylamine (0.284 g) in N,N-dimethylformamide (1.5 mL) was added ethylbromo acetate (0.269 g), and the solution was stirred at an external temperature of 50°C for 17 hours. After standing to cool, to the reaction mixture were added water and diethyl ether. The organic layer was separated, after its layer was successively washed with water and brine, and dried over anhydrous magnesium sulfate. The solvent was removed under reduced pressure to give ethyl [2-(bis(tert-butoxycarbonyl)aminomethyl)-5-fluorophenylamino]acetic acid (0.578 g).

¹H-NMR (CDCl₃) δ ppm:
1.26 (3H, t, J=7.2Hz), 1.47 (18H, s), 3.89 (2H, d, J=5.7Hz), 4.21 (2H, q, J=7.2Hz), 4.72 (2H, s), 5.28 (1H, m), 6.35-6.45 (1H, m), 6.80-6.90 (1H, m), 6.95-7.00 (1H, m)

### Reference example 27

### Ethyl (2-aminomethyl-5-fluorophenylamino)acetic acid hydrochloride

A solution of ethyl [2-(bis(tert-butoxycarbonyl)aminomethyl) -5-fluorophenylamino] acetic acid (0. 578 g) in 26 wt% hydrogen chloride-ethanol (3.0 mL) was stirred at room temperature for 3 hours. The solution was concentrated under reduced pressure. The residue was dissolved in ethanol (2.0 mL) at an external temperature of 50°C, and tetrahydrofuran (8.0 mL) was added therein. The reaction mixture was stirred at room temperature for an hour. The deposited precipitate was collected by filtration to give ethyl (2-aminomethyl-5-fluorophenylamino)acetic acid hydrochloride (0.366 g).

¹H-NMR (DMSO-d₆) δ ppm:
1.20 (3H, t, J=7.2Hz), 3.70-4.40 (6H, m), 6.45-6.55 (1H, m), 7.00-7.15 (2H, m), 8.10-8.25 (3H, br)

### Reference example 28

### 7-Fluoro-1,2,4,5-tetrahydrobenzo[e]-1,4-diazepin-3-one

A mixture of ethyl (2-aminomethyl-4-fluorophenylamino)acetic acid hydrochloride (0.218 g), sodium ethoxide (20% ethanol solution, 0.040 mL) in toluene (2.0 mL) was heated at reflux for 4 hours. After standing to cool, the mixture was concentrated under reduced pressure. To the residue was added tetrahydrofuran (15ml), and filtered. The filtrate was concentrated under reduced pressure, and the obtained crude product was purified by column chromatography on silica gel (eluent: dichloromethane-methanol) to give 7-fluoro-1,2,4,5-tetrahydrobenzo[e]-1,4-diazepin-3-one (0.0662 g).

¹H-NMR (CD₃OD) δ ppm:
3.98 (2H, s), 4.36 (2H, s);6.60-6.70 (1H, m), 6.70-6.85 (2H, m)

### Reference example 29

### tert-Butyl 4-(2-benzyloxyethoxy)-2-trifluoromethylbenzoic acid

To a stirred solution of 2-benzyloxyethanol (0.236 g) in tetrahydrofuran (3 mL) was added sodium hydride (purity 55%, 0.062 g) under ice-cooling, and the suspension was stirred at room temperature for 45 minutes. After addition of tert-butyl 4-fluoro-2-trifluoromethylbenzoic acid (0.25 g) in tetrahydrofuran (1.5 mL) with stirring under ice-cooling, the suspension was stirred at room temperature for 2.5 hours. To the stirred mixture were added ethyl acetate and water under ice-cooling. The organic layer was separated. After extraction of aqueous layer with ethyl acetate, and the collected organic layer was washed with brine and dried over anhydrous magnesium sulfate. The solvent was removed under reduced pressure. The crude product was purified by column chromatography on silica gel (eluent:hexane-ethyl acetate) to give tert-butyl 4- (2-benzyloxyethoxy)-2-trifluoromethylbenzoic acid (0.0307 g).

¹H-NMR (CDCl₃) δ ppm:
1.57 (9H, s), 3.84 (2H, t, J=4.7Hz), 4.21 (2H, t, J=4.7Hz), 4.63 (2H, s), 7.06 (1H, dd, J=8.7, 2.5Hz), 7.20-7.40 (6H, m), 7.75 (1H, d, J=8.7Hz)

### Reference example 30

### Benzyl 4-(2-acetoxyethoxy)-2-trifluoromethylbenzoic acid

To a stirred solution of benzyl 4-(2-hydroxyethoxy)-2-trifluoromethylbenzoic acid (0.12 g) and pyridine (0.042 g) in tetrahydrofuran (2 mL) was added acetyl chloride (0.033 g) under ice-cooling, the suspension was stirred at room temperature for an hour. The suspension to which was added pyridine (0.042g) and acetyl chloride (0.033 g) under ice cooling was stirred at room temperature for 30 minutes. To the mixture were added dichloromethane and water. Then, the pH value was adjusted to pH 1 with 2 mol/L hydrochloric acid. The organic layer was separated, and the solvent was removed under reduced pressure to give benzyl 4-(2-acetoxyethoxy)-2-trifluoromethylbenzoic acid (0.15 g).

¹H-NMR (CDCl₃) δ ppm:
2.10 (3H, s), 4.24 (2H, t, J=4.7Hz), 4.45 (2H, t, J=4.7Hz), 5.34 (2H, s), 7.06 (1H, dd, J=8.7, 2.5Hz), 7.29 (1H, d, J=2.5Hz), 7.30-7.50 (5H, m), 7.88 (1H, d, J=8.7Hz)

### Reference example 31

### 4-(2-Hydroxyethyl)-1,2,4,5-tetrahydrobenzo[e]-1,4-diazepin-3-one

To the mixture of (3-oxo-1,2,3,5-tetrahydrobenzo[e]-1,4-diazepin-4-yl)acetic acid (0.517 g) and N-methylmorpholine (0.356 g) in tetrahydrofuran (16 mL) was added isobutyl chloroformate (0.481 g) by dropwise under ice-methanol cooling and stirring. The reaction mixture was stirred at room temperature for 30 minutes. To the mixture was added a suspension of sodium hydride (0.151 g) in ethanol (3.5 mL) with ice-cooling in methanol, the reaction mixture was stirred under the same condition for 10 minutes. The reaction was quenched by addition of water, and the mixture was extracted with ethyl acetate. The separated aqueous layer was extracted with ethyl acetate, then the collected organic layer was washed with brine, and dried over anhydrous magnesium sulfate. The solvent was removed under reduced pressure. The crude product was purified by column chromatography on silica gel (eluent: hexane-ethyl acetate) to give 4-(2-hydroxyethyl)-1,2,4,5-tetrahydrobenzo[e]-1,4-diazepin-3-one (0.151 g).

¹H-NMR (DMSO-d₆) δ ppm:
3.35-3.50 (4H, m), 3.98 (2H, d, J=5.2Hz), 4.59 (2H, s), 4.60-4.70 (1H, m), 6.07 (1H, t, J=5.2Hz), 6.40-6.50 (2H, m), 6.85-7.00 (2H, m)

### Reference example 32

### tert-Butyl N-methanesulfonyl-[2-(3-oxo-1,2,3,5-tetrahydrobenzo[e]-1,4-diazepin-4-yl)ethyl]carbamate

To a stirred solution of 4-(2-hydroxyethyl)-1,2,4,5-tetrahydrobenzo[e]-1,4-diazepin-3-one (0.466 g), tert-butyl N-methanesulfonylcarbamate (0.529g) and triphenylphosphine (0.888 g) in tetrahydrofuran (11 mL) was added diisopropyl azodicarbonate (40% toluene solution, 0.590 g) at room temperature. The mixture was stirred at the same temperature for 11 hours. The reaction mixture was concentrated under reduced pressure, and the obtained crude product was purified by column chromatography on silica gel (eluent:hexane-ethyl acetate) to give tert-butyl N-methanesulfonyl[2-(3-oxo-1,2,3,5-tetrahydrobenzo[e]-1,4-diazepin-4-yl)ethyl]carbamate (0.633 g).

¹H-NMR (DMSO-d₆) δ ppm:
1.45-1.50 (9H, m), 2.80-4.40 (11H, m), 7.20-7.60 (4H, m)

### Reference example 33

### N-(2-Methoxyethyl)-2-(3-oxo-1,2,3,5-tetrahydrobenzo[e]-1,4-diazepin-4-yl)acetamide

N-(2-Methoxyethyl)-2-(3-oxo-1,2,3,5-tetrahydrobenzo[e]-1,4-diazepin-4-yl) acetamide was prepared with the use of (3-oxo-1,2,3,5-tetrahydrobenzo[ e]-1,4-diazepin-4-yl)acetic acid in a similar manner to that described in Reference example 8.

¹H-NMR (CDC1₃) δ ppm:
3.25-3.40 (7H, m), 4.05-4.20 (3H, m), 4.20 (2H, d, J=5.4Hz), 4.65 (2H, s), 6.20-6.35 (1H, br), 6.55-6.60 (1H, m), 6.65-6.70 (1H, m), 6.90-6.95 (1H, m), 7.05-7.15 (1H, m)

### Reference example 34

### N-Benzyloxymethyl-N-(2-methoxyethyl)-2-(3-oxo-1,2,3,5-tetrahydrobenzo[e]-1,4-diazepin-4-yl)acetamide

N-Benzyloxymethyl-N-(2-methoxyethyl)-2-(3-oxo-1,2,3,5-tetrahydrobenzo[e]-1,4-diazepin-4-yl)acetamide was prepared with the use of N-(2-methoxyethyl)-2-(3-oxo-1,2,3,5-tetrahydrobenzo[e]-1,4-diazepin-4-yl) acetamide in a similar manner to that described in Reference example 1.

¹H-NMR (CDCl₃) δ ppm:
3.20-5.10 (17H, m), 6.50-6.60 (1H, m), 6.60-6.70 (1H, m), 6.80-6.95 (1H, m), 7.05-7.10. (1H, m), 7.20-7.40 (5H, m)

### Example 1-1

### 1-(2-Chloro-4-pyrrolidin-1-yl-benzoyl)-4-methyl-1,2,4,5-tetrahydrobenzo[e]-1,4-diazepin-3-one

A mixture of 2-chloro-4-(pyrrolidin-1-yl)benzoic acid (70.4 mg), thionyl chloride (1.0 mL) and catalytic amount of N-methylpyrrolidone was stirred at room temperature for 3 hours. The mixture was concentrated under reduced pressure, the obtained residue was dissolved in dichloromethane (1 mL) (solution B). Solution B was added dropwise to a stirred solution of 4-methyl-1,2,4,5-tetrahydrobenzo[e]-1,4-diazepin-3-one (50.0 mg) and triethylamine (0.119 mL) in dichloromethane (1.0 mL) under ice-cooling and stirring. After being stirred at room temperature overnight, to the reaction mixture was added water and then extracted with dichloromethane. The organic layer was concentrated under reduced pressure. The obtained crude product was purified by column chromatography on aminopropylsilylated silica gel (eluent: dichloromethane-hexane) to give 1-(2-chloro-4-pyrrolidin-1-yl-benzoyl)-4-methyl-1,2,4,5-tetrahydro-benzo[e]-1,4-diazepin-3-one (66.4 mg).

¹H-NMR (CDCl₃) δ ppm:
1.90-2.05 (4H, m), 3.15 (3H, s), 3.15-3.30 (4H, m), 4.15-5.35 (4H, m), 5.90-8.00 (7H, m)
MS (ESI, m/z):384(M+H)⁺

### Examples 1-2 to 1-61

The following compounds of Examples 1-2 to 1-61 were obtained with the use of the corresponding 1,2,4,5-tetrahydrobenzo[e]-1,4-diazepin-3-one derivatives and benzoic acid derivatives in a similar manner to that described in Example 1-1. The structure formula and physical data of these compounds were shown in Tables 16 to 26.

**[Table 16]**

| Example | Structure formula | Compound name | 1H-NMR (solvent) or MS(m/z) |
|---|---|---|---|
| 1-2 | 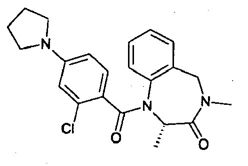 | 1-(2-chloro-4-pyrrolidin-1-ylbenzoyl)-(2S)-methyl-4-methyl-1,2,4,5-tetrahydrobenzo[e]-1,4-diazepin-3-one | 1H-NMR (CDCl3) δ ppm: 1.15-1.45 (3H, m), 1.85-2.10 (4H, m), 3.05-3.35 (8H, m), 3.80-3.95 (1H, m), 5.10-5.30 (1H, m), 5.80-7.60 (7H, m) MS(ESI, m/z): 398(M+H)+ |
| 1-3 | 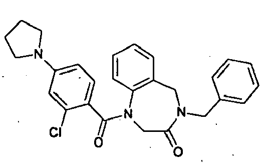 | 4-benzyl-1-(2-chloro-4-pyrrolidin-1-ylbenzoyl)-1,2,4,5-tetrahydrobenzo[e]-1,4-diazepin-3-one | 1H-NMR (CDCl3) δ ppm : 1.85-2.10 (4H, m), 3.00-3.40 (4H, m), 4.00-5.00 (6H, m), 6.00-7.55 (12H, m) MS (ESI, m/z) : 460(M+H)⁺ |
| 1-4 | 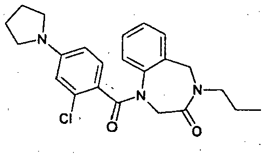 | 1-(2-chloro-4-pyrrolidin-1-ylbenzoyl)-4-propyl-1,2,4,5-tetrahydrobenzo[e]-1,4-diazepin-3-one | 1H-NMR (CDCl3) 6 ppm: 0.92 (3H, t, J=7.3Hz), 1.55-1.70 (2H, m), 1.90-2.05 (4H, m), 3.05-3.35 (4H, m), 3.50 (2H, t, J=7.3Hz), 4.05-5.55 (4H, m), 5.85-8.05 (7H, m) MS(ESI, m/z) : 412(M+H)+ |
| 1-5 | 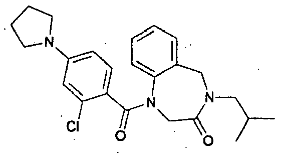 | 1-(2-chloro-4-pyrrotidin-1-ylbenzoyl)-4-isobutyl-1,2,4,5-tetrahydrobenzo[e]-1,4-diazepin-3-one | 1H-NMR (CDCl3) δ ppm: 0.91 (6H, d, J=6.6Hz), 1.85-2.15 (5H, m), 3.10-3.30 (4H, m), 3.36 (2H, d, J=7.5Hz), 3.85-5.55 (4H, m), 5.80-7.85 (7H, m) MS(ESI, m/z) : 426(M+H)+ |
| 1-6 | 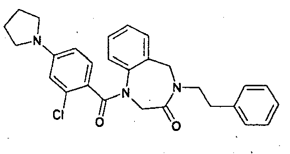 | 1-(2-chloro-4-pyrrolidin-1-ylbenzoyl)-4-phenethyl-1,2,4,5-tetrahydrobenzo[e]-1,4-diazepin-3-one | 1H-NMR(CDCI3) 6 ppm: 1.85-2.10 (4H, m), 2.91 (2H, t, J=7.3Hz), 3.10-3.35 (4H, m), 3.78 (2H, t, J=7.3Hz), 3.95-5.50 (4H, m), 5.85-7.80 (12H, m) MS(ESI, m/z) : 474(M+H)+ |
| 1-7 | 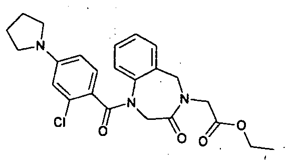 | ethyl [1-(2-chloro-4-pyrrolidin-1-ylbenzoyl)-3-oxo-1,2,3,5-tetrahydrobenzo[e]-1,4-diazepin-4-yl]acetate | 1 H-NMR (CDCl3) δ ppm: 1.27 (3H, t, J=7.4Hz), 1.90-2.10 (4H, m), 3.05-3.35 (4H, m), 4.21 (2H, q, J=7.4Hz), 4.33 (2H, s), 4.40-5.35 (4H, m), 5.85-7.80 (7H, m) MS(ESI, m/z): 456(M+H)+ |

**[Table 17]**

| Example | Structure formula | Compound name | 1H-NMR (solvent) or MS(m/z) |
|---|---|---|---|
| 1-8 | 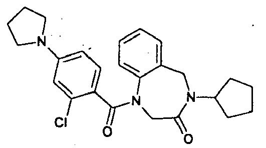 | 1-(2-chloro-4-pyrrolidin-1-ylbenzoyl)-4-cyclopentyl-1,2,4,5-tetrahydrobenzo[e]-1,4-diazepin-3-one | 1.40-2.10 (12H, m), 3.10-3.30 (4H, m), 4.00-5.00 (4H, m), 5.17 (1 H, quint, J=8.5Hz), 5.95-7.60 (7H, m) MS(ESI, m/z) : 438(M+H)+ |
| 1-9 | 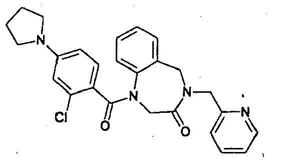 | 1-(2-chloro-4-pyrrolidin-1-ylbenzoyl)-4-pyridin-2-ylmethyl)-1,2,4,5-tetrahydrobenzo[e]-1,4-diazepin-3-one | 1 H-NMR (COC13) δ ppm: 1.85-2.10 (4H, m), 3.00-3.40 (4H, m), 4.05-5.35 (6H, m), 5.85-7.90 (10H, m), 8.55-8.65 (1H, m) MS(ESI, m/z): 461(M+H)+ |
| 1-10 | 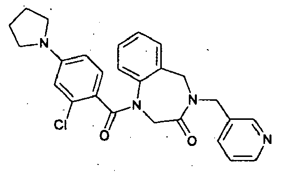 | 1-(2-chloro-4-pyrrolidin-1-ylbenzoyl)-4-pyridin-3-ylmethyl)-1,2,4,5-tetrahydrobenzo[e]-1,4-diazepin-3-one | 1H-NMR (CDCl3) δ ppm: 1.85-2.10 (4H, m), 3.00-3.40 (4H, m), 3.85-5.50 (6H, m), 5.90-7.80. (10H, m), 8.50-8.60 (1 H, m) MS(ESI, m/z): 461(M+H)+ |
| 1-11 | 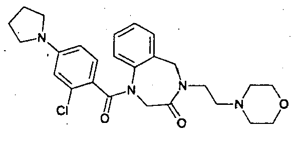 | 1-(2-chloro-4-pyrrolidin-1-ylbenzoyl)-4-(2-morpholin-4-yl-ethyl)-1,2,4,5-tetrahydrobenzo[e]-1,4-diazepin-3-one | 1H-NMR (CDCl3) δ ppm: 1.90-2.05 (4H, m), 2.45-2.60 (6H, m), 3.10-3.30 (4H, m), 3.60-3.75 (6H, m), 4.00-5.30 (4H, m), 5.85-7.75 (7H, m) MS(ESI, m/z): 483(M+H)+ |
| 1-12 | 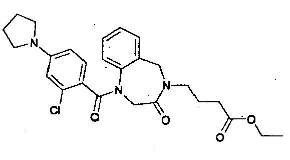 | ethyl 4-[1-(2-chloro-4-pyrrolidin-1-ylbenzoyl)-3-oxo-1,2,3,5-tetrahydrobenzo[e]-1,4-diazepin-4-yl]butylate | 1H-NMR (CDCl3) δ ppm: 1.27 (3H, t, J=7.3Hz), 1.85-2.05 (6H, m), 2.32 (2H, t, J=7.3Hz), 3.10-3.30 (4H, m), 3.58 (2H, t, J=7.3Hz), 4.15 (2H, q, J=7.3Hz), 4.20-5.00 (4H, m), 5.85-7.60 (7H, m) MS(ESI, m/z) : 484(M+H)+ |
| 1-13 | 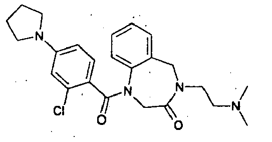 | 1-(2-chloro-4-pyrrolidin-1-ylbenzoyl)-4-(2-N,N-dimethylaminoethyl)-1,2,4,5-tetrahydrobenzo[e]-1,4-diazepin-3-one | 1 H-NMR (CDCl3) δ ppm: 1.90-2.05 (4H, m), 2.29 (6H, s), 2.49 (2H, t, J=6.6Hz), 3.10-3.35 (4H, m), 3.65 (2H, t, J=6.6Hz), 4.00-5.40 (4H, m), 5.90-7.90 (7H, m) MS(ESI, m/z) : 441(M+H)+ |

**[Table 18]**

| Example | Structure formula | Compound name | 1 H-NMR (solvent) or MS(m/z) |
|---|---|---|---|
| 1-14 | 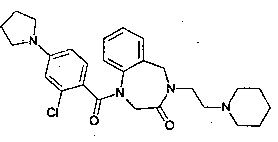 | 1-(2-chloro-4-pyrrolidin-1-ylbenzoyl)-4-(2-piperidin-1-yl-ethyl)-1,2,4,5-tetrahydrobenzo[e]-1,4-diazepin-3-one | 1H-NMR (CDCl3) δ ppm: 1.40-1.50 (2H, m), 1.55-1.65 (4H, m), 1.90-2.05 (4H, m), 2.35-2.55 (6H, m), 3.10-3.35 (4H, m), 3.67 (2H, t, J=6.6Hz), 4.00-5.45 (4H, m), 5.95-7.85 (7H, m) MS(ESI, m/z) : 481(M+H)+ |
| 1-15 | 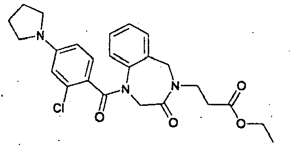 | ethyl 3-[1-(2-chloro-4-pyrrolidin-1-ylbenzoyl)-3-oxo-1,2,3,5-tetrahydrobenzo[e]-1,4-diazepin-4-yl]propionate | 1H-NMR (CDCI3) δ ppm: 1.26 (3H, t, J=7.3Hz), 1.90-2.05 (4H, m), 2.66 (2H, t, J=6.3Hz), 3.05-3.35 (4H, m), 3.75-3.85 (2H, m), 4.16 (2H, q, J=7.3Hz), 4.20-4.95 (4H, m), 6.00-7.60 (7H, m) MS(ESI, m/z) : 470(M+H)+ |
| 1-16 | 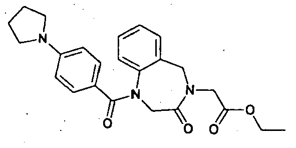 | ethyl [3-oxo-1-(4-pyrrolidin-1-ylbenzoyl)-1,2,3,5-tetrahydrobenzo[e]-1,4-diazepin-4-yl]acetate | 1 H-NMR (CDCl3) δ ppm: 1.28 (3H, t, J=7.3Hz), 1.90-2.00 (4H, m), 3.20-3.30 (4H, m), 4.22 (2H, q, J=7.3Hz), 4.37 (2H, s), 4.40-5.40 (4H, br), 6.25-6.30 (2H, m), 6.85-6.95 (1H, m), 7.15-7.30 (4H, m), 7.35-7.45 (1 H, m) MS(ESI, m/z) : 422(M+H)+ |
| 1-T7 | 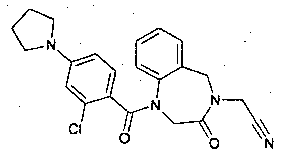 | [1-(2-chloro-4-pyrrolidin-1-ylbenzoyl)-3-oxo-1,2,3,5-tetrahydrobenzo[e]-1,4-diazepin-4-yl]acetonitrile | 1H-NMR (CDCl3) δ ppm: 1.90-2.10 (4H, m), 3.10-3.35 (4H, m), 4.10-5.25 (6H, m), 6.05-7.65 (7H, m) MS(ESI, m/z): 409(M+H)+ |
| 1-18 | 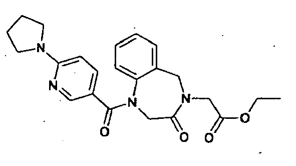 | ethyl [3-oxo-1-(6-pyrrolidin-1-ylpyridine-3-carbonyl)-1,2,3, 5-tetrahydrobenzo[e]-1,4-diazepin-4-yl]acetate | 1H-NMR (CDCl3) δ ppm: 1.28 (3H, t, J=7.2Hz), 1.90-2.05 (4H, m), 3.30-3.50 (4H, m), 3.85-5.85 (8H, m), 6.17 (1H, d, J=9.2Hz), 6.85-6.95 (1H, m), 7.20-7.30 (2H, m), 7.35-7.45 (1H, m), 7.49 (1H, dd, J=9.2, 2.2Hz), 7.96 (1H, d, J=2.2Hz) MS(ESI, m/z): 423(M+H)+ |
| 1-19 | 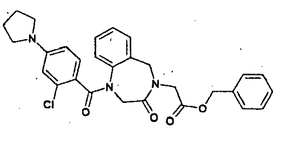 | benzyl [1-(2-chloro-4-pyrrolidin-1-ylbenzoyt)-3-oxo-1,2,3,5-tetrahydrobenzo[e]-1,4-diazepin-4-yl]acetate | 1H-NMR (CDCl3) δ ppm: 1.90-2.10 (4H, m), 3.10-3.35 (4H, m), 4.15-4.90 (6H, m), 5.10-5.25 (2H, m), 6.00-7.70 (12H, m) MS(ESI, m/z): 518(M+H)+ |

**[Table 19]**

| Example | Structure formula | Compound name | 1H-NMR (solvent) or MS(m/z) |
|---|---|---|---|
| 1-20 | 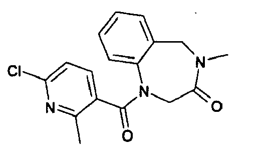 | 1-(6-chloro-2-methylpyridine-3-carbonyl)-4-methyl-1,2,3,4,5-tetrahydrobenzo[e][1,4]diaz epin-3-one | MS (ESI, m/z): 330(M+H)+ |
| 1-21 | 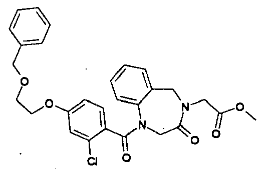 | methyl {1-[4-(2-benzyloxyethoxy)-2-chlorobenzoyl]-3-oxo-1,2,3,5-tetrahydrobenzo[e]-1,4-diazepin-4-yl}acetate | 1H-NMR (CDCl3) δ ppm: 3.65-5.70 (15H, m), 6.50-7.60 (12H, m) |
| 1-22 | 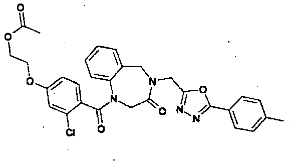 | 2-(3-chloro-4-{3-oxo-4-[5-(4-methylphenyl)-1,3,4-oxadiazol-2-ylmethyl]-2,3,4,5-tetrahydrobenzo[e]-1,4-diazepine-1-carbonyl}phenoxy)ethyl acetate | 1H-NMR (CDCl3) δ ppm: 2.00-2.20 (3H, s), 2.42 (3H, s), 4.00-5.40 (10H, m), 6.55-7.55 (9H, m), 7.86 (2H, d, J=7.6Hz) MS(ESI, m/z) : 575(M+H)+ |
| 1-23 | 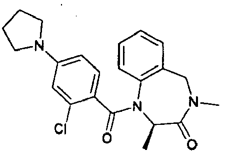 | 1-(2-chloro-4-pyrrolidin-1-ylbenzoyl)-(2R)-methyl-4-methyl-1,2,4,5-tetrahydrobenzo[e]-1,4-diazepin-3-one | 1H-NMR (CDCl3) δ ppm: 1.15-1.45 (3H, m), 1.85-2.10 (4H, m), 3.05-3.35 (8H, m), 3.80-3.95 (1H, m), 5.10-5.30 (1H, m), 5.80-7.60 (7H, m) MS(ESI, m/z) : 398(M+H)+ |
| 1-24 | 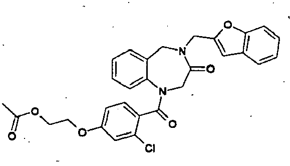 | 2-[4-(4-benzofuran-2-ylmethyl-3-oxo-2,3,4,5-tetrahydrobenzo[e]-1,4- , diazepine-1-carbonyl)-3-chlorophenoxy]ethyl acetate | 1H-NMR (CDCI3) δ ppm: 2.00-2.20 (3H, m), 3.95-5.60 (10H, m), 6.50-7.65 (12H; m) MS(ESI, m/z): 533(M+H)+ |
| 1-25 | 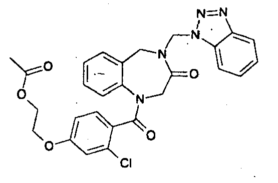 | 2-[4-(4-benzotriazole-1-ylmethyl-3-oxo-2,3,4,5-tetrahydrobenzo[e]-1,4-diazepine-1-carbonyl)-3-chloro-phenoxy]ethyl acetate | 1 H-NMR (CDCI3) δ ppm: 1.95-2.20 (3H, m), 4.00-5.50 (10H, m), 6.25-7.90 (11H, m) MS(ESI, m/z) : 534(M+H)+ |

**[Table 20]**

| Example | Structure formula | Compound name | 1 H-NMR (solvent) or MS(m/z) |
|---|---|---|---|
| 1-26 | 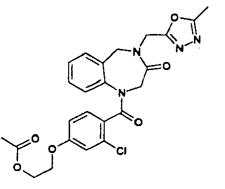 | 2-(3-chloro-4-[4-(5-methyl-1,3,4-oxadiazol-2-ylmethyl)-3-oxo-2,3,4,5-tetrahydrobenzo[e]-1,4-diazepine-1-carbonyl]phenoxy}ethyl acetate | 1 H-NMR (CDCl3) δ ppm: 2.00-2.20 (3H, m), 2.48 (3H, m), 3.40-5.60 (10H, m), 6.50-7.60 (7H, m) MS(ESI, m/z): 499(M+H)+ |
| 1-27 | 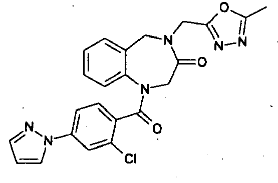 | 1-[2-chloro-4-(pyrazol-1-yl)benzoyl]-4-(5-methyl-1,3,4-oxadiazol-2-ylmethyl)-1,2.4,5-tetrahydrobenzo[e]-1,4-diazepin-3-one | MS (ESI, m/z): 463(M+H)+ |
| 1-28 | 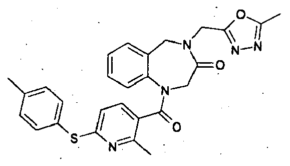 | 4-(5-methyl-1,3,4-oxadiazol-2-ylmethyl)-1-(2-methyl-6-(4-tolytsulfanyl)pyridine-3-carbonyl)-1,2,4,5-tetrahydrobenzo[e]-1,4-diazepin-3-one | MS(ESI, m/z) : 500(M+H)+ |
| 1-29 | 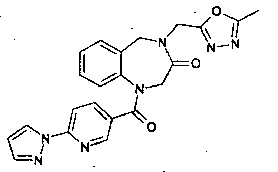 | 4-(5-methyl-1,3,4-oxadiazol-2-ylmethyl)-1-[6-(pyrazol-1-yl)pyridine-3-carbonyl]-1,2,4,5-tetrahydrobenzo[e]-1,4-diazepin-3-one | MS(ESI, m/z): 430(M+H)+ |
| 1-30 | 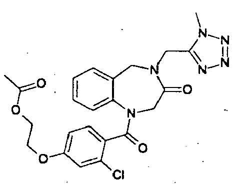 | 2-{3-chloro-4-[4-(1-methyl-1 H-tetrazol-5-ylmethyl)-3-oxo-2,3,4,5-tetrahydrobenzo[e]-1,4-diazepine-1-carbonyl]phenoxy}ethyl acetate | I H-NMR (CDCl3) δ ppm: 1.95-2.20 (3H, m), 3.85-5.20 (13H, m), 6.55-7.55 (7H, m) MS(ESt.m/z):499(M+H)+ |
| 1-31 | 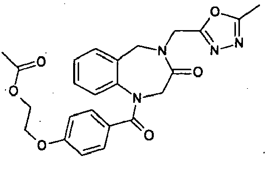 | 2-{4-[4-(5-methyl-1,3,4- oxadiazol-2-ylmethyl)-3-oxo-2,3,4,5-tetrahydrobenzo[e]-1,4-diazepine-1-carbonyl]phenoxy}ethyl acetate | 1H-NMR (CDCI3) δ ppm: 2.07 (3H, s), 2.48 (3H, s), 4.05-5.10 (10H, m), 6.65-6.85 (3H, m), 7.10-7.25 (5H, m) |

**[Table 21]**

| Example | Structure formula | Compound name | 1H-NMR (solvent) or MS(m/z) |
|---|---|---|---|
| 1-32 | 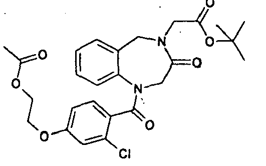 | tert-butyl {1-[4-(2-acetoxyethoxy)-2-chloro-benzoyl]-3-oxo-1,2,3,5-tetrahydrobenzo[e]-1,4-diazepin-4-yl}acetate | 1H-NMR (CDCl3) δ ppm: 1.47 (9H, s), 2.05-2.20 (3H, m), 4.00-5.00 (10H, m), 6.50-7.60 (7H, m) MS(ESI, m/z) : 517(M+H)+ |
| 1-33 | 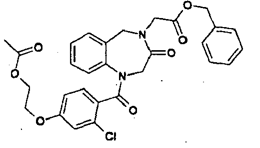 | benzyl {1-[4-(2-acetoxyethoxy)-2-chlorobenzoyl]-3-oxo-1,2,3,5-tetrahydrobenzo[e]-1,4-diazepin-4-yl}acetate | MS(ESI, m/z) : 551(M+H)+ |
| 1-34 | 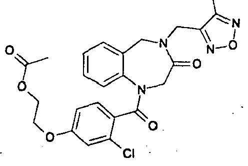 | 2-{3-chloro-4-[4-(4-methylfurazan-3-ylmethyl)-3-oxo-2,3,4,5-tetrahydrobenzo[e]-1,4-diazepine-1-carbonyl]phenoxy}ethyl acetate | 1H-NMR (CD3OD) δ ppm: 2.00-2.20 (3H, m), 2.22 (3H, s), 4.00-5.50 (10H, m), 6.50-7.60 (7H, m) MS(ESI, m/z): 499(M+H)+ |
| 1-35 | 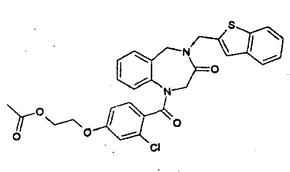 | 2-[4-(4-benzo[b]thiophen-2-ylmethyl-3-oxo-2,3,4,5-tetrahydrobenzo[e]-1,4-diazepine-1-carbonyl)-3-chlorophenoxy]ethyl acetate | 1H-NMR ,(CDCI3) δ ppm: 2.00-2.20 (3H, m), 4.00-5.50 (10H, m), 6.50-7.60 (10H, m), 7.70-7.75 (1H, m), 7.78 (1 H, d, J=7.8Hz) MS(ESI, m/z) : 549(M+H)+ |
| 1-36 | 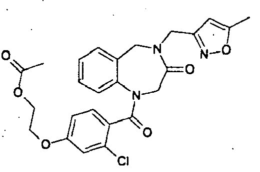 | 2-{3-chloro-4-[4-(5-methyl-isoxazole-3-ylmethyl)-3-oxo-2,3,4,5-tetrahydrobenzo[e]-1,4-diazepine-1-carbonyl]phenoxy}ethyl acetate | 1H-NMR (CD30D) δ ppm: 2.00-2.20 (3H, m), 2.39 (3H, s), 4.00-5.40 (10H, m), 5.80-6.00 (1H, m), 6.50-7.60 (7H, m) MS(ESI, m/z) : 498(M+H)+ |
| 1-37 | 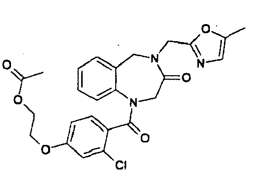 | 2-{3-chloro-4-[4-(5-methyl-oxazole-2-ylmethyl)-3-oxo-2,3,4,5-tetrahydrobenzo[e]-1,4-diazepine-1-carbonyl]phenoxy}ethyl acetate | 1H-NMR (CD30D) δ ppm: 2.00-2.20 (3H, m), 2.26 (3H, s), 3.95-5.40 (10H, m), 6.50-7.60 (8H, m) |

**[Table 22]**

| Example | Structure formula | Compound name | 1 H-NMR (solvent) or MS(m/z) |
|---|---|---|---|
| 1-38 | 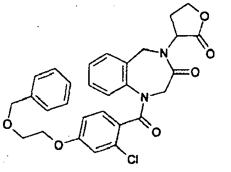 | 1-[4-(2-benzyloxyethoxy)-2-chlorobenzoyl]-4-(2-oxotetrahydrofuran-3-yl)-1,2,4,5-tetrahydrobenzo[e]-1,4-diazepin-3-one | MS(ESI, m/z) : 535(M+H)+ |
| 1-39 | 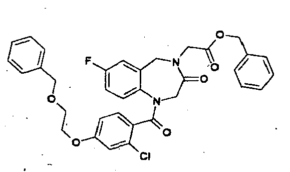 | benzyl {1-[4-(2-benzyloxyethoxy)-2-chlorobenzoyl]-7-fluoro-3-oxo-1,2,3,5-tetrahydrobenzo[e]-1,4-diazepin-4-yl}acetate | 1H-NMR (CDCl3) δ ppm : 3.70-6.20 (14H, m), 6.50-7.60 (16H, m) |
| 1-40 | 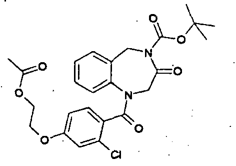 | tert-butyl 1-[4-(2-acetoxy-ethoxy)-2-chloro-benzoyl]-3-oxo-1,2,3,5-tetrahydrobenzo[e]-1,4-diazepine-4-carboxylate | 1H-NMR (CDCl3) δ ppm : 1.54 (9H, s), 2.05-2.10 (3H, m), 4.00-4.50 (8H, m), 6.50-7.65 (7H, m) |
| 1-41 | 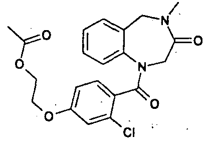 | 2-[3-chloro-4-(4-methyl-3-oxo-2,3,4,5-tetrahydro-benzo[e][1,4]diazepine-1-carbonyl)-phenoxy}-ethyl acetate | 1H-NMR (CD3OD) δ ppm: 2.00-2.20 (3H, m), 3.15 (3H, m), 4.00-5.50 (8H, m), 6.50-7.65 (7H, m) |
| 1-42 | 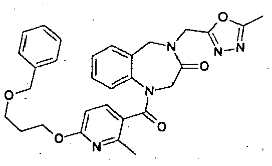 | 1-[6-(3-benzyloxy-propoxy)-2-methyl-pyridine-3-carbonyl]-4-(5-methyl-[1,3,4]oxadiazol-2-ylmethyl)-1,2,4,5-tetrahydro-benzo[e][1,4]diazepin-3-one | MS(ESI, m/z): 542(M+H)+ |
| 1-43 | 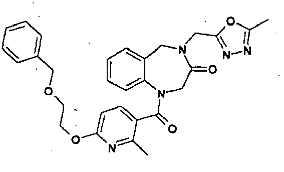 | 1-[6-(2-benzyloxy-ethoxy)-2-methyl-pyridine-3-carbonyl]-4-(5-methyl-[1,3,4]oxadiazol-2-ylmethyl)-1,2,4,5-tetrahydro-benzo[e][1,4]diazepin-3-one | MS(ESI, m/z): 528(M+H)+ |

**[Table 23]**

| Example | Structure formula | Compound name | 1H-NMR (solvent) or MS(m/z) |
|---|---|---|---|
| 1-44 | 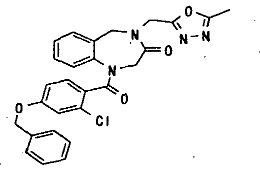 | 1-(4-benzyloxy-2-chlorobenzoyl)-4-(5-methyl-1,3,4-oxadiazol-2-ylmethyl)-1,2,4,5-tetrahydrobenzo[e]-1,4-diazepin-3-one | 1H-NMR (CDCl3) δ ppm : 2.47 (3H, s), 3.80-5.80 (8H, m), 6.55-7.70 (12H, m) |
| 1-45 | 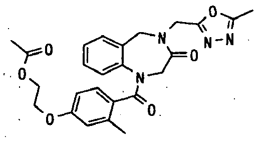 | 2-{3-methyl-4-[4-(5-methyl-1,3,4-oxadiazol-2-ylmethyl)-3-oxo-2, 3,4, 5-tetrahydrobenzo[e]-1,4-diazepine-1-carbonyl]phenoxy}ethyl acetate | 1H-NMR (CDCl3) δ ppm : 2.07 (3H, s), 2.43 (3H, s), 2.48 (3H, s), 4.00-5.10 (10H, m), 6.20-7.70 (7H, m) MS(ESI, m/z): 479 (M+H)+ |
| 1-46 | 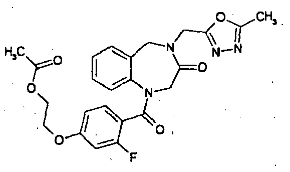 | 2-{3-fluoro-4-[4-(5-methyl- 1,3,4-oxadiazol-2-ylmethyl)-3-oxo-2,3,4,5-tetrahydrobenzo[e]-1,4-diazepine-1-carbonyl]phenoxy}ethyl acetate | 1H-NMR (CDCl3) δ ppm: 2.07 (3H, s), 2.48 (3H, s), 3.50-5.50 (10H, m), 6.10-8.10 (7H, m) MS(ESI, m/z) : 483 (M+H)+ |
| 1-47 | 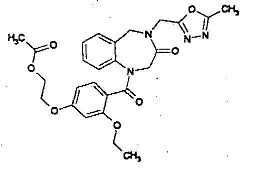 | 2-{3-ethoxy-4-[4-(5-methyl- 1,3,4-oxadiazol-2-ylmethyl)-3-oxo-2,3,4,5- tetrahydrobenzo[e]-1,4-diazepine-1-carbonyl]phenoxy}ethyl acetate | ` 1 H-NMR (CDCl3) δ ppm : 1.10-1.50 (3H, m), 2.00-2.20 (3H, m), 2.46 (3H, s), 3.50-6.00 (12H, m), 6.05-7.80 (7H, m) MS(ESI, m/z) : 509 (M+H)+ |
| 1-48 | 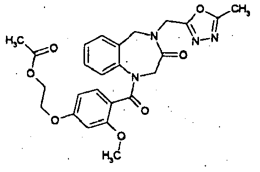 | 2-{3-methoxy-4-[4-(5- methyl-1,3,4-oxadiazol-2-ylmethyl)-3-oxo-2,3,4,5-tetrahydrobenzo[e]-1,4-diazepine-1-carbonyl]phenoxy}ethyl acetate | 1H-NMR (CDCl3) δ ppm : 2.00-2.15 (3H, m), 2.47 (3H, s), 3.30-5.50 (13H, m), 6.00-7.70 (7H, m) MS(ESI, m/z): 495(M+H)+ |
| 1-49 | 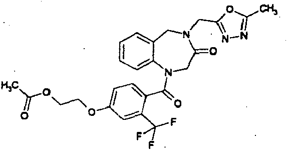 | 2-{4-[4-(5-methyl-1,3,4-oxadiazol-2-ylmethyl)-3-oxo-2,3,4,5-tetrahydrobenzo[e]-1,4-diazepine-1-carbonyl]-3-trifluoromethylphenoxy}ethyl acetate | 1H-NMR (CDCl3) δ ppm : 2.00-2.15 (3H, m), 2.45-2.55 (3H, m), 4.05-5.05 (10H, m), 6.50-7.60 (7H, m) MS(ESI, m/z): 533(M+H)+ |

**[Table 24]**

| Example | Structure formula | Compound name | 1H-NMR (solvent) or MS(m/z) |
|---|---|---|---|
| 1-50 | 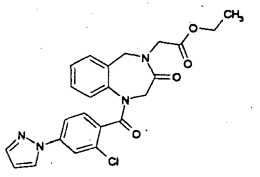 | ethyl [1-(2-chloro-4-pyrazol-1-ylbenzoyl)-3-oxo-1,2,3,5-tetrahydrobenzo[e]-1,4-diazepin-4-yl]acetate | 1H-NMR (CDCI3) δ ppm : 1.15-1.35 (3H, m),3.20-5.70 (8H, m), 6.35-8.10 (10H, m) |
| 1-51 | 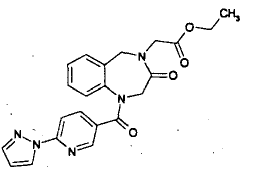 | ethyl [3-oxo-1-(6-pyrazol-1-ylpyridine-3-carbonyl)-1,2,3,5-tetrahydrobenzo[e]-1,4-diazepin-4-yl]acetate | MS(ESI, m/z) : 420(M+H)+ |
| 1-52 | 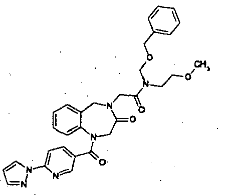 | N-benzyloxymethyl-N-(2-methoxyethyl)-2-[3-oxo-1-(6-pyrazol-1-ylpyridine-3-carbonyl)-1,2,3,5-tetrahydrobenzo[e]-1,4-diazepin-4-yl]acetamide | 1 H-NMR (CDCl3) δ ppm : 3.20-3.70 (8H, m), 3.80-6.00 (9H, m), 6.40-8.60 (15H, m) |
| 1-53 | 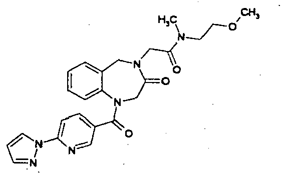 | N-(2-methoxyethyl)-N-methyl-2-[3-oxo-1-(6-pyrazol-1-ylpyridine-3-carbonyl)-1,2,3,5-tetrahydrobenzo[e]-1,4-diazepin-4-yl]acetamide | 1H-NMR (CDCl3) δ ppm : 2.90-6.00 (16H, m), 6.40-8.50 (10H, m) |
| 1-54 | 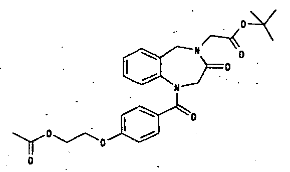 | tert-butyl {1-[4-(2-acetoxyethoxy)benzoyl]-3-oxo-1,2,3,5-tetrahydrobenzo[e]-1,4-diazepin-4-yl}acetate | 1H-NMR (CDCl3) δ ppm : 1.40-1.50 (9H, m), 2.08 (3H, s), 3.20-6.20 (10H, m), 6.50-7.50 (8H, m) |
| 1-55 | 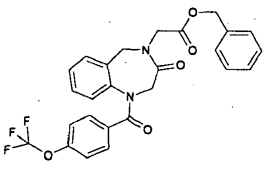 | benzyl [3-oxo-1-(4-trifluoromethoxybenzoyl)-1,2,3,5-tetrahydrobenzo[e]-1,4-diazepin-4-yl}acetate | 1 H-NMR (CDCl3) δ ppm : 3.30-6.20 (8H, m), 6.50-8.00 (13H, m) |

**[Table 25]**

| Example | Structure formula | Compound name | 1 H-NMR (solvent) or MS(m/z) |
|---|---|---|---|
| 1-56 | 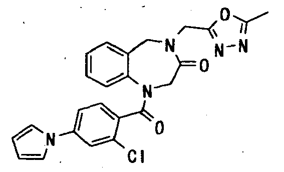 | 4-(5-methyl-1,3,4-oxadiazol-2-ylmethyl)-1-(4-pyrrol-1-ylbenzoyl)-1,2,4,5-tetrahydrobenzo[e]-1,4-diazepin-3-one | 1 H-NMR (CDCl3) δ ppm : 2.45-2.55 (3H, m), 3.50-5.60 (6H, m), 6.10-7.80 (11 H, m) MS(ESI, m/z): 462 (M+H)+ |
| 1-57 | 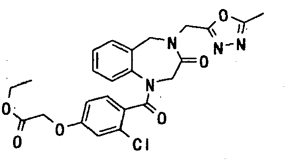 | 1-(2-chloro-4-pyrrole-1-ylbenzoyl)-4-(5-methyl-1,3,4-oxadiazol-2-ylmethyl)-1,2,4.5-tetrahydrobenzo[e]-1,4-diazepin-3-one | 1 H-NMR (CDCl3) δ ppm: 1.15-1.40 (3H, m), 2.48 (3H, s), 4.00-5.60 (10H. m), 6.50-7.60 (7H, m) MS(ESI, m/z): 499 (M+H)+ |
| 1-58 | 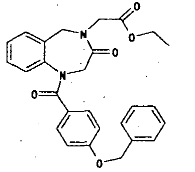 | ethyl [1-(4-benzyloxybenzoyl)-3-oxo-1,2,3,5-tetrahydrobenzo[e]-1,4-diazepin-4-yl]acetate | 1H-NMR (DMSO-d6) δ ppm: 1.18 (3H, t, J=7.1 Hz), 2.70-5.50 (10H, m), 6.85-7.55 (13H, m) |
| 1-59 | 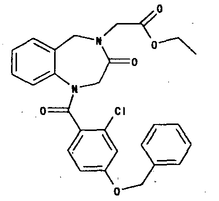 | ethyl [1-(4-benzyloxy-2-chlorobenzoyl)-3-oxo-1,2,3,5-tetrahydrobenzo[e]-1,4-diazepin-4-yl]acetate | 1 H-NMR (CDCI3) δ ppm : 1.28 (3H, t, J=7.2Hz), 4.00-5.60 (10H, m), 6.50-7.70 (12H, m) |

**[Table 26]**

| Example | Structure formula | Compound name | 1H-NMR (solvent) or MS(m/z) |
|---|---|---|---|
| 1-60 | 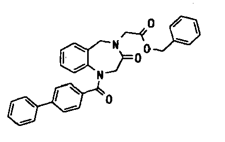 | benzyl [1-(biphenyl-4-carbonyl)-3-oxo-1,2,3,5-tetrahydrobenzo[e]-1,4-diazepin-4-yl]acetate | 1H-NMR (CDCl3) δ ppm: 3.30-6.20 (8H, m), 6.75-7.80 (18H, m) |
| 1-61 | 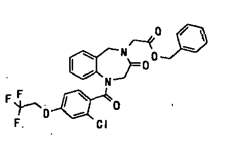 | benzyl {1-[2-chloro-4-(2,2,2-trifluoroethoxy)-benzoyl]-3-oxo-1,2,3,5-tetrahydrobenzo[e]-1,4-diazepin-4-yl}acetate . | 1 H-NMR (CDCl3) δ ppm: 4.10-5.70 (10H, m), 6.55-7.70 (12H, m) |

### Example 2-1

### 1-[2-Chloro-4-(2-hydroxyethoxy)benzoyl]-4-(5-isopropyl-1,3,4-oxadiazol-2-ylmethyl)-1,2,4,5-tetrahydrobenzo[e]-1,4-diazepin-3-one

To a stirred solution of 2-{3-chloro-4-[4-(5-isopropyl-1,3,4-oxadiazol-2-ylmethyl)-3-oxo-2,3,4,5-tetrahydrobenzo[e]-1,4-diazepine-1-carbonyl]-phenoxy}ethyl acetate (52.1 mg) in methanol (1.5mL) was added 5 mol/L aqueous solution of sodium hydroxide (0.0595 mL) at room temperature, the reaction mixture was stirred at room temperature for an hour. Two mol/L hydrochloric acid (0.148 mL) was added to the reaction mixture, and the mixture was concentrated under reduced pressure. The residue was purified by column chromatography on silica gel (eluent: ethyl acetate-methanol) to give 1-[2-chl.oro-4- (2-hydroxyethoxy)benzoyl]-4-(5-isopropyl-1,3,4-oxadiazol-2-ylmethyl)-1,2,4,5-tetrahydrobenzo[e]-1,4-diazepin-3-one (28.9 mg).

¹H-NMR (CDC1₃) δ ppm:
1.25 (6H, d, J=6.8Hz), 1.85-2.00 (1H, br), 2.95-3.15 (1H, m), 3.70-5. 40 (10H,m), 6.45-7.50 (7H, m)
MS(ESI, m/z):485(M+H)⁺

### Examples 2-2 to 2-51

The following compounds of Examples 2-2 to 2-51 were obtained with the use of the corresponding materials in a similar manner to that described in Example 2-1. The structure formula and physical data of these compounds were shown in Tables 27 to 39.

**[Table 27]**

| Example | Structure formula | Compound name | 1 H-NMR (solvent) or MS(m/z) |
|---|---|---|---|
| 2-2 | 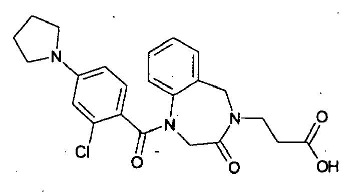 | [3-[1-(2-chloro-4-pyrrolidin-1-ylbenzoyl) -3-oxo-1,2,3,5-tetrahydrobenzo[e]-1,4-diazepin-4-yl]propionic acid | 1 H-NMR (CDCl3) δ ppm: 1.90-2.05 (4H, m), 2.73 (2H, t, J=6.3Hz), 3.10-3.30 (4H, m), 3.81 (2H, t, J=6.3Hz), 4.05-5.25 (4H, m), 6.00-7.80 (7H, m) MS(ESI, m/z) : 442(M+H)+ |
| 2-3 | 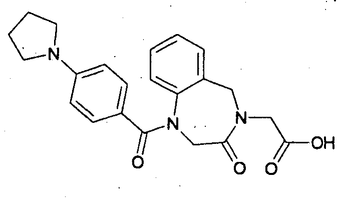 | (3-oxo-.1 -(4-pyrrolidin-1-ylbenzoyl)-1,2,3,5-tetrahydrobenzo[e]-1,4-diazepin-4-yl]acetic acid | 1 H-NMR (DMSO-d6) δ ppm: 1.85-1.95 (4H, m), 3.10-3.20 (4H, m), 4.25 (2H, s), 4.35-4.90 (4H, brm), 6.25-6.35 (2H, m), 6.85-6.90 (1H, m), 7.05-7.10 (2H, m), 7.20-7.30 (2H, m), 7.45-7.55 (1H, m), 12.75 (1H, brs) MS(ESI, m/z) : 394(M+H)+ |
| 2-4 | 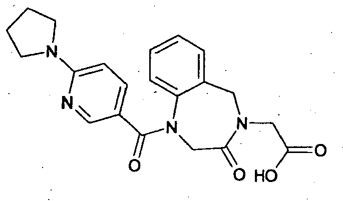 | [3-oxo-1-(6-pyrrolidin-1-ylpyridine-3-carbonyl)-1,2,3, 5-tetrahydrobenzo[e]-1,4-diazepin-4-yl]acetic acid | 1H-NMR (DMSO-d6) δ ppm: 1.75-2.00 (4H, m), 3.20-3.45 (4H, m), 3.90-5.45 (6H, m), 6.20-6.30 (1H, m), 6.90-7.05 (1H, m), 7.20-7.40 (3H, m), 7.45-7.60 (1H, m), 7.85-7.95 (1H, m), 12.75 (1H, brs) MS(ESI, m/z): 395(M+H)+ |
| 2-5 | 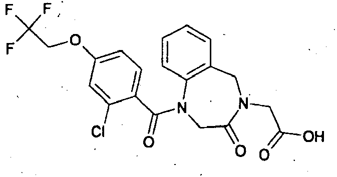 | 1-[2-chloro-4-(2,2,2-trifluoroethoxy)-benzoyl]-3-oxo-1,2,3,5-tetrahydrobenzo[e]-1,4-diazepin-4-yl acetic acid | 1 H-NMR (DMSO-d6) δ ppm: 3.95-5.50 (8H, m), 6.60-7.75 (7H, m), 12.83 (1H, brs) MS(ESI, m/z): 447(M+H)+ |
| 2-6 | 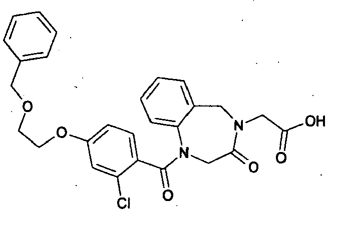 | {1-[4-(2-benzyloxy-ethoxy)-2-chloro-benzoyl]-3-oxo-1,2,3,5-tetrahydrobenzo[e]-1,4-diazepin-4-yl}acetic acid | 1H-NMR (CDCl3) δ ppm: 2.10-5.50 (12H, m), 6.50-7.65 (12H, m) MS(ESI, m/z): 509(M+H)+ |

**[Table 28]**

| Example | Structure formula a | Compound name | 1 H-NMR (solvent) or MS(m/z) |
|---|---|---|---|
| 2-7 | 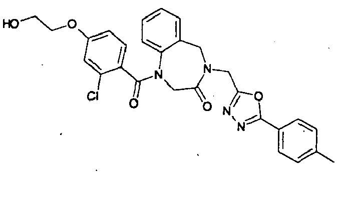 | 1-[2-chloro-4-(2-hydroxyethoxy)benzoyl]-4-[5-(4-methylphenyl)-1,3,4-oxadiazol-2-ylmethyl]-1,2,4,5-tetrahydrobenzo[e]-1,4-diazepin-3-one | 1H-NMR (CDCl3) δ ppm: 1.90-2.00 (1 H, br), 2.42 (3H, s),3.85-5.65 (10H, m), 6.55-7.60 (9H, m), 7.86 (2H, d, J=7.5Hz) MS(ESI, m/z) : 533(M+H)+ |
| 2-8 | 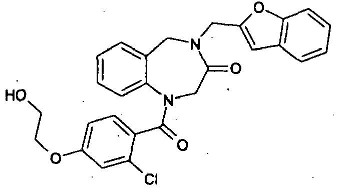 | 4-benzofuran-2-ylmethyl-1-[2-chloro-4-(2-hydroxyethoxy)-benzoyl]-1,2,4,5-tetrahydrobenzo[e]-1,4-diazepin-3-one | 1H-NMR (CDCI3) δ ppm: 80776_10 -1.80-2.05 (1H, m), 3.80-5.60 (10H, m), 6.50-7.60 (12H, m) MS(ESI, m/z): 491.2(M+H)+ |
| 2-9 | 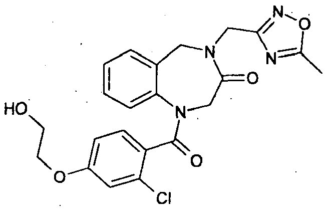 | 1-[2-chloro-4-(2-hydroxyethoxy)benzoyl]-4-(5-m ethyl-1,2,4-oxadiazol-3-ylmethyl)-1,2,4,5-tetrahydrobenzo[e]-1,4-diazepin-3-one | 1H-NMR (CDCI3) δ ppm: 2.00-2.25 (1H, br), 2.59 (3H, s); 3.85-5.80 (10H, m), 6.50-7.80 (7H, m) MS(ESI, m/z): 457.1(M+H)+ |
| 2-10 | 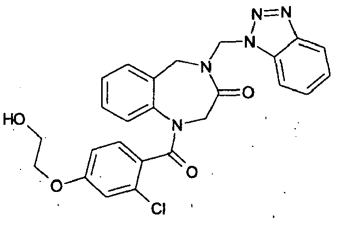 | 4-benzotriazole-1-ylmethyl-1-[2-chloro-4-(2-hydroxyethoxy)-benzoyl]-1,2,4,5-tetrahydrobenzo[e]-1,4-diazepin-3-one | 1H-NMR (CDCl3) δ ppm: 2.10-2.35 (1H, m), 3.80-6.00 (8H, m), 6.25-7.85 (12H, m), 7.95-8.15 (1H, m) MS(ESI, m/z): 492(M+H)+ |

**[Table 29]**

| Example | Structure formula | Compound name | 1 H-NMR (solvent) or MS(m/z) |
|---|---|---|---|
| 2-11 | 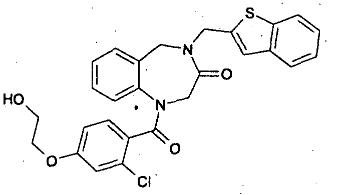 | 4-benzo[b]thiophen-2-ylmethyl-1-[2-chloro-4-(2-hydroxyethoxy)-benzoyl]-1,2,4,5-tetrahydrobenzo[e]-1,4-diazepin-3-one | 1 H-NMR (CDCl3) δ ppm: 1.85-2.05 (1H, br), 3.85-5.50 (10H, m), 6.50-7.60 (10H, m), 7.70-7.85 (2H, m) MS(ESI, m/z): 507(M+H)+ |
| 2-12 | 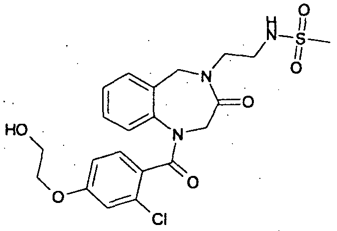 | N-(-2-{1-[2-chloro-4-(2-hydroxyethoxy)benzoyl]-3-oxo-1,2,3,5-tetrahydrobenzo[e]-1,4-diazepin-4-yl}ethyl)methanesulfona mide | 1H-NMR (CDCl3) δ ppm: 2.90-2.95 (3H, m), 3.30-3.45 (2H, m), 3.55-5.50 (11H, m), 6.50-7.65 (7H, m) |
| 2-13 | 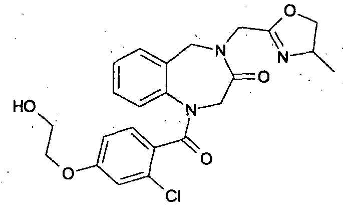 | 1-[2-chloro-4-(2-hydroxyethoxy)benzoyl]-4-(4-m ethyl-4, 5-dihydroxazole-2-ylmethyl)-1,2,4,5-tetrahydrobenzo[e]-1,4-diazepin-3-one | 1 H-NMR (CDCl3) δ ppm: 1.31 (3H, d, J=6.6Hz), 1.90-2.05 (1H, br), 3.40-5.40 (13H, m), 6.50-7.60 (7H, m) MS(ESI, m/z): 457(M)+ |
| 2-14 | 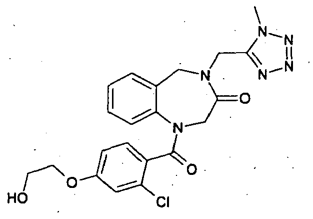 | 1-[2-chloro-4-(2-hydroxyethoxy)benzoyl]-4-(1-methyl-1H-tetrazdle-5-ylmethyl)-1,2,4,5-tetrahydrobenzo[e]-1,4-diazepin-3-one | 1 H-NMR (CDCl3) δ ppm: 2.10-2.40 (1H, m), 3.80-5.40 (13H, m), 6.50-7.60 (7H, m) MS(ESI, m/z) : 457(M+H)+ |
| 2-15 | 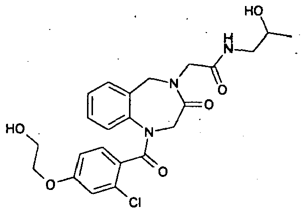 | 2-{1-[2-chloro-4-(2-hydroxyethoxy)benzoyl]-3-oxo-1,2,3,5-tetrahydrobenzo[e]-1,4-diazepin-4-yl}-N-(2-hydroxypropyl)acetamid e | 1H-NMR (CD30D) δ ppm: 1. 15 (3H, d, J=6.3Hz), 3.00-5.20 (13H, m), 6.60-7.80 (7H, m) MS(ESI, m/z): 476(M+H)+ |

**[Table 30]**

| Example | Structure formula | Compound name | 1 H-NMR (solvent) or MS (m/z) |
|---|---|---|---|
| 2-16 | 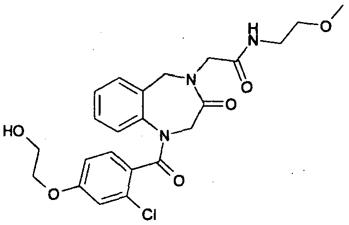 | 2-{1-[2-chloro-4-(2-hydroxyethoxy)benzoyl]-3-oxo-1,2,3,5-tetrahydrobenzo[e]-1,4-diazepin-4-yl}-N-(2-methoxyethyl)acetamide | 1H-NMR (CD3OD) δ ppm: 1.90-2.05 (1H, br), 3.29 (3H, s), 3.35-3.45 (4H, m), 3.85-5.50 (10H, m), 6.15-7.60 (7H, m) MS(ESI, m/z): 476.0(M+H)+ |
| 2-17 | 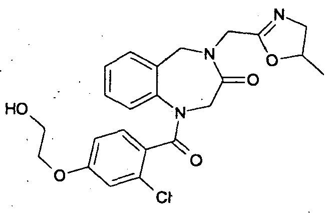 | 1-[2-chloro-4-(2-hydroxyethoxy)benzoyl]-4-(5-methyl-4,5-dihydroxazole-2-ylmethyl)-1,2,4,5-tetrahydrobenzo[e]-1,4-diazepin-3-one | 1H-NMR (CD3OD) δ ppm: 1.10-1.35 (3H, m), 1.90-2.10 (1 H, br), 3.40-5.40 (13H, m), 6.50-7.60 (7H, m) MS(ESI, m/z): 458.0(M+H)+ |
| 2-18 | 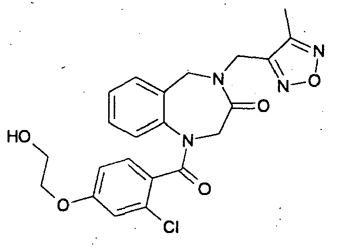 | 1-[2-chloro-4-(2-hydroxyethoxy)benzoyl]-4-(4-methylfurazan-3-ylmethyl)-1,2,4,5-tetrahydrobenzo[e]-1,4-diazepin-3-one | 1H-NMR (CD3OD) δ ppm: 1.85-1.95 (1H, br), 2.23 (3H, s), 3.20-5.20 (10H, m), 6.55-7.65 (7H, m) |
| 2-19 | 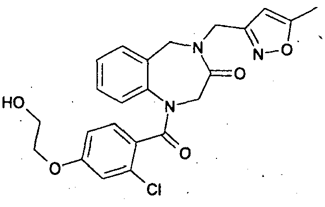 | 1-[2-chloro-4-(2-hydroxyethoxy)benzoyl]-4-(5-methylisoxazole-3-ylmethyl)-1,2,4,5-tetrahydrobenzo[e]-1,4-diazepin-3-one | 1H-NMR (CD3OD) δ ppm: 1.90-2.05 (3H, br), 2.39 (3H, s), 3.85-5.40 (10H, m); 5.85-6.05 (1H, m), 6.55-7.65 (7H, m) MS(ESI, m/z) : 456(M+H)+ |

**[Table 31]**

| Example | Structure formula | Compound name | 1 H-NMR (solvent) or MS(m/z) |
|---|---|---|---|
| 2-20 | 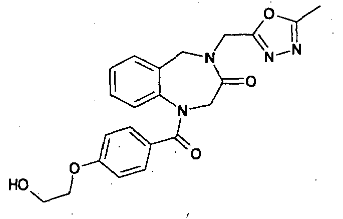 | 1-[4-(2-hydroxyethoxy)-benzoyl]-4-(5-m ethyl-1,3,4,-oxadiazol-2-ylmethyl)-1,2,4,5-tetrahydrobenzo[e]-1,4-diazepin-3-one | 1 H-NMR (CDCl3) δ ppm: 1.98 (1H, t, J=6.3Hz), 2.48 (3H, s), 3.85-5.85 (10H, m), 6.65-6.85 (3H, m), 7.10-7.25 (5H, m) MS(ESI, m/z): 423(M+H)+ |
| 2-21 | 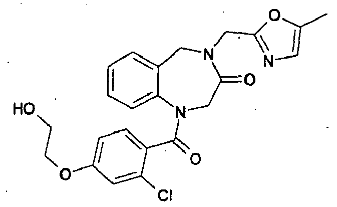 | 1-[2-chloro-4-(2-' hydroxyethoxy)benzoyl]-4-(5-methyloxazole-2-ylmethyl)-1,2,4,5-tetrahydrobenzo[e]-1,4-diazepin-3-one | 1H-NMR (CD3OD) δ ppm: 2.26 (3H, s), 3.80-6.00 (10H, m), 6.50-7.60 (8H, m) MS(ESI, m/z): 456(M+H)+ |
| 2-22 | 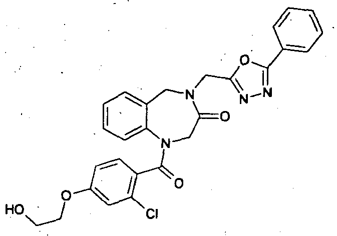 | 1-[2-chloro-4-(2-hydroxyethoxy)benzoyl]-4-(5-phenyl-1,3,4-oxadiazol-2-ylmethyl)-1,2,4,5-tetrahydrobenzo[e]-1,4-diazepin-3-one | MS(ESI, m/z): 519(M+H)+ |

**[Table 32]**

| Example | Structure formula | Compound name | 1 H-NMR (solvent) or MS(m/z) |
|---|---|---|---|
| 2-23 | 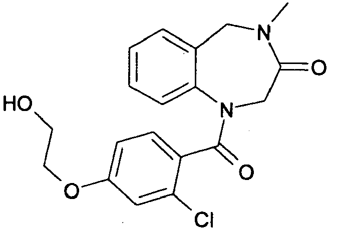 | 1-[2-chloro-4-(2-hydroxyethoxy)benzoyl]-4-methyl-1,2,4,5-tetrahydrobenzo[e]-1,4-diazepin-3-one | 1 H-NMR (CD3OD) δ ppm: 2.05-2.30 (1H, br), 3.15 (3H, s), 3.80-5.50 (8H, m), 6.50-7.65 (7H, m) MS(ESI, m/z) : 375(M+H)+ |
| 2-24 | 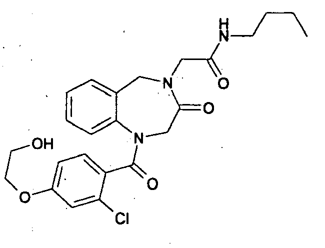 | N-butyl-2-{1-[2-chloro-4-(2-hydroxyethoxy)-benzoyl]-3-oxo-1,2,3,5-tetrahydrobenzo[e]-1,4-diazepin-4-yl}acetamide | 1H-NMR (CDCl3) δ ppm: 0.85 (3H, t, J=7.3Hz), 1.15-1.30 (2H, m), 1.35-1.45 (2H, m), 2.20-2.60 (1H, m), 3.10-3.25 (2H, m), 3.80-5.70 (10H, m), 5.95-7.80 (8H, m) MS(ESI, m/z): 474(M+H)+ |
| 2-25 | 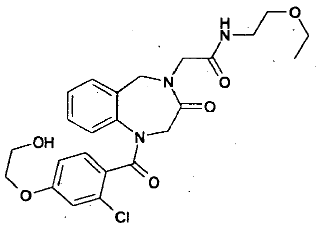 | 2-{1-[2-chloro-4-(2-hydroxyethoxy)benzoyl]-3-oxo-1,2,3,5-tetrahydrobenzo[e]-1,4-diazepin-4-yll-N-(2-ethoxyethyl)acetamide | 1H-NMR (CDCl3) δ ppm: 1.13 (3H, t, J=6.9Hz), 2.20-2.50 (1H, m), 3.35-3.50 (6H, m), 3.80-5.95 (10H, m), 6.20-7.75 (8H, m) MS(ESI, m/z): 490(M+H)+ |
| 2-26 | 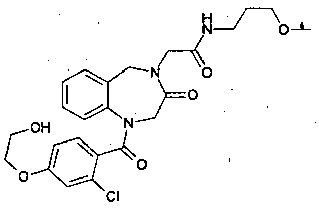 | 2-{1-[2-chloro-4-(2-hydroxyethoxy)benzoyl]-3-oxo-1,2,3,5-tetrahydrobenzo[e]-1,4-diazepin-4-yl}-N-(3-methoxypropyl)acetamid e | 1H-NMR (CDCl3) δ ppm: 1.65-1.75 (2H, m), 2.15-2.40 (1H, m), 3.10-3.45 (7H, m), 3.80-6.05 (10H, m), 6.30-7.75 (8H, m). MS(ESI, m/z): 490(M+H)+ |

**[Table 33]**

| Example | Structure formula | Compound name | 1H-NMR (solvent) or MS(m/z) |
|---|---|---|---|
| 2-27 | 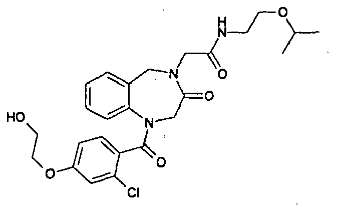 | 2-{1-[2-chloro-4-(2-hydroxyethoxy)benzoyl]-3-oxo-1,2,3,5-tetrahydrobenzo[e]-1,4-diazepin-4-yl}-N-(2-isopropoxyethyl)acetami de | 1H-NMR (CDCl3) δ ppm: 1.00-1.15 (6H, m), 2.05-2.30 (1H, m), 3.35-3.55 (5H, m), 3.85-5.85 (10H, m), 6.15-7.65 (8H, m) MS(ESI, m/z): 504(M+H)+ |
| 2-28 | 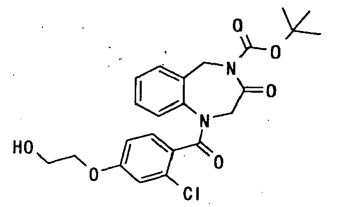 | tert-butyl 1-[2-chloro-4-(2-hydroxyethoxy)-berizoyl]-3-oxo-1,2,3,5-tetrahydrobenzo[e]-1,4-diazepine-4-carboxylate | 1H-NMR (CDCl3) δ ppm: 1.35-1.55 (9H, m), 1.80-2.20 (1H, br), 3.55-5.40 (8H, m), 6.50-7.60 (7H, m) |
| 2-29 | 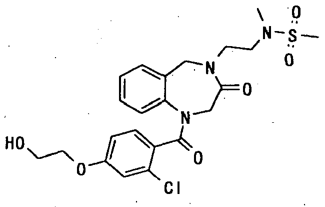 | N-(2-{1-[2-chloro-4-(2-hydroxyethoxy)benzoyl]-3-oxo-1,2,3,5-tetrahydrobenzo[e]-1,4-diazepin-4-yl}ethyl)methane-sulfonamide | MS(ESI, m/z) : 496(M+H)+ |
| 2-30 | 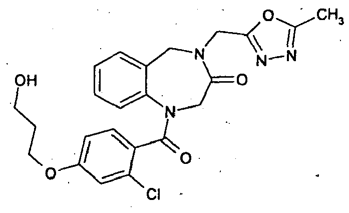 | 1-[2-chloro-4-(3-hydroxypropoxy)-benzoyl]-4-(5-methyl-1,3,4-oxadiazol-2-ylmethyl)-1,2,4,5-tetrahydrobenzo[e]-1,4-diazepin-3-one | 1H-NMR (CDCI3) 6 ppm: 1.45-1.65 (1H, br), 1.90-2.15 (2H, m), 2.48 (3H, s), 3.60-5.60 (10H, m), 6.50-7.70 (7H, m) MS(ESI, m/z): 471(M+H)+ |

**[Table 34]**

| Example | Structure formula | Compound name | 1 H-NMR (solvent) or MS(m/z) |
|---|---|---|---|
| 2-31 | 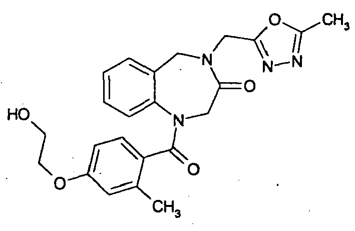 | 1-[4-(2-hydroxyethoxy)-2-methylbenzoyl]-4-(5-methyl-1,3,4-oxadiazol-2-ylmethyl)-1,2,4,5-tetrahydrobenzo[e]-1,4-diazepin-3-one | 1H-NMR (CDCl3) δ ppm: 1.90-2.00 (1H, m), 2.43 (3H, s), 2.48 (3H, s), 3.80-5.20 (10H, m), 6.20-7.70 (7H, m) MS(ESI, m/z) : 437 (M+H)+ |
| 2-32 | 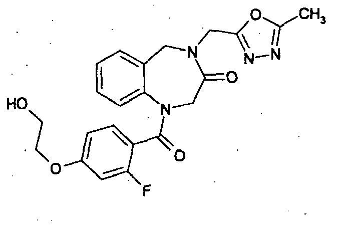 | 1-[2-fluoro-4-(2-hydroxyethoxy)benzoyl]-4-(5-methyl-1,3,4-oxadiazol-2-ylmethyl)-1,2,4,5-tetrahydrobenzo[e]-1,4-diazepin-3-one | 1H-NMR (CDCl3) δ ppm: 2.00-2.15 (1H, m), 2.47 (3H, s), 3.50-5.50 (10H, m), 6.10-8.10 (7H, m) MS(ESI, m/z): 441 (M+H)+ |
| 2-33 | 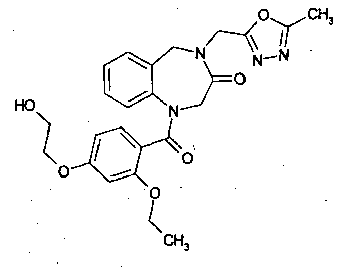 | 1-[2-ethoxy-4-(2-hydrdxyethoxy)benzoyl]-4-(5-methyl-1,3,4-oxadiazol-2-ylmethyl)-1,2,4,5-tetrahydrobenzo[e]-1,4-diazepin-3-one | 1H-NMR (CDCI3) δ ppm: 1.10-1.50 (3H, m), 2.00-2.25 (1 H, br), 2.46 (3H, s), 3.50-5.40 (12H, m), 6.00-7.60 (7H, m) MS(ESI, m/z) : 467(M+H)+ |
| 2-34 | 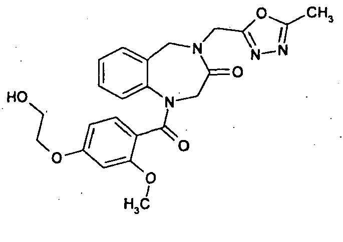 | 1-[4-(2-hydroxyethoxy)-2-methoxybenzoyl]-4-(5-methyl-1,3,4-oxadiazol-2-ylmethyl)-1,2,4, 5-tetrahydrobenzo[e]-1,4-diazepin-3-one | 1 H-NMR (CDCl3) δ ppm: 1.85-2.05 (1H, br), 2.47 (3H, s), 3.20-5.90 (13H, m), 6.00-7.80 (7H, m) MS(ESI, m/z) : 453(M+H)+ |

**[Table 35]**

| Example | Structure formula | Compound name | 1 H-NMR (solvent) or MS(m/z) |
|---|---|---|---|
| 2-35 | 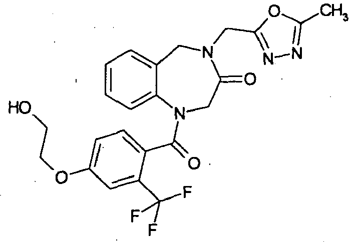 | 1-[4-(2-hydroxyethoxy)-2-trifluoromethyl-benzoyl]-4-(5-methyl-1,3,4-oxadiazol-2-yfmethyl)-1,2,4,5-tetrahydrobenzo[e]-1,4-diazepin-3-one | 1H-NMR (CDCl3) δ ppm: 1.90-2.00 (1H, br), 2.45-2.55 (3H, m), 3.30-5.60 (10H, m), 5.65-7.65 (7H, m) MS(ESI, m/z) : 491(M+H)+ |
| 2-36 | 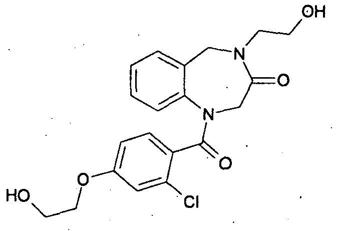 | 1-[2-chloro-4-(2-hydroxyethoxy)benzoyl]-4-(2-hydroxyethyl)-1,2,4,5-tetrahydrobenzo[e]-1,4-diazepin-3-one | 1 H-NMR (CDCI3) δ ppm: 2.00-2.80 (2H, m), 3.50-5.80 (10H, m), 6.50-8.00 (7H, m) |
| 2-37 | 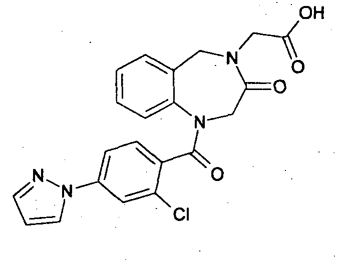 | [1-(2-chloro-4-pyrazol-1-ylbenzoyl)-3-oxo-1,2,3,5-tetrahydrobenzo[e]-1,4-diazepin-4-yl]acetic acid | 1H-NMR (DMSO-d6) δ ppm: 3.60-5.70 (6H, m), 6.50-8.70 (10H, m), 11.00-14.00 (1H, or) |
| 2-38 | 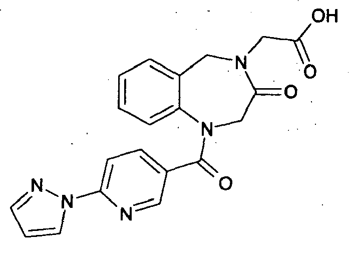 | [3-oxo-1-(6-pyrazol-1-ylpyridine-3-carbonyl)-1,2,3,5-tetrahydrobenzo[e]-1,4-diazepin-4-yl]acetic acid | MS(ESI, m/z): 392 (M+H)+ |

**[Table 36]**

| Example | Structure formula a | Compound name | 1 H-NMR (solvent) or MS(m/z) |
|---|---|---|---|
| 2-39 | 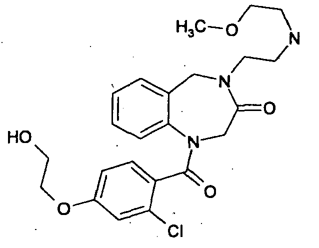 | I-Lz-cnioro-4-kz-hydroxyethoxy)benzoyl]-4-[2-(2-methoxyethyl-amino)ethyl]-1,2,4,5-tetrahydrobenzo[e]-1,4-diazepin-3-one | 1H-NMR (CDCl3) δ ppm: 2.75-2.90 (4H, m), 3.10-5.40 (15H, m), 6.50-7.80 (7H, m) |
| 2-40 | 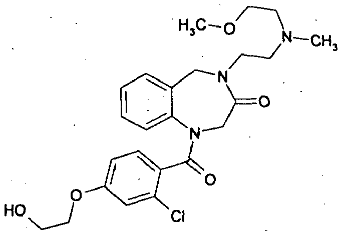 | 1-[2-chloro-4-(2-hydroxyethoXy)benzoyl]-4-{2-[(2-methoxyethyl)-methylamino]ethyl}-1,2,4,5-tetrahydrobenzo[e]-1,4-diazepin-3-one | 1H-NMR (CDCl3) δ ppm: 2.34 (3H, s), 2.55-2.70 (4H, m), 3.36 (3H, s), 3.48 (2H, t, J=5.5Hz), 3.65 (2H, t, J=6.7Hz), 3.80-5.70 (8H, m), 6.50-7.80 (7H, m) |
| 2-41 | 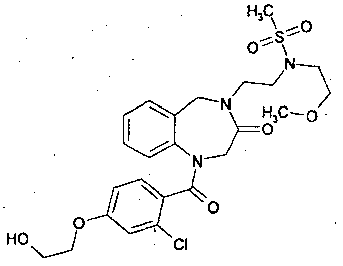 | N-(2-{1-[2-chloro-4-(2-hydroxyethoxy)benzoyl]-3-oxo-1,2,3,5-tetrahydrobenzo[e]-1,4-diazepin-4-yl)ethyl)-N-(2-methoxyethyl)-methanesulfonamide | 1H-NMR (CDCI3) δ ppm: 1.90-2.30 (1H, br), 2.92 (3H, s), 3.34 (3H, s), 3.35-5.70 (16H, m), 6.50-7.70 (7H, m) |
| 2-42 | 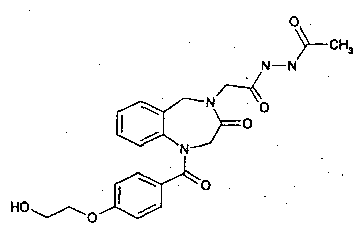 | N,1(2-(l-[4-(2-hydroxyethoxy)benzoyl]-3-oxo-1,2,3,5-tetrahydrobenzo[e]-1,4-diazepin-4-yl}acetyl)-acetohydrazide | 1H-NMR (DMSO-d6) δ ppm: 1.75-1.95 (3H, m), 3.60-3.70 (2H, m), 3.85-3.95 (2H, m), 3.95-6.00 (6H, m), 6.70-7.00 (3H, m), 7.10-7.30 (4H, m), 7.45-7.60 (1H, m), 8.95-10.10 (2H, m) |

**[Table 37]**

| Example | Structure formula. | Compound name | 1 H-NMR (solvent) 1 or MS(m/z) |
|---|---|---|---|
| 2-43 | 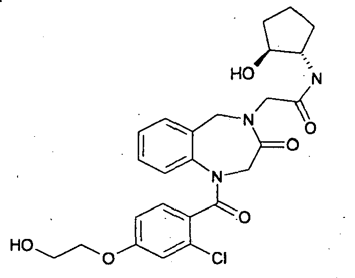 | 2-{1-[2-chloro-4-(2-hydroxyethoxy)benzoyl]-3-oxo-1,2,3,5-tetrahydrobenzo[e]-1,4-diazepin-4-yl}-N-((1S,2S)-2-hydroxycyclopentyl)-acetamide | 1 H-NMR (DMSO-d6) δ ppm: 1.10-2.00 (6H, m), 3.50-5.60 (15H, m), 6.60-7.95 (7H, m) |
| 2-44 | 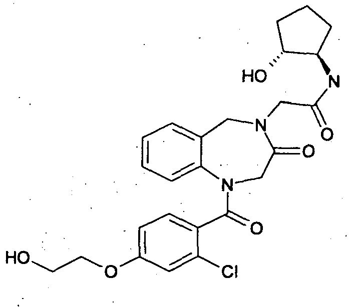 | 2-{1-[2-chloro-4-(2-hydroxyethoxy)benzoyl]-3-oxo-1,2,3,5-tetrahydrobenzo[e]-1,4-diazepin-4-yl}-N-((1 R,2R)-2-hydroxycyclopentyl)-acetamide | 1H-NMR (DMSO-d6) δ ppm: 1.10-2.00 (6H, m), 3.50-5.60 (15H, m), 6.55-7.95 (7H, m) |
| 2-45 | 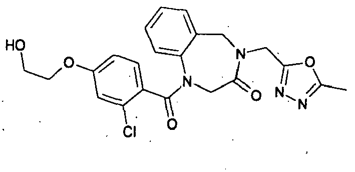 | 1-[2-chloro-4-(2-hydroxyethoxy)benzoyl]-4-(5-methyl-1,3,4-oxadiazol-2-ylmethyl)-1,2,4,5-tetrahydrobenzo[e]-1,4-diazepin-3-one | 1 H-NMR (CDCl3) δ ppm: 2.00-2.30 (1H, br), 2.48 (3H, s), 3.85-5.60 (10H, m), 6.50-7.60 (7H, m) |
| 2-46 | 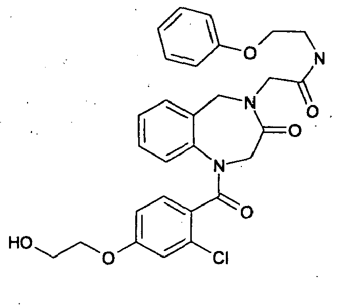 | 2-{1-[2-chloro-4-(2-hydroxyethoxy)benzoyl]-3-oxo-1,2,3,5-tetrahydrobenzo[e]-1,4-diazepin-4-yl}-N-(2-phenoxyethyl)-acetamide | 1 H-NMR (CDCI3) δ ppm: 1.80-2.00 (1H, m), 3.00-5.40 (14H, m), 6.45-7.60 (12H, m) |

**[Table 38]**

| Example | Structure formula | Compound name | 1H-NMR (solvent) or MS (m/z) |
|---|---|---|---|
| 2-47 | 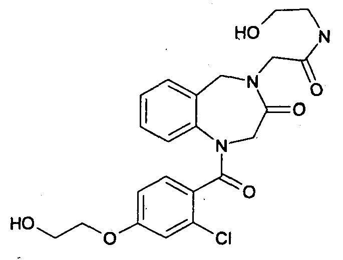 | 2-{1-[2-chloro-4-(2-hydroxyethoxy)benzoyl]-3-oxo-1,2,3,5-tetrahydrobenzo[e]-1,4-diazepin-4-yl}-N-(2-hydroxyethyl)-acetamide | 1H-NMR (CD3OD) δ ppm: 3.20-5.50 (14H, m), 6.60-7.60 (7H, m) |
| 2-48 | 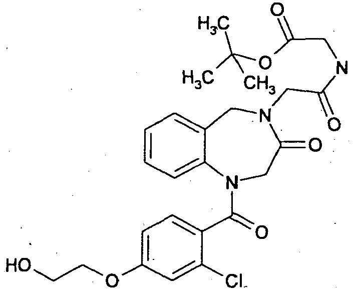 | tert-butyl (2-{1-[2-chloro-4-(2-hydroxyethoxy)benzoyl]-3-oxo-1,2,3,5-tetrahydrobenzo[e]-1,4-diazepin-4-yl}acetylamino)acetate | 1H-NMR (CDCl3) δ ppm: 1.45 (9H, s), 2.00-2.35 (1H, m), 3.40-5.70 (12H, m), 6.30-7.80 (7H, m) |
| 2-49 | 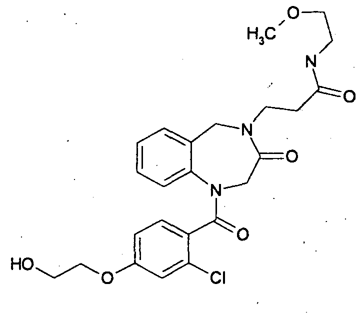 | 3-{1-[2-chloro-4-(2-hydroxyethoxy)benzoyl]-3-oxo-1,2,3,5-tetrahydrobenzo[e]-1,4-diazepin-4-yl}-N-(2-methoxyethyl)-propionamide | 1H-NMR (CDCl3) δ ppm: 1.85-2.10 (1H, br), 2.45-2.60 (2H, m), 3.25-3.50 (7H, m), 3.75-5.50 (10H, m), 5.90-7.60 (8H, m) |

**[Table 39]**

| Example | Structure formula | Compound name | 1 H-NMR (solvent) or MS (m/z) |
|---|---|---|---|
| 2-50 | 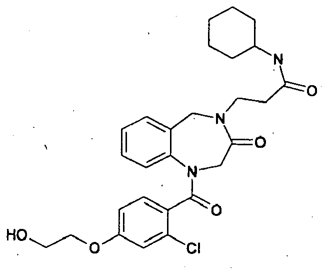 | 3-(l-[2-chloro-4-(2-hydroxyethoxy)benzoyl]-3-oxo-1, 2, 3,5-tetrahydrobenzo[e]-1,4-diazepin-4-yl}-N-cyclohexyl-propionamide | 1H-NMR (CDCl3) δ ppm: 0.90-2.10 (11 H, m), 2.40-2.55 (2H, m), 3.65-6.00 (12H, m), 6.50-7.60 (7H, m) |
| 2-51 | 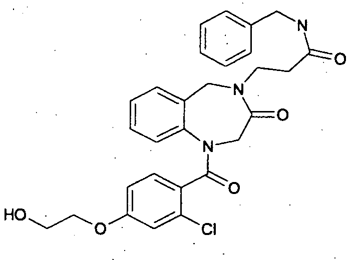 | N-benzyl-3-{1-[2-chloro-4-(2-hydroxyethoxy)benzoyl]-3-oxo-1,2, 3, 5-tetrahydrobenzo[e]-1,4-diazepin-4-yl}propionamide | 1H-NMR (CDCl3) δ ppm: 1.85-2.10 (1H, m), 2.50-2.65 (2H, m), 3.80-5.70 (12H, m), 5.95-6.25 (1H, m), 6.55-7.70 (12H, m) |

### Example 3-1

### 2-{3-Chloro-4-[4-(2-methanesulfonylaminoethyl)-3-oxo-2,3,4,5-tetrahydrobenzo[e]-1,4-diazepine-1-carbonyl]phenoxy}ethyl acetate

A solution of 2-{3-chloro-4-[4-(2-methanesulfonyl-2-tert-butoxycarbonylaminoethyl)-3-oxo-2,3,4,5-tetrahydrobenzo[e]-1,4-diazepine-1-carbonyl]phenoxy} ethyl acetate (0.203 mg) and trifluoroacetic acid (1.0 mL) in dichloromethane (1.0 mL) was stirred at room temperature for an hour, and then concentrated under reduced pressure. The residue was purified by column chromatography on silica gel (eluent: dichloromethane-methanol and hexane-ethyl acetate-ethanol) to give 2-{3-chloro-4-[4-(2-methanesulfonylaminoethyl)-3-oxo-2,3,4,5-tetrahydrobenzo[e]-1,4-diazepine-1-carbonyl]phenoxy} ethyl acetate (41.1 mg).

MS(ESI, m/z):524(M+H)⁺

### Examples 3-2 and 3-3

The following compounds of Examples 3-2 and 3-3 were obtained with the use of the corresponding materials in a similar manner to that described in Example 3-1. The structure formula and physical data of these compounds were shown in Table 40.

**[Table 40]**

| Example | Structure formula | Compound name | 1H-NMR(solvent) or MS(m/z) |
|---|---|---|---|
| 3-2 | 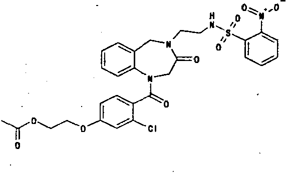 | 2-(3-chloro-4-{4-[2-(2-nitrobenzensulfonylamino)-ethyl]-3-oxo-2,3,4,5-tetrahydrobenzo[e]-1,4-diazepine-1-carbonyl}phenoxy)ethyl acetate | 1H-NMR (CDCl3) δ ppm : 2.00-2.20 (3H, m), 3.20-6.00 (12H, m), 6.50-8.20 (11H, m) |
| 3-3 | 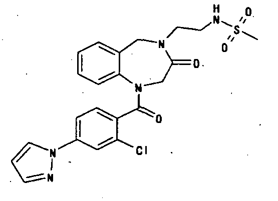 | N-{2-[1-(2-chloro-4-pyrazol-1-ylbenzoyl)-3-oxo-1,2,3,5-tetrahydrobenzo[e]-1,4-diazepin-4-yl]ethyl}methane-sulfonarivde | 1H-NMR (CDCl3) δ ppm : 2.90-5.60 (11H, m), 6.40-8.05 (10H, m) |

### Example 4

### 2-[3-Chloro-4-(3-oxo-2,3,4,5-tetrahydrobenzo[e]-1,4-diazepine-1-carbonyl)phenoxy]ethyl acetate

2-[3-Chloro-4-(3-oxo-2,3,4,5-tetrahydrobenzo[e]-1,4-diazepine-1-carbonyl)phenoxy] ethyl acetate was synthesized by using tert-butyl 1-[4-(2-acetoxyethoxy)-2-chlorobenzoyl]-3-oxo-1,2,3,5-tetrahydrobenzo[e]-1,4-diazepine-4-carboxylate according to Example 3.

¹H-NMR (CDCl₃) δ ppm:
2.00-2.15 (3H, m), 4.00-5.20 (8H, m), 6.20-7.60 (.7H, m)

### Example 5

### {1-[4-(2-Acetoxyethoxy)-2-chlorobenzoyl]-3-oxo-1,2,3,5-tetrahydrobenzo[e]-1,4-diazepin-4-yl}acetic acid

{1-[4-(2-Acetoxyethoxy)-2-chlorobenzoyl]-3-oxo-1,2,3,5-tetrahydrobenzo[e]-1,4-diazepin-4-yl}acetic acid was synthesized by using tert-butyl {1-[4-(2-acetoxyethoxy)-2-chlorobenzoyl]-3-oxo-1,2,3,5-tetrahydrobenzo[e]-1,4-diazepin-4-yl}acetate according to Example 3.

¹H-NMR (CDCl₃) δ ppm:
2.00-2.15 (3H, m), 4.00-4.95 (10H, m), 6.55-7.60 (7H, m)
MS (ESI, m/z):461(M+H)⁺

### Example 6-1

### 1-[2-Chloro-4-(2-hydroxyethoxy)benzoyl]-4-(1-methyl-1H-pyrazol-3-ylmethyl)-1,2,4,5-tetrahydrobenzo[e]-1,4-diazepin-3-one

To a stirred solution of 2-[3-chloro-4-(3-oxo-2,3,4,5-tetrahydrobenzo[e]-1,4-diazepine-1-carbonyl)phenoxy]ethyl acetate (40.0mg) in tetrahydrofuran (1.0 mL) was added sodium hydride (about60%: 10.0 mg) under ice-cooling, the mixture was stirred for an hour at room temperature. To this stirred mixture was added 3-chloromethyl-1-methyl-1H-pyrazole hydrochloride (24.9 mg) under ice-cooling, the mixture was stirred at room temperature for 1.5 hours. To this stirred reaction mixture was added sodium iodide (14.9 mg) at room temperature, the mixture was stirred at the same condition for 4 days. To the stirred reaction mixture was added 5 mol/L aqueous solution of sodium hydroxide (0.0220 mL) at room temperature, and the mixture was stirred at the same condition for 30 minutes. Water was added to the reaction mixture, which was extracted with ethyl acetate. The organic layer was washed with water and brine, and dried over anhydrous magnesium sulfate, filtered. The filtrate was concentrated under reduced pressure. The obtained crude product was purified by column chromatography on aminopropylsilylated silica gel (eluent:ethyl acetate-ethanol) to give 1-[2-chloro-4-(2-hydroxyethoxy)benzoyl]-4-(l-methyl-1H-pyrazol-3-ylmethyl)-1,2,4,5-tetrahydrobenzo[e]-1,4-diazepin-3-one (54.7 mg).

MS(ESI, m/z):455(M+H)⁺

### Examples 6-2 to 6-4

The following compounds of Examples 6-2 to 6-4 were obtained with the use of the corresponding materials in a similar manner to that described in Example 6-1. The structure formula and physical data of these compounds were shown in table 41.

**[Table 41]**

| Example | Structure formula | Compound name | 1H-NMR (solvent) or MS(m/z) |
|---|---|---|---|
| 6-2 | 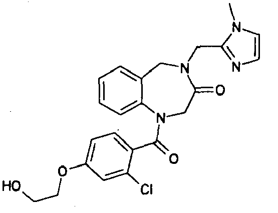 | 1-[2-chloro-4-(2-hydroxyethoxy)benzoyl]-4-(1-methyl-1H-imidazol-2-ylmethyl)-1,2,4,5-tetrahydrobenzo[e]-1,4-diazepin-3-one | 1 H-NMR (CDCl3) δ ppm: 1.80-2.80 (3H, m), 3.30-6.00 (10H, m), 6.50-8.50 (9H, m) MS(ESI, m/z) : 455(M+H)+ |
| 6-3 | 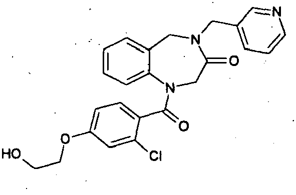 | 1-[2-chloro-4-(2-hydroxyethoxy)benzoyl]-4-pyridin-3-ylmethyl-1,2,4,5-tetrahydrobenzo[e]-1,4-diazepin-3-one | MS(ESI, m/z) : 452(M+H)+ |
| 6-4 | 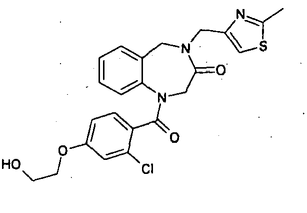 | 1-[2-chloro-4-(2-hydroxyethoxy)benzoyl]-4-(2-methylthiazol-4-ylmethyl)-1,2,4,5-tetrahydrobenzo[e]-1,4-diazepin-3-one | MS (ESI, m/z) : 472(M+H)+ |

### Example 7

### 1-(2-Chl-oro-4-pyrrolidin-1-ylbenzoyl)-4-(2-hydroxyethyl)-1,2,4,5-tetrahydrobenzo[e]-1,4-diazepin-3-one

To a stirred suspension of [1-(2-chloro-4-pyrrolidin-1-ylbenzoyl)-3-oxo-1,2,3,5-tetrahydrobenzo[e]-1,4-diazepin-4-yl]acetic acid (120 mg) and N-methylmorpholine (42.5 mg) in tetrahydrofuran (1.2 mL) was added dropwise isobutyl chloroformate (57.4 mg) under ice-methanol cooling, and the mixture was stirred under ice-methanol cooling for 20 minutes. To the reaction mixture was added dropwise a suspension of sodium tetrahydroborate (18.0 mg) in ethanol (0.25 mL) at the same temperature, and the mixture was stirred under ice-cooling for 30 minutes. To the reactionmixture was added water and then extracted with ethyl acetate. The organic layer was washed with brine. The organic layer was dried over anhydrous magnesium sulfate, filtered, and the filtrate was concentrated under reduced pressure. The obtained crude product was purified by column chromatography on aminopropylsilylated silica gel (eluent: ethyl acetate-ethanol) to give 1-(2-chloro-4-pyrrolidin-1-ylbenzoyl)-4-(2-hydroxyethyl)-1,2,4,5-tetrahydrobenzo[e] -1,4-diazepine-3-one (85.0 mg).

¹H-NMR (CDCl₃) δ ppm:
1.90-2.05 (4H, m), 2.30-2.45 (1H, br), 3.00-3.40 (4H, m), 3.65-3.90 (4H, m), 4.20-5.45 (4H, m), 5.90-7.75 (7H, m)
MS(ESI, m/z):414(M+H)⁺

### Example 8-1

### 2-{3-Chloro-4-[4-(2-hydroxyethyl)-3-oxo-2,3,4,5-tetrahydrobenzo[e]-1,4-diazepine-1-carbonyl]phenoxy}ethyl acetate

2-{3-Chloro-4-[4-(2-hydroxyethyl)-3-oxo-2,3,4,5-tetrahydrobenzo[e]-1,4-diazepine-1-carbonyl]phenoxy}ethyl acetate was synthesized by using {1-[4-(2-acetoxyethoxy)-2-chloro-benzoyl]-3-oxo-1,2,3,5-tetrahydrobenzo[e]-1,4-diazepin-4-yl}acetic acid in a similar manner to that described in Example 7.

MS(ESI, m/z):447(M+H)⁺

### Examples 8-2 and 8-3

The following compounds of Examples 8-2 and 8-3 were obtained with the use of the corresponding materials in a similar manner to that described in Example 8-1. The structure formula and physical data of these compounds were shown in Table 42.

**[Table 42]**

| Example | Structure formula | Compound name | 1H-NMR(so!vent) orMS(m/z) |
|---|---|---|---|
| 8-2 | 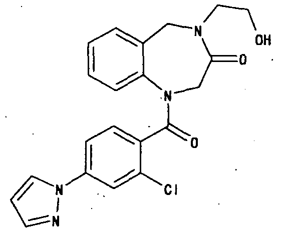 | 1-(2-chloro-4-pyrazol-1-ylbenzoyl)-4-(2-hydroxyethyl)-1,2,4,5-tetrahydrobenzo[e]-1,4-diazepin-3-one | 1H-NMR (COCl3) δ ppm: 2.10-2.35 (1H, m), 3.60-5.70 (8H, m), 6.40-8.10 (10H, m) |
| 8-3 | 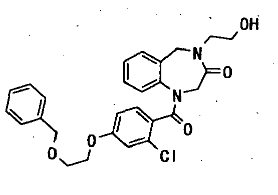 | 1-[4-(2-benzyloxy-ethoxy)-2-chlorobenzoyl]-4-(2-hydroxyethyl)-1,2,4,5-tetrahydrobenzo[e]-1,4-diazepin-3-one | 1H-NMR (COCl3) δ ppm: 2.10-2.30 (1H, br), 3.60-6.00 (14H, m), 6.50-7.70 (12H, m) |

### Example 9

### 1-(2-Chloro-4-pyrrolidin-1-ylbenzoyl)-4-(2-methoxyethyl)-1,2,4,5-tetrahydrobenzo[e]-1,4-diazepin-3-one

To a stirred suspension of 1-(2-chloro-4-pyrrolidin-1-ylbenzoyl)-4-(2-hydroxyethyl)-1,2,4,5-tetrahydrobenzo[e]-1,4-diazepin-3-one (50.0 mg) and silver(I) oxide (42.0 mg) in dichloromethane (1.0 mL) was added iodomethane (0.0380 mL) at room temperature, the reaction mixture was stirred at room temperature overnight. The insoluble material was removed by Celite filtration, and the filtrate was concentrated under reduced pressure. The obtained crude product was purified by column chromatography on silica gel (eluent:ethyl acetate) to give 1-(2-chloro-4-pyrrolidin-1-ylbenzoyl)-4-(2-methoxyethyl)-1,2,4,5-tetrahydrobenzo[e]-1,4-diazepin-3-one (6.00 mg).

¹H-NMR (CDCl₃) δ ppm:
1.85-2.05 (4H, m), 3.10-3.30 (4H, m), 3.36 (3H, s), 3.55 (2H, t, J=5.0Hz), 3.60-3.85 (2H, m), 4.15-5.55 (4H, m), 5.90-7.80 (7H, m)
MS(ESI, m/z):428(M+H)⁺

### Example10-1

### 2-{2-[1-(2-Chloro-4-pyrrolidin-1-ylbenzoyl)-3-oxo-1,2,3,5-tetrahydro-benzo[e]-1,4-diazepin-4-yl]ethyl}-isoindole-1,3-dione

To a stirred solution of 1-(2-chloro-4-pyrrolidin-1-ylbenzoyl)-4-(2-hydroxyethyl)-1,2,4,5-tetrahydrobenzo[e]-1,4-diazepin-3-one (202 mg), phthalimide (75.4 mg) and triphenylphosphine (134 mg) in tetrahydrofuran (3.0 mL) was added diisopropyl azodicarboxylate (40% toluene solution : 223 mg) at room temperature. After being stirred at room temperature overnight, the reaction mixture was concentarted under reduced pressure. The obtained residue was purified by column chromatography on silica gel (eluent:ethyl acetate-hexane) to give 2-{2-[1-(2-chloro-4-pyrrolidin-1-ylbenzoyl)-3-oxo-1,2,3,5-tetrahydro-benzo[e]-1,4-diazepin-4-yl]ethyl}isoindole-1,3-dione (176 mg).

¹H-NMR (CDCl₃) δ ppm:
1.90-2.05 (4H, m), 3.10-3.30 (4H, m), 3.70-4.00 (4H, m), 4.20-4.90 (4H, m), 6.15-7.35 (7H, m), 7.65-7.75 (2H, m), 7.80-7.90 (2H, m)
MS(ESI, m/z):543(M+H)^{+ +}

### Examples 10-2 to 10-4

The following compounds of Examples 10-2 to 10-4 were obtained with the use of the corresponding materials in a similar manner to that described in Example 10-1. N-tert-Butoxycarbonylmethanesulfonamide derivative in Example 10-2, N-tert-butoxycarbonyl-2-nitrobenzenesulfonamide derivative in Example 10-3, the similar material used in Example 10-1 to that used in Example 10-4 were used as materials bearing an active proton, respectively. The structure formula and physical data of these compounds were shown in Table 43.

**[Table 43]**

| Example | Structure formula | Compound name | 1H-NMR (solvent) |
|---|---|---|---|
| 10-2 | 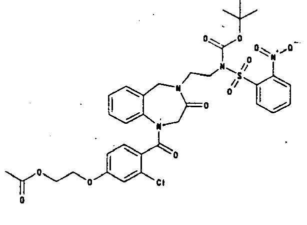 | 2-[4-(4-{2-[tert-butoxycarbonyl-(2-nitrobenzenesulfonyl)-amino]ethyl}-3-oxo-2,3,4,5-tetrahydrobenzo[e]-1,4-diazepine-1-carbonyl)-3-chlorophenoxy]ethyl acetate | 1H-NMR (CDCl3) δ ppm: 1.20-1.50 (9H, m), 1.75-2.20 (3H, m), 3.40-5.80 (12H, m), 6.40-7.85 (11H, m) |
| 10-3 | 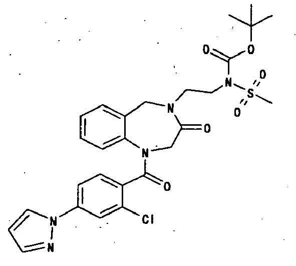 | N-tert-butoxycarbonyl-N-{2-[1-(2-chloro-4-pyrazol-1-ylbenzoyl)-3-oxo-1,2,3,5-tetrahydrobenzo[e]-1,4-diazepin-4-yl]ethyl}methanesulfon-amide | 1H-NMR (CDCI3) δ ppm: 1.40-1.60 (9H, m), 3.00-6.00 (11 H, m), 6.40-8.00 (10H, m) |
| 10-4 | 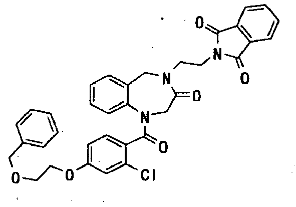 | 2-(2-{1-[4-(2-benzyloxyethoxy)-2-chlorobenzoyl]-3-oxo-1,2,3,5-tetrahydrobenzo[e]-1,4-diazepin-4-yl}ethyl)isoindole-1,3-dione | 1 H-NMR (CDCl3) δ ppm: 3.40-5.50 (14H, m), 6.55-7.95 (16H, m) |

### Example 11

### 4-(2-Aminoethyl)-1-(2-chloro-4-pyrrolidin-1-ylbenzoyl)-1,2,4,5-tetrahydrobenzo[e]-1,4-diazepin-3-one

To a stirred solution of 2-{2-[1-(2-chloro-4-pyrrolidin-1-ylbenzoyl)-3-oxo-1,2,3,5-tetrahydrobenzo[e]-1,4-diazepin-4-yl]ethyl}isoindole-1, 3-dione (155 mg) in methanol (3.0mL) wasaddedhydrazinemonohydrate (15.7mg) underice-cooling After being heated to reflux for 2 hours, the reaction mixture was concentarted under reduced pressure. The obtained residue was purified by column chromatography on aminopropylsilylated silica gel (eluent:ethyl acetate-ethanol) to give 4-(2-aminoethyl)-1-(2-chloro-4-pyrrolidin-1-ylbenzoyl)-1,2,4,5-tetrahydrobenzo[e]-1,4-diazepin-3-one (39.0 mg).

¹H-NMR (CDCl₃) δ ppm:
1.90-2.05 (4H, m), 2.90-4.95 (12H, m), 6.10-7.40 (7H, m)
MS(ESI, m/z):413(M+H)⁺

### Example 12

### N-{2-[1-(2-Chloro-4-pyrrolidin-1-ylbenzoyl)-3-oxo-1,2,3,5-tetrahydrobenzo[e]-1, 4-diazepin-4-yl] ethyl} benzamide

To a stirred solution of 4-(2-aminoethyl)-1-(2-chloro-4-pyrrolidin-1-ylbenzoyl)-1,2,4,5-tetrahydrobenzo[e]-1,4-diazepin-3-one (35.0 mg) and triethylamine (0.0280 mL) in dichloromethane (1.0 mL) was added benzoyl chloride (0.0140 mL) under ice-cooling. The reaction mixture was stirred at room temperature overnight. To the reaction mixture was added water and then extracted with dichloromethane. The organic layer was washed with water and brine. The organic layer was dried over anhydrous magnesium sulfate, filtered, and the filtrate was concentrated under reduced pressure. The obtained crude product was purified by column chromatography on aminopropylsilylated silica gel (eluent:ethyl acetate-hexane) to give N-{2-[1-( 2-chloro-4-pyrrolidin-1-ylbenzoyl)-3-oxo-1,2,3,5-tetrahydrobenzo[e]-1,4-diazepin-4-yl] ethyl} benzamide (26.0 mg).

¹H-NMR (CDCl₃) δ ppm:
1.90-2.05 (4H, m), 3.05-3.30 (4H, m), 3.60-3.75 (2H, m), 3.75-3.90 (2H, m), 4.15-5.55 (4H, m), 5.90-8.10 (13H, m)
MS (ESI, m/z) :517 (M+H)⁺

### Example 13

### 2-{3-Chloro-4-[4-(2-methanesulfonyl-2-tert-butoxycarbonyl aminoethyl)-3-oxo-2,3,4,5-tetrahydrobenzo[e]-1,4-diazepine-1-carbonyl]phenoxy}ethyl acetate

Diethyl azodicarboxylate Solution (40%) in toluene (0.121 mL) was added to a stirred solution of 2-{3-chloro-4-[4-(2-hydroxyethyl)-3-oxo-2,3,4,5-tetrahydrobenzo[e]-1,4-diazepine-1-carbonyl]phenoxy}ethyl acetate (59.0 mg), N-tert-butoxycarbonylmethanesulfonamide (38.7 mg) and triphenylphosphine (69.2 mg) in tetrahydrofuran (1.0 mL) at room temperature. After being stirred at room temperature for 20 hours, the reaction mixture was concentarted under reduced pressure. The residue was purified by column chromatography on silica gel (eluent:dichloromethane-methanol) to give 2-{3-chloro-4-[4-(2-methanesulfonyl-2-tert-butoxycarbonylaminoethyl)-3-oxo-2,3,4,5-tetrahydrobenzo[e]-1,4-diazepine-1-carbonyl]phenoxy} et hyl acetate (203 mg).

MS(ESI, m/z):624(M+H)⁺

### Example 14-1

### 2-(3-Chloro-4-{4-[2-(methanesulfonylmethylamino)ethyl]-3-oxo-2,3,4,5-tetrahydrobenzo[e]-1,4-diazepine-1-carbonyl}-phenoxy)ethyl acetate

Iodomethane (0.1 mL) was added to a stirred mixture of 2-{3-chloro-4-[4-(2-methanesulfonylaminoethyl)-3-oxo-2,3,4,5-tetrahydrobenzo[e]-1,4-diazepine-1-carbonyl]-phenoxy} ethyl acetate (12.3 mg) and potassium carbonate (6.5 mg) in N,N-dimethylformamide (0.8mL) at room temperature. After being stirred at room temperature for 16 hours, the reaction mixture was partitioned between etyl acetate and water. The organic layer was washed with water and brine, dried over anhydrous magnesium sulfate, concentrated under reduced pressure. The residue was purified by column chromatography on silica gel (eluent:dichloromethane-methanol) to give 2-(3-chloro-4-{4-[2-(methanesulfonylmethylamino)ethyl]-3-oxo-2,3,4,5-tetrahydrobenzo[e]-1,4-diazepine-1-carbonyl}phenoxy)ethyl acetate(3.3 mg).

MS(ESI, m/z):538(M+H)⁺

### Examples 14-2 to 14-4

The following compounds of Examples 14-2 to 14-4 were obtained with the use of the corresponding materials in a similar manner to that described in Example 14-1. The structure formula and physical data of these compounds were shown in Table 44.

**[Table 44]**

| Example | Structure formula | Compound name | 1H-NMR (solvent) |
|---|---|---|---|
| 14-2 | 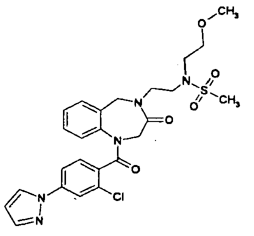 | N-{2-[1-(2-chloro-4-pyrazol-1-ylbenzoyl)-3-oxo-1,2,3,5-tetrahydrobenzo[e]-1,4-diazepin-4-yl]ethyl}-N-(2-methoxyethyl)methanesulfonamide | 1H-NMR (CDCl3) δ ppm: 3.80-6.00 (18H, m), 6.40-8.05 (10H, m) |
| 14-3 | 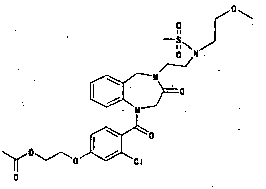 | 2-[3-chloro-4-(4-[2-[methanesulfonyl-(2-methoxyethyl)amino]ethyl]-3-oxo-2.3,4,5-tetrahydrobenzo[e]-1,4-diazepine-1-carbonyl)phenoxy]ethyl acetate | 1 H-NMR (CDCl3) δ ppm: 2.11 (3H, s), 3.20-4.60 (22H, m), 6.80-7.05 (2H, m), 7.15-7.55 (5H, m) |
| 14-4 | 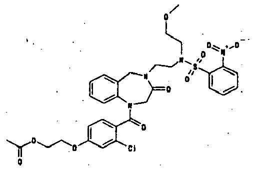 | 2-[3-chloro-4-(4-(2-[(2-methoxyethyl)-(2-nitrobenzenesulfonyl)amino]ethyl]-3-oxo-2,3,4,5-tetrahydrobenzo[e]-1,4-diazepine-1-carbonyl)phenoxy]ethyl acetate | 1H-NMR (CDCl3) δ ppm: 1.75-2.20 (3H, m), 3.00-5.70 (19H, m), 6.30-8.30 (11H, m) |

### Example 15

### 1-(2-Chloro-4-pyrrolidin-1-ylbenzoyl)-4-(1H-tetrazol-5-ylmethyl)-1,2,4,5-tetrahydrobenzo[e]-1,4-diazepin-3-one

Sodium azide (59.1 mg) was added to a stirred solution of [1-(2-chloro-4-pyrrolidin-1-ylbenzoyl)-3-oxo-1,2,3,5-tetrahydrobenzo[e]-1,4-diazepin-4-yl]acetonitrile (93.0 mg) and trimethylamine hydrochloride (65.2 mg) in N-methylpyrrolidone (1.5 mL) at room temperature. The reaction mixture was stirred at 130°C for 7 hours. After standing to cool to around room temperature, the reaction mixture was poured into 1 mol/L hydrochloric acid and then extracted with ethyl acetate. The organic layer was washed with water. The organic layer was dried over anhydrous magnesium sulfate, filtered, and the filtrate was concentrated under reduced pressure. The residue was added ethyl acetate and triturated, and the precipitate was collected by filtration to give 1-(2-chloro-4-pyrrolidin-1-ylbenzoyl)-4-(1H-tetrazol-5-ylmethyl)-1,2,4,5-tetrahydrobenzo[e]-1,4-diazepin-3-one (55.0 mg)

¹_{H}-NMR (CDCl₃) δ ppm:
1.85-2.05 (4H, m), 3.00-3.35 (4H, m), 4.00-5.70 (6H, m), 5.95-7.95 (7H, m), 14.05-15.30 (1H, br)
MS(ESI, m/z):452(M+H)⁺

### Example 16

### Methyl [1-(2-chloro-4-pyrrolidin-1-ylbenzoyl)-3-oxo-1,2,3,5-tetrahydrobenzo[e]-1,4-diazepin-4-yl]acetate

To a stirred suspension of [1-(2-chloro-4-pyrrolidin-1-ylbenzoyl)-3-oxo-1,2,3,5-tetrahydrobenzo[e]-1,4-diazepin-4-yl] acetic acid (50.0 mg) and potassium carbonate (32.3 mg) in N,N-dimethylformamide (2.0mL) was added iodomethane (0.0109 mL) at room temperature, the reaction mixture was stirred at room temperature overnight. To the mixture were added water and ethyl acetate, and the aqueous layer was extracted with ethyl acetate. The organic layer was washed with brine. The organic layer was dried over anhydrous magnesium sulfate, filtered, and the filtrate was concentrated under reduced pressure. The obtained crude product was purified by column chromatography on aminopropylsilylated silica gel (eluent:ethyl acetate). To the obtained product were added dichloromethane and water, and the separated organic layer was concentrated under reduced pressure to give methyl[1-(2-chloro-4-pyrrolidin-1-ylbenzoyl)-3-oxo-1,2,3,5-tetrahydrobenzo[e]-1,4-diazepin-4-yl]acetate (51.0 mg).

¹H-NMR (CDCl₃) δ ppm:
1.90-2.10 (4H, m), 3.10-3.35 (4H, m), 3.70-3.85 (3H, m), 4.10-5.20 (6H, m), 5.95-7.85 (7H, m)
MS(ESI, m/z):442(M+H)⁺

### Example 17

### 1-(2-Chloro-4-pyrrolidin-1-ylbenzoyl)-4-(3-ethyl-1,2,4-oxadiazol-5-ylmethyl)-1,2,4,5-tetrahydrobenzo[e]-1,4-diazepin-3-one

To a solution of N-hydroxypropionamidine (30.5 mg) in tetrahydrofuran (4.0 mL) was added sodium hydride (about 60% : 14.3 mg) at 60°C, and the mixture was stirred at 60°C for 0.5 hour. After being cooled to room temperature, to the mixture was added a solution of methyl [1-(2-chloro-4-pyrrolidin-1-ylbenzoyl)-3-oxo-1,2,3,5-tetrahydrobenzo[e]-1,4-diazepin-4-yllacetate (51.0 mg) in tetrahydrofuran (3.0mL), and the reaction mixture was stirred under heating to reflux overnight. The reaction mixture was concentrated under reduced pressure. To the residue were added water and dichloromethane, and the separated organic layer was washed with brine. The organic layer was dried over anhydrous magnesium sulfate, filtered, and the filtrate was concentrated under reduced pressure. The obtained crude product was purified by column chromatography on silica gel (eluent: ethyl acetate- hexane) to give 1-(2-chloro-4-pyrrolidin-1-ylbenzoyl)-4-(3-ethyl-1,2,4-oxadiazol-5-ylmethyl)-1,2,4,5-tetrahydrobenzo[e]-1,4-diazepin-3-one (6.10 mg).

¹H-NMR (CDCl₃) δ ppm:
1.34 (3H, t, J=7.6Hz), 1.90-2.05 (4H, m), 2.78 (2H, q, J=7.6Hz), 3.10-3.35 (4H, m), 4.10-5.50 (6H, m), 6.00-7.85 (7H, m),
MS(ESI, m/z):480(M+H)⁺

### Example 18-1

### 1-[4-(2-Hydroxyethoxy)benzoyl]-3-oxo-1,2,3,5-tetrahydrobenzo[ e]-1,4-diazepin-4-ylacetic acid

Two mol/L aqueous solution of sodium hydroxide (0.500 mL) was added to a suspension of ethyl {1-[4-(2-acetoxyethoxy)benzoyl]-3-oxo-1,2,3,5-tetrahydrobenzo[e]-1,4-diazepin-4-yl} acetate (0.0497 g) in ethanol (2.0 mL) at room temperature. The reaction liquid was stirred at room temperature for 3 hours, and then the reaction was quenched with 2 mol/L hydrochloric acid (0.500 mL). Toluene was added to the reaction liquid, and then the solvent was removed under reduced pressure. The residue was purified by column chromatography on octadecylsilylated silica.gel (eluent: methanol-water) to give 1-[4-(2-hydroxyethoxy)benzoyl]-3-oxo-1,2,3,5-tetrahydrobenzo[e]-1,4-diazepin-4-ylacetic acid (0.0293 g).

¹H-NMR (DMSO-d₆) δ ppm:
2.30 (1H, s), 2.50-5.50 (10H, m), 6.65-7.60 (8H, m)
MS(ESI, m/z):385(M+H)⁺

### Examples 18-2 to 18-4

The following compounds of Examples 18-2 to 18-4 were obtained with the use of the corresponding materials in a similar manner to that described in Example 18-1. The structure formula and physical data of these compounds were shown in Table 45.

**[Table 45]**

| Example | Structure formula | Compound name | 1 H-NMR (solvent) or ms(m/z) |
|---|---|---|---|
| 18-2 | 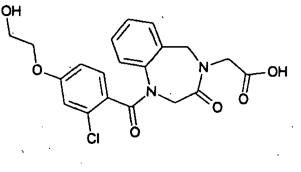 | 1-[2-chloro-4-(2-hydroxyethoxy)benzoyl]-3-oxo-1,2,3,5-tetrahydrobenzo[e]-1,4-diazepin-4-yl acetic acid | 1H-NMR (DMSO-d6) δ ppm: 3.15-5.10 (10H, m), 6.65-7.60 (7H, m), 12.78 (1H, brs) MS(ESI, m/z): 419 (M+H)+ |
| 18-3 | 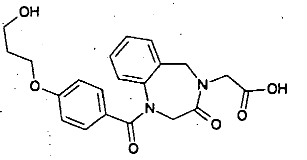 | 1-[4-(3-hydroxypropoxy)benzoyl]-3-oxo-1,2,3,5-tetrahydrobenzo[e]-1,4-diazepin-4-yl acetic acid | 1H-NMR (DMSO-d6) δ ppm: 1.75-1.95 (2H, m), 2.30 (1H, s), 3.15-5.05 (10H, m), 6.70-7.55 (8H, m) MS(ESI, m/z): 399 (M+H)+ |
| 18-4 | 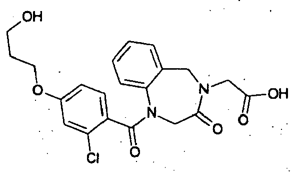 | 1-[2-chloro-4-(3-hydroxypropoxy)benzoyl]-3-oxo-1,2,3,5-tetrahydrobenzo[e]-1,4-diazepin-4-yl acetic acid | 1H-NMR (DMSO-d6) δ ppm: 1.70-1.95 (2H, m), 2.30 (1H.s), 3.40-5.00 (10H, m), 6.60-7.70 (7H, m) MS(ESI, m/z): 433 (M+H)+ |

### Example 19

### {1-[2-Chloro-4-(2-hydroxyethoxy)benzoyl]-3-oxo-1,2,3,5-tetrahydrobenzo[e]-1,4-diazepin-4-yl}acetohydrazide

A solution of benzyl {1-[4-(2-acetoxyethoxy)-2-chlorobenzoyl]-3-oxo-1,2,3,5-tetrahydrobenzo[e]-1,4-diazepin-4-yl}acetate (0.086 g) and hydrazine monohydrate (0.0156 g) in ethanol (3.0 mL) was heated to reflux for 15 hours. After standing to cool, the mixture was added dichloromethane, and then the organic layer was separated. The organic layer was dried over anhydrous magnesium sulfate, and concentrated under reduced pressure. The obtained crude product was purified by column chromatography on silica gel (eluent:ethanol) to give {1-[2-chloro-4-(2-hydroxyethoxy)benzoyl]-3-oxo-1,2,3,5-tetrahydrobenzo[e]-1,4-diazepin-4-yl}acetohydrazide (0.070 g).

. ¹H-NMR (DMSO-d₆) δ ppm:
3.55-5.00 (10H, m), 6.60-7.70 (7H, m), 8.80-9.30 (2H, m)
MS(ESI, m/z):433(M+H)⁺

### Example 20

### 4-(5-Amino-1,3,4-oxadiazol-2-ylmethyl)-1-[2-chloro-4-(2-hydroxyethoxy)benzoyl]-1,2,4,5-tetrahydrobenzo[e]-1,4-diazepin-3-one

To the stirred mixture of cyanogen bromide (10.7 mg) and potassium hydrogen carbonate (13.4 mg) in water (0.2 mL) was added a solution of 1-[2-chloro-4-(2-hydroxyethyl)-1-ylbenzoyl]-3-oxy-1,2,3,5-tetrahydrobenzo[e]-1,4-diazepin-4-ylacetohydrazide (29.0 mg) in methanol (0.4 mL) at room temperature. After being stirred for 18 hours at room temperature, the reaction mixture was purified by column chromatography on silica gel (eluent: methanol-dichloromethane) to give the crude product. The crude product was suspended in diethylether to give 4-(5-amino-1,3,4-oxadiazol-2-ylmethyl)-1-[2-chloro-4-(2-hydroxyethoxy)benzoyl]-1,2,4,5-tetrahydrobenzo[e]-1,4-diazepin-3-one (16.1 mg).

¹H-NMR (CDCl₃) δ ppm:
3.55-5.80 (10H, m), 6.60-7.70 (9H, m)

### Example 21-1

### 2-[1-(2-Chloro-4-pyrrolidin-1-ylbenzoyl)-3-oxo-1,2,3,5-tetrahydrobenzo[e]-1,4-diazepin-4-yl]-N-methylacetamide

To a solution of [1-(2-chloro-4-pyrrolidin-1-ylbenzoyl)-3-oxo-1,2,3,5-tetrahydrobenzo[e]-1,4-diazepin-4-yl]acetic acid (80.0 mg), methylamine hydrochloride (13.9 mg), 4-dimethylaminopyridine (25.1 mg) and triethylamine (0.0290 mL) in N,N-dimethylformamide (1.6 mL) was added 1-ethyl-3-(N,N-dimethylaminopropyl)carbodiimide hydrochloride (43.0 mg) at room temperature, the reaction mixture was stirred at room temperature overnight. The reaction mixture was poured into 1 mol/L hydrochloric acid and then extracted with ethyl acetate. The organic layer was washed with water. The organic layer was dried over anhydrous magnesium sulfate, filtered, and the filtrate was concentrated under reduced pressure. The obtained crude product was purified by column chromatography on aminopropylsilylated silica gel (eluent: ethyl acetate-ethanol) to give 2-[1- (2-chloro-4-pyrrolidin-1-ylbenzoyl)-3-oxo-1,2,3,5-tetrahydrobenzo[e]-1,4-diazepin-4-yl]-N-methylacetamide (26.0 mg).

¹H-NMR (CDCl₃) δ ppm:
1.90-2.05 (4H, m), 2.70-2.85 (3H, m), 3.05-3.40 (4H, m), 4.17 (2H, s), 4.25-5.70 (4H, m), 5.85-8.05 (8H, m)
MS(ESI, m/z):441(M+H)⁺

### Examples 21-2 to 21-52

The following compounds of Examples 21-2 to 21-52 were obtained with the use of the corresponding materials in a similar manner to that described in Example 21-1. The structure formula and physical data of these compounds were shown in Tables 46 to 57.

**[Table 46]**

| Example | Structure formula | Compound name | 1H-NMR (solvent) or MS(m/z) |
|---|---|---|---|
| 21-2 | 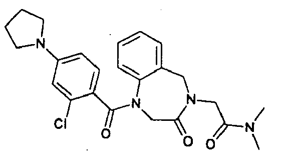 | 2-[1-(2-chloro-4-pyrrolidin-1-ylbenzoyl)-3-oxo-1,2,3,5-tetrahydrobenzo[e]-1,4-diazepin-4-yl]-N,N-dimethylacetamide | 1 H-NMR (CDCI3) δ ppm: 1.90-2.05 (4H, m), 2.97 (3H, s), 3.00 (3H, s), 3.10-3.30 (4H, m), 3.95-5.50 (6H, m), 5.95-8.20 (7H, m) MS(ESI, m/z) : 455(M+H)+ |
| 21-3 | 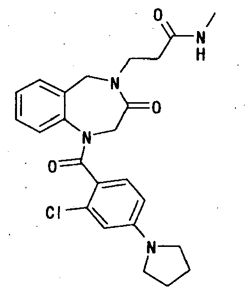 | 3-[1-(2-chloro-4-pyrrolidin-1-ylbenzoyl)-3-oxo-1,2,3,5-tetrahydrobenzo[e]-1,4-diazepin-4-yl]-N-methylpropionamide | 1 H-NMR (CDCI3) δ ppm: 1.90-2.05 (4H, m), 2.51 (2H, t, J=6.3Hz), 2.77 (3H, d, J=4.7Hz), 3.10-3.30 (4H, m), 3.83 (2H, t, J=6.3Hz), 4.25-5.40 (4H, m), 5.95-7.85 (8H, m) MS(ESI, m/z) : 455(M+H)+ |
| 21-4 | 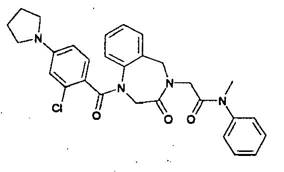 | 2-[1-(2-chloro-4-pyrrolidin-1-ylbenzoyl)-3-oxo-1,2,3,5-tetrahydrobenzo[e]-1,4-diazepin-4-yl]-N-methyl-N-phenylacetamide | 1 H-NMR (CDCI3) δ ppm: 1.90-2.05 (4H, m), 3.10-3.25 (4H, m), 3.29 (3H, s), 3.85-5.45 (6H, m), 5.95-7.80 (12H, m) MS(ESI, m/z) : 517(M+H)+ |
| 21-5 | 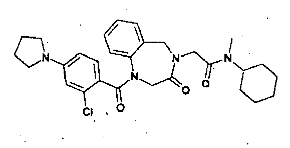 | 2-[1-(2-chloro-4-pyrrolidin-1-ylbenzoyl)-3-oxo-1,2,3,5-tetrahydrobenzo[e]-1,4-diazepin-4-yl]-N-cyclohexyl-N-methylacetamide | 1 H-NMR (CDCl3) δ ppm: 1.00-1.85 (10H, m), 1.90-2.05 (4H, m), 2.80-2.90 (3H, m), 3.05-3.35 (4H, m), 3.45-5.40 (7H, m), 5.95-7.75 (7H, m) MS(ESI, m/z) : 523(M+H)+ |
| 21-6 | 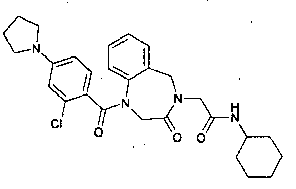 | 2-[1-(2-chloro-4-pyrrolidin-1-ylbenzoyl)-3-oxo-1,2,3,5-tetrahydrobenzo[e]-1,4-diazepin-4-yl]-N-cyclohexylacetamide | 1 H-NMR (CDCl3) δ ppm: 0.95-1.40 (5H, m), 1.45-1.85 (5H, m), 1.90-2.10 (4H, m), 3.10-3.30 (4H, m), 3.65-3.80 (1 H, m), 3.95-5.45 (6H, m), 5.75-7.80 (8H, m) MS(ESI, m/z): 509(M+H)+ |

**[Table 47]**

| Example | Structure formula | Compound name | 1H-NMR (solvent) orMS(m/z) |
|---|---|---|---|
| 21-7 | 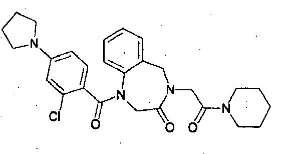 | 1-(2-chloro-4-pyrrolidin-1-ylbenzoyl)-4-(2-oxo-2-piperidine-1-ylethyl)-1,2,4,5-tetrahydrobenzo[e]-1,4-diazepin-3-one | 1H-NMR (CDCl3) δ ppm: 1.45-1.70 (6H, m), 1.90-2.05 (4H, m), 3.10-3.30 (4H, m), 3.30-3.45 (2H, m), 3.50-3.60 (2H, m), 4.05-5.40 (6H, m), 6.05-7.80 (7H, m) MS(ESI, m/z) : 495(M+H)+ |
| 21-8 | 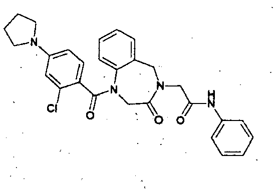 | 2-[1-(2-chloro-4-pyrrolidin-1-ylbenzoyl)-3-oxo-1,2,3,5-tetrahydrobenzo[e]-1,4-diazepin-4-yl]-N-phenylacetamide | 1 H-NMR (CDCl3) δ ppm: 1.90-2.05 (4H, m), 3.10-3.30 (4H, m), 4.31 (2H, s), 4.40-5.50 (4H, m), 6.05-7.60 (12H, m), 8.23 (1 H, brs). MS(ESI, m/z): 503(M+H)+ |
| 21-9 | 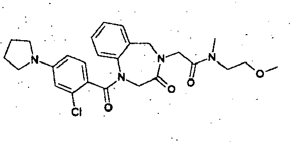 | 2-[1-(2-chloro-4-pyrrolidin-1-ylbenzoyl)-3-oxo-1,2,3,5-tetrahydrobenzo[e]-1,4-diazepin-4-yl]-N-(2-methoxyethyl)-N-methylacetamide | 1 H-NMR (CDCl3) δ ppm: 1.90-2.05 (4H, m), 2.95-3.10 (3H, m), 3.15-3.30 (4H, m), 3.30-3.40 (3H, m), 3.45-3.60 (4H, m), 3.90-5.05 (6H, m), 5.95-7.60 (7H, m) MS(ESI, m/z): 499(M+H)+ |
| 21-10 | 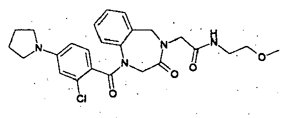 | 2-[1-(2-chloro-4-pyrrolidin-1-ylbenzoyl)-3-oxo-1,2,3,5-tetrahydrobenzo[e]-1,4-diazepin-4-yl]-N-(2-methoxyethyl)acetamide | 1 H-NMR (CDCI3) δ ppm: 1.90-2.05 (4H, m), 3.15-3.25 (4H, m), 3.27 (3H, s), 3.35-3.45 (4H, m), 4.19 (2H, s), 4.25-5.45 (4H, m), 6.05-7.70 (8H, m) MS(ESI, m/z) : 485(M+H)+ |
| 21-11 | 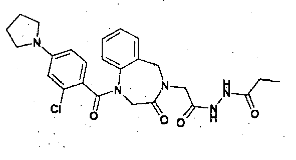 | propionic acid N'-{2-[1-(2-chloro-4-pyrrolidin-1-ylbenzoyl)-3-oxo-1,2,3,5-tetrahydrobenzo[e]-1,4-diazepin-4-yl]acetyl]hydrazide | 1 H-NMR (CDCI3) δ ppm: 1.17 (3H, t, J=7.6Hz), 1.90-2.15 (4H, m), 2.27 (2H, q, J=7.6Hz), 3.10-3.40 (4H, m), 4.00-5.50 (6H, m), 6.00-7.70 (7H, m), 7.75-8.00 (1H, br), 8.50-8.90 (1H, br) MS(ESI, m/z): 498(M+H)+ |

**[Table 48]**

| Example | Structure formula | Compound name | 1H-NMR (solvent) or MS(m/z) |
|---|---|---|---|
| 21-12 | 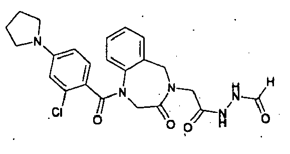 | 1-(2-{1-[2-chloro-4-(pyrrolidin-1-yl)benzoyl]-3-oxo-1,2,3,5-tetrahydrobenzo[e]-1,4-diazepin-4-yl)acetyl)-2-formylhydrazide | 1.90-2.05 (4H, m), 3.05-3.35 (4H, m), 4.00-5.35 (6H, m), 6.00-7.50 (7H, m), 7.90-8.15 (1H, m), 8.15-9.40 (1H, br) MS(ESI, m/z) : 470(M+H)+ |
| 21-13 | 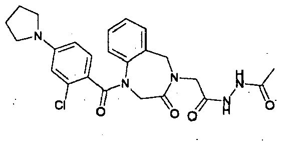 | acetic acid N'-{2-[1-(2-chloro-4-pyrrolidin-1-ylbenzoyl) -3-oxo-1,2,3,5-tetrahydrobenzo[e]-1,4-diazepin-4-yl]acetyl)hydrazide | 1H-NMR (CDCl3) δ ppm: 1.90-2.05 (7H, m), 3.10-3.30 (4H, m), 4.10-5.40 (6H, m), 6.00-7.80 (7H, m), 8.30-8.60 (1H, br), 8.80-9.40 (1 H, br) MS(ESI, m/z): 484(M+H)+ |
| 21-14 | 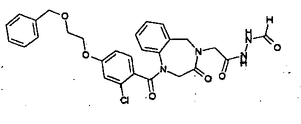 | form N'-(2-{1-(4-(2-benzyloxyethoxy)-2-chlorobenzoyl]-3-oxo-1,2,3,5-tetrahydrobenzo[e]-1,4-diazepin-4-yl)acetyl)hydrazide | 1H-NMR (CDCl3) δ ppm: 3.65-5.20 (12H, m), 6.50-7.60 (12H, m), 7.90-8.15 (1H, m), 8.20-9.50 (1H, br) MS(ESI, m/z) : 551(M+H)+ |
| 21-15 | 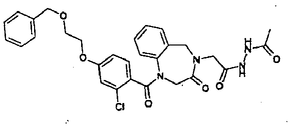 | acetic acid N'-(2-{1-[4-(2-benzyloxyethoxy)-2-chlorobenzoyl]-3-oxo-1,2,3,5-tetrahydrobenzo[e]-1,4-diazepin-4-yl}acetyl)hydrazide | 1H-NMR (CDCI3) δ ppm: 2.03 (3H, s), 3.70-5.30 (12H, m), 6.55-7.55 (12H, m), 7.70-7.90 (1H, br), 8.40-8.70 (1H, br) MS(ESI, m/z) : 565(M+H)+ |
| 21-16 | 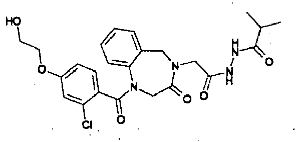 | isobutyric acid N'-(2-{1-[2-chloro-4-(2-hydroxyethoxy)benzoyl]-3-oxo-1,2,3,5-tetrahydrobenzo[e]-1,4-diazepin-4-yl}acetyl)hydrazide | 1H-NMR (CDCl3) δ ppm: 1.15-1.25 (6H, m), 2.10-2.30 (1H, br), 2.40-2.55 (1H, m), 3.85-5.40 (10H, m), 6.55-7.55 (7H, m) MS(ESI, m/z) : 503(M+H)+ |

**[Table 49]**

| Example | Structure formula | Compound name | 1H-NMR (solvent) or MS(m/z) |
|---|---|---|---|
| 21-17 | 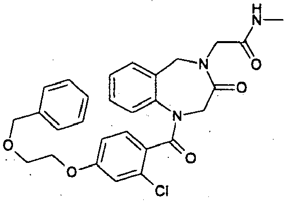 | 2-{1-[4-(2-benzyloxyethoxy)-2-chlorobenzoyl]-3-oxo-1,2,3,5-tetrahydrobenzo[e]-1,4-diazepin-4-yl}-N-methylacetamide | MS(ESI, m/z) : 522(M+H)+ |
| 21-18 | 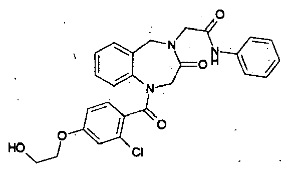 | 2-{1-[2-chloro-4-(2-hydroxyethoxy)benzoyl]-3-oxo-1,2,3,5-tetrahydrobenzo[e]-1,4-diazepin-4-yl}-N-phenylacetamide | 1H-NMR (CDCl3) δ ppm: 1.90-2.00 (1H, m), 3.85-5.40 (10H, m), 6.55-7.60 (12H, m), 7.95-8.30 (1H, m) |
| 21-19 | 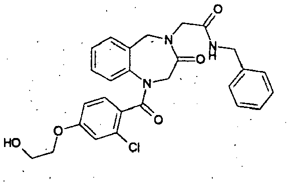 | N-benzyl-2-{1-[2-chloro-4-(2-hydroxyethoxy)benzoyl]-3-oxo-1,2,3,5-tetrahydrobenzo[e]-1,4-diazepin-4-yl}acetamide | MS(ESI, m/z) : 508(M+H)+ |
| 21-20 | 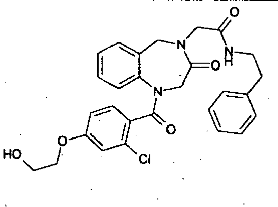 | 2-11-[2-chloro-4-(2-hydroxyethoxy)benzoyl]-3-oxo-1,2,3,5-tetrahydrobenzo[e]-1,4-diazepin-4-yl}-N-phenethylacetamide | MS(ESI, m/z) : 522(M+H)+ |

**[Table 50]**

| Example | Structure formula | Compound name | 1H-NMR (solvent) or MS(m/z) |
|---|---|---|---|
| 21-21 | 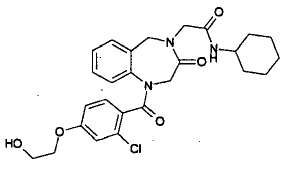 | 2-11-[2-chloro-4-(2-hydroxyethoxy)benzoyl]-3-oxo-1,2,3,5-tetrahydrobenzo[e]-1,4-diazepin-4-yl}-N-cyclohexylacetamide | MS(ESI, m/z): 500(M+H)+ |
| 21-22 | 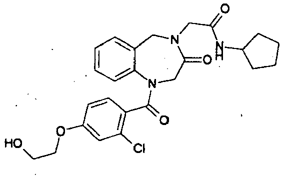 | 2-{1-[2-chloro-4-(2-hydroxyethoxy)benzoyl]-3-oxo-1,2,3,5-tetrahydrobenzo[e]-1,4-diazepin-4-yl}-N-cyclopentylacetamide | MS(ESI, m/z): 486(M+H)+ |
| 21-23 | 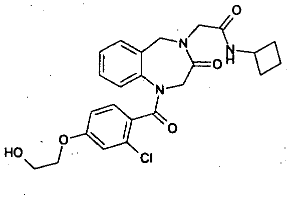 | 2-[1-[2-chloro-4-(2-hydroxyethoxy)benzoyl]-3-oxo-1,2,3,5-tetrahydrobenzo[e]-1,4-diazepin-4-yl]-N-cyclobutylacetamide | MS(ESI, m/z): 472(M+H)+ |
| 21-24 | 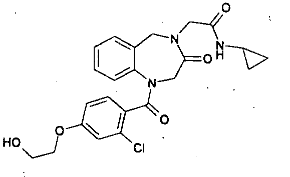 | 2-{1-[2-chloro-4-(2-hydroxyethoxy)benzoyl]-3-oxo-1,2,3,5-tetrahydrobenzo[e]-1,4-diazepin-4-yl}-N-cyclopropylacetamide | MS(ESI, m/z): 458(M+H)+ |
| 21-25 | 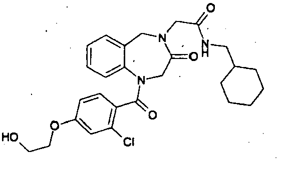 | 2-[1-[2-chloro-4-(2-hydroxyethoxy)benzoyl]-3-oxo-1,2,3,5-tetrahydrobenzo[e]-1,4-diazepin-4-yl]-N-cyclohexylmethylacetamide | MS(ESI, m/z): 514(M+H)+ |

**[Table 51]**

| Example | Structure formula | Compound name | 1H-NMR (solvent) or MS(m/z) |
|---|---|---|---|
| 21-26 | 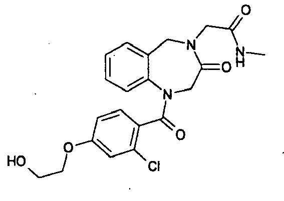 | 2-{1-[2-chloro-4-(2-hydroxyethoxy)benzoyl]-3-oxo-1,2,3,5-tetrahydrobenzo[e]-1,4-diazepin-4-yl}-N-methylacetamide | MS(ESI, m/z) : 432(M+H)+ |
| 21-27 | 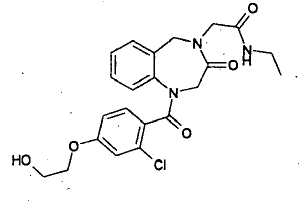 | 2-{1-[2-chloro-4-(2-hydroxyethoxy)benzoyl]-3-oxo-1,2,3,5-tetrahydrobenzo[e]-1,4-diazepin-4-yl)-N-ethylacetamide | MS(ESI, m/z): 446(M+H)+ |
| 21-28 | 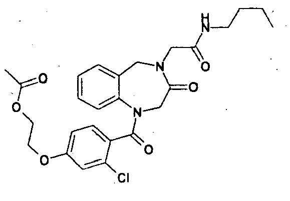 | 2-[4-(4-butylcarbamoylmethyl-3-oxo-2,3,4,5-tetrahydrobenzo[e]-1,4-diazepine-1-carbonyl)-3-chlorophenoxy]ethyl acetate | 1H-NMR (CDCI3) δ ppm: 0.86 (3H, t, J=7.3Hz), 1.15-1.30 (2H, m), 1.35-1.45 (2H, m), 2.00-2.20 (3H, m); 3.15-3.25 (2H, m), 4.00-5.25 (10H, m), 5.85-7.60 (8H, m) MS(ESI, m/z) : 516(M+H)+ |
| 21-29 | 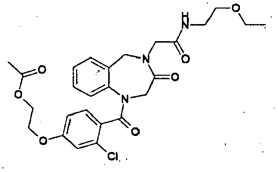 | 2-(3-chloro-4-{4-[(2-ethoxyethylcarbamoyl)methy l]-3-oxo-2,3,4,5-tetrahydrobenzo[e]-1,4-diazepine-1-carbonyl)phenoxy]ethyl acetate | 1H-NMR (CDCI3) δ ppm: 1.13 (3H, t, J=6.9Hz), 2.00-2.20 (3H, m), 3.35-3.50 (6H, m), 4.00-5.05 (10H, m), 6.10-7.60 (8H, m) MS(ESI, m/z) : 532(M+H)-E |

**[Table 52]**

| Example | Structure formula | Compound name | 1 H-NMR (solvent) or MS(m/z) |
|---|---|---|---|
| 21-30 | 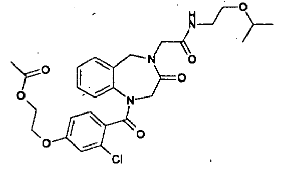 | 2-(3-chloro-4-{4-[(2-isopropoxyethylcarbamoyl)-methyl]-3-oxo-2,3,4,5-tetrahydrobenzo[e]-1,4-diazepine-1-carbonyl}phenoxy}ethyl acetate | 1H-NMR (CDCl3) δ ppm: 1.00-1.15 (6H, m), 2.00-2:20 (3H, m), 3.30-3.55 (5H, m), 4.00-5.25 (10H, m), 6.15-7.60 (8H, m) MS(ESI, m/z): 546(M+H)+ |
| 21-31 | 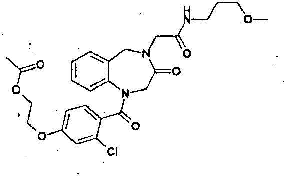 | 2-(3-chloro-4-{4-[(3-methoxypropylcarbamoyl)-methyl]-3-oxo-2,3,4,5-tetrahydrobenzo[e]-1,4-diazepine-1-carbonyl)phenoxy)ethyl acetate | 1H-NMR (CDCl3) δ ppm: 1.65-1.75 (2H, m), 2.00-2.20 (3H, m), 3.10-3.45 (7H, m), 4.00-5.50 (10H, m), 6.30-7.60 (8H, m) MS(ESI, m/z): 532(M+H)+ |
| 21-32 | 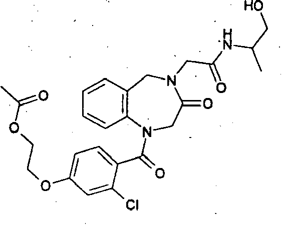 | 2-(3-chloro-4-{4-[(2-hydroxy-1-methylethylcarbamoyl)-methyl]-3-oxo-2,3,4,5-tetrahydrobenzo[e]-1,4-diazepine-1-carbonyl}phenoxy)ethyl acetate | 1 H-NMR (CDCl3) δ ppm: 1.1 1-(3H, d, J=6.8Hz), 2.00-2.20 (3H, m), 3.40-3.70 (2H, m), 3.90-5.40 (11H, m), 6.40-7.60 (7H, m) MS(ESI, m/z): 518(M+H)+ |
| 21-33 | 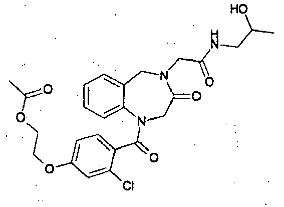 | 2-(3-chloro-4-{4,-[(2-hydroxypropylcarbamoyl)-methyl]-3-oxo-2,3,4,5-tetrahydrobenzo[e]-1,4-diazepine-1-carbonyl}phenoxy)ethyl acetate | 1 H-NMR (CDCI3) δ ppm: 1.13 (3H, d, J=6.3Hz), 2.00-2.20 (3H, m), 2.40-2.80 (1 H, m), 3.00-3.15 (1 H, m), 3.35-3.50 (1 H, m), 3.80-5.60 (11 H, m), 6.35-7.70 (7H, m) |

**[Table 53]**

| Example | Structure formula | Compound name | 1H-NMR (solvent) or MS(m/z) |
|---|---|---|---|
| 21-34 | 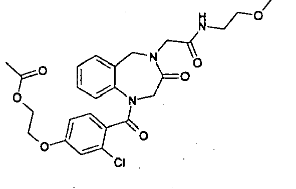 | 2-(3-chloro-4-{4-[(2-methoxyethylcarbamoyl)-methyl]-3-oxo-2,3,4,5-tetrahydrobenzo[e]-1,4-diazepine-1-carbonyl}phenoxy)ethyl acetate | 1H-NMR (CD30D) δ ppm: 2.05-2.15 (3H, m), 3.29 (3H, s), 3.35-3.50 (4H, m), 4.00-5.50 (10H, m), 6.15-7.70 (7H, m) |
| 21-35 | 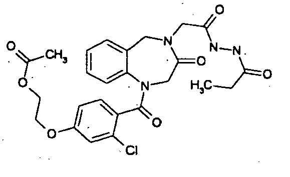 | 2-(3-chloro-4-{3-oxo-4-[2-oxo-2-(N'-propionylhydrazino)ethyl]-2,3,4,5-tetrahydrobenzo[e]-1,4-diazepine-1-carbonyl}phenoxy)ethyl acetate | 1H-NMR (CDCl3) δ ppm: 1.05-1.20 (3H, m), 2.00-2.15 (3H, m), 2.20-2.35 (2H, m), 3.90-5.70 (10H, m), 6.50-7.60 (7H, m), 7.90-9.20 (2H, br) MS(ESI, m/z) : 531(M+H)+ |
| 21-36 | 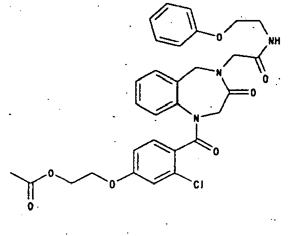 | 2-(3-chloro-4-[3-oxo-4-[(2-phenoxyethylcarbamoyl)-methyl]-2,3,4,5-tetrahydrobenzo[e]-1,4-diazepine-1-carbonyl}phenoxy)ethyl acetate | 1H-NMR (CDCl3) δ ppm: 2.00-2.20 (3H, m), 3.55-5.80 (14H, m), 6.30-7.70 (12H, m) |
| 21-37 | 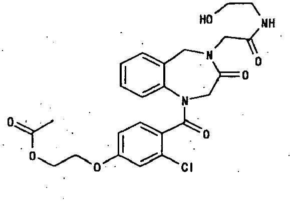 | 2-(3-chloro-4-{4-[(2-hydroxyethylcarbamoyl)-methyl]-3-oxo-2,3,4,5-tetrahydrobenzo[e]-1,4-diazepine-1-carbonyl}phenoxy)ethyl acetate | 1H-NMR (CDCl3) δ ppm: 2.00-2.20 (3H, m), 2.30-2.70 (1 H, m), 3.30-3.80 (4H., m), 4.00-5.70 (10H, m), 6.25-7.65 (7H, m) |

**[Table 54]**

| Example | Structure formula | Compound name | 1H-NMR (solvent) or MS(m/z) |
|---|---|---|---|
| 21-38 | 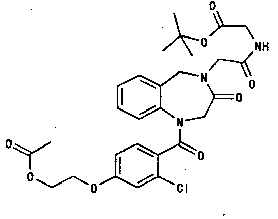 | tert-butyl (2-{1-[4-(2-acetoxyethoxy)-2-chlorobenzoyt]-3-oxo- . 1,2,3,5-tetrahydrobenzo[e]-1,4-diazepin-4-yl}acetylamino)acetate | 1H-NMR (CDCl3) δ ppm: 1.45 (9H, s), 2.00-2.20 (3H, m), 3.85-5.70 (12H, m), 6.25-7.65 (8H, m) |
| 21-39 | 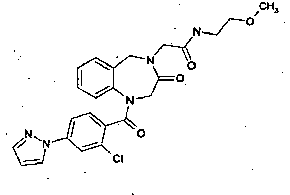 | 2-[1-(2-chloro-4-pyrazol-1-ylbenzoyl)-3-oxo-1,2,3,5-tetrahydrobenzo[e]-1,4-diazepin-4-yl]-N-(2-methoxyethyl)acetamide | 1H-NMR (CDCl3) δ ppm: 3.20-5.70 (13H, m), 6.10-8.00 (10H, m) |
| 21-40 | 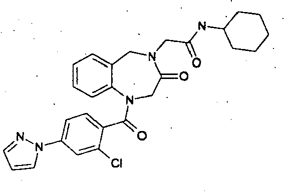 | 2-[1-(2-chloro-4-pyrazole-1-ylbenzoyl)-3-oxo-1,2,3,5-tetrahydrobenzo[e]-1,4-diazepin-4-yl]-N-cyclohexylacetamide | 1H-NMR (CDCl3) δ ppm: 0.70-1.90 (10H, m), 3.60-5.70 (7H, m), 5.70-8.20 (10H, m) |
| 21-41 | 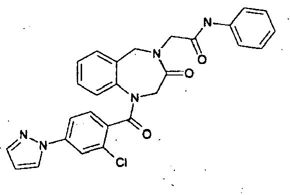 | 2-[1-(2-chloro-4-pyrazol-1-ylbenzoyl)-3-oxo-1,2,3,5-tetrahydrobenzo[e]-1,4-diazepin-4-yl]-N-phenylacetamide | 1H-NMR (CDCl3) δ ppm: 3.50-6.00 (6H, m), 6.40-8.40 (15H, m) |

**[Table 55]**

| Example | Structure formula | Compound name | 1H-NMR (solvent) or MS(m/z) |
|---|---|---|---|
| 21-42 | 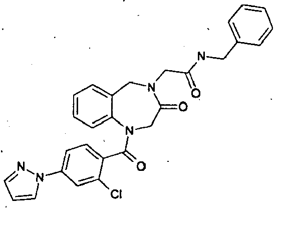 | N-benzyl-2-[1-(2-chloro-4-pyrazole-1-ylbenzoyl)-3-oxo-1,2,3,5-tetrahydrobenzo[e]-1,4-diazepin-4-yl]acetamide | 1H-NMR (CDCl3) δ ppm: 3.50-5.80 (8H, m), 6.20-8.20 (15H, m) |
| 21-43 | 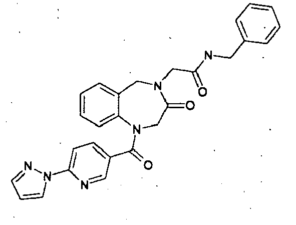 | N-benzyl-2-[3-oxo-1-(6-pyrazol-1-ylpyridine-3-carbonyl)-1,2,3,5-tetrahydrobenzo[e]-1,4-diazepin-4-yl]acetamide | 1H-NMR (CDCl3) δ ppm: 3.30-5.50 (8H, m), 6.40-8.50 (15H, m) |
| 21-44 | 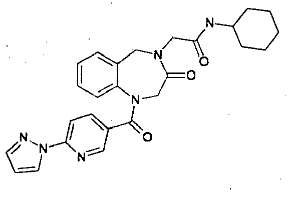 | N-cyclohexyl-2-[3-oxo-1-(6-pyrazol-1-ylpyridine-3-carbonyl)-1,2,3,5-tetrahydrobenzo[e]-1,4-diazepin-4-yl]acetamide | 1H-NMR (CDCl3) δ ppm: 0.60-2.00 (10H, m), 3.20-5.70 (7H, m), 6.90-8.50 (10H, m) |
| 21-45 | 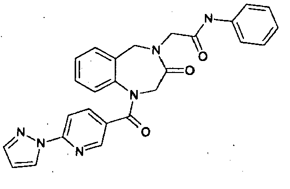 | 2-[3-oxo-1-(6-pyrazol-1-ylpyridine-3-carbonyl)-1,2,3,5-tetrahydrobenzo[e]-1,4-diazepin-4-yl]-N-phenylacetamide | 1H-NMR (CDCl3) δ ppm: 3.30-6.20 (6H, m), 6.40-8.60 (15H, m) |

**[Table 56]**

| Example | Structure formula | Compound name | 1H-NMR( solvent) or MS(m/z) |
|---|---|---|---|
| 21-46 | 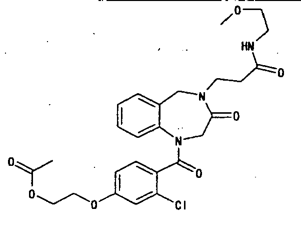 | 2-(3-chloro-4-{4-[2-(2-methoxyethylcarbamoyl)-ethyl]-3-oxo-2,3,4,5-tetrahydrobenzo[e]-1,4-diazepine-1-carbonyl}phenoxy)ethyl acetate | 1H-NMR (CDCl3) δ ppm: 2.00-2.20 (3H, m), 2.45-2.60 (2H, m), 3.25-3.50 (7H, m), 3.70-5.75 (10H, m), 6.00-7.60 (8H, m) |
| 21-47 | 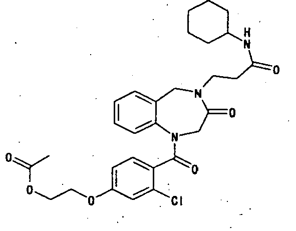 | 2-[3-chloro-4-[4-(2-cyclohexylcarbamoylethyl)-3-oxo-2,3,4,5-tetrahydrobenzo[e]-1,4-diazepine-1-carbonyl]phenoxy}ethyl acetate | 1H-NMR (CDCl3) δ ppm: 0.80-1.90 (10H, m), 1.95-2.20 (3H, m), 2.40-2.70 (2H, m), 3.60-5.90 (12H, m), 6.50-7.70 (7H, m) |
| 21-48 | 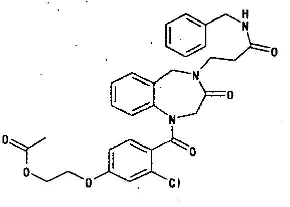 | 2-{4-[4-(2-benzylcarbamoylethyl)-3-oxo-2,3,4,5-tetrahydrobenzo[e]-1,4-diazepine-1-carbonyl]-3-chlorophenoxy}ethyl acetate | 1H-NMR (CDCl3) δ ppm: 2.00-2.20 (3H, m), 2.50-2.65 (2H, m), 3.70-5.80 (12H, m), 6.10-5.70 (12H, m) |
| 21-49 | 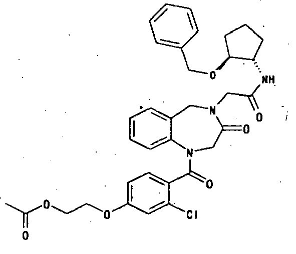 | 2-(4-[4-[((1S,2S)-2-benzyloxycyclopentylcarbam oyl)methyl]-3-oxo-2,3,4,5-tetrahydrobenzo[e]-1,4-diazepine-1-carbonyl}-3-chlorophenoxy)ethyl acetate | 1H-NMR (CDCl3) δ ppm: 1.20-1.70 (6H, m), 2.00-2.20 (3H, m), 3.60-5.50 (14H, m), 5.80-7.80 (13H, m) |

**[Table 57]**

| Example | Structure formula | Compound name | 1H-NMR(solvent) or MS(m/z) |
|---|---|---|---|
| 21-50 | 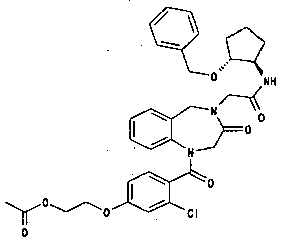 | 2-(4-[4-[((1R,2R)-2-benzyloxycyclopentyl-carbamoyl)methyl]-3-oxo-2,3,4,5-tetrahydrobenzo[e]-1,4-diazepine-1-carbonyl}-3-chlorophenoxy)ethyl acetate | 1 H-NMR (CDCl3) δ ppm: 1.20-1.80 (6H, m), 2.00-2.20 (3H, m), 3.60-5.50 (14H, m), 5.80-7.70 (13H, m) |
| 21-51 | 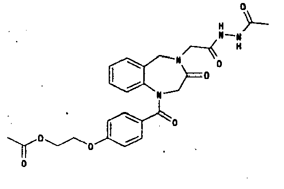 | 2-(4-{4-[2-(N'-acetylhydrazino)-2-oxoethyl]-3-oxo-2,3,4,5-tetrahydrobenzo[e]-1.4-diazepine-1-carbonyl}phenoxy)ethyl acetate | ¹H-NMR (DMSO-d₆) δ ppm:1.75-2.05 (6H, m), 3.50-6.00 (10H, m), 6.70-7.60 (8H, m), 9.00-10.25 (2H, m) |
| 21-52 | 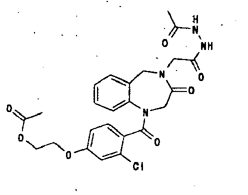 | 2-(4-{4-[2-(N'-acetylhydrazino)-2-oxoethyl]-3-oxo-2,3,4,5-tetrahydrobenzo[e]-1,4-diazepine-1-carbonyl}-3-chlorophenoxy)ethyl acetate | 1H-NMR (CDCl3) δ ppm : 1.95-2.20 (6H, m), 3.65-5.70 (10H, m), 6.50-7.70 (7H, m), 7.90-9.20 (2H, m) |

### Example 22

### {1-[4-(2-Benzyloxyethoxy)-2-chlorobenzoyl]-3-oxo-1,2,3,5-tetrahydrobenzo[e]-1,4-diazepin-4-yl}acetylazide

To a stirred mixture of {1-[4- (2-benzyloxyethoxy)-2-chlorobenzoyl]-3-oxo-1,2,3,5-tetrahydrobenzo[e]-1,4-diazepin-4-yl}acetic acid (50.9 mg), triethylamine (12.1 mg) and toluene (0.30 mL) was added diphenylphoshoryl azide (33.0 mg) at room temperature, and the reactionmixturewas stirred at roomtemperature for 2 hours. To the stirred reaction mixture was added triethylamine (5.10 mg) at room temperature, the reaction mixture was stirred at room temperature for an hour. To the stirred reaction mixture was added water at room temperature, and then extracted with ethyl acetate. The organic layer was washed with brine. The organic layer was dried over anhydrous magnesium sulfate, filtered, and the filtrate was concentrated under reduced pressure to give {1-[4-(2-benzyloxyethoxy)-2-chlorobenzoyl]-3-oxo-1,2,3,5-tetrahydrobenzo[e]-1,4-diazepin-4-yl}acetylazide (35.4 mg).

¹H-NMR (DMSO-d₆) δ ppm:
3.65-3.80 (2H, m), 4.05-4.15 (2H, m), 4.35-4.90 (8H, m), 6.65-6.80 (1H, m), 6.85-7.60 (11H, m)

### Example 23

### 1-{1-[4-(2-Benzyloxyethoxy)-2-chlorobenzoyl]-3-oxo-1,2,3,5-tetrahydrobenzo[e]-1,4-diazepin-4-ylmethyl}-3-methylurea

A solution of {1-[4-(2-benzyloxyethoxy)-2-chlorobenzoyl]-3-oxo-l,2,3,5-tetrahydrobenzo[e]-1,4-diazepin-4-yl} acetylazide (53.4 mg) in benzene (1.0 mL) was heated to reflux for an hour. To the stirred mixture were added tetrahydrofuran (1.0 mL) and 40% methylamine methanol solution (0.500 mL) at room temperature, the reaction mixture was stirred at room temperature for 2 hours. The reaction mixture was concentrated under reduced pressure, and the obtained residue was purified by column chromatography on silica gel (eluent: dichloromethane-methanol) to give 1-{1-[4-(2-benzyloxyethoxy)-2-chlorobenzoyl]-3-oxo-1,2,3,5-tetrahydrobenzo[e]-1,4-diazepin-4-ylmethyl}-3-methylurea (17.9 mg).

MS(ESI, m/z):537(M+H)⁺

### Example 24

### 2-(3-Chloro-4-{4-[2-(N'-isobutylhydrazino)-2-oxoethyl]-3-oxo-2,3,4,5-tetrahydrobenzo[e]-1,4-diazepine-1-carbonyl} phenoxy)ethyl acetate

To a stirred solution of isobutyric acid N'-(2-{1-[2-chloro-4-(2-hydroxyethoxy)benzoyl]-3-oxo-1,2,3,5,-tetrahydrobenzo[e]-1,4-diazepin-4-yl}acetyl)hydrazide (59.1 mg) and pyridine (0.0140 mL) in tetrahydrofuran (1.5 mL) was added acetyl chloride (0.0100 mL) under ice-cooling, the reaction mixture was stirred at room temperature for 1. 5 hours. To the-stirred reaction mixture were added acetyl chloride (0. 0100 mL) and pyridine (0.0140 mL) under ice-cooling, the reaction mixture was stirred at room temperature for 3.5 hours. To the stirred reaction mixture were added acetyl chloride (0.0100 mL) and pyridine (0.0140 mL) under ice-cooling, the reaction mixture was stirred at room temperature for an hour. The reaction mixture was purified by column chromatography on silica gel (eluent:ethyl acetate-methanol) to give 2-(3-chloro-4-{4-[2-(N'-isobutylhydrazino)-2-oxoethyl]-3-oxo-2,3,4,5-tetrahydrobenzo[e]-1,4-diazepine-1-carbonyl} phenoxy)ethyl acetate (53.9 mg).

¹H-NMR (CDCl₃) δ ppm:
1.10-1.25 (6H, m), 2.05-2.15 (3H, m), 2.40-2.55 (1H, m), 4.00-5.40 (10H, m), 6.50-7.60 (7H, m), 8.05-8.25 (1H, m), 8.80-9.05 (1H, m)
MS(ESI, m/z):545(M+H)⁺

### Example 25-1

### 1-[4-(2-Benzyloxyethoxy)-2-chlorobenzoyl]-4-1,3,4-oxadiazol-2-ylmethyl-1,2,4,5-tetrahydrobenzo[e]-1,4-diazepin-3-one

To a stirred solution of triphenylphosphine (77.4 mg) in dichloromethane (1.0 mL) was added carbon tetrachloride (0.0910 mL) at room temperature, and the mixture was stirred at room temperature for 20 minutes. To the mixture were successively added triethylamine (0.0660 mL) and a solution of form N'-(2-{1-[4-(2-benzyloxyethoxy)-2-chlorobenzoyl]-3-oxo-1,2,3,5-tetrahydrobenzo[e] -1,4-diazepin-4-yl}acetyl) hydrazide (65.1 mg) in dichloromethane (1.5 mL), the reaction mixture was stirred at room temperature for 3 hours. The reaction mixture was purified by column chromatography on silica gel (eluent:ethyl acetate-hexane-methanol) to give 1-[4-(2-benzyloxyethoxy)-2-chlorobenzoyl]-4-1,3,4-oxadiazol-2-ylmethyl)-1,2,4,5-tetrahydrobenzo[e]-1,4-diazepin-3-one (44.6 mg).

¹H-NMR (CDCl₃) δ ppm:
3.70-5.40 (12H, m), 6.55-7.60 (12H, m), 8.37 (1H, s)
MS(ESI, m/z):533(M+H)⁺

### Examples 25-2 to 25-6

The following compounds of Examples 25-2 to 25-6 were obtained with the use of the corresponding materials in a similar manner to that described in Example 25-1. The structure formula and physical data.of these compounds were shown in Table 58.

**[Table 58]**

| Example | Structure formula | Compound name | 1H-NMR (solvent) or MS(m/z) |
|---|---|---|---|
| 25-2 | 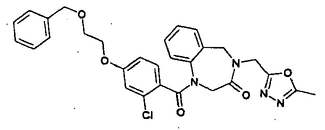 | 1-[4-(2-benzyloxyethoxy)-2-chlorobenzoyl]-4-(5-methyl-1,3,4-oxadiazol-2-ylmethyl)-1,2,4,5-tetrahydrobenzo[e]-1,4-diazepin-3-one | 1H-NMR (CDCI3) δ ppm: 2.47 (3H, s), 3.65-5.50 (12H, m), 6.55-7.60 (12H, m) MS(ESI, m/z): 547(M+H)+ |
| 25-3 | 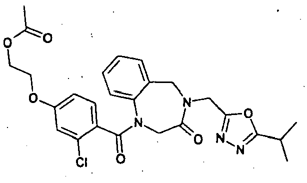 | 2-{3-chloro-4-[4-(5-isopropyl-1,3,4-oxadiazol-2-ylmethyl)-3-oxo-2,3,4,5-tetrahydrobenzo[e]-1,4-diazepine-1-carbonyl]phenoxy}ethyl acetate | 1 H-NMR (CDCl3) δ ppm: 1.33 (6H, d, J=6.2Hz), 2.05-2.20 (3H, m), 3.05-3.20 (1H, m), 4.00-5.50 (10H, m), 6.55-7.85 (7H, m) MS(ESI, m/z): 527(M+H)+ |
| 25-4 | 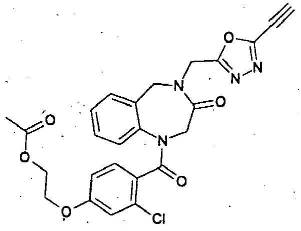 | 2-{3-chloro-4-[4-(5-ethynyl-1,3,4-oxadiazol-2-ylmethyl)-3-oxo-2,3,4,5-tetrahydrobenzo[e]-1,4-diazepine-1-carbonyl]phenoxy}ethyl acetate | MS(ESI, m/z) : 509(M+H)+ |
| 25-5 | 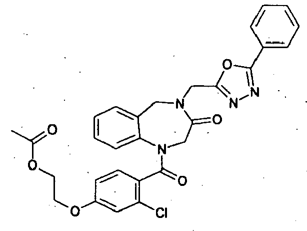 | 2-{3-chloro-4-[3-oxo-4-(5-phenyl-1,3,4-oxadiazol-2-ylmethyl)-2,3,4,5-tetrahydrobenzo[e]-1,4-diazepine-1-carbonyl]phenoxy}ethyl acetate | MS(ESI, m/z): 561(M+H)+ |
| 25-6 | 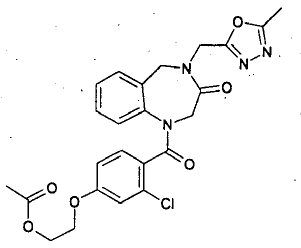 | 2-{3-chloro-4-[4-(5-methyl-1,3,4-oxadiazol-2-ylmethyl)-3-oxo-2.3,4,5-tetrahydrobenzo[e]-1,4-diazepine-1-carbonyl]-phenoxy}ethyl acetate | 1H-NMR (CDCI3) δ ppm: 2.00-2.20 (3H, m), 2.48 (3H, s), 3.20-5.60 (10H, m), 6.50-7.60 (7H, m) MS(ESI, m/z) : 499(M+H) + |

### Example 26

### 1-(2-Chloro-4-pyrrolidin-1-ylbenzoyl)-4-(3-methyl-1,2,4-oxadiazol-5-ylmethyl)-1,2,4,5-tetrahydrobenzo[e]-1,4-diazepin-3-one

A solution of 2-[1-(2-chloro-4-pyrrolidin-1-ylbenzoyl)-3-oxo-1,2,3,5-tetrahydrobenzo[e]-1,4-diazepin-4-yl]acetamide (25.0 mg) in N,N-dimethylacetamide dimethyl acetal (1.5 mL) was stirred at 120°C for an hour. The mixture was concentrated under reduced pressure. To the obtained residue were added 70% acetic acid (1.0 mL), 5 mol/L aqueous solution of sodium hydroxide (0.0500 mL), and hydroxylammonium chloride (4.90 mg), and the reaction mixture was stirred at room temperature overnight. To the reaction mixture were added water and ethyl acetate, and the separated organic layer was washed with brine. The organic layer was dried over anhydrous magnesium sulfate, filtered, and the filtrate was concentrated under reduced pressure. The obtained crude product was purified by preparative thin layer chromatography on aminopropylsilylated silica gel (eluent: ethyl acetate-hexane) to give 1-(2-chloro-4-pyrrolidin-1-ylbenzoyl)-4-(3-methyl-1,2,4-oxadiazol-5-ylmethyl)-1,2,4,5-tetrahydrobenzo[e]-1,4-diazepin-3-one (7.90 mg).

¹H-NMR (CDCl₃) δ ppm:
1.90-2.05 (4H, m), 2.42 (3H, s), 3.10-3.35 (4H, m), 4.20-5.40 (6H, m), 6.00-7.80 (7H, m)
MS(ESI, m/z):466(M+H)⁺

### Example 27

### 2-{1-[2-Chloro-4-(2-hydroxyethoxy)benzoyl]-3-oxo-1,2,3,5-tetrahydrobenzo[e]-1,4-diazepin-4-yl}-N-hydroxyacetamidine

A mixture of {1-[2-chloro-4-(2-hydroxyethoxy)benzoyl]-3-oxo-1,2,3,5-tetrahydrobenzo[e]-1,4-diazepin-4-yl}acetonitrile (177 mg), hydroxylammonium chloride (154 mg), potassium carbonate (306 mg) and ethanol (5.0 mL) was heated to reflux for an hour. After the mixture was cooled to room temperature, the precipitate was removed by filtration. The filtrate was concentrated under reduced pressure, and the obtained residue was purified by column chromatography on aminopropylsilylated silica gel (eluent:ethyl acetate-methanol) to give 2-{1-[2-chloro-4-(2-hydroxyethoxy)-benzoyl]-3-oxo-1,2,3,5-tetrahydrobenzo[e]-1,4-diazepin-4-yl}-N-hydroxyacetamidine (80.0 mg).

¹H-NMR (CDCl₃) δ ppm:
1.85-2.10 (1H, br), 3.80-5.20 (11H, m), 6.35-7.60 (7H, m)
MS(ESI, m/z):433(M+H)⁺

### Example 28

### 2-{4-[4-(2-Acetoxyimino-2-acetylaminoethyl)-3-oxo-2,3,4,5-tetrahydrobenzo[e]-1,4-diazepine-1-carbonyl]-3-chloro phenoxy}ethyl acetate

To a stirred solution of 2-{1-[2-chloro-4-(2-hydroxyethoxy)benzoyl]-3-oxo-l,2,3,5-tetrahydrobenzo[e]-1,4-diazepin-4-yl}-N-hydroxyacetamidine (40.0mg) in pyridine (3.0mL) was added acetyl chloride (0.0330mL) under ice-cooling, the reaction mixture was stirred at room temperature for 12 hours. The mixture was concentrated under reduced pressure, and the obtained residue was purified by column chromatography on silica gel (eluent:ethyl acetate-methanol) to give 2-{4-{4-(2-acetoxyimino-2-acetylaminoethyl)-3-oxo-2,3,4,5-tetrahydrobenzo[e]-1,4-diazepine-1-carbonyl]-3-chlorophenoxy}ethyl acetate (33.1 mg).

MS(ESI, m/z):559(M+H)⁺

### Example 29

### 2-{3-Chloro-4-[4-(5-methyl-[1,2,4] oxadiazol-3-ylmethyl) -3-oxo-2,3,4,5-tetrahydrobenzo[ e]-1,4-diazepine-1-carbonyl]-phenoxy}ethyl acetate

2-{4-[4-(2-Acetoxyimino-2-acetylaminoethyl)-3-oxo-2,3,4,5-tetrahydrobenzo[e]-1,4-diazepine-1-carbonyl]-3-chlorophenoxy}ethyl acetate (33.1 mg) was dissolved in pyridine (3.0 mL), and the solution was stirred at 100°C for 2.5 hours. The mixture was concentrated under reduced pressure, and the obtained residue was purified by column chromatography on aminopropylsilylated silica gel (eluent:ethyl acetate-hexane) to give 2-{3-chloro-4-[4-(5-methyl-[1,2,4] oxadiazol-3-ylmethyl)-3-oxo-2,3,4,5-tetrahydrobenzo[e]-1,4-diazepine-1-carbonyl]phenoxy}ethyl acetate (15.3 mg).

¹H-NMR (CDCl₃) δ ppm:
2.05-2.20 (3H, m), 2.59 (3H, s), 4.00-6.00 (10H, m), 6.50-7.80 (7H, m)
MS(ESI, m/z):499(M+H)⁺

### Example 30

### 2-{3-Chloro-4-[4-(5-methyl-4,5-dihydrooxazol-2-ylmethyl)-3-oxo-2,3,4,5-tetrahydrobenzo[e]-1,4-diazepine-1-carbonyl]-phenoxy}ethyl acetate

To a stirred solution of 2-(3-chloro-4-{4-[(2-hydroxypropylcarbamoyl)methyl]-3-oxo-2,3,4,5-tetrahydrobenzo[e]-1,4-diazepine-1-carbonyl}phenoxy)ethyl acetate (365 mg) in tetrahydrofuran (5.0 mL) was added (methoxycarbonylsulfamoyl)-triethylammonium hydroxide (252 mg) at room temperature, the solution was stirred at 60°C for an hour. After being cooled to room temperature, the mixture was concentrated under reduced pressure. The obtained crude product was purified by column chromatography on aminopropylsilylated silica gel (eluent:ethyl acetate-methanol) to give 2-{3-chloro-4-[4-(5-methyl-4,5-dihydrooxazol-2-ylmethyl)- 3-oxo-2,3,4,5-tetrahydrobenzo[e]-1,4-diazepine-1-carbonyl]phenoxy}ethyl acetate (153 mg).

¹H-NMR (CD₃OD) δ ppm:
1.15 (3H, d, J=6.3Hz), 2.05-2.15 (3H, m), 3.00-5.20 (13H, m), 6.60-7.80 (7H, m)
MS(ESI, m/z) :500(M+H)⁺

### Example 31-1

### {1-[4-(2-Acetoxyethoxy)-2-chloro-benzoyl]-3-oxo-1,2,3,5-tetrahydrobenzo[e]-1,4-diazepin-4-yl} acetic acid

To a stirred solution of benzyl {1-[4-(2-acetoxyethoxy)-2-chlorobenzoyl]-3-oxo-1,2,3,5-tetrahydrobenzo[e]-1,4-diazepin-4-yl}acetate (734 mg) in tetrahydrofuran (10.0 mL) was added 10% palladium-carbon (150 mg) at room temperature under an argon atmosphere, the suspension was stirred under a hydrogen atmosphere at room temperature for 0.5 hour. The catalyst was removed by Celite filtration, the solvent was removed under reduced pressure to give {1-[4-(2-acetoxyethoxy)-2-chloro-benzoyl]-3-oxo-1,2,3,5-tetrahydrobenzo[e]-1,4-diazepin-4-yl}acetic acid (632 mg).

¹H-NMR (CDCl₃) δ ppm:
2.00-2.15 (3H, m), 4.00-5.60 (10H, m), 6.50-7.60 (7H, m)

### Examples 31-2 to 31-19

The following compounds of Examples 31-2 to 31-19 were obtained with the use of the corresponding materials in a similar manner to that described in Example 31-1. The structure formula and physical data of these compounds were shown in Tables 59 to 63.

**[Table 59]**

| Example | Structure formula | Compound name | 1 H-NMR (solvent) or MS(m/z) |
|---|---|---|---|
| 31-2 | 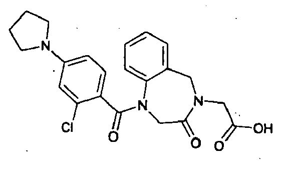 | [1-(2-chloro-4-pyrrolidin-1-ylbenzoyl) -3-oxo-1,2,3,5-tetrahydrobenzo[e]-1,4-diazepin-4-yl]acetic acid | 1H-NMR(DMSO-d6) δ ppm: 1.80-1.95 (4H, m), 3.05-3.25 (4H, m), 4.21 (2H, s), 4.25-4.95 (4H, m), 6.10-7.55 (7H, m), 12.74 (1H, brs) MS(ESI, m/z) : 428(M+H)+ |
| 31-3 | 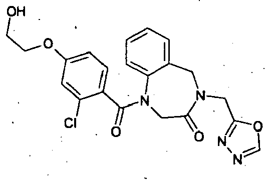 | 1-[2-chloro-4-(2-hydroxyethoxy)benzoyl]-4-1,3,4,-oxadiazole-2-ylmethyl-1,2,4,5-tetrahydrobenzo[e]-1,4-diazepin-3-one | 1H-NMR (CDCl3) δ ppm: 1.90-2.15 (1H, br), 3.60-5.60 (10H, m), 6.50-7.60 (7H, m), 8.20-8.45 (1H, m) MS(ESI, m/z) : 443(M+H)+ |
| 31-4 | 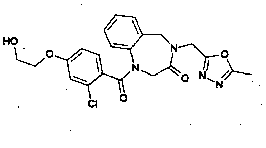 | 1-[2-chloro-4-(2-hydroxyethoxy)benzoyl]-4-(5-methyl-1,3,4,-oxadiazol-2-ylmethyl)-1,2,4,5-tetrahydrobenzo[e]-1,4-diazepin-3-one | 1H-NMR (CDCl3) δ ppm: 1.85-2.05 (1H, br), 2.48 (3H, s), 3.85-5.60 (10H, m), 6.55-7.60 (7H, m) MS(ESI, m/z) : 457(M+H)+ |
| 31-5 | 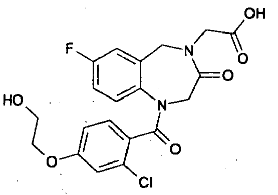 | {1-[2-chloro-4-(2-hydroxyethoxy)benzoyll-7-fluoro-3-oxo-1,2,3,5-tetrahydrobenzo[e]-1,4-diazepin-4-yl}acetic acid | 1H-NMR(DMSO-d6) δ ppm: 3.60-5.05 (10H, m), 6.70-7.60 (7H, m), 12.81 (1 H, brs) |
| 31-6 | 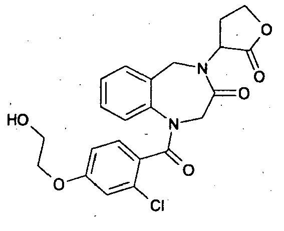 | 1-[2-chloro-4-(2-hydroxyethoxy)benzoyl]-4-(2-oxotetrahydrofuran-3-yl)-1,2,4,5-tetrahydrobenzo[e]-1,4-diazepin-3-one | 1H-NMR (CDCl3) d ppm: 0.80-1.40 (2H, m), 1.85-2.70 (3H, m), 3.65-5.80 (8H, m), 5.70-7.60 (7H, m) |

**[Table 60]**

| Example | Structure formula | Compound name | 1H-NMR (solvent) or MS(m/z) |
|---|---|---|---|
| 31-7 | 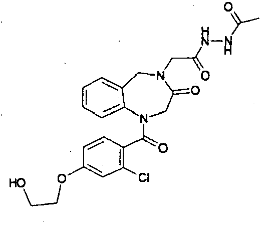 | acetic acid N'-(2-{1-[2-chloro-4-(2-hydroxyethoxy)benzoyl]-3-oxo-1,2,3,5-tetrahydrobenzo[e]-1,4-diazepin-4-yl}acetyl)hydrazide | MS(ESI, m/z): 473(M-H)- |
| 31-8 | 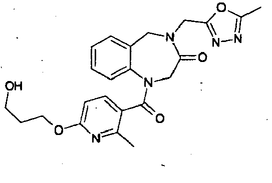 | 1-[6-(3-hydroxypropoxy)-2-methylpyridine-3-carbonyl]-4-(5-methyl-1,3,4-oxadiazol-2-ylmethyl)-1,2,4,5-tetrahydrobenzo[e]-1,4-diazepin-3-one | 1H-NMR (CD30D) δ ppm: 1.85-2.00 (2H, m), 2.45-2.60 (6H, m), 2.85-3.00 (1 H, m), 3.60-3.75 (2H, m), 4.40-5.20 (8H, m), 6.15-7.50 (7H, m) MS(ESI, m/z): 452(M+H)+ |
| 31-9 | 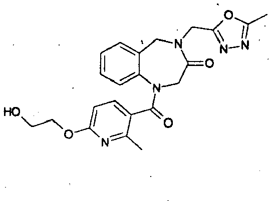 | 1-[6-(2-hydroxyethoxy)-2-methylpyridine-3-carbonyl]-4-(5-methyl-1,3,4-oxadiazol-2-ylmethyl)-1,2,4,5-tetrahydrobenzo[e]-1,4-diazepin-3-one | 1H-NMR (CD3OD) δ ppm: 2.45-2.60 (6H, m), 3.35-3.50 (1H, m), 3.85-3.95 (2H, m), 4.35-5.20 (8H, m), 6.10-7.30 (6H, m) MS(ESI, m/z): 438(M+H)+ |
| 31-10 | 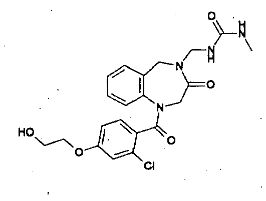 | 1-{1-[2-chloro-4-(2-hydroxyethoxy)benzoyl]-3-oxo-1,2,3,5-tetrahydrobenzo[e]-1,4-diazepin-4-ylmethyl}-3-methylurea | 1H-NMR (CDCl3) δ ppm: 2.00-6.00 (14H, m), 6.40-8.40 (7H, m) MS(ESI, m/z) : 469(M+Na)+ |

**[Table 61]**

| Example | Structure formula | Compound name | 1 H-NMR (solvent) or MS(m/z) |
|---|---|---|---|
| 31-11 | 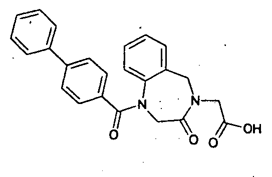 | 1-(biphenyl-4-carbonyl)-3-oxo-1,2,3,5-tetrahydrobenzo[e]-1,4-diazepin-4-ylacetic acid | 1H-NMR (DMSO-d6) δ ppm: 3.50-5.65 (6H, m), 6.60-7.70 (13H, m), 12.84 (1H, brs) MS(ESI, m/z): 401 (M+H)+ |
| 31-12 | 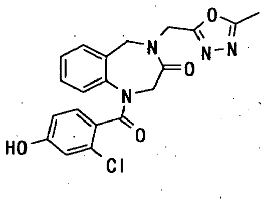 | 1-(2-chloro-4-hydroxyb6nzoyl)-4-(5-methyl-1,3,4,-oxadiazol-2-ylmethyl)-1,2,4,5-tetrahydrobenzo[e]-1,4-diazepin-3-one | 1H-NMR (CD30D) δ ppm: 2.47 (3H, s), 4.00-5.70 (6H, m), 6.35-7.75 (7H, m) MS(ESI, m/z) : 413(M+H)+ |
| 31-13 | 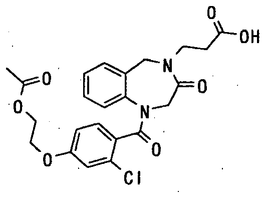 | 3-{1-[4-(2-acetoxyethoxy)-2-chlorobenzoyl]-3-oxo-1,2,3,5-tetrahydrobenzo[e]-1,4-diazepin-4-yl}propionic acid | 1H-NMR (CDCl3) δ ppm: 2.00-2.20 (3H, m), 2.65-2.80 (2H, m), 3.70-6.00 (10H, m), 6.50-7.80 (7H, m) MS(ESI, m/z): 475(M+H)+ |
| 31-14 | 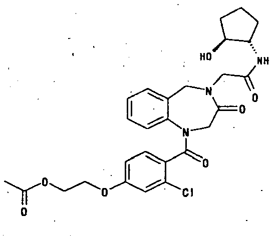 | 2-(3-chloro-4-[4-[((l S,2S)-2-hydroxycyclopentyl-carbamoyl)methyl]-3-oxo-2,3,4,5-tetrahydrobenzo[e]-1,4-diazepine-1-carbonyl}phenoxy)ethyl acetate | 1H-NMR (CDCI3) d ppm: 1.20-2.25 (9H, m), 3.70-5.70 (12H, m), 6.00-7.70 (8H, m) |

**[Table 62]**

| Example | Structure formula | Compound name | 1 H-NMR (solvent) or MS(m/z) |
|---|---|---|---|
| 31-15 | 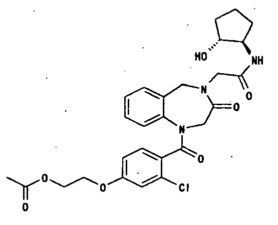 | 2-(3-chloro-4-{4-[((1 R,2R)-2-hydroxycyclopentyl-carbamoyl)methyl]-3-oxo-2,3,4,5-tetrahydrobenzo[e]-1,4-diazepine-1-carbonyl}phenoxy)ethyl acetate | 1H-NMR (CDCl3) d ppm: 1.20-1.80 (6H, m), 2.00-2.20 (3H, m), 3.60-5.50 (12H, m), 5.80-7.70 (8H, m) |
| 31-16 | 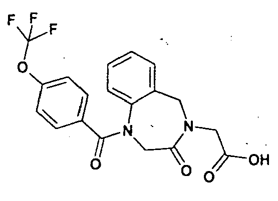 | 3-oxo-1-(4-trifluoromethoxybenzoyl)-3-oxo-1,2,3,5-tetrahydrobenzo[e]-1,4-diazepin-4-ylacetic acid | 1H-NMR(DMSO-d6) δ ppm: 3.50-5.50 (6H, m), 6.60-8.10 (8H, m), 12.91 (1H, brs) |
| 31-17 | 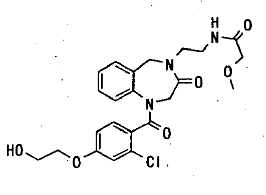 | N-(2-{1-[2-chloro-4-(2-hydroxyethoxy)benzoyl]-3-oxo-1,2,3,5-tetrahydrobenzo[e]-1,4-diazepin-4-yl}ethyl)-2-methoxyacetamide | 1H-NMR (CDCl3) δ ppm: 2.40-2.70 (1H, m), 3.00-6.00 (17H, m), 6.50-7.60 (7H, m) |
| 31-18 | 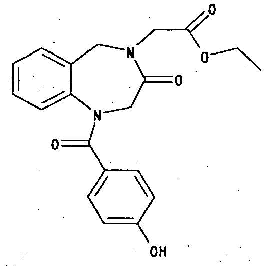 | ethyl [1-(4-hydroxybenzoyl)-3-oxo-1,2,3,5-tetrahydrobenzo[e]-1,4-diazepin-4-yl]acetate | MS(ESI, m/z): 369(M+H)+ |

**[Table 63]**

| Example | Structure formula | Compound name | 1 H-NMR (solvent) or HS(m/z) |
|---|---|---|---|
| 31-19 | 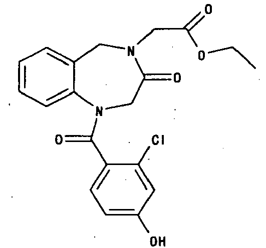 | ethyl [1-(2-chloro-4-hydroxybenzoyl)-3-oxo-1,2,3,5-tetrahydrobenzo[e]-1,4-diazepin-4-yl]acetate | MS(ESI, m/z): 403(M+H)+ |

### Example 32

### {1-[2-Chloro-4-(2-hydroxyethoxy)benzoyl]-3-oxo-1,2,3,5-tetrahydrobenzo[e]-1,4-diazepin-4-yl}acetonitrile

To a stirred solution of 2-{1-[2-chloro-4-(2-hydroxyethoxy)benzoyl]-3-oxo-1,2,3,5-tetrahydrobenzo[e]-1,4-diazepin-4-yl}acetamide (41.2 mg) and triethylamine (0.0480 mL) in dichloromethane (1.5 mL) was added trifluoroacetic anhydride (0.0350 mL) at room temperature, the reaction mixture was stirred at room temperature for 15 hours. At the same condition, to the reaction mixture were added trifluoroacetic anhydride (0.0350 mL) and triethylamine (0.0480 mL), the reaction mixture was stirred at room temperature for an hour. The reaction mixture was concentrated under reduced pressure, and the obtained residue was purified by column chromatography on aminopropylsilylated silica gel (eluent:ethyl acetate-methanol) to give{1-[2-chloro-4-(2-hydroxyethoxy)benzoyl]-3-oxo-1,2,3,5-tetrahydrobenzo[e]-1,4-diazepin-4-yl}acetonitrile (33.2 mg).

¹H-NMR (CDCl₃) δ ppm:
1.85-2.00 (1H, m), 3.85-5.50 (10H, m), 6.60-7.65 (7H, m)
MS(ESI, m/z):400(M+H)⁺

### Example 33

### [1-(2-Chloro-4-pyrrolidin-1-ylbenzoyl)-3-oxo-1,2,3,5-tetrahydrobenzo[e]-1,4-diazepin-4-yl]acetohydrazide

A mixture of benzyl [1-(2-chloro-4-pyrrolidin-1-ylbenzoyl)-3-oxy-1,2,3,5-tetrahydrobenzo[e]-1,4-diazepin-4-yl] acetate (0.293 g) andhydrazinemonohydrate (0.0567 g) inmethanol (5.0 mL) was heated to reflux for 15 hours. To the reaction mixture was added water, and then extracted with dichloromethane. This organic layer was dried over anhydrous magnesium sulfate, and concentrated under reduced pressure. The residue was dissolved in dichloromethane, to the mixture was added diethyl ether. The deposited precipitate was collected by filtration to give [1-(2-chloro-4-pyrrolidin-1-ylbenzoyl)-3-oxo-1,2,3,5-tetrahydrobenzo[e]-1,4-diazepin-4-yl] acetohydrazide (0.175 g).

1.90-2.10 (4H, m), 3.10-3.35 (4H, m), 3.60-5.00 (6H, m), 6.00-7.70 (7H, m)
MS(ESI, m/z) : 442 (M+H) ⁺

### Example 34

### 4-Methyl-1-(2-methyl-6-pyrrolidin-1-ylpyridine-3-carbonyl)-1,2,4,5-tetrahydrobenzo[e]-1,4-diazepin-3-one

A solution of 1-(6-chloro-2-methylpyridine-3-carbonyl)-4-methyl-1,2,4,5-tetrahydrobenzo[e]-1,4- diazepin-3-one (30.0 mg) and pyrrolidine (0.0380 mL) in N-methylpyrrolidone (0.5 mL) was stirred at an external temperature of 100°C for 10 hours. After being cooled, to the reaction solution was added water, and then extracted with ethyl acetate. The separated organic layer was washed with water and brine. The organic layer was dried over anhydrous magnesium sulfate, filtered. The filtrate was concentrated under reduced pressure and the obtained crude product was purified by column chromatography on silica gel (eluent:ethyl acetate-hexane). Fractions containing the object substance were collected, then concentrated under reduced pressure, and the crystallization was employed with the addition of ethyl acetate and diisopropyl ether to the residue. The deposited crystal was collected by filtration to;give4-methyl-1-(2-methyl-6-pyrrolidin-1-ylpyridine-3-carbonyl)-1,2,4,5-tetrahydrobenzo[e]-1,4- diazepine-3-one (18.8 mg).

¹H-NMR (CDCl₃) δ ppm:
1.90-2.00 (4H, m), 2.52 (3H; s), 3.16 (3H, s), 3.30-3.45 (4H, m), 4.20-5.20 (4H, brm), 5.87 (1H, dd, J=8.8Hz), 6.80-7.00 (2H, m), 7.15-7.35 (3H, m)

### Example 35

### 2-[1-(2-Chloro-4-pyrrolidin-1-ylbenzoyl)-3-oxo-1,2,3,5-tetrahydrobenzo[e]-1,4-diazepin-4-yl]acetamide

A mixture of [1-(2-chloro-4-pyrrolidin-1-ylbenzoyl)-3-oxo-1,2,3,5-tetrahydrobenzo[e]-1,4-diazepin-4-yl]acetic acid (50.0 mg), N;N-carbodiimidazole (20.0 mg) and tetrahydrofuran (3.0 mL) was stirred at room temperature for 0.75 hour. To the mixture was added 28% aqueous solution of ammonia (0. 500 mL), and the mixture was stirred at room temperature overnight. The solvent was concentrated under reduced pressure, and the obtained residue was purified by column chromatography on aminopropylsilylated silica gel (eluent: ethyl acetate-methanol) and silylated silica gel with benzenesulfonic acid (eluent:methanol) to give 2-[1-(2-chloro-4-pyrrolidin-1-ylbenzoyl)-3-oxo-1,2,3,5-tetrahydrobenzo[e]-1,4-diazepin-4-yl]acetamide (25.9 mg).

¹H-NMR (CD₃OD) δ ppm:
1.90-2.10 (4H, m), 3.10-3.30 (4H, m), 4.00-5.00 (6H, m), 6.00-7.60 (7H, m)
MS(ESI, m/z) :427 (M+H) ⁺

### Example 36

### 2-{1-[2-Chloro-4-(2-hydroxyethoxy)benzoyl]-3-oxo-1,2,3,5-tetrahydrobenzo[e]-1,4-diazepin-4-yl}acetamide

To a stirred solution of {1-[2-chloro-4-(2-hydroxyethoxy)benzoyl]-3-oxo-1,2,3,5-tetrahydrobenzo[e]-1,4-diazepin-4-yl}acetic acid (96.1 mg) and di-tert-butyl dicarbonate (150 mg) in N,N-dimethylformamide (2.0mL) were added ammonium carbonate (53.7 mg) and pyridine (0.0556mL) at room temperature, the reaction mixture was stirred at room temperature for 11 hours. The reaction mixture was diluted with water and then extracted with ethyl acetate. The organic layer was concentrated under reduced pressure, and the obtained crude product was purified by column chromatography on aminopropylsilylated silica gel (eluent: ethyl acetate-methanol) to give 2-{1-[2-chloro-4- (2-hydroxyethoxy) benzoyl]-3-oxo-1,2,3,5-tetrahydrobenzo[e]-1,4-diazepin-4-yl} acetamide (46.6 mg).

¹H-NMR (CD₃OD) δ ppm:
3.70-4.75 (10H, m), 6.60-7.65 (7H, m)

### Example 37-1

### 1-[2-Chloro-4-(2-hydroxyethoxy)benzoyl]-4-(5-ethyl-1,3,4-oxadiazol-2-ylmethyl)-1,2,4,5-tetrahydrobenzo[e]-1,4-diazepin-3-one

### (Process A)

To a stirred solution of {1-[4-(2-acetoxyethoxy)-2-chlorobenzoyl] -3-oxo-1,2, 3, 5-tetrahydrobenzo[e]-1,4-diazepin-4-yl}acetic acid (80.0 mg), propionohydrazide (16.9 mg) and hydroxybenzotriazole monohydrate (39.9mg) in N,N-dimethylformamide (1.0 mL) was added 1-ethyl-3-(dimethylaminopropyl)carbodiimide hydrochloride (50.0 mg) at room temperature, the solution was stirred at the same temperature for an hour. To the solution were added water and ethyl acetate, the mixture was stirred for 5 minutes. The organic layer was separated. The combined organic layer was concentrated under reduced pressure. The crude product was purified by column chromatography on aminopropylsilylated silica gel (eluent:hexane-ethyl acetate-methanol) to give 2-(3-chloro-4-{3-oxo-4-[2-oxo-2-(N'-propionylhydrazino)-ethyl]-2,3,4,5-tetrahydrobenzo[e]-1,4-diazepine-1-carbonyl}phenoxy)ethyl acetate (74.8 mg).
¹H-NMR (CDCl₃) δ ppm:
1.05-1.20 (3H, m), 2.00-2.15 (3H, m), 2.20-2.35 (2H, m), 3.90-5.70 (10H, m), 6.50-7.60 (7H, m), 7.90-9.20 (2H, br)
MS(ESI, m/z):531(M+H)⁺

### (Process B)

A suspension of polymer-bounded triphenylphosphine (0.15g, load:2.2 mmol/g) in carbon tetrachloride (1.0 mL) was stirred at room temperature for 20 minutes. Acetonitrile (1.0mL) of the acetate derivatives obtained in Process A (0.075 g) and diisopropylethylamine (0.073 g) was added to it, the mixture was stirred at 60°C for 30 minutes. An insoluble material was removed by filtration, and the filtrate was concentrated under reduced pressure to give 2-{3-chloro-4-[4-(5-ethyl-1,3,4-oxadiazol-2-ylmethyl)-3-oxo-2,3,4,5-tetrahydrobenzo[e]-1,4-diazepine-1-carbonyl]-phenoxy} ethyl acetate(0.114 g).
¹H-NMR (CDCl₃) δ ppm:
1.25-1.40 (3H, m), 2.00-2.20 (3H, m), 2.75-2.90 (2H, m), 3.70-5.50 (10H, m), 6.50-7.80 (7H, m)
MS(ESI, m/z):513(M+H)⁺

### (Process C)

To a stirred solution of the acetate derivatives obtained in Process B (72.3 mg) in methanol (1.0 mL) was added 2 mol/L aqueous solution of sodium hydroxide (0.036 mL) at room temperature, and the mixture was stirred at room temperature for 45 minutes. Two mol/L aqueous solution of sodium hydroxide (0.070 mL) was added to the mixture, and the mixture was stirred at room temperature for 3.5 hours. Two mol/L aqueous solution of sodium hydroxide (0.036 mL) was added to the mixture, and the mixture was stirred at room temperature for 0.5 hour. To the mixture was added 2 mol/L hydrochloric acid (0.176 mL). To the mixture were successively added water and ethyl acetate, and the organic layer was separated. The aqueous layer was extracted with ethyl acetate, the organic layer was combined and the solvent was removed under reduced pressure. The crude product was purified by column chromatography on aminopropylsilylated silica gel (eluent:hexane-ethyl acetate) to give 1-[2-chloro-4-(2-hydroxyethoxy)benzoyl]-4-(5-ethyl-1,3,4-oxadiazol-2-ylmethyl)-1,2,4,5-tetrahydrobenzo[e]-1,4-diazepin-3-one (39.8 mg).
¹H-NMR (CDCl₃) δ ppm:
1.25-1.40 (3H, m), 1.85-2.05 (1H, m), 2.75-2.90 (2H, m), 3.80-5.70 (10H, m), 6.50-7.60 (7H, m)
MS(ESI, m/z):471(M+H)⁺

### Example 37-2

### 1-[2-Chloro-4-(2-hydroxyethoxy)benzoyl]-4-(5-propyl-1,3,4-oxadiazol-2-ylmethyl)-1,2,4,5-tetrahydrobenzo[e]-1,4-diazepin-3-one

2-(4-{4-[2-(N-Butylylhydrazino)-2-oxoethyl]-3-oxo-2,3,4,5-tetrahydrobenzo[e]-1,4-diazepine-1-carbonyl}-3-chlorophenoxy)ethyl acetate was obtained by condensation of ll-[4-(2-acetoxyethoxy)-2-chloro-benzoyl]-3-oxo-1,2,3,5-tetrahydrobenzo[e]-1,4-diazepin-4-yl}acetic acid with butyrohydrazide in a similar manner to that described in Process A of Example 37-1. MS(ESI, m/z):545(M+H)⁺
2-{3-Chloro-4-[4-(5-propyl-1,3,4-oxadiazol-2-ylmethyl)-3-oxo-2,3,4,5-tetrahydrobenzo[e]-1,4-diazepine-1-carbonyl]-phenoxy}ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-(5-propyl-1,3,4-oxadiazol-2-ylmethyl)-1,2,4,5-tetrahydrobenzo[e]-1,4-diazepin-3-one.
¹H-NMR (CDCl₃) δ ppm:
0.80-1.10 (3H, m), 1.70-1.85 (2H, m), 2.00-2.30 (1H, m), 2.70-2.90 (2H, m), 3.50-5.70 (10H, m), 6.50-7.70 (7H, m)
MS(ESI, m/z):485(M+H)⁺

### Example 37-3

### 4-(5-Butyl-1,3,4-oxadiazol-2-ylmethyl)-1-[2-chloro-4-(2-hydroxyethoxy)benzoyl]-1,2,4,5-tetrahydrobenzo[e]-1,4-diazepin-3-one

2-(3-Chloro-4-{3-oxo-4-[2-oxo-2-(N'-pentanoylhydrazino)ethyl]-2,3,4,5-tetrahydrobenzo[e]-1,4-diazepine-1-carbonyl}phenoxy)ethyl acetate was obtained by condensation of {1-[4-(2-acetoxyethoxy)-2-chloro-benzoyl]-3-oxo-1,2,3,5-tetrahydrobenzo[e] 1,4-diazepin-4-yl} acetic acid with pentanohydrazide in a similar manner to that described in Process A of Example 37-1. MS(ESI, m/z):559(M+H)⁺
2-{3-Chloro-4-[4-(5-butyl-1,3,4-oxadiazol-2-ylmethyl)-3-oxo-2,3,4,5-tetrahydrobenzo[e]-1,4-diazepine-1-carbonyl]-phenoxy} ethyl acetate was obtained by cyclization of the obtained acetate derivative in a similar manner to that described in Process B of Example 37-1, and was de-acetylated in a similar manner to that described in Process C of Example 37-1 to give 4-(5-butyl-1,3,4-oxadiazol-2-ylmethyl)-1-[2-chloro-4-(2-hydroxyethoxy)-benzoyl]-1,2,4,5-tetrahydrobenzo[e]-1,4-diazepin-3-one.
¹H-NMR (CDCl₃) δ ppm:
0.92 (3H, t, J=7.4Hz), 1.30-1.45 (2H, m), 1.65-1.80 (2H,m), 1.85-2.05 (1H, m), 2.78 (2H, t, J=7.4Hz), 3.80-5.70 (10H, m), 6.50-7.70 (7H, m)
MS(ESI, m/z):499(M+H)⁺

### Example 37-4

### 4-(5-tert-Butyl-1,3,4-oxadiazol-2-ylmethyl)-1-[2-chloro-4-(2-hydroxyethoxy)benzoyl]-1,2,4,5-tetrahydrobenzo[e]-1,4-diazepin-3-one

2-[3-Chloro-4-(4-{2-[N'-(2,2-dimethylpropionyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl} acetic acid with pivalohydrazide in a similar manner to that described in Process A of Example 37-1. MS(ESI, m/z):559(M+H)⁺
2-{3-Chloro-4-[4-(5-tert-butyl-1,3,4-oxadiazol-2-ylmethyl)-3-oxo-2,3,4,5-tetrahydrobenzo[e]-1,4-diazepine-1-carbonyl] phenoxy} ethyl acetate was obtained by cyclization of the obtained acetate derivative in a similar manner to that described in process B of Example 37-1, and was de-acetylated in a similar manner to that described in process C of Example 37-1 to give 4-(5-tert-butyl-1,3,4-oxadiazol-2-ylmethyl)-1-[2-chloro-4-(2-hydroxyethoxy)benzoyl]-1,2,4,5-tetrahydrobenzo[e]-1,4-diazepin-3-one.
¹H-NMR (CDCl₃) δ ppm:
1.34 (9H, s), 1.85-2.05 (1H, m), 3.70-5.70 (10H, m), 6.50-7.70 (7H, m)
MS(ESI, m/z):499(M+H)⁺

### Example 37-5

### 1-[2-chloro-4-(2-hydroxyethoxy)benzoyl]-4-[5-(2-methoxyethyl)-1,3,4-oxadiazole-2-ylmethyl]-1,2,4,5-tetrahydrobenzo[e]-1,4-diazepin-3-one

2-[3-Chloro-4-(4-{2-[N'-(3-methoxypropionyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 3-methoxypropionohydrazide in a similar manner to that described in Process A of Example 37-1. MS(ESI, m/z):561(M+H)⁺
2-(3-Chloro-4-{4-[5-(2-methoxyethyl)-1,3,4-oxadiazol-2-ylmethyl]-3-oxo-2,3,4,5-tetrahydrobenzo[e]-1,4-diazepine-1-carbonyl} phenoxy) ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-(5-(2-methoxyethyl)-1,3,4-oxadiazol-2-ylmethyl)-1,2,4,5-tetrahydrobenzo[e]-1,4-diazepin-3-one.
¹H-NMR (CDCl₃) δ ppm:
1.90-2.10 (1H, m), 3.07 (2H, t, J=6.5Hz), 3.35 (3H, s), 3.75 (2H, t, J=6.5Hz), 3.80-5.70 (10H, m), 6.50-7.70 (7H, m)
MS(ESI, m/z):501(M+H)⁺

### Example 37-6

### 1-[2-Chloro-4-(2-hydroxyethoxy)benzoyl]-4-[5-(4-fluorophenyl)-1,3,4-oxadiazol-2-ylmethyl]-1,2,4,5-tetrahydrobenzo[e]-1,4-diazepin-3-one

2-[3-Chloro-4-(4-{2-[N'-(4-fluorobenzoyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 4-fluorobenzohydrazide in a similar manner to that described in Process A of Example 37-1.
2-(3-Chloro-4-{4-[5-(4-fluorophenyl)-1,3,4-oxadiazol-2-ylmethyl]-3-oxo-2,3,4,5-tetrahydrobenzo[e]-1,4-diazepine-1-carbonyl}phenoxy)ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(4-fluorophenyl)-1,3,4-oxadiazol-2-ylmethyl]-1,2,4,5-tetrahydrobenzo[e]-1,4-diazepin-3-one.
¹H-NMR (CDCl₃) δ ppm:
1.85-2.10 (1H, m), 3.80-5.70 (10H, m), 6.55-8.10 (11H, m)
MS(ESI, m/z):537(M+H)⁺

### Example 37-7

### 1-[2-Chloro-4-(2-hydroxyethoxy)benzoyl]-4[5-(3-fluorophenyl)-1,3,4-oxadiazol-2-ylmethyl]-1,2,4,5-tetrahydrobenzo[e]-1,4-diazepin-3-one

2-[3-Chloro-4-(4-{2-[N'-(3-fluorobenzoyl)hydrazino]-2-oxoethyl}-3-oxo-2,3,4,5-tetrahydrobenzo[e]-1,4-diazepine-1-carbonyl)phenoxy]ethyl acetate was obtained by.condensation of {1-[4-(2-acetoxyethoxy)-2-chloro-benzoyl]-3-oxo-1,2,3,5-tetrahydrobenzo[e]-1,4-diazepin-4-yl}acetic acid with 3-fluorobenzohydrazide in a similar manner to that described in Process A of Example 37-1.
2-(3-Chloro-4-{4-[5-(3-fluorophenyl)-1,3,4-oxadiazol-2-ylmethyl]-3-oxo-2,3,4,5-tetrahydrobenzo[e]-1,4-diazepine-1-carbonyl}phenoxy)ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-{5-(3-fluorophenyl)-1,3,4-oxadiazol-2-ylmethyl]-1,2,4,5-tetrahydrobenzo[e]-1,4-diazepin-3-one.
¹H-NMR (CDC1₃) δ ppm:
1.90-2.10 (1H, m), 3.80-5.70 (10H, m), 6.50-7.90 (11H, m)
MS(ESI, m/z):537(M+H)⁺

### Example 37-8

### 1-[2-Chloro-4-(2-hydroxyethoxy)benzoyl]-4-[5-(2-fluorophenyl)-1,3,4-oxadiazol-2-ylmethyl]-1,2,4,5-tetrahydrobenzo[e]-1,4-diazepin-3-one

2-[3-Chloro-4-(4-{2-[N'-(2-fluorobenzoyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 2-fluorobenzohydrazide in a similar manner to that described in Process A of Example 37-1.
2-(3-chloro-4-{ 4-[5-(2-fluorophenyl)-1,3,4-oxadiazol-2-ylmethyl]-3-oxo-2,3,4,5-tetrahydrobenzo[e]-1,4-diazepine-1-carbonyl}phenoxy)ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(2-fluorophenyl)-1,3,4-oxadiazol-2-ylmethyl]-1,2,4,5-tetrahydrobenzo[e]-1,4-diazepin-3-one.
¹H-NMR (CDCl₃) δ ppm:
1.90-2.10 (1H, m), 3.80-5.70 (10H, m), 6.50-8.10 (11H, m)
MS(ESI, m/z):537(M+H)⁺

### Example 37-9

### 1-[2-Chloro-4-(2-hydroxyethoxy)benzoyl]-4-[5-(3-methylphenyl)]-1,3,4-oxadiazol-2-ylmethyl)-1,2,4,5-tetrahydrobenzo[e]-1,4-diazepin-3-one

2-[3-Chloro-4-(4-{ 2-[N'-(3-methylbenzoyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl} acetic acid with 3-methylbenzohydrazide in a similar manner to that described in Process A of Example 37-1.
2-[3-Chloro-4-{3-oxo-4-[5-(3-methylphenyl)]-1,3,4-oxadiazol-2-ylmethyl}-2,3,4,5-tetrahydrobenzo[e]-1,4-diazepine-1-carbonyl] phenoxy] ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(3-methylphenyl)]-1,3,4-oxadiazol-2-ylmethyl]-1,2,4,5-tetrahydrobenzo[e]-1,4-diazepin-3-one.
¹H-NMR (CDCl₃ δ ppm:
1.85-2.05 (1H, m), 2.42 (3H, s), 3.85-5.70 (10H, m), 6.55-7.90 (11H, m)
MS(ESI, m/z):533(M+H)⁺

### Example 37-10

### 1-[2-Chloro-4- (2-hydroxyethoxy) benzoyl]-4-[5-(2-methylphenyl)]-1,3,4-oxadiazol-2-ylmethyl)-1,2,4,5-tetrahydrobenzo[e]-1,4-diazepin-3-one

2-[3-Chloro-4-(4-{2-[N'-(2-methylbenzoyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 2-methylbenzohydrazide in a similar manner to that described in Process A of Example 37-1.
2-[3-Chloro-4-{3-oxo-4-[5-(2-methylphenyl)]-1,3,4-oxadiazol-2-ylmethyl}-2,3,4,5-tetrahydrobenzo[e]-1,4-diazepine-1-carbonyl]phenoxy] ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(2-methylphenyl)]-1,3,4-oxadiazol-2-ylmethyl)-1,2,4,5-tetrahydrobenzo[e]-1,4-diazepin-3-one.
¹H-NMR (CDCl₃) δ ppm:
1.95-2.20 (1H, m), 2.42 (3H, s), 3.85-5.70 (10H, m), 6.50-7.90 (11H, m)
MS(ESI, m/z):533(M+H)⁺

### Example 37-11

### 1-[2-Chloro-4-(2-hydroxyethoxy)benzoyl]-4-[5-(4-methoxyphenyl)-1,3,4-oxadiazol-2-ylmethyl]-1,2,4,5-tetrahydrobenzo[e]-1,4-diazepin-3-one

2-[3-Chloro-4-(4-{2-[N'-(4-methoxybenzoyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl} acetic acid with 4-methoxybenzohydrazide in a similar manner to that described in Process A of Example 37-1.
2-(3-chloro-4-{4-[5-(4-methoxyphenyl)-1,3,4-oxadiazol-2-ylmethyl]-3-oxo-2,3,4,5-tetrahydrobenzo[e]-1,4-diazepine-1-carbonyl} phenoxy) ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(4-methoxyphenyl)-1,3,4-oxadiazol-2-ylmethyl]-1,2,4,5-tetrahydrobenzo[e]-1,4-diazepin-3-one.
¹H-NMR (CDCl₃) δ ppm:
1.85-2.15 (1H, m), 3.70-5.70 (13H, m), 6.50-8.15 (11H, m)
MS(ESI, m/z):549(M+H)⁺

### Example 37-12

### 1-[2-Chloro-4-(2-hydroxyethoxy)benzoyl]-4-[5-(3-methoxyphenyl)-1,3,4-oxadiazol-2-ylmethyl]-1,2,4,5-tetrahydrobenzo[e]-1,4-diazepin-3-one

2-[3-Chloro-4-(4-{2-[N'-(3-methoxybenzoyl) hydrazino] -2-oxoethyl}-3-oxo-2,3,4,5-tetrahydrobenzo[e]-1,4-diazepine-1-carbonyl)phenoxy] ethyl acetate was obtained in a similar manner to that described by condensation of {1-[4-(2-acetoxyethoxy)-2-chloro-benzoyl]-3-oxo-1,2,3,5-tetrahydrobenzo[e]-1,4-diazepin-4-yl} acetic acid with 3-methoxybenzohydrazide in Process Aof Example 37-1.
2-(3-Chloro-4-{4-[5-(3-methoxyphenyl)-1,3,4-oxadiazol-2-ylmethyl]-3-oxo-2,3,4,5-tetrahydrobenzo[e]-1,4-diazepine-1-carbonyl}phenoxy) ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(3-methoxyphenyl)-1,3,4-oxadiazol-2-ylmethyl]-1,2,4,5-tetrahydrobenzo[e] -1,4-diazepin-3-one.
¹H-NMR (CDCl₃) δ ppm:
1.85-2.15 (1H, m), 3.80-5.70 (13H, m), 6.50-7.70 (11H, m)
MS(ESI, m/z):549(M+H)⁺

### Example 37-13

### 1-[2-Chloro-4-(2-hydroxyethoxy)benzoyl]-4-[5-(2-methoxyphenyl)-1,3,4-oxadiazol-2-ylmethyl]-1,2,4,5-tetrahydrobenzo[e]-1,4-diazepin-3-one

2-[3-Chloro-4-(4-[2-[N'-(2-methoxybenzoyl)hydrazino]-2-oxoethyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 2-methoxybenzohydrazide in a similar manner to that described in Process A of Example 37-1.
2-(3-Chloro-4-{4-{5-(2-methoxyphenyl)-1,3,4-oxadiazol-2-ylmethyl]-3-oxo-2,3,4,5-tetrahydrobenzo[e]-1,4-diazepine-1-carbonyl}phenoxy) ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-[5-(2-methoxyphenyl)-1,3,4-oxadiazol-2-ylmethyl]-1,2,4,5-tetrahydrobenzo[e]-1,4-diazepin-3-one.
¹H-NMR (CDCl₃) δ ppm:
1.80-2.10 (1H, m), 3.80-5.70 (13H, m), 6.50-7.90 (11H, m)
MS(ESI, m/z):549(M+H)⁺

### Example 37-14

### 1-[2-Chloro-4-(2-hydroxyethoxy)benzoyl]-4-(5-pyridin-4-yl-1,3,4-oxadiazol-2-ylmethyl)-1,2,4,5-tetrahydrobenzo[e]-1,4-diazepin-3-one

2-[3-Chloro-4-(3-oxo-4-{2-oxo-2-[N'-(pyridine-4-carbonyl)hydrazino]ethyl)-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl} acetic acid with isonicotinohydrazide in a similar manner to that described in Process A of Example 37-1.
2-{3-Chloro-4-[3-oxo-4-(5-pyridin-4-yl-1,3,4-oxadiazol-2-ylmethyl)-2,3,4,5-tetrahydrobenzo[e]-1,4-diazepine-1-carbonyl] phenoxy} ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-(5-pyridin-4-yl-1,3,4-oxadiazol-2-ylmethyl)-1,2,4,5-tetrahydrobenzo[e]-1,4-diazepin-3-one.
¹H-NMR (CDCl₃) δ ppm:
1.90-2.10 (1H, m), 3.80-5.70 (10H, m), 6.50-7.65 (7H, m), 7.84 (2H, d, J=4.8Hz), 8.80 (2H, d, J=4.8Hz)
MS(ESI, m/z):520(M+H)⁺

### Example 37-15

### 1-[2-Chloro-4-(2-hydroxyethoxy)benzoyl]-4-(5-pyridin-3-yl-1,3,4-oxadiazol-2-ylmethyl)-1,2,4,5-tetrahydrobenzo[e]-1,4-diazepin-3-one

2-[3-Chloro-4-(3-oxo-4-{2-oxo-2-[N'-(pyridine-3-carbonyl)hydrazino]ethyl}-2,3,4,5-tetrahydrobenzo[e]-1,4-diazepine-1-carbonyl)phenoxy] ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with nicotinohydrazide in a similar manner to that described in Process A of Example 37-1. MS(ESI, m/z):580(M+H)⁺
2-{3-Chloro-4-[3-oxo-4-(5-pyridin-3-yl-1,3,4-oxadiazol-2-ylmethyl)-2,3,4,5-tetrahydrobenzo[e]-1,4-diazepine-1-carbonyl] phenoxy} ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-(5-pyridin-3-yl-1,3,4-oxadiazol-2-ylmethyl)-1,2,4,5-tetrahydrobenzo[e]-1,4-diazepin-3-one.
¹H-NMR (CDCl₃) δ ppm:
1.90-2.40 (1H, m), 3.80-5.70 (10H, m), 6.55-7.65 (8H, m), 8.20-8.40 (1H, m), 8.70-8.85(1H, m), 9.15-9.25 (1H, m)
MS(ESI, m/z):520(M+H)⁺

### Example 37-16

### 1-[2-Chloro-4-(2-hydroxyethoxy)benzoyl]-4-(5-pyridin-2-yl-1,3,4-oxadiazol-2-ylmethyl)-1,2,4,5-tetrahydrobenzo[e]-1,4-diazepin-3-one

2-[3-Chloro-4-(3-oxo-4-{2-oxo-2-[N'-(pyridine-2-carbonyl)hydrazino]ethyl}-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with pyridine-2-carbohydrazide in a similar manner to that described in Process A of Example 37-1. MS(ESI, m/z):580(M+H)⁺
2-{3-Chloro-4-[3-oxo-4-(5-pyridin-2-yl-1,3,4-oxadiazol-2-ylmethyl)-2,3,4,5-tetrahydrobenzo[e]-1,4-diazepine-1-carbonyl] phenoxy} ethyl acetate was obtained by cyclization of the obtained acetate derivative in a similar manner to that described in Process B of Example 37-1, and was de-acetylated in a similar manner to that described in Process C of Example 37-1 to give 1-[2-chloro-4-(2-hydroxyethoxy)benzoyl]-4-(5-pyridin-2-yl-1,3,4-oxadiazol-2-ylmethyl)- 1,2,4,5-tetrahydrobenzo[e]-1,4-diazepin-3-one.
¹H-NMR (CDCl₃) δ ppm:
1.85-2.10 (1H, m), 3.80-5.70 (10H, m), 6.55-7.65 (8H, m), 7.85-7.95 (1H, m), 8.10-8.30 (1H, m), 8.75-8.85 (1H, m)
MS(ESI, m/z):520(M+H) ⁺

### Example 37-17

### 1-[2-Chloro-4-(2-hydroxyethoxy)benzoyl]-4-[5-(2-piperidin-1-ylethyl)-1,3,4-oxadiazol-2-ylmethyl]-1,2,4,5-tetrahydrobenzo[e]-1,4-diazepin-3-one

2-[3-Chloro-4-(3-oxo-4-{2-oxo-2-[N'-(3-piperidin-1-ylpropionyl)hydrazino]ethyl}-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 3-piperidin-1-ylpropionohydrazide in a similar manner to that described in Process A of Example 37-1. MS(ESI, m/z):614(M+H)⁺
2-(3-Chloro-4-{3-oxo-4-[5-(2-piperidin-1-ylethyl)-1,3,4-oxadiazol-2-ylmethyl]-2,3,4,5-tetrahydrobenzo[e]-1,4-diazepine-1-carbonyl} phenoxy) ethyl acetate was obtained by cyclization of the above obtained acetate derivative in a similarmanner to that described in process B of Example 37-1. MS(ESI, m/z) :596 (M+H) ⁺
The above obtained acetate derivative was de-acetylated in a similar manner to that described in Process C of Example 37-1 to give 1-[2-chloro-4-(2-hydroxyethoxy)benzoyl]-4-[5-(2-piperidin-1-ylethyl)-1,3,4-oxadiazol-2-ylmethyl]-1,2,4,5-tetrah ydrobenzo[e]-1,4-diazepin-3-one.
¹H-NMR (CDCl₃) δ ppm:
1.35-1.65 (6H, m), 1.80-2.05 (1H, m), 2.35-2.55 (4H, m), 2.65-2.80 (2H, m), 2.90-3.05(2H, m), 3.80-5.40 (10H, m), 6.55-7.60 (7H, m) MS(ESI, m/z):554(M+H)⁺

### Example 37-18

### 1-[2-Chloro-4-(2-hydroxyethoxy)benzoyl]-4-[5-(2-morpholin-4-ylethyl)-1,3,4-oxadiazol-2-ylmethyl]-1,2,4,5-tetrahydrobenzo[e]-1,4-diazepin-3-one

2-[3-Chloro-4-(4-{ 2-[N'-(3-morpholin-4-ylpropionyl) hydrazino]-2-oxoethyl}-3-oxo-2,3,4,5-tetrahydrobenzo[e]-1,4-diazepine-1-carbonyl)phenoxy]ethyl acetate was.obtained by condensation of {1-[4-(2-acetoxyethoxy)-2-chloro-benzoyl]-3-oxo-1,2,3,5-tetrahydrobenzo[e]-1,4-diazepin-4-yl} acetic acid with 3-morpholin-4-ylpropionohydrazide in a similar manner to that described in Process A of Example 37-1. MS(ESI, m/z):616(M+H)⁺
2-(3-Chloro-4-{4-[5-(2-morpholin-4-ylethyl)-1,3,4-oxadiazol-2-ylmethyl]-3-oxo-2,3,4,5-tetrahydrobenzo[e]-1,4-diazepine-1-carbonyl}phenoxy)ethyl acetate was obtained by cyclization of the obtained acetate derivative in a similar manner to that described in Process B of Example 37-1. MS (ESI, m/z):598(M+H)⁺
The obtained acetate derivative was de-acetylated in a similar manner to that described in Process C of Example 37-1 to give 1-[2-chloro-4-(2-hydroxyethoxy)benzoyl]-4-[5-(2-morpholin-4-ylethyl)-1,3,4-oxadiazol-2-ylmethyl]-1,2,4,5-tetrahydrobenzo[e]-1,4-diazepin-3-one.
¹H-NMR (CDCl₃) δ ppm:
1.80-2.00 (1H, m), 2.40-2.55 (4H, m), 2.70-2.80 (2H, m), 2.90-3.05 (2H, m), 3.60-3.70(4H, m), 3.85-5.40 (10H, m), 6.55-7.60 (7H, m)
MS(ESI, m/z):556(M+H)⁺

### Example 37-19

### 1-[2-Chloro-4-(2-hydroxyethoxy)benzoyl]-4-[2-(5-methyl-1,3,4-oxadiazol-2-yl)ethyl]-1,2,4,5-tetrahydrobenzo[e]-1,4-diazepin-3-one

2-(4-{4-[3-(N'-Acetylhydrazino)-3-oxopropyl]-3-oxo-2,3,4,5-tetrahydrobenzo[e]-1,4-diazepine-1-carbonyl}-3-chlorophenoxy)ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with acetohydrazide in a similar manner to that described in process A of Example 37-1. MS(ESI, m/z):531(M+H)⁺
2-(3-Chloro-4-{4-[2-(5-methyl-1,3,4-oxadiazol-2-yl)ethyl]-3-oxo-2,3,4,5-tetrahydrobenzo[e]-1,4-diazepine-1-carbonyl} phenoxy) ethyl acetate was obtained by cyclization of the above obtained acetate derivative in a similar manner to that described in Process B of Example 37-1. MS(ESI, m/z):513(M+H)⁺
The above obtained acetate derivative was de-acetylated in a similar manner to that described in Process C of Example 37-1 to give 1-[2-chloro-4-(2-hydroxyethoxy)benzoyl]-4-[2-(5-methyl-1,3,4-oxadiazol-2-yl)ethyl]-1,2,4,5-tetrahydrobenzo[e]-1,4-diazepin-3-one.
¹H-NMR (CDCl₃) δ ppm:
1. 95-2.20 (1H, m), 2.49 (3H, s), 3.05-3.20 (2H, m), 3.80-5.70 (10H,
m), 6.50-7.70(7H, m)
MS(ESI, m/z):471(M+H)⁺

### Example 37-20

### 1-[2-Chloro-4-(2-hydroxyethoxy)benzoyl]-4-{2-[5-(2-methoxyethyl)-1,3,4-oxadiazol-2-yl]ethyl}-1,2,4,5-tetrahydrobenzo[e]-1,4-diazepin-3-one

2-[3-Chloro-4-(4-{3-[N'-(3-methoxypropionyl)hydrazino]-3-oxopropyl}-3-oxo-2,3,4,5-tetrahydrobenzo[e]-1,4-diazepine-1-carbonyl)phenoxy]ethyl acetate was obtained by condensation of {1-[4-(2-acetoxyethoxy)-2-chloro-benzoyl]-3-oxo-1,2,3,5-tetrahydrobenzo[e]-1,4-diazepin-4-yl}acetic acid with 3-methoxypropionohydrazide in a similar manner to that described in Process A of Example 37-1. MS(ESI, m/z):575(M+H)⁺
2-[3-Chloro-4-(4-[2-[ 5-(2-methoxyethyl)-1,3,4-oxadiazol-2-yl]ethyl]-3-oxo-2,3,4,5-tetrahydrobenzo[e]-1,4-diazepine-1-carbonyl)phenoxy] ethyl acetate was obtained by cyclization of the above obtained acetate derivative in a similar manner to that described in Process B of Example 37-1. MS(ESI, m/z):557(M+H)⁺
The above obtained acetate derivative was de-acetylated in a similar manner to that described in Process C of Example 37-1 to give 1-[2-chloro-4-(2-hydroxyethoxy)benzoyl]-4-{2-[5-(2-methoxyethyl)-1,3,4-oxadiazol-2-yl]:ethyll-1,2,4,5-tetrahydrobenzo[e]-1,4-diazepin-3-one.
¹H-NMR (CDC1₃) δ ppm:
1.90-2.20 (1H, m), 3.05-3.25 (4H, m), 3.36 (3H, s), 3.77 (2H, t, J=6.4Hz), 3.85-5.80 (10H, m), 6.50-7.70(7H, m)
MS(ESI;-m/.z):515(M+H)⁺

### Example 38-1

### 1-(2-Chloro-4-ethoxybenzoyl)-4-(5-methyl-1,3,4-oxadiazol-2-ylmethyl)-1,2,4,5-tetrahydrobenzo[e]-1,4-diazepin-3-one

To a stirred mixture of 1-(2-chloro-4-hydroxybenzoyl)-4-(5-methyl-1,3,4-oxadiazol-2-ylmethyl)-1,2,4,5-tetrahydrobenzo[e]-1,4-diazepin-3 (0.0413 g) and cesium carbonate (0.0815 g) in N,N-dimethylformamide (1.5 mL) was added iodoethane (0. 0343 g) at room temperature, and the mixture was stirred at 60°C for an hour. To the mixture were added water and ethyl acetate, and the organic layer was separated. The aqueous layer was extracted with ethyl acetate, and then the collected organic layer was successively washed with water and brine, and dried over anhydrous magnesium sulfate. The solvent was removed under reduced pressure. The obtained crude product was purified by column chromatography on aminopropylsilylated silica gel (eluent:hexane-ethyl acetate) to give 1-(2-chloro-4-ethoxybenzoyl)-4-(5-methyl-1,3,4-oxadiazol-2-ylmethyl)-1,2,4,5-tetrahydrobenzo[e]-1 ,4-diazepin-3-one (0.311 g).

¹H-NMR (CDC1₃) δ ppm:
1.25-1.55 (3H, m), 2.48 (3H, s), 3.70-5.70 (8H, m), 6.45-7.65 (7H, m)
MS(ESI, m/z):441(M+H)⁺

### Examples 38-2 to 38-10

The following compounds of Examples 38-2 to 38-10 were obtained with the use of the corresponding materials in a similar manner to that described in Example 37-1. The structure formula and physical data of these compounds were shown in Tables 64 and 65.

**[Table 64]**

| Example | Structure formula | Compound name | 1H-NMR (solvent) or MS(m/z) |
|---|---|---|---|
| 38-2 | 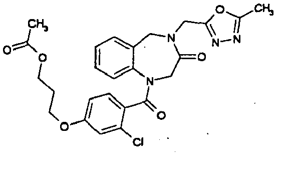 | 3-{3-chloro-4-[4-{5-methyl-1,3,4,-oxadiazol-2-ylmethyl)-3-oxo-2,3,4,5-tetrahydrobenzo[e]-1,4-diazepine-1-carbonyl]phenoxy}propyl acetate | MS(ESI, m/z) : 513(M+H)+ |
| 38-3 | 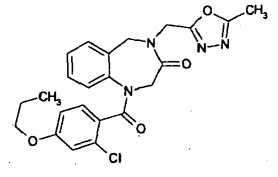 | 1-(2-chloro-4-propoxybenzoyl)-4-(5-methyl-1,3,4,-oxadiazol-2-ylmethyl)-1,2,4,5-tetrahydrobenzo[e]-1,4-diazepin-3-one | 1H-NMR (CDCl3) δ ppm: 0.80-1.20(3H, m), 1.65-1.95 (2H, m), 2.48 (3H,s), 3.60-5.80 (8H, m), 6.45-7.65 (7H, m) MS(ESI, m/z) :-455(M+H)+ |
| 38-4 | 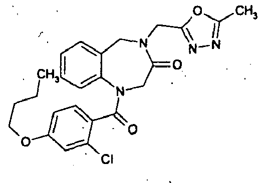 | 1-(2-chloro-4-butoxybenzoyl)-4-(5-methyl-1,3,4,-oxa diazol-2-ylmethyl)-1,2,4,5-tetrahydrobenzo[e]-1,4-diazepin-3-one | 1H-NMR (CDCl3) δ ppm: 0.85-1.10(3H, m), 1.35-1.95 (4H, m), 2.48 (3H ,s), 3.70-5.80 (8H, m), 6.45-7.65 (7H, m) MS(ESI, m/z) : 469(M+H)+ |
| 38-5 | 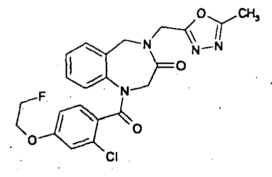 | 1-[2-chloro-4-(2-fluoroethoxy)benzoyl]-4-(5-methyl-1,3,4,-oxadiazol-2-ylmethyl)-1,2,4,5-tetrahydrobenzo[e]-1,4-diazepin-3-one | 1H-NMR (CDCl3) δ ppm: 2.40-2.55 (3H, m), 3.90-5.80 (10H, m), 6.55-7.65 (7H, m) MS(ESI, m/z): 459(M+H)+ |

**[Table 65]**

| Example | Structure formula | Compound name | 1H-NMR (solvent) or MS(m/z) |
|---|---|---|---|
| 38-6 | 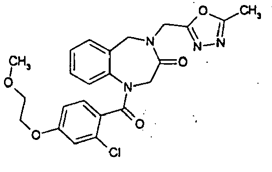 | 1-[2-chloro-4-(2-methoxyethoxy)benzoyl]-4-(5-methyl-1,3,4,-oxadiazol-2-ylmethyl)-1,2,4,5-tetrahydrobenzo[e]-1,4-diazepin-3-one | 1H-NMR (CDCl3) δ ppm: 2.47 (3H, s), 3.30-5.80 (13H, m), 6.50-7.65 (7H, m) MS(ESI, m/z) : 471 (M+H)+ |
| 38-7 | 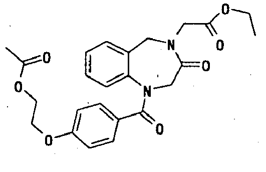 | ethyl{1-[4-(2-acetoxyethoxy)benzoyl]-3-oxo-1,2,3,5-tetrahydrobenzo[e]-1,4-diazepin-4-yl}acetate | 1H-NMR (CDCl3) δ ppm: 1.28 (3H, t, J=7.2Hz), 2.05-2.15 (3H, m), 3.30-6.00 (12H, m), 6.65-7.45 (8H, m) |
| 38-8 | 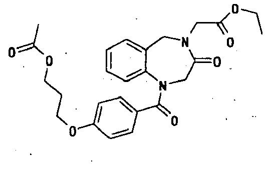 | ethyl {1-[4-(2-acetoxypropoxy)benzoyl]-3-oxo-1,2,3,5-tetrahydrobenzo[e]-1,4-diazepin-4-yllacetate | 1 H-NMR (CDCl3) δ ppm: 1.28 (3H, t, J=7.2Hz), 2.00-2.15 (5H, m), 3.30-6.00 (12H, m), 6.60-7.50 (8H, m) |
| 38-9 | 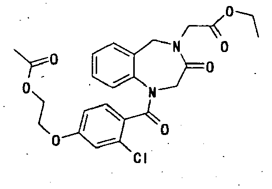 | ethyl {1-[4-(2-acetoxyethoxy)-2-chlorobenzoyl]-3-oxo-1,2,3,5-tetrahydrobenzo[e]-1,4-diazepin-4-yljacetate | 1 H-NMR (CDCI3) δ ppm: 1.28 (3H, t, J=7.2Hz), 2.00-2.15 (3H, m), 3.50-6.00 (12H, m), 6.50-7.60 (7H, m) |
| 38-10 | 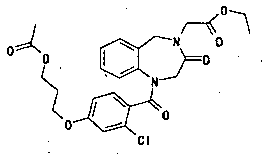 | ethyl {1-[4-(2-acetoxypropoxy)-2-chlorobenzoyl]-3-oxo-1,2,3,5-tetrahydrobenzo[e]-1,4-diazepin-4-yl}acetate | 1 H-NMR (CDCI3) δ ppm: 1.28 (3H, t, J=7.1 Hz), 2.00-2.20 (5H, m), 3.50-6.00 (12H, m), 6.50-7.65 (7H, m) |

### Example 39

### 2-[3-Chloro-4-(4-{2-[(2-methoxyethyl)methylamino]ethyl}-3-oxo-2,3,4,5-tetrahydrobenzo[e]-1,4-diazepine-1-carbonyl)-phenoxy]ethyl acetate

To a stirred solution of 2-(3-chloro-4-{4-[2-(2-methoxyethylamino)ethyl]-3-oxo-2,3,4,5-tetrahydrobenzo[e]-1,4-diazepine-1-carbonyl}phenoxy)ethyl acetate (0.037 g) in tetrahydrofuran (0.30 mL) were successively added sodium hydride (0.021g, purity:60%) and iodomethane (0.0120 g) under ice-cooling, the reaction mixture was stirred at room temperature for 3 hours. The reaction was quenched by addition of water under the same condition, to which was added ethyl acetate, and then the organic layer was separated. The aqueous layer was extracted with ethyl acetate. The collected organic layer was washed with brine, washed with anhydrous magnesium sulfate. The solvent was removed under reduced pressure and the obtained crude product was purified by column chromatography on aminopropylsilylated silica gel (eluent:hexane-ethyl acetate) to give 2-[3-chloro-4-(4-{2-[(2-methoxyethyl)methylamino]-ethyl}-3-oxo-2,3,4,5-tetrahydrobenzo[e]-1,4-diazepine-1-carbonyl)phenoxy]ethyl acetate (0.00740 g).

¹H-NMR (CDCl₃) δ ppm:
2.00-2.20.(3H, m), 2.34 (3H, s), 2.45-2.80 (4H, m), 3.36 (3H, s), 3.48 (2H, t, J=5.5Hz), 3.65 (2H, t, J=6.6Hz), 3.80-5.80 (8H, m), 6.50-7.85 (7H, m)

### Example 40

### {1-[4-(2-Acetoxyethoxy)benzoyl]-3-oxo-1,2,3,5-tetrahydrobenzo[e]-1,4-diazepin-4-yl}acetic acid

To a stirred solution of tert-butyl 1-[4-(2-acetoxyethoxy)benzoyl]-3-oxo-1,2,3,5-tetrahydrobenzo[e]-1,4-diazepin-4-yl}acetate (0.158 g) in dichloromethane (0.70 mL) was added trifluoroacetic acid (0.700 mL) under ice-cooling, and the reaction mixture was stirred at room temperature for 1.5 hours. The mixture was concentrated under reduced pressure. To the residue were added ethanol and toluene, the mixture was concentrated under reduced pressure again. The obtained crude product was purified by recrystallization from ethyl acetate to give {1-[4-(2-acetoxyethoxy)benzoyl]-3-oxo-1,2,3,5- tetrahydrobenzo[e]-1,4-diazepin-4-yl}acetic acid (0.0920 g).

¹H-NMR (DMSO-d₆) δ ppm:
2.00 (3H, s), 3.50-5.80 (10H, m), 6.75-7.00 (3H, m), 7.15-7.30 (4H, m), 7.45-7.55 (1H, m), 12.00-13.50 (1H, br)

### Example 41

### 2-(3-Chloro-4-{4-[2-(2-methoxyethylamino)ethyl]-3-oxo-2,3,4,5-tetrahydrobenzo[e]-1,4-1-carbonyl}phenoxy)ethyl acetate

To a stirred suspension of 2-[3-chloro-4-(4-{2-[(2-methoxyethyl)-(2-nitrobenzenesulfonyl)amino]ethyl}-3-oxo-2,3,4,5-tetrahydrobenzo[e]-1,4-diazepine-1-carbonyl)-phenoxy]ethyl acetate (0.112 g) and potassium carbonate (0.116 g) in N,N-dimethylformamide (0.80 mL) was added benzenethiol (0.037 g) at room temperature, the reaction mixture was stirred at the same condition for 30 minutes. To the mixture were added ethyl acetate and water, and the organic layer was separated. The aqueous layer was extracted with ethyl acetate / toluene. The collected organic layer was washed with brine, and dried over anhydrous magnesium sulfate. The solvent was removed under reduced pressure, and the residue was purified by column chromatography on aminopropylsilylated silica gel (eluent:ethyl acetate) to give 2-(3-chloro-4-{4-[2-(2-methoxyethylamino)ethyl]-3-oxo-2,3,4,5-tetrahydrobenzo[e]-1,4-1-carbonyl}phenoxy)ethyl acetate (0.0593 g).

¹H-NMR (CDCl₃) δ ppm:
2.00-2.20 (3H, m), 2.60-5.90 (19H, m), 6.50-7.80 (7H, m)

### Example 42 and Example 43

### N-(2-Methoxyethyl)-2-[3-oxo-1-(6-pyrazol-1-ylpyridine-3-carbonyl)-1,2,3,5-tetrahydrobenzo[e]-1,4-diazepin-4-yl] acetamide

N-Hydroxymethyl-N-(2-methoxyethyl)-2-[3-oxo-1-(6-pyrazol-1-ylpyridine-3-carbonyl)-1,2,3,5-tetrahydrobenzo[e]-1,4-diazepin-4-yl]acetamide
To a stirred solution of N-benzyloxymethyl-N-(2-methoxyethyl)-2-[3-oxo-1-(6-pyrazol-1-ylpyridine-3-carbonyl)-1,2,3,5-tetrahydrobenzo[e]-1,4-diazepin-4-yl] acetamide (0.0599g) in dichloromethane (1.0 mL) was added trifluoroacetic acid (1.0 mL) at room temperature, the reaction mixture was stirred at the same condition for 2 hours. To the mixture were successively added water (5.0 mL), 2 mol/L aqueous solution of sodium hydroxide (6.5 mL) and ethyl acetate (5.0 mL) . To the mixture was added ethyl acetate (5.0 mL), and the organic layer was separated. The aqueous layer was extracted with ethyl acetate, and then the collected organic layer was washed with brine. The organic layer was dried over anhydrous magnesium sulfate, and concentrated under reduced pressure. The residue was purified by column chromatography on aminopropylsilylated silica gel (eluent:ethyl acetate-ethanol) to give N-(2-methoxyethyl)-2-[3-oxo-1-(6-pyrazol-1-ylpyridine-3-carbonyl)-1,2,3,5-tetrahydrobenzo[e]-1,4-diazepin-4-yl] acetamide (0.0131 g) and N-hydroxymethyl-N-(2-methoxyethyl)-2-[3-oxo-1-(6-pyrazol-1-ylpyridine-3-carbonyl)-1,2,3,5-tetrahydrobenzo[e]-1,4-diazepin-4-yl]acetamide (0.0131 g).

### (N-(2-Methoxyethyl)-2-[3-oxo-1-(6-pyrazol-1-ylpyridine-3-carbonyl)-1,2,3,5-tetrahydrobenzo[e]-1,4-diazepin-4-yl] acetamide)

¹H-NMR (CDCl₃) δ ppm:
3.20-6.00 (13H, m), 6.25-6.50 (2H, m), 6.80-6.95 (1H, m), 7.15-7.35 (2H, m), 7.40-7.50 (1H, m), 7.60-7.75 (2H, m), 7.82 (1H, d, J=8.5Hz), 8.26 (1H, s), 8.46 (1H, d, J=2.6Hz)
(N-Hydroxymethyl-N-(2-methoxyethyl)-2-[3-oxo-1-(6-pyrazol-1-yl-pyridine-3-carbonyl)-1,2,3,5-tetrahydrobenzo[e]-1,4-diazepin-4-yl] acetamide)
¹H-NMR (CDCl₃) δ ppm:
1.50-2.00 (1H, br), 3.20-6.00 (15H, m), 6.40-6.50 (1H, m), 6.70-7.90 (7H, m), 8.20-8.55 (2H, m)

### Example 44

### 4-(2-Aminoethyl)-1-[4-(2-benzyloxyethoxy)-2-chlorobenzoyl]-1,2,4,5-tetrahydrobenzo[e]-1,4-diazepin-3-one

4-(2-Aminoethyl)-1-[4-(2-benzyloxyethoxy)-2-chlorobenzoyl]-1,2,4,5-tetrahydrobenzo[e]-1,4-diazepin-3-one was obtained with the use of 2-(2-{1-[4-(2-benzyloxyethoxy)-2-chlorobenzoyl]-3-oxo-1,2,3,5-tetrahydrobenzo[e]-1,4-diazepin-4-yl}ethyl)isoindole-1,3-dione in a similar manner to that described in Example 11.

¹H-NMR (CDCl₃) δ ppm:
2.85-5.70 (14H, m), 6.55-7.65 (12H, m)

### Example 45

### Methyl (2-{1-[2-chloro-4-(2-hydroxyethoxy)benzoyl]-3-oxo-1,2,3,5-tetrahydrobenzo[e]-1,4-diazepin-4-yl}acetylamino)acetate

To a stirred mixture of (2-{1-[2-chloro-4-(2-hydroxyethoxy)benzoyl]-3-oxo-1,2,3,5-tetrahydrobenzo[e]-1,4-diazepin-4-yl} acetylamino) acetic acid in tetrahydrofuran-methanol (0.18-0.18 mL) was added (trimethylsilyl)diazomethane (6.30 mg) at room temperature, and the mixture was stirred at room temperature for an hour. The reaction mixture was concentrated under reduced pressure, and the residue was purified by column chromatography on silica gel (eluent:ethyl acetate-ethanol) to give methyl (2-{1-[2-chloro-4-(2-hydroxyethoxy)benzoyl]-3-oxo-1,2,3,5-tetrahydrobenzo[e]-1,4-diazepin-4-yl}acetylamino)acetate (4.20 mg).

¹H-NMR (CDC1₃) δ ppm:
1.80-2.20 (1H, br), 3.30-5.80. (15H, m), 6.35-7.80 (7H, m)

### Example 46-1

### 1-(2-Chloro-4-pyrrolidin-1-ylbenzoyl)-4-(5-ethyl-1,3,4-oxadiazol-2-ylmethyl)-1,2,4,5-tetrahydrobenzo[e]-1,4-diazepin-3-one

A solution of propionic acid N'-(2-[1-(2-chloro-4-pyrrolidin-1-ylbenzoyl)-3-oxo-1,2,3,5-tetrahydrobenzo[e]-1,4-diazepin-4-yl] acetyl} hydrazide (35.0mg) in phosphoryl chloride (1.0 mL) was stirred at 100°C for an hour. The reaction mixture was concentrated under reduced pressure, then the residue were added water and ethyl acetate. The separated organic layer was washed with brine. The organic layer was dried over anhydrous magnesium sulfate, filtered, and the filtrate was concentrated under reduced pressure. The obtained crude product was purified by column chromatography on silica gel (eluent:ethyl acetate-methanol) to give 1-(2-chloro-4-pyrrolidin-1-ylbenzoyl)-4-(5-ethyl-1,3,4-oxadiazol-2-ylmethyl)-1,2,4,5-tetrahydrobenzo[e]-1,4-diazepin-3-one (3.00 mg) .

¹H-NMR (CDCl₃) δ ppm:
1.33 (3H, t, J=7.6Hz), 1.90-2.05 (4H, m), 2.81 (2H, q, J=7.6Hz), 3.10-3.30 (4h, m), 4.30-5.60 (6H, m), 6.00-7.70 (7H, m)
MS(ESI, m/z):480(M+H)⁺

### Examples 46-2 and 46-3

The following compounds of Examples 46-2 and 46-3 were obtained with the use of the corresponding materials in a similar manner to that described in Example 46-1. The structure formula and physical data of these compounds were shown in Table 66.

**[Table 66]**

| Example | Structure formula | Compound name | 1H-NMR (solvent) or MS(m/z) |
|---|---|---|---|
| 46-2 | 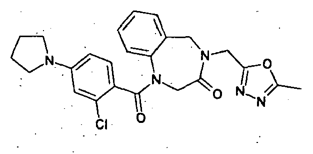 | 1-(2-chloro-4-pyrrolidin-1-ylbenzoyl)-4-(5-methyl-1,3,4,-oxadiazol-2-ylmethyl)-1,2,4,5-tetra hydrobenzo[e]-1,4-diazepin-3-one | 1 H-NMK (CDCl3) δ ppm: 1.90-2.05 (4H, m), 2.48 (3H, s), 3.10-3.30 (4H, m), 4.20-5.50 (6H, m), 6.00-7.70 (7H, m) MS(ESI, m/z) : 466(M+H)+ |
| 46-3 | 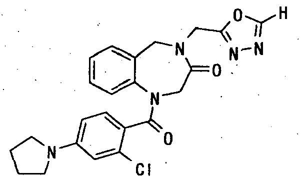 | 1-(2-chloro-4-pyrrolidin-1-ylbenzoyl)-4-1,3,4,-oxadiazol-2-ylmethyl)-1.2,4,5-tetrahydrobenzo[e]-1,4-diazepin-3-one | ¹H-NMR (CDCl₃) δ ppm: 1.90-2.05 (4H, m), 3.10-3.30 (4H, m), 4.10-5.40 (6H, m), 6.00-7.60 (7H, m), 8.30-8.40 (1 H, m) MS(ESI, m/z): 452(M+H)+ |

### Example 47

### 1-[2-Chloro-4-(2-hydroxy-2-methylpropoxy)benzoyl]-4-(5-methyl-1,3,4-oxadiazol-2-ylmethyl)-1,2,4,5-tetrahydrobenzo[e]-1,4-diazepin-3-one

To a stirred suspension of 1-(2-chloro-4-hydroxybenzoyl)-4-(5-methyl-1,3,4-oxadiazol-2-ylmethyl)-1,2,4,5-tetrahydrobenzo[e]-1,4-diazepin-3-one (50.0 mg) and cesium carbonate (59.2 mg) in N,N-dimethylformamide (1 mL) was added 2,2-dimethyloxirane (26.2 mg) at room temperature, the mixture was stirred at an external temperature of 60 °C for 2 days. To the stirred mixture were added cesium carbonate (59.2mg) and2,2-dimethyloxirane (26.2 mg) at room temperature, the mixture was stirred for 2 days. To the mixture were added water and ethyl acetate under ice-cooling, and the organic layer was separated. The aqueous layer was extracted with ethyl acetate, and then the collected organic layer was washed with brine, and dried over anhydrous magnesium sulfate. The solvent was removed under reduced pressure. The obtained crude product was purified by column chromatography on aminopropylsilylated silica gel (eluent:hexane-ethyl acetate-methanol) to give 1-[2-chloro-4-(2-hydroxy-2- methylpropoxy)benzoyl]-4-(5-methyl-1,3,4-oxadiazol-2-ylmethyl)-1,2,4,5-tetrahydrobenzo[e]-1,4-diazepin-3-one (16.4 mg).

¹H-NMR (CDCl₃) δ ppm:
1.20-1.50 (6H, m), 2.00-2.20 (1H, m), 2.48 (3H, s), 3.50-6.00 (8H, m), 6.50-7.60 (7H, m)
MS(ESI, m/z):485(M+H)⁺

### Example 48

### N-(2-{1-[4-(2-Benzyloxyethoxy)-2-chlorobenzoyl]-3-oxo-1,2,3,5-tetrahydrobenzo[e]-1,4-diazepin-4-yl}ethyl)-2-methoxyacetamide

N-(2-{1-[4-(2-Benzyloxyethoxy)-2-chlorobenzoyl]-3-oxo-1,2,3,5-tetrahydrobenzo[e]-1,4-diazepin-4-yl}ethyl)-2-methoxyacetamide was obtained with the use of 4-(2-aminoethyl)-1-[4-(2-benzyloxyethoxy)-2-chlorobenzoyl] -1,2,4,5-tetrahydrobenzo[e]-1,4-diazepin-3-one and methoxyacetyl chloride in a similar manner to that described in Example 12.

¹H-NMR (CDC1₃) δ ppm:
3.10-5.50 (19H, m), 6.50-7.60 (12H, m)

### Example 49

### 2-{3-Chloro-4-[4-(4-methyl-4,5-dihydrooxazol-2-ylmethyl)-3-oxo-2,3,4,5-tetrahydrobenzo[e]-1,4-diazepine-1-carbonyl]-phenoxy}ethyl acetate

To a stirred solution of 2-(3-chloro-4-{ 4-[(2-hydroxy-1-methylethylcarbamoyl)methyl]-3-oxo-2,3,4,5-tetrahydrobenzo[e]-1,4-diazepine-1-carbonyl}phenoxy)ethyl acetate (80.0 mg) and diisopropylethylamine (59.9 mg) intetrahydrofuran (2.0mL) was added methanesulfonyl chloride (21.2 mg) under ice-cooling, the mixture was stirred at room temperature for 12 hours. To the stirred mixture were successively added diisopropylethylamine (59.9 mg) and methanesulfonyl chloride (21.2 mg) at room temperature, the solution was stirred at room temperature for 2.5hours and at 40°C for 2 hours. After standing to cool, to the mixture were successively added methanol (0.20 mL) and 5 mol/L aqueous solution of sodium hydroxide (93.0 µL), and the mixture was stirred at room temperature for 0.5 hour. To the mixture was added methanol (0.40 mL), and the mixture was stirred at room temperature for 20 minutes. To the mixture was added water and then extracted with ethyl acetate. The separated aqueous layer was extracted with ethyl acetate, and then the collected organic layer was washed with brine, dried over anhydrous magnesium sulfate. The solvent was removed under reduced pressure to give 2-{3-chloro-4-[4-(4-methyl-4,5-dihydrooxazol-2-ylmethyl)-3-oxo-2,3,4,5-tetrahydrobenzo[e]-1,4-diazepine-1-carbonyl]-phenoxy}ethyl acetate (64.0 mg).

MS(ESI, m/z):500(M+H)⁺

### Example 50

### (2-{1-[2-Chloro-4-(2-hydroxyethoxy)benzoyl]-3-oxo-1,2,3,5-tetrahydrobenzo[e]-1,4₋diazepin-4-yl}acetylamino)acetic acid

(2-{1-[2-Chloro-4-(2-hydroxyethoxy)benzoyl)-3-oxo-1,2,3,5-tetrahydrobenzo[e]-1,4-diazepin-4-yl}acetylamino)acetic acid was obtained with the use of tert-butyl (2-{1-[4-(2-acetoxyethoxy)-2-chlorobenzoyl]-3-oxo-1,2,3,5-tetrahydrobenzo-[e]-1,4-diazepin-4-yl}acetylamino)acetate in a similar manner to that described in Example 3.

¹H-NMR (CD₃OD) δ ppm:
3.70-5.30 (12H, m), 6.60-7.70 (7H, m)

### Example 51

### 1-[2-Chloro-4-(2-hydroxyethoxy)benzoyl]-4-(5-cyclopropyl-1,3,4-oxadiazol-2-ylmethyl)-1,2,4,5-tetrahydrobenzo[e]-1,4-diazepin-3-one

(Process A) To a stirred solution of {1-[4-(2-acetoxyethoxy)-2-chloro-benzoyl]-3-oxo-1,2,3,5-tetrahydrobenzo[e]-1,4-diazepin-4-yl}acetic acid (0.600 g), tert-butyl hydrazinecarboxylate (0.181 g) and hydroxybenzotriazole monohydrate (0.299 g) in N,N-dimethylformamide (6.0 mL) was added 1-ethyl-3-(dimethylaminopropyl) carbodiimide hydrochloride (0.374 g) at room temperature, the solution was stirred at room temperature for 2 hours. To the reaction solution was added water, and then extracted with dichloromethane. The separated organic layer was dried over anhydrous magnesium sulfate, filtered. The filtrate was concentrated under reduced pressure, and the residue was purified by column chromatography on aminopropylsilylated silica gel (eluent:ethyl acetate-ethanol) to give 2-[3-chloro-4-(4-{2-[N-(2,2-dimethylpropionyl)hydrazino]-2-oxoethyl}-3-oxo-2,3,4,5-tetrahydrobenzo[e]-1,4-diazepine-1-carbonyl)phenoxy]ethyl acetate (0.694 g).
(Process B) Trifluoroacetic acid (1.8 mL) was slowly added dropwise to a stirred solution of the acetate derivative (obtained in Process A) in dichloromethane (1.8 mL) under ice-cooling, and the mixture was stirred at the same condition for 20 minutes. The mixture was stirred at room temperature for 20 minutes, and then the solvent was removed under reduced pressure. The residue was diluted by addition of ethyl acetate, and then to an aqueous solution of sodium hydrogen carbonate was added slowly the mixture under ice-cooling. The organic layer was extracted with ethyl acetate, dried over anhydrous magnesium sulfate, and filtered. The filtrate was concentrated under reduced pressure, and the residue was purified by column chromatography on silica gel (eluent:ethyl acetate-ethanol) to give 2-[3-chloro-4-(4-hydrazinocarbonylmethyl-3-oxo-2,3,4,5-tetrahydrobenzo[e]-1,4-diazepine-1-carbonyl) phenoxy]ethyl acetate (0.308 g).
(Process C) To a stirred solution of the acetate derivative (obtained in process B) (80.0ml), cyclopropanecarboxylic acid (15.2 mg) and hydroxybenzotriazole monohydrate (38.7 mg) in N,N-dimethylformamide (0.84 mL) was added 1-ethyl-3-(dimethyl aminopropyl)carbodiimide hydrochloride (48.5 mg) at room temperature, the mixture solution was stirred at room temperature for 8 hours. To the reaction mixture solution was added water and then extracted with ethyl acetate. The separated organic layer was washed with water, dried over anhydrous magnesium sulfate and filtered. The filtrate was concentrated under reduced pressure, and the deposited crystal was washed with ethyl acetate-hexane, and then collected by filtration to give 2-{3-chloro-4-[4-(N'-cyclopropylhydrazinocarbonylmethyl)-3-oxo-2,3,4,5-tetrahydrobenzo[e]-1,4-diazepine-1-carbonyl]phenoxy}ethyl acetate (0.0464 g).
(Process D) To polymer-bounded triphenylphosphine (86.0mg, load:2.2 mmol/g) was added carbon tetrachloride, and the mixture solution was stirred at room temperature for 20 minutes. To the stirredmixture solution were added the acetate derivatives (obtained in process C) (44.7 mg) and a solution of diisopropylethylamine (42.5 mg) in acetonitrile (0.80 mL) at room temperature, the mixture was stirred at an external temperature of 75 °C overnight. After standing to cool, to the mixture solution was added polymer-bounded triphenylphosphine (70.6.mg), it was stirred at an external temperature of 75°C for 2 hours. After standing to cool, an insoluble material was removed by filtration. The filtrate was concentrated under reduced pressure, and the residue was purified by column chromatography on aminopropylsilylated silica gel (eluent:ethyl acetate) to give 2-{3-chloro-4-[4-(5- cyclopropyl-l,3,4-oxadiazol-2-yl-methyl)-3-oxo-2,3,4,5-tetraliydrobenzo[e]-1,4-diazepine-1-carbonyl]phenoxy}ethyl acetate (48.6 mg).
(Process E) To a stirred solution of the acetate derivative (obtained in Process C) (43.2 mg) in methanol (1. 6 mL) was added 2 mol/L aqueous solution of sodium hydroxide (0.0500 mL) at room temperature, and the mixture was stirred at room temperature for 15 minutes. Twomol/L hydrochloric acid (0.125 mL) was added under ice-cooling. The mixture was concentrated undr reduced pressure. To the residue was added water and then extracted with dichloromethane. The separated organic layer was concentrated under reduced pressure, and the residue was purified by column chromatography on aminopropylsilylated silica' gel (eluent:ethyl acetate-ethanol) to give 1-[2-chloro-4-(2-hydroxyethoxy)benzoyl]-4-(5-cyclopropyl-1,3,4-oxadiazol-2-ylmethyl)-1,2,4,5-tetrahydrobenzo[e]-1,4-diazepin-3-one (35.3 mg).

MS(ESI, m/z):483(M+H)⁺

### Example 52

### 1-[2-Chloro-4-(4-hydroxybutoxy)benzoyl]-4-(5-methyl-1,3,4-oxadiazol-2-ylmethyl)-1,2,4,5-tetrahydrobenzol e]-1,4-diazepin-3-one

(Process A) To a stirred suspension of the 1-(2-chloro-4-hydroxybenzoyl)-4-(5-methyl-1,3,4-oxadiazol-2-ylmethyl)-1,2,4,5-tetrahydrobenzo[e]-1,4-diazepin-3-one (60.0 mg) and cesium carbonate (118 mg) in N,N-dimethylformamide (1.5 mL) was added 4-iodobutyl acetate (77.4 mg) at room temperature, the mixture was stirred at an external temperature of 60°C for 1.5 hours. After standing, to cool, to the mixture were added water and ethyl acetate, and the organic layer was separated. The aqueous layer was extracted with ethyl acetate, then the collected organic layer was washed with water and brine, and dried over anhydrous magnesium sulfate. The solvent was removed under reduced pressure to give 4-{3-chloro-4-[4-(5-methyl-1,3,4-oxadiazol-2-ylmethyl)-3-oxo-2,3,4,5-tetrahydrobenzo[e]-1,4-diazepin-1-carbonyl]phenoxy}butyl acetate (76.6 mg).
(Process B) To a stirred solution of the acetate derivative (obtained in Process A) (76.6 mg) in methanol (1.5 mL) was added 2 mol/L aqueous solution of sodium hydroxide (0.29 mL) at room temperature, and the mixture was stirred at room temperature for an hour. After being added 2 mol/L hydrochloric acid (0.29 mL) at the same condition, to the mixture were added water and dichloromethane. The organic layer was separated and then was concentrated under reduced pressure. The crude product was purified by column chromatography on aminopropylsilylated silica gel (eluent:hexane-ethyl acetate-methanol) to give 1-[2-chloro-4-(4-hydroxybutoxy)-benzoyl] -.4-(E-methyl-1, 3,4-oxadiazol-2-ylmethyl) -1,2,4, 5-tetrahydrobenzo[e]-1,4-diazepin-3-one (40.0 mg).

¹H-NMR (CDCl₃) δ ppm:
1.35-1.50 (1H, br)., 1.60-2.00 (4H, m), 2.48 (3H, s), 3.35-5. 80 (10H, m), 6.45-7.70 (7H, m)
MS (ESI, m/z): 485(M+H)⁺

### Example 53

### 1-[2-Chloro-4-(2-hydroxyethoxy)benzoyl]-4-(5-ethynyl-1,3,4-oxadiazol-2-ylmethyl)-1,2,4,5-tetrahydrobenzo[e]-1,4-diazepin-3-one

1-[2-Chloro-4-(2-hydroxyethoxy)benzoyl]-4-(5-ethynyl-1,3,4-oxadiazol-2-ylmethyl)-1,2,4,5-tetrahydrobenzo[e]-1,4-diazepin-3-one was obtained with the use of 2-{3-chloro-4-[4-(5-ethynyl-1,3,4-oxadiazol-2-ylmethyl)-3-oxo-2,3,4,5-tetrahydrobenzo[e]-1,4-diazepine-1-carbonyl] phenoxy} ethyl acetate instead of 2-{ 3-chloro-4-[4-(5-isopropyl-1,3,4-oxadiazol-2-ylmethyl)-3-oxo-2,3,4,5-tetrahydrobenzo[e]-1,4-diazepine-1-carbonyl] phenoxy} ethyl acetate in a similar manner to that described in Example 2-1.

¹H-NMR (CDCl₃) δ ppm:
1.80-6.00 (12H, m), 6.50-7.80 (7H, m)
MS(ESI, m/z):467(M+H)⁺

### Test example 1

### Binding experiment for human V2 receptor

Test compounds were dissolved in dimethylsulfoxide at 10 mM. The affinities for human V2 receptor were determined by an inhibition against [³H] -Arginie vasopressin (AVP) (Perkin elmer Japan) binding to the human V2 receptor using CHO cell membranes expressed human V2 receptor (Packard BioScience). Cell membranes suspension was prepared by suspending the cell membranes as mentioned above with Assay buffer (50 mM Tris-HCl, 10 mM MgCl2, 0.1% bovine serum albumin (BSA), pH7.4) at an adequate protein concentration. The test compound solutions were prepared by diluting the dimethylsulfoxide solution of the test compound as mentioned above with Assay buffer at final concentration of 10 nM, 100 nM, 1 µM and 10 µM (in this diluting procedure, concentration of .dimethylsulfoxide was adjusted to be 0.5% at each concentration of the test compound) . Cell membranes suspension (50 µL), 3 nM of [³H] -AVP (50 µL), Assay buffer (50 µL) and the solution of the test compounds at each concentrations (50 µL) were added to MultiScreen 96-well plate (Millipore) and the mixture was incubated for an hour at 25 °C with shaking slightly. After filtration by aspiration, the plate was washed with ice-cold washing buffer (50 mM Tris-HCl, 10 mM MgC12, pH 7.4) for three times. After dry the plate, Microscinti-20 (Packard) was added to each well and the plate was shaken slightly, and then radioactivities of each well were counted with a micro plate scintillation counter, TopCount (Packard). Non-specific binding of [³H]-AVP for cell membranes was determined by adding 1 µM of cold AVP substituted for the test compounds. The concentration of the test compounds inhibiting the specific binding of [³H] -AVP by 50 % was considered as IC50 value. The Ki value of the test compounds was calculated from Kd value determined by the method described below and was considered as indication of affinity to the human V2 receptor. The results were shown in Table 67 as below.

### The calculation of Kd value of [³H] -AVP for the cell membranes expressed human V2 receptor

The suspension of the cell membranes expressed human V2 receptor was prepared with diluting adequately with the Assay buffer as mentioned above. In consideration for the radioactivity of [³H]-AVP, six different concentrations (final concentration; ranging from approximately 100 pM to 6000 pM) of [³H] -AVPwereprepared by serial dilution by from 2 to 3 fold with the Assay buffer. The cell membranes suspension (50 µL), each concentrations of [³H] -AVP (50 µL), the Assay buffer (50 µL) and 1 µM of cold AVP or the Assay buffer were added to MultiScreen 96-well plate (Millipore) and the mixture was incubated for an hour at 25 °C with shaking slightly. The specific bindings (B value) of each concentrations of the [3H] -AVP were determined by the method as mentioned above and free-bound contents (F value) at each concentrations of the [³H] -AVP were determined. The Kd value was calculated by Scatchard analysis using the B value and the F value.

**[Table 67]**

| Test compound | Ki (nM) |
|---|---|
| Example 1 -7 | 86. 6 |
| Example 15 | 97. 7 |
| Example 31 - 11 | 103.0 |
| Example 2-5 | 197.0 |
| Example 2-21 | 322. 4 |

### Test example 2

### The study to confirm the aqonism of human V2 receptor

The experiment to confirm the response of the test compounds to human V2 receptor was carried out using the cells prepared as described below in order to use this confirmation study to see the agonism of human V2 receptor of the test compounds.
The test compounds were dissolved in dimethylsulfoxide at 10 mM and solutions of the test compounds were prepared by 10-fold serial dilution with the Assay buffer (0.1 % BSA, 20 mM HEPES/Hank's balanced salt solution, pH 7. 4) at the concentration of 0.1 1 nM to 10 µM, which were used in this study.
Human V2 receptor couples with Gs protein, one of the G-coupling protein, and consequently produces cyclic adenosine 3', 5'-monophosphate (cAMP) via adenylate cyclase. This cAMP produce can be substituted to intracellular increase of Ca²⁺ with coexpressing Gqs, a chimeric protein, and human V2 receptor (Mol. Pharmacol., Vol. 50, pp. 885-890, 1996). The responses of the test compounds for human V2 receptor were quantified by measuring this intracellular Ca²⁺. The changes of intracellular Ca²⁺ after adding the test compounds at each concentration as mentioned above (0.1 nM to 10 µM) were measured with a FlexStation (Molecular Devices) using a FLIPR CALCIUM ASSAY KIT (Molecular Devices).
The intrinsic activities (IA) of the test compounds were calculated from the maximum response of the test compounds as that of AVP was considered to be 1. In case of a full agonist, the EC50 values of the test compounds were calculated as the maximum response of AVP was considered to be 100% and in the case of a partial agonist, EC50 values of test compounds were calculated as the maximum response of own test compounds were considered to be 100%. The concentration that achieved to 50% response of the maximum .response in a concentration-response curve was considered as EC50 value. The values of EC50 obtained in this study were shown in Table 68 described below as indications of the agonism of human V2 receptor.

### The preparation of the cells using confirmation study of an agonism of human V2 receptor (HEK293 cells coexpressed human V2 receptor and Gqs chimeric protein)

HEK293 cells (American Type Culture Collection) were incubated in the Eagle's Minimum Essential Medium (EMEM, Invitrogen) containing 1 mM sodium pyruvate, nonessential amino acids (0.1 mM), streptomycin (100 µg/mL), penicillin (100 U/mL), 10% fetal calf serum (Sanko chemicals) in an incubator with 5% CO2 at 37°C. The transfection was carried out by adding pCI-neo hV2 expression vector, expression vector inserted with the sequence coding Gqs chimeric protein (pLEC-Gqs5, LiveWare, Molecular Devices) and Lipofectamine2000 (Invitrogen), all diluted with OPTI-MEM I Reduced Serum Medium I (Invitrogen), to the cell suspension which was prepared from the confluent cells suspended with EMEM as mentioned above without antibiotics at 1X10⁶ cells/mL. After the transfection, the cells (HEK293 cells coexpressed human V2 receptor and Gqs chimeric protein) were incubated in an incubator with 5% CO2 for two days and were used as cells for confirming agonism of human V2 receptor, and used for assessment of the test compounds. The expression plasmid vector of human V2 receptor represented as pCI-neo/hV2 above was constructed by the method as described below.

### The method of construction of human V2 receptor expression plasmid vector

cDNA library was obtained from reverse transcription of human kidney total RNA using SuperScript II RNase H-reverse transcriptase (Invitrogen) and oligo dT. The DNA fragment encoding human V2 receptor was amplified by the PCR method using the cDNA library as a template, primers used in combination of each forward primer (sequence no. 1-3 shown below) and each reverse primer (sequence no. 4-6 shown below) respectively and pfu DNA polymerase (Stratagene). This amplifiedDNAfragment andpCR-blunt kit (Invitrogen), a cloning plasmid vector, were ligated by a general method of the kit. The ligate-productions were introduced into E. coli TOP10 cells (Invitrogen) by the general method, and the transformant cells were selected by LB agar medium containing 50 µg/mL of kanamycin. One of the transformant was grown in LB liquid medium and the vectors were extracted from the transformant and purified. The vectors were clevaged with restriction enzyme Eco RI to obtain DNA fragments. As the same time, pCI-neo (Promega), a mammalian expression plasmid vector, was digested by restriction enzyme Eco RI and treated with calf intestinal alkaline phosphatase to protect from a self ligation. Then this pCI-neo and the DNA fragments obtained by Eco RI digestion as mentioned above were ligated by Quick ligation Kit (New England BioLabs). After the ligated-productions were introduced into E. coli TOP10 cells by a general method, the transformants were selected with LB agar medium containing 100 µg/mL of ampicillin. One of the transformant was grown in LB liquid medium and the vectors were extracted from the transformant and purified. The sequence of the DNA fragment inserted at multi-cloning site of this vector was determined and corresponded to the sequence of human V2 receptor administered as accession no. AF030626 in GenBank/EMBL data base. This expression plasmid vector encoding human V2 receptor was termed as pCI-neo/hV2.

Sequence no.1 AGTCCGCACATCACCTCCAG
Sequence no.2 ATGCTCATGGCGTCCACCAC
Sequence no.3 GCCCTCAGAACACCTGC
Sequence no.4 GCTCCTCACGATGAAGTGTC
Sequence no.5 GCAAGACACCCAACAGCTCC
Sequence no.6 GCTGAGCTTCTCAAAGCCTCT

[Table 68]

| Test compound | Agonism of human V2 receptor EC50 (nM) | IA |
|---|---|---|
| Example 1-7 | 4 | 0.92 |
| Example 15 | 1 | 1.09 |
| Example.31 -1 | 39 | 0.35 |
| Example 2-5 | 10 | 1.03 |
| Example 1 -29 | 50 | 0.93 |
| Example 2-21 | 6 | 1.09 |
| Example 31 -8 | 44 | 1.18 |

### Test example 3

### The study of antidiuretic effect-the confirmation study of antidiuretic effect on the diuretic activity induced by loading hypotonic solution in the anesthetized rats infused with hypotonic solution

It has been reported that plasma AVP level was decreased with intravenous infusion of hypotonic solution (J. Endocrinol., Vol. 141, pp. 59-67, 1994). The antidiuretic effect of the test compounds in the rats induced a diuretic condition was determined by the method as reported by Angchanpen et al (Br. J. Pharmacol., Vol. 93, pp. 151-155, 1988). The test compounds were dissolved in dimethylsulfoxide at 10 mM, and used in the study. Male SD rats (200-400 g weight) were anesthetized with 100 mg/kg of Inactin (SIGMA)intraperitonealy and each cannula was inserted into trachea, jugular vein, bladder and femoral vein, respectively. The hypotonic solution (0.3% NaCl, 0.83% glucose) was infused via femoral vein at 9 mL/hour. The urine volume obtained via cannula inserted into bladder was measured every 10 minutes. After steady-state of urine volume for three 10 minutes periods, test compounds prepared as mentioned above were administered intravenously at 10 µg/kg via cannula inserted into jugular vein. Dimethylsulfoxide/saline (0.3%) was used as a vehicle.
The average of urine volume measured for three 10 minutes periods before the administration was defined as the pre value (0%). After the administration of the test compounds, the urine volume was measured every 10 minutes. The antidiuretic effect, namely the decrease of the urine volume, induced by administration of test compounds was calculated from the decreasing rate of urine volume (minus %) against the pre value. Because maximum decreasing rate of the urine volume after adminiatration of vehicle was -20% in this study, the period that decreasing rate of urine volume restored to -20% after administration of the test compounds was considered as indication of duration time of the test compounds. Each of the result was shown in Table 69.

**[Table 69]**

| Test compound | Antidiuretic effect (decreasing rate of urine volume) | Duration time |
|---|---|---|
| Untreated | 0% | - |
| Example 1 -7 | -68. 6% | 30 minutes |

The abbreviations used above were shown below.
AVP: arginine vasopressin
[³H]-AVP: tritium labeled-vasopressin
HEPES: 4-(2-Hydroxyethyl)-1-piperazineethanesulfonic Acid
HEK: human embryonic kidney
Tris: 2-Amino-2-hydroxymethl-1,3-propanediol
CHO: chinese hamster ovary cell

### Test example 4

### The experiment for inhibitory effect on cytochrome P-450 (CYP3A4) originated from human liver microsome

The test compounds dissolved in dimethylsulfoxide at 10 mM were added to 0.1 M phosphate buffer (pH 7.4) containing 10 mM MgCl2, and human liver microsome (1.0 mg/mL) as enzyme and testosterone (50 µM) as a substrate for CYP3A4 were added to this 0.1M phosphate buffer in order to prepare phosphate buffer solution of the test compounds at final concentration of 10 µM (final volume 0.2 mL). The reaction was started by the addition of NADPH (1.0 mg/mL) and then incubated for 5 minutes at 37°C. After acetonitrile was added to stop the reaction, the residual concentration of testosterone was determined using LC/MS/MS, and calculated the rate of metabolism of substrate from the ratio of content of testosterone at end to that at start (A value) . As the same way, the experiment except for the test compounds was carried out and remaining testosterone was determined in the same way as mentioned above. The rate of metabolism of substrate was calculated from the ratio of content of testosterone at end to that at start of the reaction (B value) as control value. The ratio of A value against B value was determined as a degree of incidence of test compounds on metabolic rate, which was shown in Table 70. As shown in Table 11, it was suggested that the test compounds of the present invention rarely affected metabolism of substrate for cytochrome P-450 (CYP3A4) originated from human liver microsome.

**[Table 70]**

| Test compound | (A value/B va)ue)X100 |
|---|---|
| Example 1 -7 | 89. 9 |

### Test example 5

### Acute toxicity test

Male SD rats (250-300 g weight) were divided into some groups (N=3) and each cannula was inserted into trachea and jugular vein under anesthesia with urethane (1.5 g/kg, subcutaneously). The test compound solutions were prepared with an adequate solvent to be a dosage of 3 mg/kg, and then the soluton was administered intravenously via cannula. The survival rate was determined for an hour experimental period. As the result was shown in Table 71, it was not observed that animal was dead and then it was suggested that the compounds of the present invention had low toxicity.

**[Table 71]**

| Test compound | Dead |
|---|---|
| Example 1 -7 | 0/3 |

### Industrial Applicability

The compound represented by the above general formula (I) of the present invention, for example, by a binding experiment for human V2 receptor or a study to confirm the agonism of human V2 receptor, is showed a strong agonism of human V2 receptor. Thence the compound represented by the above general formula (I) of the present invention can decrease urine volume significantly. Therefore the compound represented by the above general formula (I) of the present invention has an antidiuretic activity on the profile based on the present activity and a release activity of coagulation factor VIII and von-Wiliebrand factor, is useful for various dysuria, a large volume of urine or bleeding tendency, is preferably as an agent for the treatment or prevention of micturition, urinary incontinence, enuresis, diabetes insipidus (for example, central diabetes insipidus or nephrogenic diabetes insipidus), nocturia, nocturnal enuresis, overactive bladder, hemophilia, von-Wiliebrand disease, congenital/acquired dysfunction of blood platelets, spontaneous bleeding or the like. In addition, the compound of the present invention has a very weak inhibition activity against cytochrome P-450 (CYP) enzyme, and can be used without anxiety, in case of using for the elderly person or combination with other agents.

## Claims

1. An aromatic amide derivative represented by the general formula: 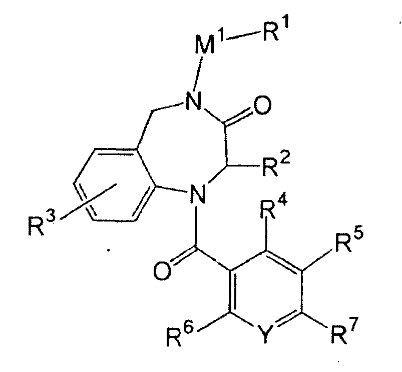 wherein R¹ represents a hydrogen atom or a C₁₋₆ alkyl group which may have a substituent selected from the following (Substituent group A) ;
(Substituent group A)
a hydroxy group, a halogen atom, a thiol group, a cyano group, a C₃₋₁₀ cycloalkyl group, a C₁₋₆ alkoxy group, a halo(C₁₋₆ alkyl) group, a C₆₋₁₀ aryl group, a C₆₋₁₀ aryloxy group, -COOR^{A1} (in the formula, R^{A1} is a hydrogen atom, a C₃₋₁₀ cycloalkyl group, a C₁₋₆ alkyl group or a C₆₋₁₀ aryl (C₁₋₆ alkyl) group), -CONHNR^{A2}R^{A3} (in the formula, R^{A2} and R^{A3} are independently a hydrogen atom, a formyl group, a C₂₋₇ acyl group, a C₁₋₆alkoxy(C₂₋₇ acyl) group, a heteroarylcarbonyl group, an alicyclic amino (C₂₋₇ acyl) group, a C₁₋₆ alkoxycarbonyl (C₂₋₇ acyl) group or a C₆₋₁₀ arylcarbonyl group), -CONR^{A4}R^{A5} (in the formula, R^{A4} and R^{A5} are independently a hydrogen atom, a C₁₋₆ alkyl group, a C₁₋₆ alkoxy(C₁₋₆ alkyl) group, a hydroxy(C₁₋₆ alkyl) group, a C₃₋₁₀ cycloalkyl group, a hydroxy (C₃₋₁₀ cycloalkyl) group, a C₆₋₁₀ aryl(C₁₋₆ alkyl) group, a carboxy (C₁₋₆ alkyl) group, a C₁₋₆ alkoxycarbonyl (C₁₋₆ alkyl) group, a heteroaryl group or a C₆₋₁₀ aryl group, or -NR^{A4}R^{A5} forms an alicyclic amino group), -NR^{A6}R^{A7} (in the formula, R^{A6} and R^{A7} are independently a hydrogen atom, a C₁₋₆ alkyl group, a C₁₋₆ alkoxy(C₁₋₆ alkyl) group, a C₁₋₆ alkoxy(C₂₋₇ acyl) group, a C₂₋₇ acyl group, a C₆₋₁₀ aryl group, a C₆₋₁₀ arylcarbonyl group, a C₁₋₆ alkylsulfonyl group, a C₆₋₁₀ arylsulfonyl group or a heteroarylcarbonyl group, or -NR^{A6}R^{A7} forms an alicyclic amino group), -SO₂NR^{A8}R^{A9} (in the formula, R^{A8} and R^{A9} are independently a hydrogen atom, a C₁₋₆ alkyl group or a C₁₋₆ alkoxy(C₁₋₆ alkyl) group), a heterocycloalkyl group, a group represented by a general formula: 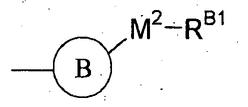 wherein B ring is a C₆₋₁₀ aryl group or a heteroaryl group, R^{B1} is a hydrogen atom, a halogen atom, a cyano group, a C₃₋₁₀ cycloalkyl group, a C₁₋₆ alkoxy group, a halo(C₁₋₆ alkyl) group, a C₁₋₆ alkoxy (C₁₋₆ alkyl) group, a hydroxy(C₁₋₆ alkyl) group, -COOR^{B11} (in the formula, R^{B11} is a hydrogen atom, a C₁₋₆ alkyl group or a C₆₋₁₀ aryl (C₁₋₆ alkyl) group), -CONR^{B12}R^{B13} (in the formula, R^{B12} and R^{B13} are independently a hydrogen atom, a C₁₋₆ alkyl group, a C₁₋₆ alkoxy(C₁₋₆ alkyl) group or a hydroxy (C₁₋₆ alkyl) group, or -NR^{B12}R^{B13} forms an alicyclic amino group), -NR^{B14}R^{B15} (in the formula; R^{B14} and R ^{B15} are independently a hydrogen atom, a C₁₋₆ alkyl group, a C₂₋₇ acyl group, a C₆₋₁₀ arylcarbonyl group, a C₆₋₁₀ aryl group, a heteroaryl group, an alicyclic amino-subsutituted (C₁₋₆alkyl) group, a C₁₋₆alkylsulfonyl group or a C₆₋₁₀ arylsulfonyl group, or -NR ^{B14} R ^{B15} forms an alicyclic amino group), or -SO₂NR^{B16}R^{B17} (in the formula, R^{B16} and R^{B17} are independently a hydrogen atom, a C₁₋₆ alkyl group or a C₁₋₆ alkoxy(C₁₋₆ alkyl) group, or -NR^{B16}R^{B17} forms an alicyclic amino group), M² is a single bond or a C₁₋₄ alkylene group; or a group represented by the general formula: 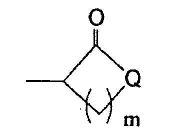 or 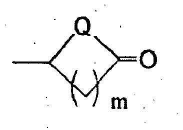 wherein Q is -O- or -NR^{C}- (in the formula, R^{C} is a hydrogen atom or a C₁₋₆ alkyl group), m is an integer from 1 to 4,
R² is a hydrogen atom or C₁₋₆ alkyl group;
R³ is a hydrogen atom, a halogen atom, a hydroxy group, a C₁₋₆ alkyl group or a C₁₋₆ alkoxy group;
R⁴, R⁵ and R⁶ are independently a hydrogen atom, a halogen atom, a C₁₋₆ alkyl group, a C₁₋₆ alkoxy group'or a halo(C₁₋₆ alkyl) group; R⁷ is a hydrogen atom, a halogen atom, a hydroxy group, a C₁₋₆ alkyl group, a halo(C₁₋₆ alkyl) group , a hydroxy(C₁₋₆ alkyl) group, a halo (C₁₋₆ alkoxy) C₁₋₆ alkyl group , a C₆₋₁₀ aryl group, a heteroaryl group, a heterocycloalkyl group, _{-NR}^{D1}_{R}^{D2} (in the formula, R^{D1} and R ^{D2} are independently a hydrogen atom, a C₁₋₆ alkyl group, a hydroxy (C₁₋₆ alkyl) group, a halo(C₁₋₆ alkyl) group or a C₁₋₆ alkoxy(C₁₋₆ alkyl) group, or -NR^{D1}R^{D2} forms an alicyclic amino group), -O-R^{D3} [ in the formula, R^{D3} is a hydrogen atom, a C₁₋₆ alkyl group, a C₂₋₇ acyloxy-substituted (C₁₋₆ alkyl) group, a hydroxy(Cl_6alkyl) group, a C₁₋₆ alkoxy (C₁₋₆ alkyl) group, a halo (C₁₋₆ alkyl) group, a halo (C₁₋₆ alkoxy)C₁₋₆ alkyl group, a C₁₋₆ alkoxycarbonyl(C₁₋₆ alkyl) group, a C₆₋₁₀ aryl group or a heteroaryl group], a C₆₋₁₀ aryl[C₁₋₆ alkoxy (C₁₋₆ alkyl)] group or a C₃₋₈ cycloalkyl group;
M¹ is a single bond, a C₁₋₄ alkylene group, -CO-, -NR^{E}- (in the formula, R^{E} is a hydrogen atom, a C₁₋₆ alkyl group or a C₂₋₇ acyl group) or -SO₂-;
Y is N or CR^{E} (in the formula, R^{E} is a hydrogen atom, a halogen atom, a C₁₋₆ alkyl group or a halo(C₁₋₆ alkyl) group;
or a pharmaceutically acceptable salt thereof, or a prodrug thereof.

2. An aromatic amide derivative represented by the general formula: 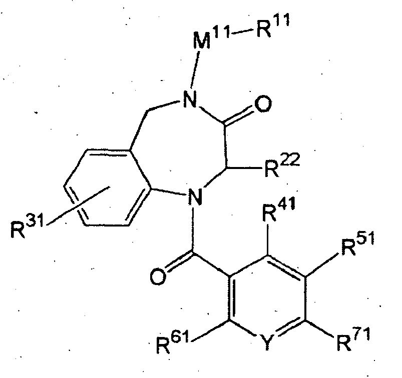 wherein R¹¹ is a hydrogen atom or a C₁₋₆ alkyl group which may have a substituent selected from the following (Substituent group A1);
(Substituent group A1)
a hydroxy group, a halogen atom, a thiol group, a cyano group, a C₃₋₁₀ cycloalkyl group, a C₁₋₆ alkoxy group, a halo(C₁₋₆ alkyl) group, a C₆₋₁₀ aryl group, a C₆₋₁₀ aryloxy group, -COOR^{A1} (in the formula, R^{A1} is a hydrogen atom, a C₃₋₁₀ cycloalkyl group, a C₁₋₆ alkyl group or a C₆₋₁₀ aryl(C₁₋₆ alkyl) group), -CONHNR^{A2}R^{A3} (in the formula, R^{A2} and R^{A3} are independently a hydrogen atom, a C₂₋₇ acyl group, a C₁₋₆ alkoxy(C₂₋₇ acyl) group, a C₁₋₆ alkoxycarbonyl-subsutituted (C₂₋₇ acyl) group or C₆₋₁₀ arylcarbonyl group), -CONR^{A41}R^{A51} (in the formula, R^{A41} is a hydrogen atom, and R^{A51} is a C₁₋₆ alkyl group, a C₁₋₆ alkoxy(C₁₋₆ alkyl) group, a hydroxy(C₁₋₆ alkyl) group, a heteroaryl group or a C₆₋₁₀ aryl group), -NR^{A6}R^{A7} (in the formula, in case that _{R}^{A6} is a hydrogen atom, R^{A7} is a C₂₋₇ acyl group, a C₆₋₁₀ arylcarbonyl group or a heteroarylcarbonyl group, or in case that R^{A6} is a C₁₋₆ alkyl group, a C₆₋₁₀ aryl group or a heteroarylcarbonyl group, R^{A7} is a C₁₋₆ alkylsulfonyl group or a C₆₋₁₀ arylsulfonyl group), -SO₂NR^{A8}R^{A9} (in the formula, R^{A8} is a hydrogen atom, R^{A9} is a C₁₋₆ alkyl group or a C₁₋₆ alkoxy(C₁₋₆ alkyl) group), a heterocycloalkyl group, a group represented by the general formula: 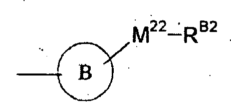 wherein B ring is a C₆₋₁₀ aryl group or a heteroaryl group, R^{B2} is a hydrogen atom, a halogen atom, a cyano group, a C₃₋₁₀ cycloalkyl group, a C₁₋₆ alkoxy group, a halo(C₁₋₆ alkyl) group, a C₁₋₆ alkoxy(C₁₋₆ alkyl) group, a hydroxy(C₁₋₆ alkyl) group, -COOR^{B21} (in the formula, R^{B21} is a hydrogen atom, a C₁₋₆ alkyl group or a C₆₋₁₀ aryl (C₁₋₆ alkyl) group), -CONR^{B22}R^{B23} (in the formula, R^{B22} and R^{B23} are independently a hydrogen atom, a C₁₋₆ alkyl group, a C₁₋₆ alkoxy(C₁₋₆ alkyl) group or a hydroxy(C₁₋₆ alkyl) group, or -NR^{B22}R^{B23} forms an alicyclic amino group), -NR^{B24}R^{B25} (in the formula, R^{B24} and R^{B25} are independently a hydrogen atom, a C₁₋₆ alkyl group, a C₂₋₇ acyl group, a C₆₋₁₀ arylcarbonyl group, C₁₋₆ alkylsulfonyl group or a C₆₋₁₀ arylsulfonyl group, or -NR^{B24}R^{B25} forms an alicyclic amino group), -SO₂NR^{B26}R^{B27} (in the formula, _{R}^{B26} and _{R}^{B27} are independently a hydrogen atom, a C₁₋₆ alkyl group or a C₁₋₆ alkoxy(C₁₋₆ alkyl) group, or -NR^{B26}R^{B27} forms an alicyclic amino group), M²² is a single bond or a C₁₋₄ alkylene group, or a group represented by the general formula: 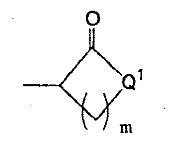 or 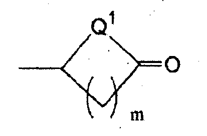 wherein Q¹ is -NR^{C}- (in the formula, R^{C} is a hydrogen atom or a C₁₋₆ alkyl group), and m is an integer from 1 to 4,
R²² is a hydrogen atom or a methyl group;
R³¹ is a hydrogen atom, a halogen atom, a hydroxy group or a C₁₋₆ alkyl group;
R⁴¹, R⁵¹ and R⁶¹ are independently a hydrogen atom, a halogen atom, a C₁₋₃ alkyl group, a C₁₋₆ alkoxy group or a halo(C₁₋₃ alkyl) group; R⁷¹ is a hydrogen atom, a halogen atom, a hydroxy group, a C₁₋₆ alkyl group, a halo (C₁₋₆ alkyl) group, a hydroxy (C₁₋₆ alkyl) group, a halo(C₁₋₆ alkoxyl)C₁₋₆ alkyl group, a C₆₋₁₀ aryl group, a heteroaryl group, a C₃₋₈ cycloalkyl group, a heterocycloalkyl group, -NR^{D11}R^{D22} (in the formula, R^{D11} and R^{D22} are independently a hydrogen atom, a C₁₋₆ alkyl group, a hydroxy(C₁₋₆ alkyl) group, a halo(C₁₋₆ alkyl) group or a C₁₋₆ alkoxy(C₁₋₆alkyl) group, or -NR^{D11}R^{D22} forms an alicyclic amino group), -O-R^{D33} (in the formula, R^{D33} is a hydrogen atom, a C₁₋₆ alkyl group, a C₂₋₇ acyloxy-substituted (C₁₋₆ alkyl) group, a hydroxy(C₁₋₆ alkyl) group, a C₁₋₆ alkoxy(C₁₋₆ alkyl) group, a halo(C₁₋₆ alkyl) group or a halo (C₁₋₆ alkoxy)C₁₋₆ alkyl group), or a C₆₋₁₀ aryl[C₁₋₆ alkoxy(C₁₋₆ alkyl)] group;
M¹¹ is a single bond or a C₁₋₄ alkylene group;
Y is N or CR^{F} (in the formula, R^{F} is a hydrogen atom, a halogen atom, a C₁₋₆ alkyl group or a halo(C₁₋₆ alkyl) group;
or a pharmaceutically acceptable salt thereof, or a prodrug thereof.

3. An aromatic amide derivative as claimed in claims 2 wherein R²² and R³¹ are a hydrogen atom; Y is CH, or a pharmaceutically acceptable salt thereof, or a prodrug thereof.

4. An aromatic amide derivative as claimed in claims 3 wherein R⁶¹ is a hydrogen atom or a halogen atom, or a pharmaceutically acceptable salt thereof, or a prodrug thereof.

5. An aromatic amide derivative as claimed in claim 4 wherein R⁷¹ is a hydroxy (C₁₋₆ alkyl) group, a halo (C₁₋₆ alkoxyl)C₁₋₆ alkyl group, a C₆₋₁₀ aryl group, a heteroaryl group, a heterocycloalkyl group, -NR^{D11}R^{D22} (in the formula, R^{D11} and R^{D22} are independently a C₁₋₆ alkyl group, a hydroxy(C₁₋₆ alkyl) group, a halo(C₁₋₆ alkyl) group or a C₁₋₆ alkoxy(C₁₋₆ alkyl) group, or -NR^{D11}R^{D22} forms an alicyclic amino group), -O-R^{D33} [in the formula, R^{D33} is a hydrogen atom, a C₁₋₆ alkyl group, a C₂₋₇ acyloxy-substituted (C₁₋₆ alkyl) group, a hydroxy (C₁₋₆ alkyl) group, a C₁₋₆ alkoxy (C₁₋₆ alkyl) group, a halo (C₁₋₆ alkyl) group or a halo (C₁₋₆alkoxy)C₁₋₆alkyl group], or a C₆₋₁₀ aryl[C₁₋₆ alkoxy (C₁₋₆ alkyl)] group, more preferably a C₆₋₁₀ aryl group, a heteroaryl group, heterocycloalkyl group, -NR^{D11}R^{D22} [ in the formula, R^{D11} and R^{D22} are independently a hydrogen atom, a C₁₋₆ alkyl group, a hydroxy(C₆₋₁₀ alkyl) group, a halo(C₁₋₆ alkyl) group or a C₁₋₆ alkoxy(C₁₋₆ alkyl) group, or -NR^{D11}R^{D22} forms an alicyclic amino group], -O-R^{D33} [in the formula, R^{D33} is a hydrogen atom, a C₁₋₆ alkyl group, a C₂₋₇ acyloxy-substituted (C₁₋₆ alkyl) group, a hydroxy(C₁₋₆ alkyl) group, a C₁₋₆ alkoxy(C₁₋₆ alkyl) group, a halo C₁₋₆ alkyl) group, or a halo(C₁₋₆ alkoxy)C₁₋₆ alkyl group]; or a pharmaceutically acceptable salt thereof, or a prodrug thereof.

6. An aromatic amide derivative as claimed in claim 5 wherein R¹¹ is a hydrogen atom or a C₁₋₆ alkyl group which may have a substituent selected from a group consisting of the following (Substituent group A2)
(Substituent group A2)
a hydroxy group, a C₃₋₁₀ cycloalkyl group, a C₁₋₆ alkoxy group, a halo(C₁₋₆ alkyl) group, -COOR^{A1} (in the formula, R^{A1} is a hydrogen atom, a C₃₋₁₀ cycloalkyl group, a C₁₋₆ alkyl group or a C₆₋₁₀ aryl (C₁₋₆ alkyl) group), _{-CONR}^{A41}_{R}^{A51} (in the formula, _{R}^{A41} is a hydrogen atom, and R^{A51} is a C₁₋₆ alkyl group, a C1_6 alkoxy(C₁₋₆ alkyl) group,-a hydroxyl (C₁_₆ alkyl) group, a heteroaryl group or a C₆₋₁₀ aryl group), or a group represented by the general formula: 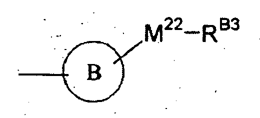 wherein B ring is a C₆₋₁₀ aryl group or a heteroaryl group, R^{B3} is a hydrogen atom, a C₃₋₁₀ cycloalkyl group, a C₁₋₆ alkoxy group, a halo(C₁₋₆ alkyl) group, -COOR^{B21} (in the formula, _{R}^{B21} is a hydrogen atom, a C₁₋₆ alkyl group or a C₆₋₁₀ aryl (C₁₋₆alkyl) group), -CONR^{B22}R^{B23} (in the formula, R^{B22} and R^{B23} are independently a hydrogen atom, a C₁₋₆ alkyl group, a C₁₋₆ alkoxy(C₁₋₆ alkyl) group or a hydroxyl (C₁₋₆ alkyl) group, or -NR^{B22}R^{B23} forms an alicyclic amino group), a C₁₋₆ alkoxy(C₁₋₆ alkyl) group or a hydroxy(C₁₋₆ alkyl) group, and M²² is a single bond or a C₁₋₄ alkylene group; or a pharmaceutically acceptable salt thereof, or a prodrug thereof.

7. An aromatic amide derivative as claimed in claim 6 wherein R¹¹ is a hydrogen atom or a C₁₋₆ alkyl group which may have a substituent selected from a group consisting of the following (Substituent group A3).
(Substituent group A3)
a hydroxy group, a C₃₋₁₀ cycloalkyl group, a C₁₋₆ alkoxy group, a halo(C₁₋₆ alkyl) group, -COOR^{A1} [in the formula, R^{A1} is a hydrogen atom, a C₃₋₁₀ cycloalkyl group, a C₁₋₆ alkyl group or a C₆₋₁₀ aryl(C₁₋₆ alkyl) group], -CONR^{A41}R^{A51} [in the formula, R^{A41} is a hydrogen atom, and R^{A51} is a C₁₋₆ alkyl group, a C₁₋₆ alkoxy(C₁₋₆ alkyl) group, a hydroxy(C₁₋₆ alkyl) group, a heteroaryl group or a C₆₋₁₀ aryl group], or a group represented by the general formula: 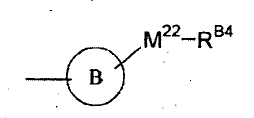 wherein B ring is a C₆₋₁₀ aryl group or a heteroaryl group, R^{B4} is a hydrogen atom, a C₃₋₁₀ cycloalkyl group, a C₁₋₆ alkoxy group or a halo(C₁₋₆ alkyl) group, and M²² is a single bond or a C₁₋₄ alkylene group; or a pharmaceutically acceptable salt thereof, or a prodrug thereof.

8. An aromatic amide derivative as claimed in claim 1 which is a compound selected from a group consisting of the following group and a pharmaceutically acceptable salt thereof.

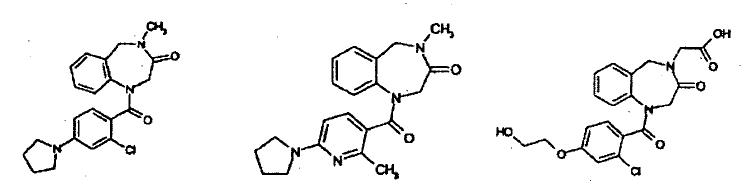

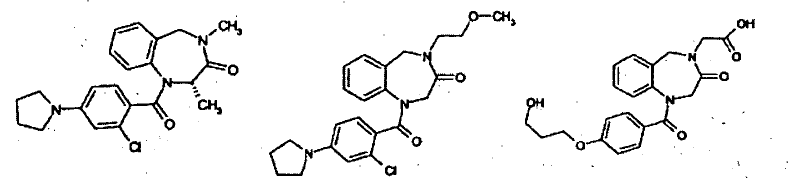

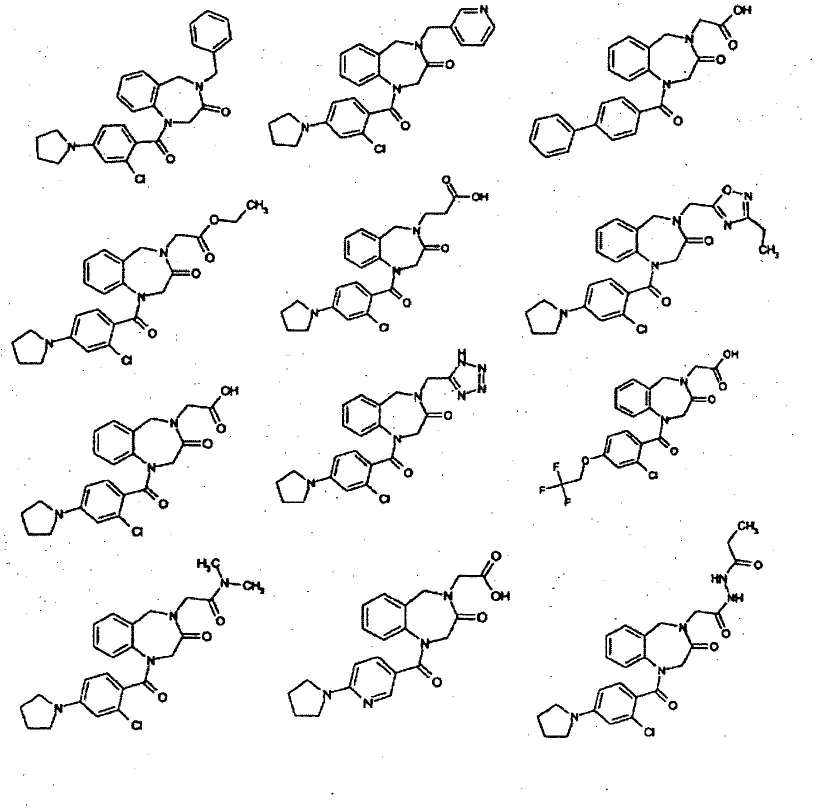

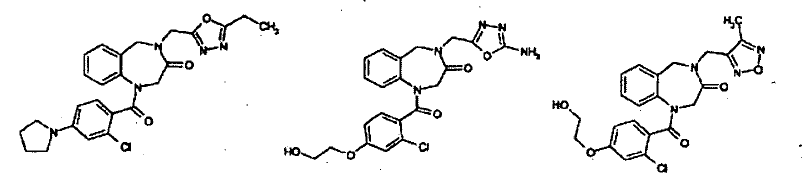

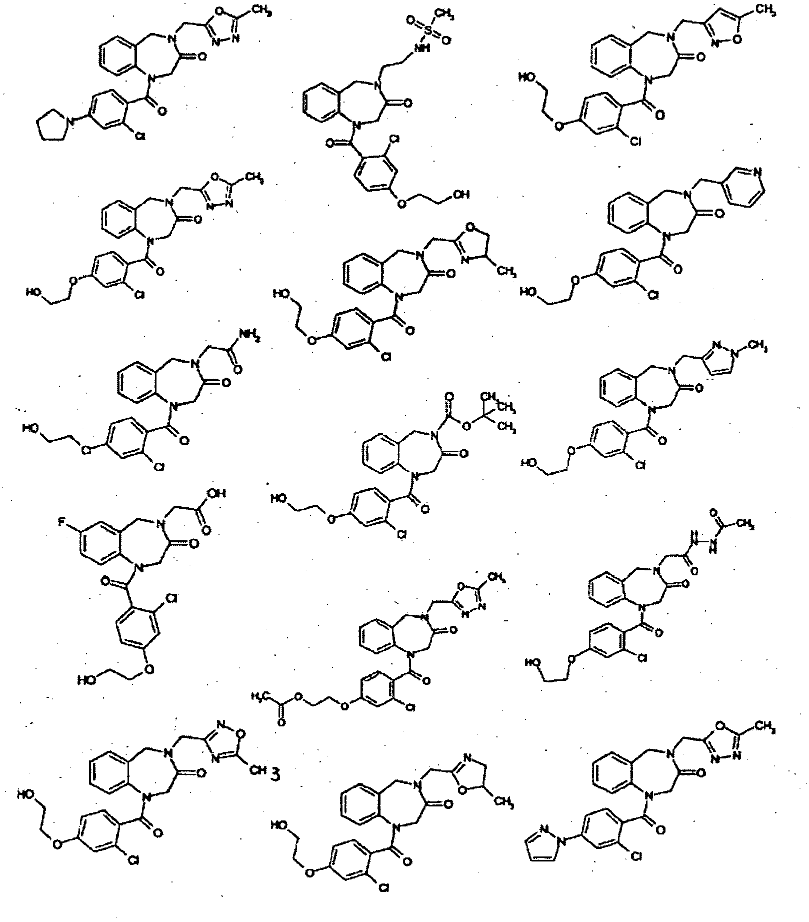

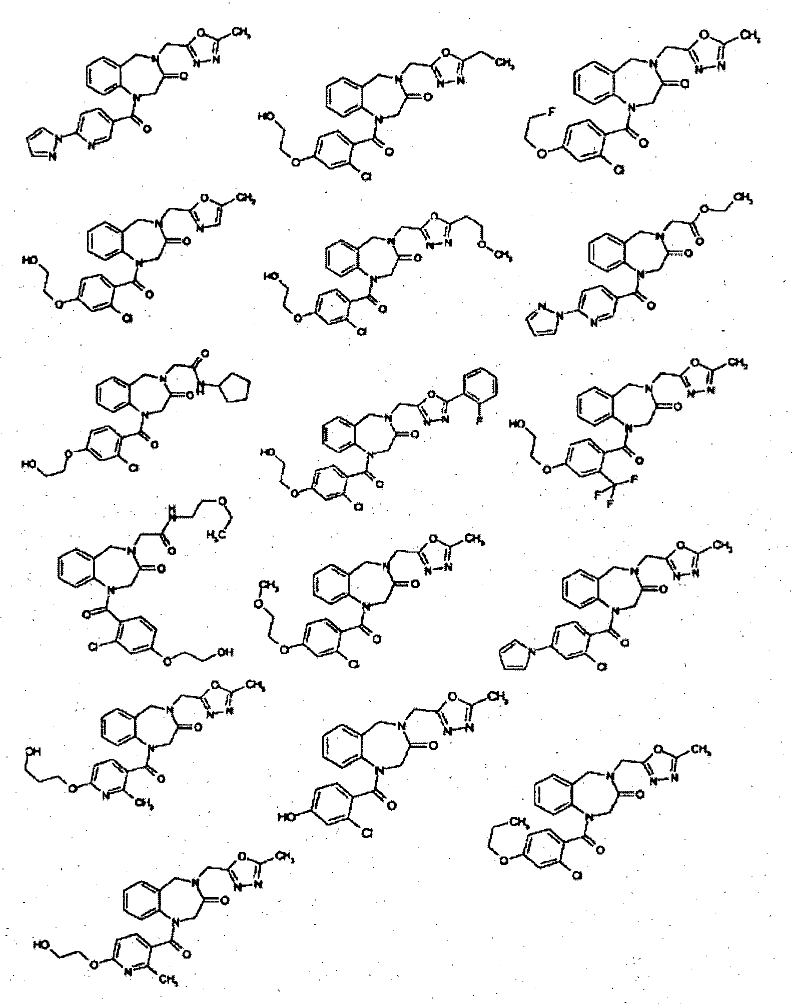

9. A pharmaceutical composition comprising as an active ingredient an aromatic amide derivative as claimed in any one of claims 1 to 8 or a pharmaceutically acceptable salt thereof, or a prodrug thereof.

10. A pharmaceutical composition as claimed in claim 9, which is a human type-2 arginine vasopressin receptor agonist.

11. An agent for the treatment or prevention of a disease associated with an increasing of urine volume, comprising as an active ingredient an aromatic amide derivative as claimed in any one of claims 1 to 8 or a pharmaceutically acceptable salt thereof, or a prodrug thereof.

12. An agent for the treatment or prevention of a disease associated with an increasing of number of micturition, comprising as an active ingredient an aromatic amide derivative as claimed in any one of claims 1 to 8 or a pharmaceutically acceptable salt thereof, or a prodrug thereof.

13. An agent for the treatment or prevention of a disease associated with diabetes insipidus, nocturia, nocturnal enuresis, overactive bladder, hemophilia or von-Wiliebrand disease, comprising as an active ingredient an aromatic amide derivative or a pharmaceutically acceptable salt thereof, or a prodrug thereof, as claimed in any one of claims 1 to 8.

14. A pharmaceutical composition comprising in combination an aromatic amide derivative as claimed in any one of claims 1 to 8 or a pharmaceutically acceptable salt thereof, or a prodrug thereof, and at least one agent selected from a group consisting of agents for the treatment of diabetes insipidus, nocturia, nocturnal enuresis, overactive bladder, hemophilia, other than a human type-2 arginine vasopressin receptor agonist.

15. A pharmaceutical composition comprising in combination Drug Group 1; consisting of an aromatic amide derivative as claimed in any one of claims 1 to 8 or a pharmaceutically acceptable salt thereof, or a prodrug thereof, and Drug Group 2; at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-channel opener, a sensory nerve blocking agent, a β-adrenergic agonist, an acetylcholinesterase inhibitor and anti-inflammatory agent.

16. A use of an aromatic amide derivative as claimed in any one of claims 1 to 8 or a pharmaceutically acceptable salt thereof, or a prodrug thereof, for the manufacture of an agent for the treatment or prevention of diabetes insipidus, nocturia, nocturnal enuresis, overactive bladder, hemophilia or von-Wiliebrand disease.

17. A ethod for the treatment or prevention of diabetes insipidus, nocturia, nocturnal enuresis, overactive bladder, hemophilia or von-Wiliebrand disease which comprises administering an effective amount of an aromatic amide derivative as claimed in any one of claims 1 to 8 or a pharmaceutically acceptable salt thereof, or a prodrug thereof.

18. A ethod for the treatment or prevention of diabetes insipidus, nocturia, nocturnal enuresis, overactive bladder, hemophilia or von-Wiliebrand disease which comprises administering in combination each effective amount of Drug Group 1; an aromatic amide derivative as claimed in any one of claims 1 to 8 or a pharmaceutically acceptable salt thereof, or a prodrug thereof, and Drug Group 2; at least one agent selected from a group consisting of an α₁-adrenoceptor blocker, a cholinergic blocking agent, a cholinergic agent, an antispasmodic agent, an anti-androgen agent, an antidepressant, a calcium antagonist, a potassium-cnannei opener, a sensory nerve blocking agent, a β-adrenergic agonist, an acetylcholinesterase inhibitor and anti-inflammatory agent.
